# EUROPEAN PATENT APPLICATION

(11) **EP 2 045 333 A1**
(43) Date of publication of application: **08.04.2009**
(21) Application number: 07291213.2
(22) Date of filing: 05.10.2007
(51) Int. Cl.: C12Q 1/68

(54) **Method for detecting ralstonia solanacearum race 3 biovar 2**

(71) Applicant: INSTITUT NATIONAL DE LA RECHERCHE AGRONOMIQUE (INRA), 75007 Paris Cédex 07 (FR)
(72) Inventor: Boucher, Christian, 31650 Auzielle (FR); Elbaz, Mounira, 2042 Tunis (TN); Genin, Stéphane, 31450 Donneville (FR); Guidot, Alice, 69300 Caluire (FR); Prior, Philippe, 97432 Ravine des Cabris (FR)
(74) Representative: Colombet, Alain André

(57) **Abstract**

The invention concerns a method for the detection of *Ralstonia solanacearum* race 3 biovar 2 strains in a medium, comprising the determination of the presence or the absence in a sample of the medium, of:
(i) at least one first nucleic acid target having a sequence selected from the group constituted of SEQ ID NO: 1-49, complementary sequences thereof, and homologous sequences thereof, or
(ii) at least one fragment of said first target nucleic acid, wherein said fragment is not constituted of or comprised in a sequence selected from the group constituted of SEQ ID NO: 111-140;

whereby, if said first nucleic acid target or fragment thereof is present in the sample, it is determined that *Ralstonia solanacearum* race 3 biovar 2 strain is present in the medium.

## Description

### Field of the invention

The present invention relates to a method for detecting race 3 biovar 2 *Ralstonia solanacearum* strains.

### Background of the invention

*Ralstonia solanacearum* is a Gram-negative soil-borne plant pathogen with thousands of distinct strains in a heterogeneous species complex. *Ralstonia solanacearum* causes bacterial wilt, which is globally distributed and economically destructive. It is thus considered the single most destructive bacterial plant disease because of its unusually broad host range. The bacterium attacks plants in over 200 different families, including dicots and monocots, and annual plants as well as trees. Economically important crop hosts include: tomato, potato, pepper, tobacco, peanut, ornamentals, banana, plantain, and eucalyptus. Losses due to bacterial wilt are known to be enormous but cannot be accurately estimated because of its large but undocumented impact on subsistence agriculture and because planting of wilt-susceptible crops have been abandoned altogether in many parts of the world. Specifically, economic losses to the potato industry in the world have been estimated 950 million US $ and the potato brown rot strain of *Ralstonia solanacearum* was listed in the USA as a Bioterrorism Select Agent (BSA).

The pathogen usually enters host roots from the soil, multiplies in the root cortex, and colonizes the xylem vessels, so the bacteria spread rapidly throughout the host via the plant's own vascular system. Symptoms vary according to host, but rapid wilting and death are the common elements.

To unravel the genetic diversity within this species complex, a new classification scheme was proposed that distinguishes four phylotypes:
- phylotype I corresponds to the "Asiaticum" division 1 of Cook et al (1989) and contains strains belonging to biovars 3 (GMI1000), 4 and 5;
- phylotype II corresponds to the "Americanum" division 2 of Cook and contains strains belonging to race 1 biovar 1, race 2 biovar 1 (e.g. Moko disease-causing strains such as Molk2), race 3 biovar 2 (e.g. IPO1609 and UW551) and race 3 biovar 2T strains;
- phylotype III contains strains from Africa and the Indian Ocean, which belong to biovars 1, 2 and 2T;
- phylotype IV is reported highly heterogeneous; it contains strains from Indonesia, some strains from Japan, and a single strain from Australia, belonging to biovars 1, 2 and 2T; phylotype IV also contains the closely related species *Ralstonia syzigii* and the blood disease bacterium (BDB).

Each phylotype can be further subdivided into sequevars based on differences in partial sequence of the endoglucanase gene (eg/). The phylotyping scheme is broadly consistent with the former phenotypic and molecular typing schemes (Fegan and Prior, 2005: Prior and Fegan 2005), and adds valuable information about the geographical origin and in some cases the pathogenicity of strains. It is believed that, after the race/biovar classification, the phylotype classification scheme is to become the core organizing principle for assigning a particular strain a phylogenetic position with a predictive value on potential host range (Fegan and Prior, 2006; Wicker et al., 2007).

Whole genome sequencing was decisive in unravelling the broad genetic diversity encompassed within this organism with unusually broad host range. Thus a metagenomic microarray from sequence data of a broad host range tomato phylotype I strain (GMI1000) has been developed which has enabled comparative genomic hybridizations demonstrating that a third of the *Ralstonia solanacearum* genome is constituted of variable genes probably acquired by horizontal gene transfers. The distribution of variable genes between strains is related to the phylotype classification (Guidot et al, 2007).

Recent phylogenetic evidences indicated that strains that fit with the definition of the potato brown rot agent were placed into the phylotype IIB sequevar 1 and 2, *i*.*e*. the biovar 2 Andean strains of *Ralstonia solanacearum* historically known as race 3 biovar 2. These strains are highly pathogenic to potato and adapted to low temperatures. Some strains in that group were also reported to carry an enlarged host range including tomato, and *Geranium rosa* (Carmeille *et al.,* 2006).

Given, the important economic impact of potato brown rot, it is highly desirable to develop methods for specifically detecting race 3 biovar 2 *Ralstonia solanacearum* strains.

Thus, WO 2004/042016 relates to real-time PCR primers and probes useful for the detection of such strains. The primers and probes enable the detection of a nucleic acid sequence which is specific for race 3 biovar 2 *Ralstonia solanacearum* strains.

However, it appears that the nucleic acid sequence detected by the primers and probes of WO 2004/042016 is that of a mobile genetic element, since this sequence encodes part of a protein homologous to the Mu-like phage of the ORF35 of the B3 bacteriophage from *Pseudomonas aeruginosa* (Accession Q7AX27).

In general, mobile genetic elements should be avoided in the frame of the specific detection of a pathogenic microorganism, since particular strains of the pathogenic microorganism could lack the element or, conversely, other unrelated microorganisms could harbour the element, thereby yielding respectively false negative and false positive results.

Accordingly, it is an object of the present invention to develop a method for detecting race 3 biovar 2 *Ralstonia solanacearum* strains which involves the detection of specific nucleic acid sequences which do not belong to mobile genetic elements.

### Description of the invention

The present invention arises from the identification, by the inventors, of genomic portions of *Ralstonia solanacearum* which are specific of the race 3 biovar 2 strains of *Ralstonia solanacearum*.

Thus the present invention relates to a method for the detection of *Ralstonia solanacearum* race 3 biovar 2 in a medium, comprising the determination of the presence or the absence in a sample of the medium, of:
(i) at least one first nucleic acid target having a sequence selected from the group constituted of SEQ ID NO: 1-49, complementary sequences thereof, and homologous sequences thereof, or
(ii) at least one fragment of said first nucleic acid target, wherein said fragment is preferably not constituted of or comprised in a sequence selected from the group constituted of SEQ ID NO: 111-140;
whereby, if said first nucleic acid target or fragment thereof is present in the sample, it is determined that *Ralstonia solanacearum* race 3 biovar 2 is present in the medium.

The present invention also relates to the use of:
(i) at least one first nucleic acid target having a sequence selected from the group constituted of SEQ ID NO: 1-49, complementary sequences thereof, and homologous sequences thereof, or
(ii) at least one fragment of said first target nucleic acid, wherein said fragment is preferably not constituted of or comprised in a sequence selected from the group constituted of SEQ ID NO: 111-140;
for detecting *Ralstonia solanacearum* race 3 biovar 2.

As intended herein the "medium" can be of natural or synthetic origin. Where the medium is of synthetic origin, it can in particular be a solid or liquid bacterial culture medium, Where the medium is of natural origin, it can notably be water, soil, or a biological tissue, in particular a plant tissue. Preferably, the medium is selected from the group constituted of a potato tissue, a tomato tissue, and a geranium tissue. These plant tissues are particularly prone to infection by *Ralstonia solanacearum* race 3 biovar 2. However, it is particularly preferred that the medium is a potato tissue, since *Ralstonia solanacearum* race 3 biovar 2 is the causative agent of potato brown rot. Preferably, the potato tissue should be the tuber, which constitutes the potato tissue most likely to contain *Ralstonia solanacearum* race 3 biovar 2.

As intended herein a "sample" relates to a portion of the medium liable to contain nucleic acids. In particular, it is preferred that the sample is obtained from the medium by a nucleic acid extraction. Numerous methods exist, which are well known to one of skill in the art, for extracting nucleic acids from a medium, in particular from a plant tissue.

As intended herein "nucleic acid" preferably relates to RNA or DNA.

As intended herein "determination" of the presence or the absence of a nucleic acid in a sample relates to the detection of the full length nucleic acid in itself, but also to fragments of the nucleic acid. The present inventors have shown that genetic material transfers which have occurred in the course of evolution and which have structured the genome of *Ralstonia solanacearum* race 3 biovar 2 concerned genomic patches often involving several genes. These genomic patches correspond to SEQ ID NO: 1-49. Accordingly, the detection of fragments of these genomic patches is generally indicative of the presence of the nucleic acid as a whole. Besides, even if portions of SEQ ID NO: 1-49 should be lacking in a particular race 3 biovar 2 strain of *Ralstonia solanacearum,* it will be clear to one of skill in the art that the detection of fragments of conserved portions of SEQ ID NO: 1-49 is sufficient to unambiguously determine the presence of *Ralstonia solanacearum* race 3 biovar 2.

Thus, as intended herein a "fragment" should be of a length such that it can be considered by one of skill in the art that it presents essentially no identity with nucleic acids of the same length which can be found in *Ralstonia solanacearum* strains which are not of race 3 biovar 2. Preferably, fragments according to the invention comprise at least 9 nucleotides, more preferably at least 15 nucleotides and most preferably at least 18 nucleotides.

Exemplary fragments of the above-mentioned genomic patches notably encompass gene and inter-gene sequences SEQ ID NO: 50-110.

Some short portions of SEQ ID NO: 1-49 are of a known length but of undetermined sequence. It will be apparent to one of skill in the art that the above-defined fragments of SEQ ID NO: 1-49 should preferably not be constituted of these short portions.

The inventors have also found that the genomic patches they identified comprised sequence derived from mobile genetic elements. These mobile genetic elements are represented by SEQ ID NO: 111-140. Accordingly, the method and use according to the invention should preferably not determine the presence or absence of these mobile genetic elements or fragments thereof as first target nucleic acids.

As intended herein "homologous sequences" relate to natural variants of sequences SEQ ID NO: 1-49, which can be found in strains of *Ralstonia solanacearum* race 3 biovar 2. Thus the homologous sequences notably encompass:
- sequences which are derived from sequences SEQ ID NO: 1-49 by insertion, deletion or substitution of at least one nucleotide;
- sequences which are liable to hybridize under stringent conditions to the complementary sequences of SEQ ID NO: 1-49;
- sequences which present at least 95% identity to sequences SEQ ID NO: 1-49;
provided the homologous sequences are specific to *Ralstonia solanacearum* race 3 biovar 2.

In a particular embodiment of the above-defined method and use, the determination comprises at least one step of hybridization of the nucleic acid target or fragment thereof with a probe or a primer. Preferably, the probe or primer is a fragment of nucleic acid having a sequence selected from the group constituted of SEQ ID NO: 1-49, homologous sequences thereof, and complementary sequences thereof. More preferably, the probe or primer is selected from the group constituted of SEQ ID NO: 141-386.

In another particular embodiment of the above-defined method and use, the determination comprises at least one step of nucleic acid amplification.

Preferably, where the determination comprises a step of nucleic acid amplification, the determination is implemented by a method selected from PCR and NASBA. NASBA is notably described in Compton (1991) Nature 350:91-92.

Also preferably, the determination is implemented by a method selected from Southern blotting, Northern blotting, dot blots, and nucleic acid micro or macro-array hybridization,

In a particular embodiment of the invention the above-defined method and use also comprise the determination of the presence or the absence in a sample of the medium, of:
(i) at least one second nucleic acid target having a sequence selected from the group constituted of SEQ ID NO: 111-140, complementary sequences thereof, and homologous sequences thereof, or
(ii) at least one fragment of said second nucleic acid target.

Indeed, since the at least one first target sequence is preferably not a mobile genetic elements, the determination of the presence or absence of nucleic acids corresponding to mobile genetic elements can be effected in a further step.

The present invention also relates to a nucleic acid having a sequence selected from the group constituted of SEQ ID NO: 1-22, SEQ ID NO: 50-110, SEQ ID NO: 111-140, SEQ ID NO: 141-246, and SEQ ID NO: 247-386 and their complementary sequences.

The present invention also relates to a nucleic acid micro or macro-array comprising a plurality of nucleic acid probes arranged onto a solid support, wherein the nucleic acid probes are fragments of nucleic acids having sequences selected from the group constituted of SEQ ID NO: 1-49, complementary sequences thereof, and homologous sequences thereof, provided that preferably at least one of the nucleic acid probes is not a fragment of a nucleic acid having a sequence selected from the group consisting of SEQ ID NO: 111-140.

Preferably, in the above-defined nucleic acid micro-array, the nucleic acid probes comprise SEQ ID NO: 247-386.

The present invention also relates to a kit intended for the detection of *Ralstonia solanacearum* race 3 biovar 2 in a medium, comprising at least:
- two primers suitable to amplify a portion of a nucleic acid having a sequence selected from the group constituted of SEQ ID NO: 1-49 and complementary sequences thereof, provided that said portion is preferably not comprised in a nucleic acid having a sequence selected from the group consisting of SEQ ID NO: 111-140 and complementary sequences thereof;
- optionally one detectable nucleic acid probe suitable to hybridize to said amplified portion.

### EXAMPLE

### Materials and methods :

### • Bacterial strains used in this study

**Table 1** provides the list of the 14 race 3 biovar 2 strains and 45 non-race 3 biovar 2 strains used herein together with their geographical origin, host of origin. The 14 race 3 biovar 2 strains corresponded to phylotype IIB, sequevar 1 based on the classification scheme proposed by Fegan & Prior (2005).

### • Microarray :

A 12X draft of the genome sequence from strain IPO1609 (a race 3 biovar 2 strain accessible from the *Collection Française de Bactéries Phytopathogènes* under accession number CFBP 6926) has been established in collaboration with Genoscope and annotated by the inventors. The deduced amino acid sequence of the genes thus identified was compared with the amino acid sequence of the genes previously identified in strain GMI1000 (Salanoubat *et al.,* 2002). All the genes from IPO1609 that did not have a homolog (less than 40% identity over at least 80% of both the query and subject sequences) will be further referred to as "potential IPO1609-specific genes". For each of these genes a specific representative 70 mer-oligonucleotide was designed using ROSO algorithm (Reymond, 2004). These oligonucleotides were chosen as having no significant homology with any part of the genome of strain GMI1000 and were used to generate a microarray as previously reported for the construction of the GMI1000 microarray (Occhialini *et al.,* 2005). The "IPO1609 partial microarray" thus generated also includes additional oligonucleotides representative of the IPO1609 allelic variants for a limited number of genes conserved but significantly divergent between strains GMI1000 and IPO1609 (mostly including type III-secretion dependant pathogenicity effectors) therefore permitting the distinction of the two allelic forms of a given gene. Finally, a limited number of oligonucleotides representative of particular intergenic regions were also included on the microarray. The sequence of each individual oligonucleotide spotted on the microarray has been deposited at *Agence de Protection des Programmes* (APP) under accession number IDDN.FR.001.300024.000.R.P.2006.000.10300.

### • Genomic DNA extraction, DNA labelling, microarray hybridization, hybridization signal measurement and analysis.

These have been performed as previously described (Guidot *et al.,* 2007) except that standard control DNA used for all genome hybridizations experiments which were performed consisted of an equimolar combination of the genomic DNA from three sequenced strains GMI1000, IPO1609 and Molk2 (IPO1609 and Molk2 genomes being unpublished) (Molk2 is accessible from the *Collection Française de Bactéries Phytopathogènes* under accession number CFBP 6925 and from the ATCC under accession number BAA-1115). Analysis was conducted as previously described using ImaGene and GeneShight (BioDiscovery) softwares. A gene was considered as being absent in the tested strain when the base 2 logarithm of the ratio of the normalized hybridization signal of the tested strain over the normalized hybridization signal with the control DNA was lower to -1.

### • PCR validation

The list of candidate race 3 biovar 2-specific genes deduced from comparative genomic hybridizations (CGH) experiments was checked by PCR amplification. The PCR primers used are given in **Table 2.** The primers were designed to amplify one genomic fragment from each gene. When possible, one of the two primers for each gene was designed inside the oligonucleotide spotted on the microarray. PCR were conducted in 25 µl reaction mixture containing 10 ng DNA from each tested strain, 25 pmol of each primer (UR), 1.5 mM MgCl₂, 200 µM of each four dNTP, 0.5 U of Red Gold Star Taq DNA polymerase (Eurogentec) and the buffer supplied by the manufacturer. PCR amplifications were performed as follows: an initial denaturation step at 96°C for 5 min followed by 30 cycles of 94°C for 15 s., 59°C for 30 s., and 72°C for 30 s., with a final extension step of 72°C for 10 min. Negative (PCR reaction mixture without DNA) and positive (IPO1609 DNA) controls were included in each experiment. The multiplex PCR described by Fegan & Prior (2005) for the *Ralstonia solanacearum* phylotype identification was conducted on each tested DNA as an amplification positive control.

### • In silico comparison of strains UW551 with IPO1609, GMI1000 and Molk2 strains

The deduced amino acid sequence for each predicted gene from strain UW551 was BlastX compared to the genomic sequence of strain IPO1609 in order to identify the predicted proteins of UW551 that have no counterpart covering at least 80% of their length. These proteins designated as "potential UW551-specific proteins" were then compared with BlastX to the genomic sequence of strain GMI1000 and Molk2. All the proteins that did not have a counterpart covering at least 80% of the length of the query sequence with at least 40% identity where then individually compared using BlastP with the predicted proteins from strains GMI1000 and Molk2. This was performed in order to eliminate false candidates that could remain in the list due to frameshift in the nucleotide sequence.

### Results:

### • Analysis of the distribution of "potential IPO1609-specific genes" among a collection of strains representative of the diversity of Ralstonia solanacearum.

Comparative genomic hybridizations (CGH) on the microarray have been performed to compare the lists of genes between 11 race 3 biovar 2 strains and 20 non-race 3 biovar 2 strains **(Table 1).** This analysis identifies a set of 137 oligonucleotides which are present in at least 10 of the 11 race 3 biovar 2 strains and absent from at least 19 of the 20 non-race 3 biovar 2 strains. These oligonucleotides were representative of 79 genes and 38 intergenic regions from the IPO1609 genome. These genes and intergenes therefore were considered as "candidate race 3 biovar 2 specific genomic regions". A large proportion of these regions forms clusters in the IPO1609 genome and sometime a few genes mapping within these clusters were not found in the list of thus defined "candidate race 3 biovar 2 specific regions". Based on
i) the known mosaic structure of *Ralstonia solanacearum* genome that suggests that sets of genes could have been acquired through horizontal gene transfers or lost through deletions (Salanoubat et al., 2002 ; Guidot *et al.,* 2007),
ii) on the consideration that some genes might be missing from this list due to the fact that hybridization data are missing in a limited number of cases, and
iii) taking into account the possibility that certain genes that score next to the cutoff value could have been miss-categorized,
the inventors included into the list of the "race 3 biovar 2 specific regions" all the sets of 1 or 2 contiguous genes that were not originally detected as being race 3 biovar 2 -specific but that are located within a race 3 biovar 2 -specific gene cluster. Based on these criteria 34 additional genes were included into the list of race 3 biovar 2 -specific genes. These additional genes are identified with a star (*) in **Table 3.**

When compared using BlastP with the sequence of the predicted proteins from strains GMI1000 and Molk2, 5 of these genes were found to have a counterpart in at least one of these strains and were therefore eliminated from the final list of race 3 biovar 2 -specific genes presented in **Table 3.** This final list includes a total of 151 genes or intergenic regions organized in 18 clusters and 15 additional individual genes or intergenic regions.

### • Validation of candidate race 3 biovar 2-specific genomic regions.

The list of candidate race 3 biovar 2-specific genomic regions given in **Table 3** has been established based on the analysis of a limited number of strains. Therefore the inventors validated this list on a larger collection of strains representative of the diversity found in *Ralstonia solanacearum.*

A total of eight race 3 biovar 2-strains (among-which six were also used for CGH experiments) and 32 non-race 3 biovar 2 strains (seven of which were also used for CGH experiments) were used for this validation **(Table 1**). This validation was conducted by PCR amplification of one genomic fragment from each candidate race 3 biovar 2-specific region. All tested DNA could be amplified using the multiplex PCR for the *Ralstonia solanacearum* phylotype identification as described by Fegan and Prior (2005) therefore confirming that the strains tested actually correspond to *Ralstonia solanacearum* isolates.

Each genomic region that gave a positive amplification from non-race 3 biovar 2 strains or a negative amplification from race 3 biovar 2 strains was excluded from the list of "race 3 biovar 2 specific genomic regions". The results validated the specificity of eleven gene clusters and eleven individual genes or intergenic regions **(Table 4)**. Among these genes, 27 were predicted to be parts of mobile genetic elements (bacteriophage or insertion sequences).

### • Identification of additional candidate race 3 biovar 2-specific genes in strain UW551

A genomic draft for the race 3 biovar 2 virulent isolate UW551 is publicly available (Gabriel *et al.* 2006). Because strain IPO1609 probably harbours a genome deletion that significantly impairs its virulence compared to UW551, the inventors decided to compare the genome sequences for these two strains. In doing this 328 predicted genes from UW551 that had no counterpart in IPO1609 were identified. Based on the same criteria, *in silico* comparison of these 328 genes with the genome sequence of strain Molk2 and GMI1000 reduced this set to 94 UW551 specific genes. Comparison of these 94 predicted proteins using BlastP with the proteins of GMI1000 and Molk2 eliminated 67 proteins that were not previously detected as having a counterpart in GMI1000 or Molk2 due to the presence of a potential frameshift mutation in the corresponding genes, therefore leading to a final list of the 27 UW551 specific genes shown in **Table 5.**

### List of tables

**Table 1:** List of *Ralstonia solanacearum* strains used in this study (ND not determined, NO no object)
**Table 2:** List of the oligonucleotides used for PCR amplification of candidate race 3 biovar 2 specific genes. (a) Sequences in bold correspond to part of the oligonucleotide spotted on the microarray
**Table 3:** List of candidate race 3 biovar 2-specific genomic regions.*Added genes correspond to the genes that have been included into the list based on their location within clusters of race 3 biovar 2 specific gene clusters.
**Table 4:** Validated race 3 biovar 2 -specific genomic regions together with sequences of the PCR primers used for gene amplification. Black boxes highlight genes from mobile genetic elements (bacteriophage or insertion sequences).
**Table 5:** List of additional candidate race 3 biovar 2-specific genes identified from strain UW551.

**Table 1**

| **Strain ID** | **Host** | **Origin** | **Race** | **Biovar** | **CGH on microarray** | **PCR verification** |
|---|---|---|---|---|---|---|
| IPO1609 | Potato | Netherlands | 3 | 2 | Yes | control + |
| JT516 | Potato | Reunion Is. | 3 | 2 | Yes | Yes |
| CMR34 | Tomato | Cameroon | 3 | 2 | Yes | Yes |
| RE | Potato | Uruguay | 3 | 2 | Yes | Yes |
| AP31 H | Potato | Uruguay | 3 | 2 | Yes | |
| AP42H | Potato | Uruguay | 3 | 2 | Yes | Yes |
| TB1H | Potato | Uruguay | 3 | 2 | Yes | |
| TB2H | Potato | Uruguay | 3 | 2 | Yes | |
| TC1H | Potato | Uruguay | 3 | 2 | Yes | Yes |
| TB10 | Potato | Uruguay | 3 | 2 | Yes | |
| ETAC | Potato | Uruguay | 3 | 2 | Yes | |
| RM | Potato | Uruguay | 3 | 2 | Yes | Yes |
| PSS525 | Potato | Taiwan | 3 | 2 | | Yes |
| CMR24 | Potato | Cameroon | 3 | 2 | | Yes |
| CIP10 | Potato | Peru | 3 | 2T | | Yes |
| NCPPB3987 | Potato | Brazil | 3 | 2T | | Yes |
| Molk2 | Banana | Philippines | 2 | 1 | Yes | |
| CIP418 | Peanut | Indonesia | 2 | 1 | Yes | |
| UW9 | Heliconia | Costa Rica | 2 | 1 | | Yes |
| CFBP1183 | Heliconia | Costa Rica | 2 | 1 | | Yes |
| UW163 | Plantain | Peru | 2 | 1 | Yes | |
| Ant75 | Heliconia | Martinique | NO | 1 | Yes | |
| Ant80 | Anthurium | Martinique | NO | 1 | Yes | |
| Ant307 | Anthurium | Martinique | NO | 1 | Yes | |
| JY200 | Anthurium | Martinique | NO | 1 | Yes | |
| JY201 | Anthurium | Martinique | NO | 1 | Yes | |
| Ant1121 | Anthurium | Martinique | NO | 1 | Yes | Yes |
| CFBP6797 | Solanum | Martinique | NO | 1 | | Yes |
| CFBP7014 | Anthurium | Trinidad | NO | 1 | | Yes |
| TOM=T1 | Tomato | Uruguay | 2 | 1 | Yes | Yes |
| B34 | Banana | Brazil | 2 | 1 | Yes | Yes |
| A3909 | Heliconia | Hawai | 2 | 1 | Yes | Yes |
| CIP239 | Potato | Brazil | 1 | 1 | | Yes |
| CIP301 | Potato | Peru | 1 | 1 | | Yes |
| CFBP2957 | Tomato | Martinique | 1 | 1 | | Yes |
| CMR39 | Tomato | Cameroon | 1 | 1 | | Yes |
| ICMP7963 | Potato | Kenya | 1 | 1 | | Yes |
| CFBP6942 | Solanum | Cameroon | NO | 2T | Yes | Yes |
| CFBP6941 | Tomato | Cameroon | NO | 2T | Yes | |
| CMR43 | Potato | Cameroon | NO | 2T | | Yes |
| CIP358 | Potato | Cameroon | NO | 2T | | Yes |
| CFBP3059 | Eggplant | Burkina | NO | 1 | Yes | Yes |
| | | Faso | | | | |
| CMR66 | Solanum | Cameroon | NO | 2T | | Yes |
| JT525 | Pelargonium | Reunion Is. | NO | 1 | | Yes |
| JT528 | Potato | Reunion Is. | NO | 1 | | Yes |
| J25 | Potato | Kenya | NO | 2T | | Yes |
| NCPPB332 | Potato | Zimbabwe | NO | 1 | | Yes |
| GMI1000 | Tomato | Guyana | 1 | 3 | Yes | |
| CMR134 | Solanum | Cameroon | 1 | 3 | Yes | |
| CIP365 | Potato | Philippines | 1 | 3 | | Yes |
| R288 | Morus alba | China | 1 | 5 | | Yes |
| PSS358 | Tomato | Taiwan | 1 | 3 | Yes | |
| PSS190 | Tomato | Taiwan | 1 | 3 | Yes | |
| PSS219 | Tomato | Taiwan | 1 | 3 | | Yes |
| ACH732 | Tomato | Australia | NO | 2 | | Yes |
| Psi07 | Tomato | Indonesia | NO | 2T | | Yes |
| Psi36 | Tomato | Indonesia | NO | 2T | | Yes |
| MAFF301558 | Potato | Japan | NO | 2T | | Yes |
| R. syzygii | Clove | Indonesia | NO | NO | Yes | Yes |
| R28 | | | | | | |

**Table 2**

| **Gene name** | **Primer name** | **Primer sequence (a)** | **Product size (bp)** | **SEQ ID NO : 1** |
|---|---|---|---|---|
| IPO_00030 | IPO_00030_L | **ACTTGGAGAGATTTACGGAGGAG** | 200 | 387 |
| | IPO_00030_R | AAGCAAACGAGATAAGGGAGAAC | | 388 |
| IPO_00031 | IPO_00031_L | AAACACAATTCACCTTCCTGATG | 185 | 389 |
| | IPO_00031_R | **GGCCACTAGACTTTCCAGTGAT** | | 390 |
| IPO_00034 | IPO_00034_L | AGCTTACCTGCTGTTCGACATCT | 818 | 391 |
| | IPO_00034_R | **GTTCTTCGTGATAGCGGAGACT** | | 392 |
| IPO_00043 | IPO_00043_L | CTGAATTCGAAAAGGATAGAGCA | 206 | 141 |
| | IPO_00043_R | CTCGACAAACTCTTGCAACTGAC | | 142 |
| IPO_00044 | IPO_00044_L | **CTATGCAGAAGCGTTGCTTGTT** | 191 | 143 |
| | IPO_00044_R | CTTTAGCGAGCACAAGATTGAGT | | 144 |
| IPO_00045 | IPO_00045_L | **GAGATCGTTGGAAACATCAAGAC** | 165 | 145 |
| | IPO_00045_R | GTGAACCACTATTGCCGGTATC | | 146 |
| IPO_00233 | IPO_00233_L | AGAACTGCCAAGTTCGACTACCT | 207 | 147 |
| | IPO_00233_R | **CATTCCAACGTTCAGATGGTTAT** | | 148 |
| IPO_00234 | IPO_00234_L | GCAGAAAAGATATCCCCTGCAC | 214 | 149 |
| | IPO_00234_R | **TTCGAGTACAAATGTAGGCTTCC** | | 150 |
| IPO_00875 | IPO_00875_L | GATCAGATGGAGCAAAGAACACT | 221 | 151 |
| | IPO_00875_R | TATTGAAACTCTTCACGGGTCAT | | 152 |
| IPO_00876 | IPO_00876_L | **TTTCGACCAAGAAAAGCATAGAG** | 238 | 153 |
| | IPO_00876_R | ATTTCTGTGCCCACTACGAACTA | | 154 |
| IPO_00877 | IPO_00877_L | TGGTGTTCTAACTGTGGAAGGTT | 163 | 155 |
| | IPO_00877_R | TACCGCCAGTCATATCAGTTCTT | | 156 |
| IPO_00878 | IPO_00878_L | **ATTAGACTGATCAAGGCATGGAA** | 152 | 157 |
| | IPO_00878_R | CCTTCATTATTGAGACGGTCAAG | | 158 |
| IPO_00879 | IPO_00879_L | **ATGTTTGTGCTACTGGTCAGTCC** | 223 | 159 |
| | IPO_00879_R | CCTTCACTTGCAGATAATGGAAC | | 160 |
| IPO_00881 | IPO_00881_L | AAAGAAGCTCAAGGAGATCAAGG | 201 | 161 |
| | IPO_00881_R | **AACAGCAGGTTGTGATACTGCAT** | | 162 |
| IPO_01030 | IPO_01030_L | TATGAATGGGTTGATAGCGTTCT | 165 | 393 |
| | IPO_01030_R | **CCATATCCACCGATAAACAACAT** | | 394 |
| IPO_01058 | IPO_01058_L | **CGAGCTCATCGTTATCGACAT** | 140 | 163 |
| | IPO_01058_R | AAGCTCTTGGACTAGGACGATCT | | 164 |
| IPO_01131 | IPO_01131_L | **CACGATATGACCACGATCAACTA** | 195 | 395 |
| | IPO_01131_R | GTAGACACGAATCACGTCTCCAT | | 396 |
| IPO_01132 | IPO_01132_L | **ACATCAACGACCCTTACTGTCC** | 147 | 397 |
| | IPO_01132_R | GACCATAGTCATCGCTGCTTAAC | | 398 |
| IPO_01137 | IPO_01137-1_L | CTATCCCGCAGAAGGTATTCAAC | 186 | 399 |
| | IPO_01137-1_R | AGTCATAGGCGTCTCGGTACTT | | 400 |
| | IPO_01137-2_L | ACACTCTGTTCACCAAGTACGG | 217 | 401 |
| | IPO_01137-2_R | **CTTTGAAACTGGAGGAACAGCTT** | | 402 |
| IPO_01259 | IPO_01259_L | **TGAAATGCTCAAAGACAAACAGA** | 235 | 165 |
| | IPO_01259_R | ATCGTACAGGTCATTGCCAAAT | | 166 |
| IPO_01260 | IPO_01260_L | TACAACCTGAAGAGGATCTCGAA | 225 | 167 |
| | IPO_01260_R | **AAAGCCGGTCATAGAGGACATAG** | | 168 |
| IPO_01311 | IPO_01311_L | **CAACCAGACCATCTACAAGATCC** | 165 | 169 |
| | IPO_01311_R | GCTTCATACTCAAATCGAACACC | | 170 |
| IPO_01312 | IPO_01312_L | AACTCCAACTTGCTTGACTGTTC | 208 | 171 |
| | IPO_01312_R | GGATGAACTTCGTTCGATTGAG | | 172 |
| IPO_01314 | IPO_01314_L | GAAGCTCGGTGATATCGAAAC | 287 | 173 |
| | IPO_01314_R | GGTGATCGCTGTCGATAATTT | | 174 |
| IPO_01362 | IPO_01362_L | **AATTGGGTATACGTGATCTGTGG** | 280 | 243 |
| | IPO_01362_R | TCGGGTAAGACGAAGCTGACTA | | 244 |
| IPO_02090 | IPO_02090_L | CAATAGAAATTGCCGAGGTGATA | 171 | 239 |
| | IPO_02090_R | **CCTTGATAAGGATGTTCAACGAC** | | 240 |
| IPO_02092 | IPO_02092_L | AACACTCAAAAGCTGACCATCAT | 151 | 403 |
| | IPO_02092_R | **CAACCTTGATCTGTTCGGAGAC** | | 404 |
| IPO_02095 | IPO_02095_L | GTTGCAATGCTGGTTTCCAAG | 157 | 405 |
| | IPO_02095_R | **GTCATGGACGAGAAATCGATAC** | | 406 |
| IPO_02097 | IPO_02097_L | **CTCAGAGGATCTGTTCATCGACT** | 160 | 407 |
| | IPO_02097_R | GTTGAAGACGCCGAAGAAAAA | | 408 |
| IPO_02098 | IPO_02098_L | **ACGAGATTCCTGAAGCTGAGGT** | 192 | 409 |
| | IPO_02098_R | CTTGCAGAACCTGACACATGA | | 410 |
| IPO_02102 | IPO_02102_L | GTATCAGAAAGGCCAGCTACACA | 207 | 175 |
| | IPO_02102_R | **CTGATTGCCAATATTCGATTCTC** | | 176 |
| IPO_02140 | IPO_02140_L | AAGGGCAATGGCTTTTTCTGT | 153 | 177 |
| | IPO_02140_R | **GAGACTGATAAATCAGCGTTTCC** | | 178 |
| IPO_02141 | IPO_02141_L | GCTTTCTACGTCGCCTCAGTAT | 225 | 179 |
| | IPO_02141_R | **GTAACGGTCTGATTCTTGAGGTTT** | | 180 |
| IPO_02142 | IPO_02142_L | CCACTACCCCTGTTTCCATTC | 245 | 181 |
| | IPO_02142_R | AAACCTGTAGTCGTCGTCCTTG | | 182 |
| IPO_02143 | IPO_02143_L | **AGACGTTGGACAACATCAACC** | 189 | 183 |
| | IPO_02143_R | AGTTCTGTTCGTCGTCGTGAAT | | 184 |
| IPO_02144 | IPO_02144_L | **CCAGTATTCCAAGACGCTATCC** | 189 | 185 |
| | IPO_02144_R | AACGGATACAGCAGCAGGTTT | | 186 |
| IPO_02145 | IPO_02145_L | ATACCCAGCGCGTATTCCAA | 164 | 187 |
| | IPO_02145_R | **GTTGAGGCAACACCAGACAG** | | 188 |
| IPO_02146 | IPO_02146_L | CTTGAGAAAGCTTTTGGTGAAGA | 166 | 189 |
| | IPO_02146_R | **CTTTGAGACCTTCCCAGGCTAA** | | 190 |
| IPO_02147 | IPO_02147_L | TAAGAGCAGGCTATGGACAACAT | 209 | 191 |
| | IPO_02147_R | AATACCAGCACACAGAAGGTCAG | | 192 |
| IPO_02148 | IPO_02148_L | **CTGATTTCCATGTACCTGCATC** | 235 | 193 |
| | IPO_02148_R | ACATCAGCACGTTCTTGTAGAGC | | 194 |
| IPO_02149 | IPO_02149_L | GACGATGAGTTGCTGAAGTACC | 237 | 195 |
| | IPO_02149_R | TGAAGGTAATGGTCACAGCTTTT | | 196 |
| IPO_02150 | IPO_02150_L | **ATCCGGTTCCTTCTGATGATCT** | 124 | 197 |
| | IPO_02150_R | CTTCAACTTCACGTGTTCAATCA | | 198 |
| IPO_02151 | IPO_02151_L | GCTAGTCCACACGACAAAATCAT | 163 | 199 |
| | IPO_02151_R | **GTGACTAGCTTGGCGATCTTCT** | | 200 |
| IPO_02152 | IPO_02152_L | **AGGTGAAGTGCTCGAAATCCT** | 284 | 201 |
| | IPO_02152_R | CTTCTTCCTTCTCGATGCCTTC | | 202 |
| IPO_02153 | IPO_02153_L | TTATGTGGCTTTCTCTGGCAATA | 228 | 203 |
| | IPO_02153_R | **ACAAACGTCCAGTCGTCAATC** | | 204 |
| IPO_02154 | IPO_02154_L | CATTTATCTCTGGTCTTGGCTTG | 173 | 205 |
| | IPO_02154_R | **ACAGCCAAACTGACAAGATCG** | | 206 |
| IPO_02155 | IPO_02155_L | GCATGAAAATGTCTACGTTCCTC | 491 | 207 |
| | IPO_02155_R | ATGGTGATAGTGCGGAATGAC | | 208 |
| IPO_02156 | IPO_02156_L | **CAGACATGTTCTGCGAAGGAT** | 283 | 209 |
| | IPO_02156_R | GAGGAACGTAGACATTTTCATGC | | 210 |
| IPO_02157 | IPO_02157_L | TGTAATGACCAAGCAAGAACTCA | 112 | 211 |
| | IPO_02157_R | **CACGGTGTCAAGAATGGTTTC** | | 212 |
| IPO_02158 | IPO_02158_L | ATTTCAAACGCCAAGCCTTTAAC | 165 | 213 |
| | IPO_02158_R | **GTTCATCGAAAGCGATGTTCTC** | | 214 |
| IPO_02159 | IPO_02159_L | GCCTATTTGGACCGAAGAAGAC | 394 | 215 |
| | IPO_02159_R | ACTTCTTGAGGCATCTCGGTTT | | 216 |
| IPO_02160 | IPO_02160_L | GCAGCATTGATGACAAGTTCC | 339 | 217 |
| | IPO_02160_R | AGTATTGGTAAAGGCGTTGCAC | | 218 |
| IPO_02162 | IPO_02162_L | AAGGCTAAGGGGGAGTAAGTCAT | 548 | 411 |
| | IPO_02162_R | **AGGTAGTTGCGTACTTGGTCGTA** | | 412 |
| IPO_02163 | IPO_02163_L | **AGCAACCGAAGATATCGACCT** | 284 | 219 |
| | IPO_02163_R | GCCCAAGCGAAATCAACTCTT | | 220 |
| IPO_02165 | IPO_02165_L | CTTTCCCAGTCAATACATTCCAG | 220 | 221 |
| | IPO_02165_R | CGTTATCACATAAGCCACATCAA | | 222 |
| IPO_02166 | IPO_02166_L | **AAACAACACGCTGTTGAGCAT** | 195 | 223 |
| | IPO_02166_R | CTTCTTTGCGCACCAATAATC | | 224 |
| IPO_02923 | IPO_02923_L | **GCGCAAATTAAACACTACAAAGG** | 225 | 413 |
| | IPO_02923_R | ATCCCCAATCTCCTTTATCACTC | | 414 |
| IPO_02924 | IPO_02924_L | GAAGGTAAATCCAAAGGAAATGG | 232 | 415 |
| | IPO_02924_R | AATTGACTTCGGCTCGTATTCTT | | 416 |
| IPO_02927 | IPO_02927_L | AATTCCATGAGTGGAGTGATTTTT | 245 | 417 |
| | IPO_02927_R | **AGATCATGAGCCTCAGCATTATTA** | | 418 |
| IPO_03123 | IPO_03123_L | GAACGGAGCCATAGTGATGAAG | 946 | 419 |
| | IPO_03123_R | AGAGTCGCTAACACAAGTCATCC | | 420 |
| IPO_03132 | IPO_03132_L | AGGGAATCAAATCGCTCATCT | 226 | 245 |
| | IPO_03132_R | AGAAGAAGCCCATGATGACAGAG | | 246 |
| IPO_03302 | IPO_03302_L | **CGTGTATATCGGCAGTCAAGAAG** | 227 | 225 |
| | IPO_03302_R | CTAAGAAATGAAAGGTGGGGTTC | | 226 |
| IPO_03306 | IPO_03306_L | GGATGACTTGTGTTAGCGACTCT | 171 | 227 |
| | IPO_03306_R | **GAATACGATCCTCCACAATCAAA** | | 228 |
| IPO_03560 | IPO_03560_L | **CACGATCATATATGGGTCCAGTT** | 173 | 421 |
| | IPO_03560_R | GGTTCTTTTTGATCGTAGCCTTT | | 422 |
| IPO_03656 | IPO_03656_L | **TCTGTTCCGAGTATCACCTTGTT** | 156 | 423 |
| | IPO_03656_R | CATAGTATTCGCAGTCCAGATCC | | 424 |
| IPO_03659 | IPO_03659_L | GGATGTGGGGAGGTTTATTAGTC | 192 | 425 |
| | IPO_03659_R | **TTCATCAACTCCTCAGGAATCAG** | | 426 |
| IPO_03742 | IPO_03742_L | AAGATATGTGCCAGCTACCACTG | 206 | 427 |
| | IPO_03742_R | **AGCATATACAGTCCCTCGTATGC** | | 428 |
| IPO_03816 | IPO_03816_L | **ATTTCAACGACCTGCATCAGA** | 272 | 429 |
| | IPO_03816_R | CCACACCAGGTTCTTCTTGTTC | | 430 |
| IPO_04000 | IPO_04000_L | GTAAGGTCTGCAAGGACATCATC | 179 | 431 |
| | IPO_04000_R | **GTGGCTGCGATAGAACTTGTAGT** | | 432 |
| IPO_04001 | IPO_04001_L | **GACCATCTTTCAGTGGTGGA** | 110 | 433 |
| | IPO_04001_R | TAATGCCCTAAACTTTCCTGATG | | 434 |
| IPO_04002 | IPO_04002_L | TGTTTGATCTGAGCAAGTTTGTG | 250 | 435 |
| | IPO_04002_R | AGAAACTCATCCGCAAGGTC | | 436 |
| IPO_04003 | IPO_04003_L | GTAAGGAGAAGTGATGTCGGAAC | 283 | 437 |
| | IPO_04003_R | **GTCCTTGTTCTTGAACTGGTACG** | | 438 |
| IPO_04004 | IPO_04004_L | CTTGACGTCTGACAACCAAGTAG | 342 | 241 |
| | IPO_04004_R | ATAAGATAAACAGGTCGGCCTTC | | 242 |
| IPO_04067 | IPO_04067_L | **CATGCCAAGGAACACATCAAG** | 203 | 439 |
| | IPO_04067_R | AAGTATTTGTTGCCGTGGTACTC | | 440 |
| IPO_04353 | IPO_04353_L | AGGTACTCGGCTATCACAATCAC | 202 | 441 |
| | IPO_04353_R | **ATGCATTCTCCATGTATTCCATC** | | 442 |
| IPO_04521 | IPO_04521_L | TGAAGCACTGTCTATCAACCAGA | 222 | 229 |
| | IPO_04521_R | TTTGTCCTAGTCACAGCACTGAA | | 230 |
| IPO_04523 | IPO_04523_L | GACTTCGGCTATCTGGAGAAAAT | 217 | 231 |
| | IPO_04523_R | TCTTAGCAGGTTTAGGCTGAGTG | | 232 |
| IPO_04524 | IPO_04524_L | CATCTTCAAGGATGACTCTCTGG | 235 | 233 |
| | IPO_04524_R | **GAAGAAGTGACCAGGCTGAATTT** | | 234 |
| IPO_04525 | IPO_04525_L | ACTCAGTGACGAAGAGGTTGAAG | 250 | 235 |
| | IPO_04525_R | ACGAGTAGCTTCAATGGTGTCTT | | 236 |
| IPO_04526 | IPO_04526_L | ACACTATGCCTGCTGACTTGAA | 169 | 237 |
| | IPO_04526_R | AGGTGACTTCAACAATGTTAGGC | | 238 |
| IPO_04530 | IPO_04530_L | GAGACTCGGTTCAACAAGAAAAA | 207 | 443 |
| | IPO_04530_R | **CTTAGACGCACTAGCGAAATACG** | | 444 |

| | | | | |
|---|---|---|---|---|
| a) sequences in bold correspond to part of the oligonucleotide spotted on the microarray | | | | |

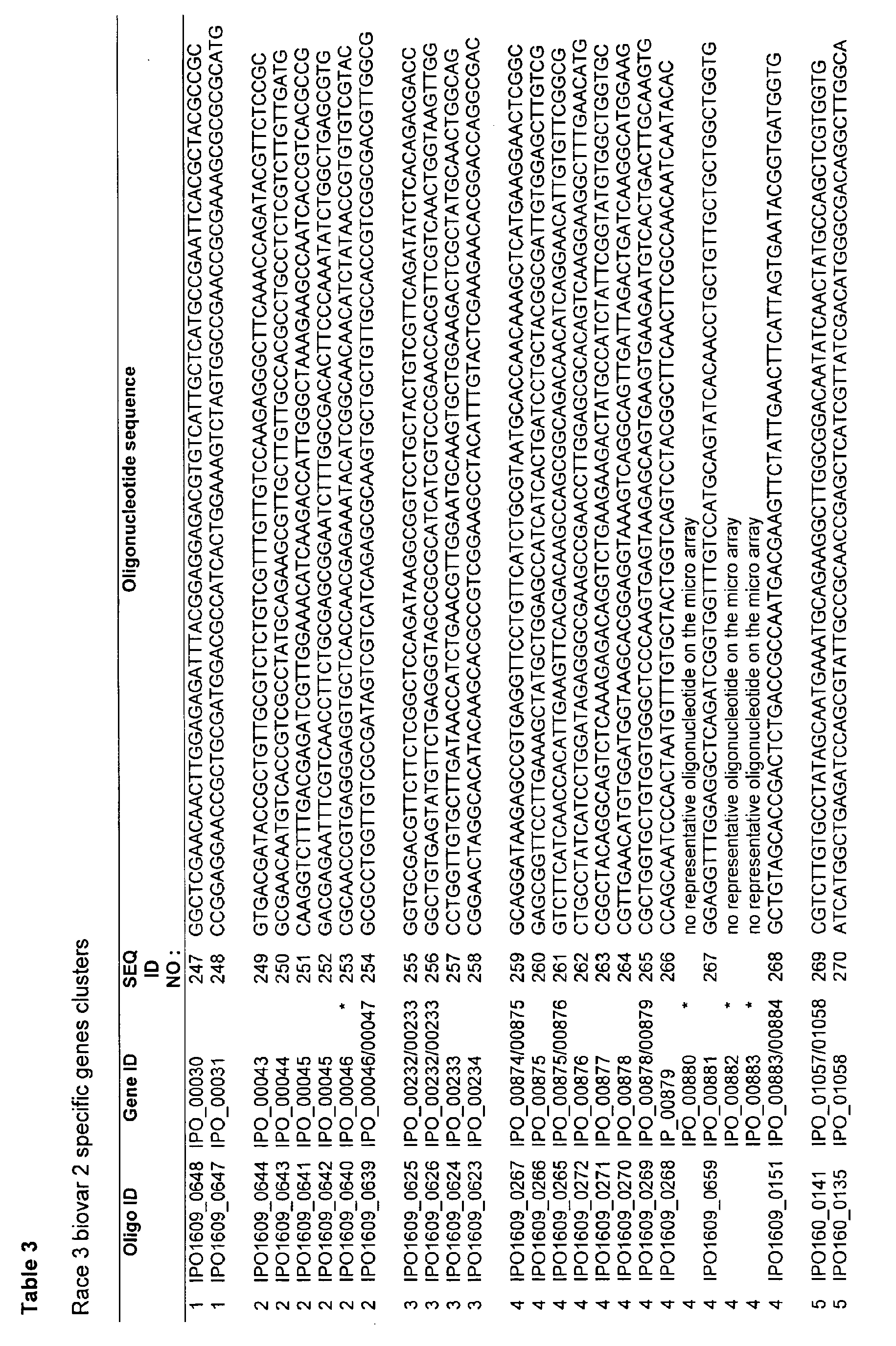

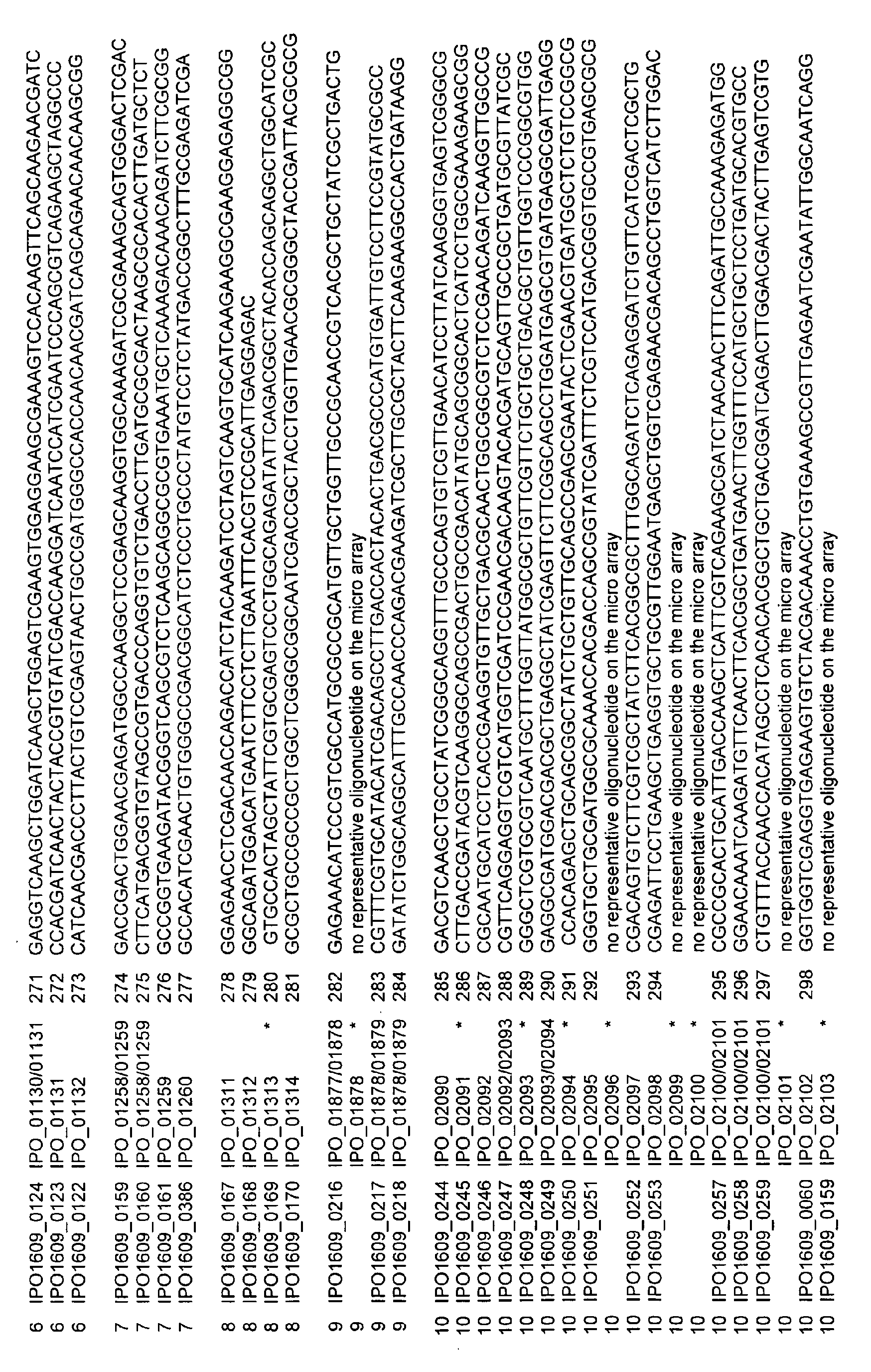

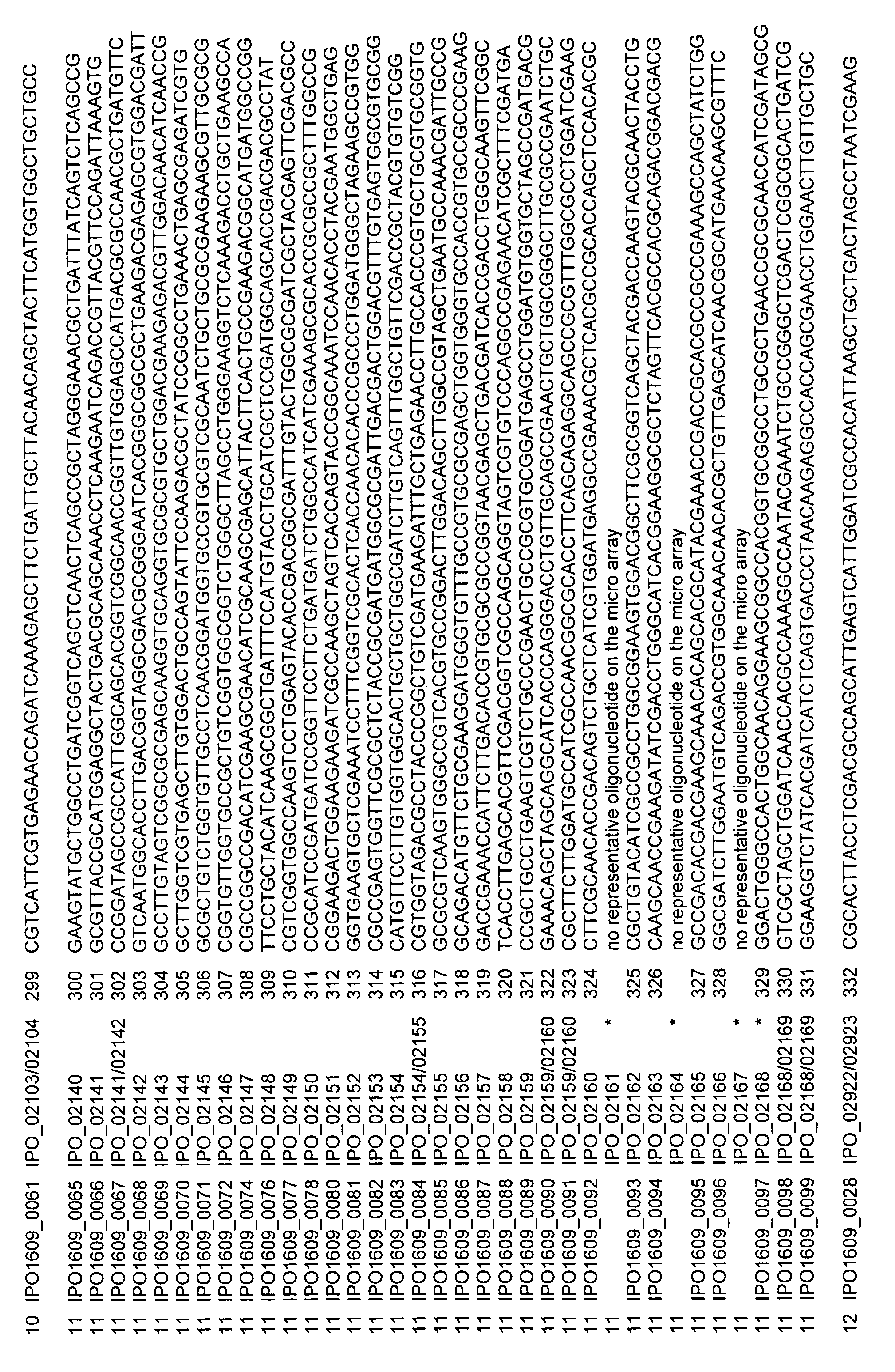

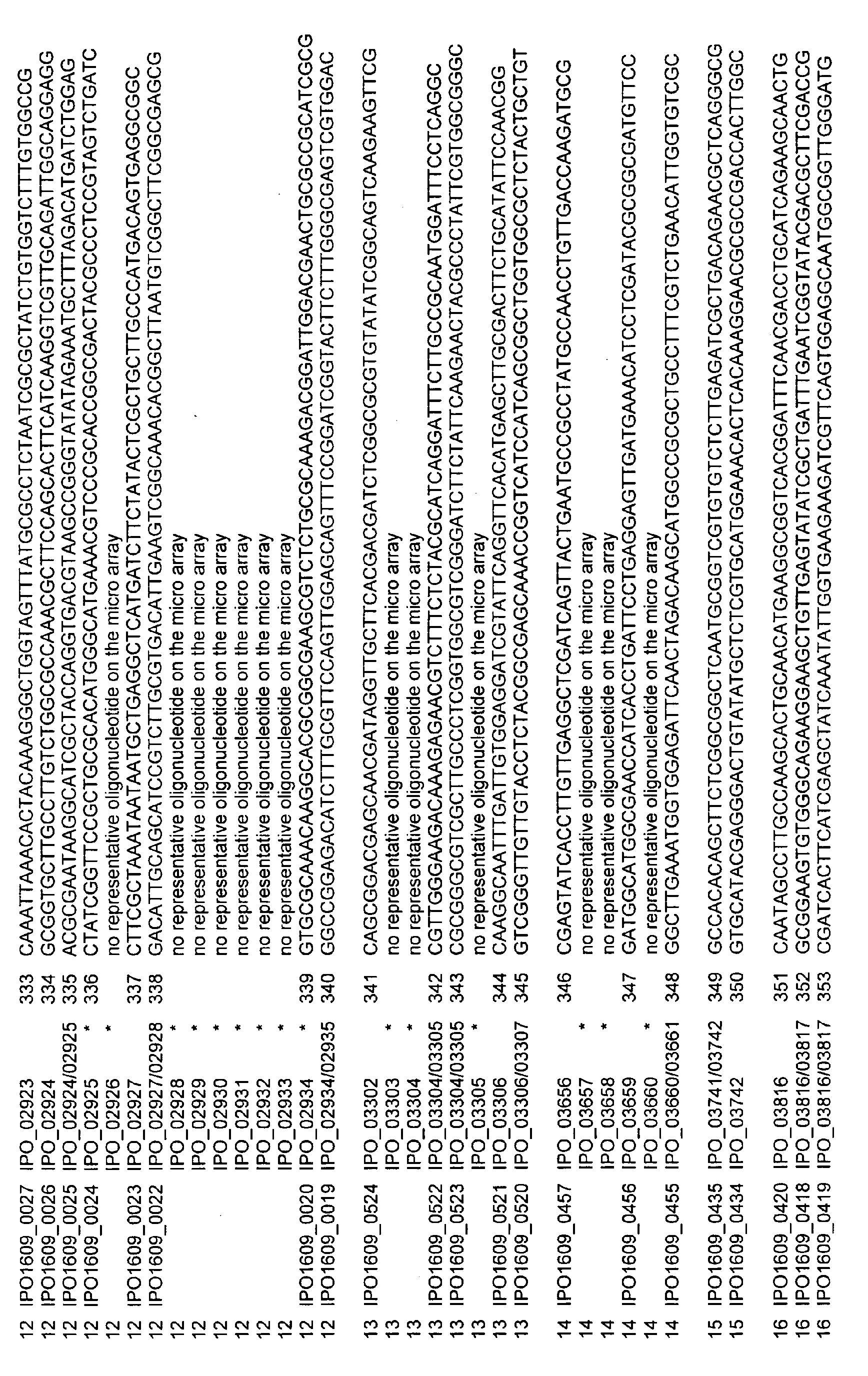

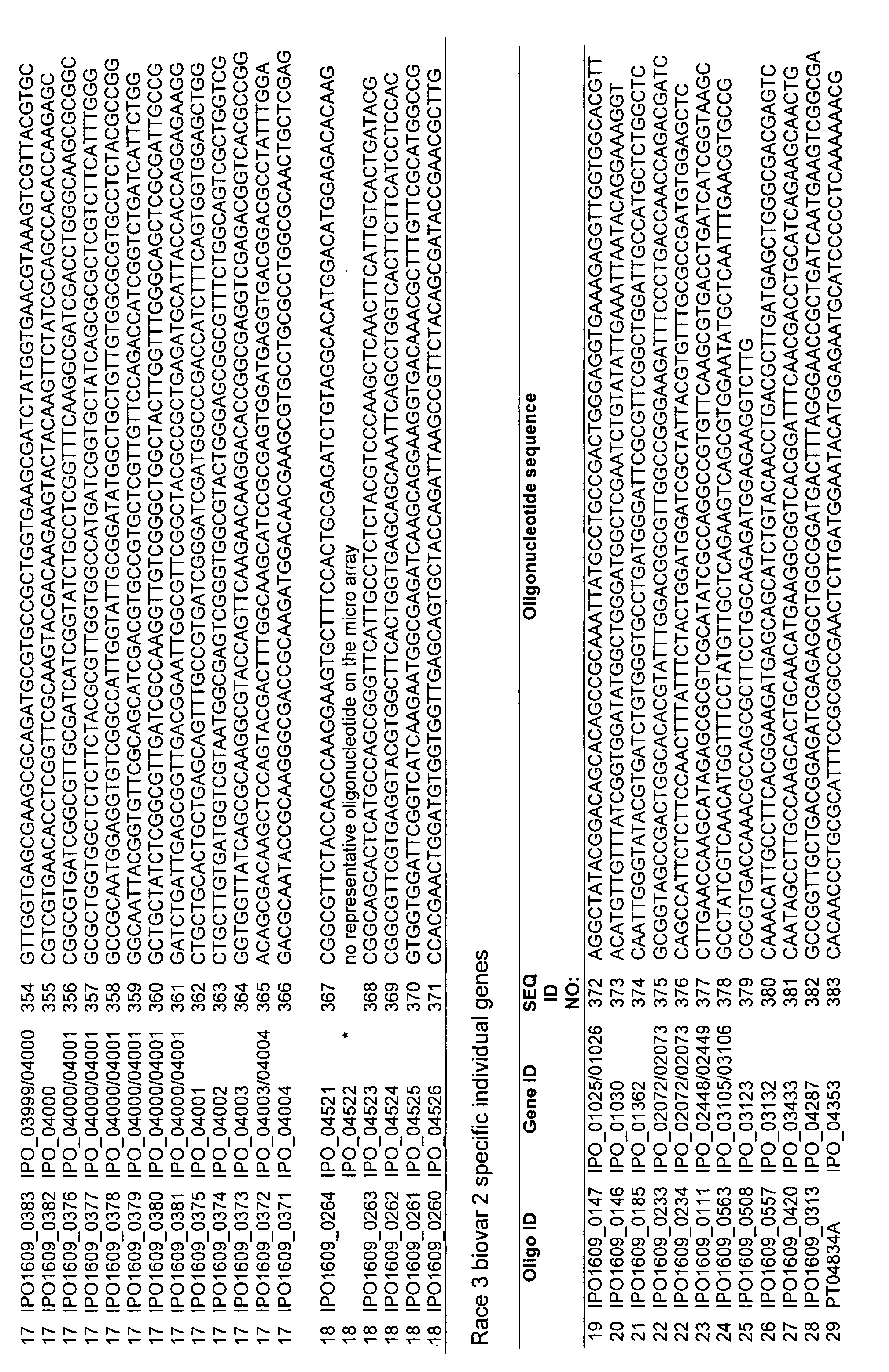

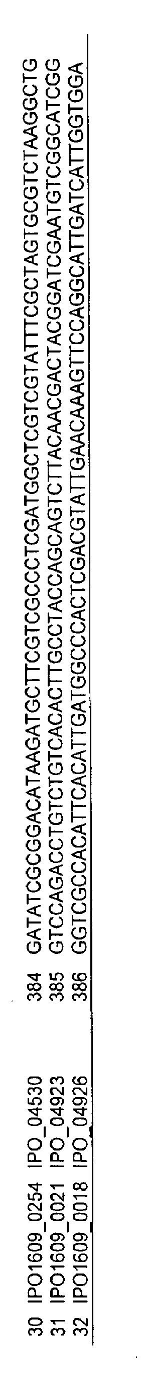

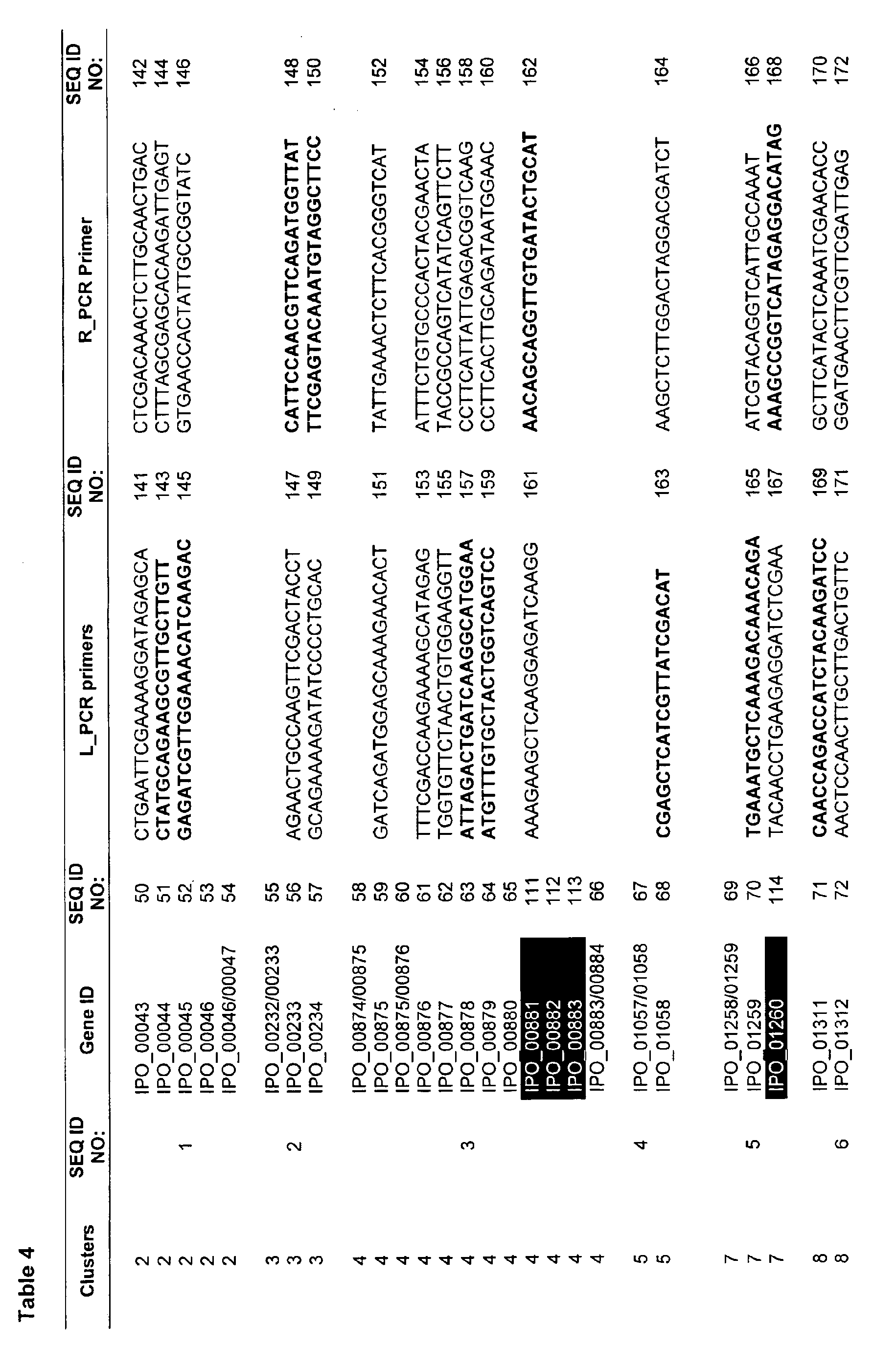

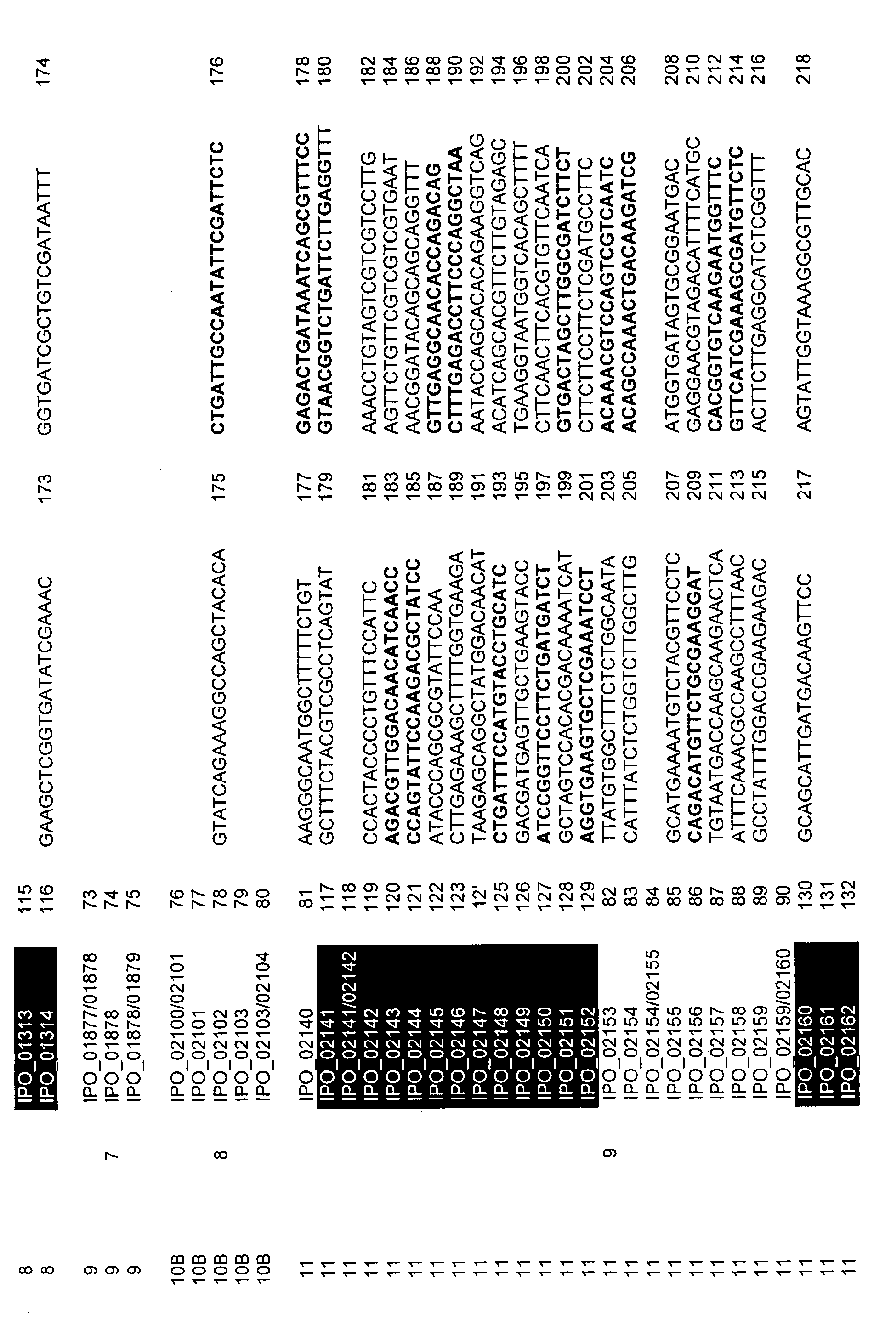

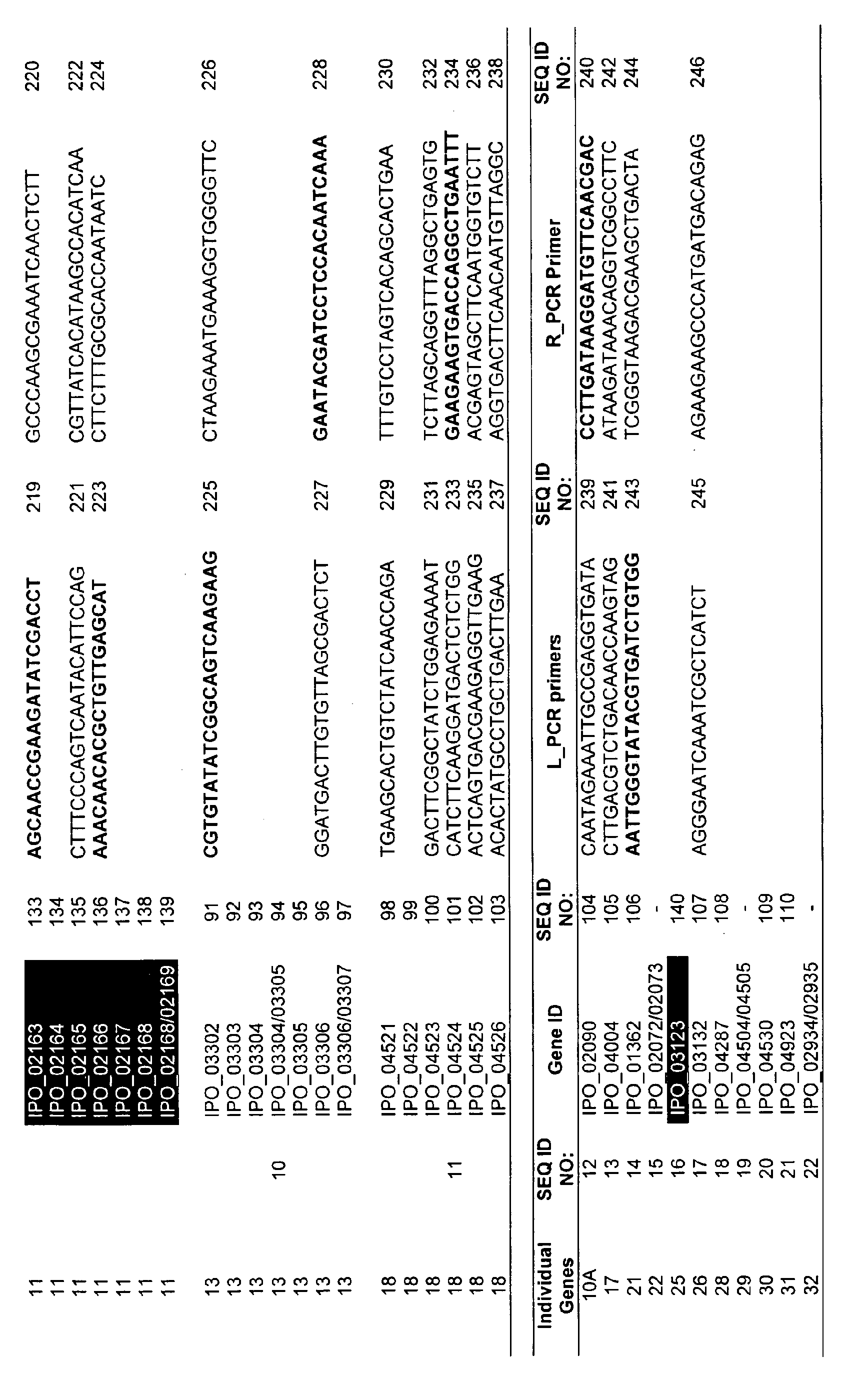

**Table 5**

| **Gene ID** | **GenBank Accession number** | **SEQ ID NO:** |
|---|---|---|
| RRSL_00004 | NZ_AAKL01000174 | 23 |
| RRSL_00093 | NZ_AAKL01000099 | 24 |
| RRSL_00419 | NZ_AAKL01000074 | 25 |
| RRSL_00442 | NZ_AAKL01000066 | 26 |
| RRSL_00500 | NZ_AAKL01000026 | 27 |
| RRSL_00600 | NZ-AAKLO1000073 | 28 |
| RRSL_00696 | NZ_AAKL01000059 | 29 |
| RRSL_00772 | NZ_AAKL01000058 | 30 |
| RRSL_01930 | NZ_AAKL01000033 | 31 |
| RRSL_01998 | NZ_AAKL01000044 | 32 |
| RRSL_02069 | NZ_AAKL01000022 | 33 |
| RRSL_02232 | NZ_AAKL01000025 | 34 |
| RRSL_02410 | NZ_AAKL01000024 | 35 |
| RRSL_02412 | NZ_AAKL01000024 | 36 |
| RRSL_02430 | NZ_AAKL01000024 | 37 |
| RRSL_03158 | NZ_AAKL01000027 | 38 |
| RRSL_03244 | NZ_AAKL01000017 | 39 |
| RRSL_03346 | NZ_AAKL01000012 | 40 |
| RRSL_03351 | NZ_AAKL01000012 | 41 |
| RRSL_03359 | NZ_AAKL01000012 | 42 |
| RRSL_03472 | NZ_AAKL01000008 | 43 |
| RRSL_03712 | NZ_AAKL01000013 | 44 |
| RRSL_03795 | NZ_AAKL01000009 | 45 |
| RRSL_03985 | NZ_AAKL01000007 | 46 |
| RRSL_04009 | NZ_AAKL01000007 | 47 |
| RRSL_04153 | NZ_AAKL01000004 | 48 |
| RRSL_04507 | NZ_AAKL01000002 | 49 |

### References:

Carmeille A, Prior P, Kodja H, Chiroleu F, Luisetti J, Besse P (2006) Evaluation of resistance to race 3, biovar 2 of Ralstonia solanacearum in tomato germplasm. J. Phytopathol. 154:398-402
Cook, D., and L. Sequeira. 1994. Strain differentiation of Pseudomonas solanacearum by molecular genetic methods, p. 77-94. In A. C. Hayward and G. L. Hartman (ed.), BActerial wilt- The disease and ist causative agent, Pseudomonas solanacearum. CAB International, Wallingford, UK.
Fegan, M., and P. Prior. (2005) How complex is the "Ralstonia solanacearum species complex", p. 449-462. In C. Allen, P. Prior and C. Hayward (ed.), Bacterial Wilt: the Disease and the Ralstonia solanacearum species complex. APS Press, St. Paul, MN, USA.
Fegan, M., and Prior, P. 2006. Diverse members of the Ralstonia solanacearum species complex cause bacterial wilts of banana. Australasian Plant Pathology 35, 2: 93-101.
Gabriel DW, Allen C, Schell M, Denny TP, Greenberg JT, Duan YP, Flores-Cruz Z, Huang Q, Clifford JM, Presting G, González ET, Reddy J, Elphinstone J, Swanson J, Yao J, Mulholland V, Liu L, Farmerie W, Patnaikuni M, Balogh B, Norman D, Alvarez A, Castillo JA, Jones J, Saddler G, Walunas T, Zhukov A, Mikhailova N. (2006) Identification of open reading frames unique to a select agent: Ralstonia solanacearum race 3 biovar 2. 1: Mol Plant Microbe Interact. 2006 Jan;19(1):69-79.
Guidot A, Prior P, Schoenfeld J, Carrere S, Genin S, Boucher C (2007) Genomic structure and phylogeny of the plant pathogen Ralstonia solanacearum inferred from gene distribution analysis. J Bacteriol. 189:377-87.
Occhialini A, Cunnac S, Reymond N, Genin S, Boucher C (2005) Genome-wide analysis of gene expression in Ralstonia solanacearum reveals that the hrpB gene acts as a regulatory switch controlling multiple virulence pathways. Mol Plant Microbe Interact. 18:938-49.
Prior P., Fegan M., 2005. Recent development in the phylogeny and classification of Ralstonia solanacearum. Acta Horticulturae. (ISHS) 695:127-136.
Reymond N, Charles H, Duret L, Calevro F, Beslon G, Fayard JM.(2004) ROSO: optimizing oligonucleotide probes for microarrays. Bioinformatics. 20:271-3
Salanoubat, M., S. Genin, F. Artiguenave, J. Gouzy, S. Mangenot, M. Arlat, A. Billault, P. Brottier, J. C. Camus, L. Cattolico, M. Chandler, N. Choisne, C. Claudel-Renard, S. Cunnac, N. Demange, C. Gaspin, M. Lavie, A. Moisan, C. Robert, W. Saurin, T. Schiex, P. Siguier, P. Thébault, M. Whalen, P. Wincker, M. Levy, J. Weissenbach, and C. A. Boucher (2002) Genome sequence of the plant pathogen Ralstonia solanacearum. Nature 415:497-502.
Wicker E., Grassart L., Coranson-Beaudu R., Mian D., Guilbaud C., Fegan M. and Prior P. (2007). Ralstonia solanacearum strains in Martinique (French West Indies) exhibiting a new pathogenic potential. Applied and Environmental Microbiology. In press

## Claims

1. A method for the detection of *Ralstonia solanacearum* race 3 biovar 2 strains in a medium, comprising the determination of the presence or the absence in a sample of the medium, of:
(i) at least one first nucleic acid target having a sequence selected from the group constituted of SEQ ID NO: 1-49, complementary sequences thereof, and homologous sequences thereof, or
(ii) at least one fragment of said first nucleic acid target, wherein said fragment is not constituted of or comprised in a sequence selected from the group constituted of SEQ ID NO: 111-140;
whereby, if said first nucleic acid target or fragment thereof is present in the sample, it is determined that *Ralstonia solanacearum* race 3 biovar 2 strain is present in the medium.

2. The method according to claim 1, wherein the medium is selected from the group constituted of a potato tissue, a tomato tissue, and a geranium tissue.

3. The method according to claim 1 or 2, wherein the medium is a potato tissue.

4. The method according to claim 3, wherein the potato tissue is the tuber.

5. The method according to any of claims 1 to 4, wherein the sample is obtained from the medium by a nucleic acid extraction.

6. The method according to any of claims 1 to 5, wherein the determination comprises at least one step of hybridization of the nucleic acid target or fragment thereof with a probe or a primer.

7. The method according to claim 6, wherein the probe or primer is a fragment of nucleic acid having a sequence selected from the group constituted of SEQ ID NO: 1-49, homologous sequences thereof, and complementary sequences thereof.

8. The method according to claim 6 or 7, wherein the probe or primer is selected from the group constituted of SEQ ID NO: 141-386.

9. The method according to any of claims 1 to 8, wherein the determination comprises at least one step of nucleic acid amplification.

10. The method according to any of claims 6 to 9, wherein the determination is implemented by a method selected from PCR and NASBA.

11. The method according to any of claims 6 to 8, wherein the determination is implemented by a method selected from Southern blotting, Northern blotting, dot blots, and nucleic acid micro or macro-array hybridization.

12. The method according to any of claims 1 to 11, comprising the determination of the presence or the absence in a sample of the medium, of:
(i) at least one second nucleic acid target having a sequence selected from the group constituted of SEQ ID NO: 111-140, complementary sequences thereof, and homologous sequences thereof, or
(ii) at least one fragment of said second target nucleic acid.

13. A nucleic acid having a sequence selected from the group constituted of SEQ ID NO: 1-22, SEQ ID NO: 50-110, SEQ ID NO: 111-140, SEQ ID NO: 141-246, and SEQ ID NO: 247-386 and their complementary sequences.

14. A nucleic acid micro or macro-array comprising a plurality of nucleic acid probes arranged onto a solid support, wherein the nucleic acid probes are fragments of nucleic acids having sequences selected from the group constituted of SEQ ID NO: 1-49, complementary sequences thereof, and homologous sequences thereof, provided that at least one of the nucleic acid probes is not a fragment of a nucleic acid having a sequence selected from the group consisting of SEQ ID NO: 111-140.

15. The nucleic acid micro or macro-array according to claim 14, wherein the nucleic acid probes comprise SEQ ID NO: 247-386.

16. A kit intended for the detection of *Ralstonia solanacearum* race 3 biovar 2 in a medium, comprising at least:
- two primers suitable to amplify a portion of a nucleic acid having a sequence selected from the group constituted of SEQ ID NO: 1-49 and complementary sequences thereof, provided that said portion is not comprised in a nucleic acid having a sequence selected from the group consisting of SEQ ID NO: 111-140 and complementary sequences thereof;
- optionally one detectable nucleic acid probe suitable to hybridize to said amplified portion.

17. The use of:
(i) at least one first nucleic acid target having a sequence selected from the group constituted of SEQ ID NO: 1-49, complementary sequences thereof, and homologous sequences thereof, or
(ii) at least one fragment of said first target nucleic acid, wherein said fragment is not constituted of or comprised in a sequence selected from the group constituted of SEQ ID NO: 111-140;
for detecting *Ralstonia solanacearum* race 3 biovar 2 strains.


SEQUENCE LISTING

<110> INSTITUT NATIONAL DE LA RECHERCHE AGRONOMIQUE (INRA)

<120> Method for detecting Ralstonia solanacearum race 3 biovar 2

<130> BET07P0857

<160> 444

<170> PatentIn version 3.4

<210> 1
<211> 5600
<212> DNA
<213> Ralstonia solanacearum

<400> 1
tcagaacttc ccatcaaccc ggatgctgct ggcgatttcc tttgccagct ggtgcccagc 60

ctccacaggc cggtcaaaga ctgaaacgct gagcaggtac atgccgccca tcgttgggat 120

atagaacagg tcttgagcac ccggctggtt tcgcaaatgg acagtcgcga ggttgtgcag 180

cgtttgtcgc cccgagatat cgactgtctc gtaaggcgtc cattcgatga tgacgctgcg 240

ctttgcgggc agccccgttg agcgctcgac aaactcttgc aactgacgag caaggcggtt 300

tttcgtttgg tccggcattg cggagaacgt atctggtttg aagccctctt ggacaacaaa 360

cgacagagac gcaacagcgg tatcgtcacc tggtcccgag acggccaaca gcgtgcggct 420

tcgcctggtg tgagcggcat cagcctgtgc tctatccttt tcgaattcag ccgtgtctac 480

gcgccaggcc gtaggcacgg acagtgacac accgtcaacg cgctgtgtag acatctcgtc 540

ggcgacagcg tgagcagcga atccaatgct cgcgaaaaca gcagcaagaa gggcaagaat 600

gcgcatgggg tgacccggat tttgaatcga gtgaagcata gcgcaatgcc ctcaagcttt 660

cgtttgtgaa cagatcgtag tggcaacatc ggacagctgc tcttgctgga ggtgtgccta 720

caacccgcat ctggtcgata tcagttctag ccaaggtctg gtcaaccggt gaatagctta 780

aaccgccgga tatcagcgcc tgagttgacg ttgtatcggt ggttgctgaa gtgtcctggc 840

aattggtgct cactactgct ccagcgaaac gacgtcgcga atcgattcga actcgcgcac 900

gaggtcaggc tggtgttggt gtagataggc ttgcaccggc tcacactcga gaagcctcac 960

gaggtaccct ttagcgagca caagattgag tacgtcttgg ccgtaggtct gttccacgat 1020

tttgtactgc tcctgcaggt tgctcatctc ccgctccatc ttcgccatct gctcgtgcgt 1080

cacgcctgtt agcttggccg gcttctttcc atcaacaaga cgagaggcag gcgtggcaac 1140

aagcaacgct tctgcatagg cgacggtgac attgttcgcg gcgatcatca attccacgca 1200

ctcgacctgt cgcgttggcc gcatcttgcg aatcgcgcgg gctacctctg gcgaaaactg 1260

ccggtctctc aacaggtcga cggcctcagg gcaaatgcct tccaacaggt tcaatttttt 1320

cacgatctgg gagatgtcta cggacagcac cttggcaagg cgttccggtg aaacgccacg 1380

ctcaatggca cgccggatca tgaagtgctc ctggacgctc gacaggcggt tgatgcggtt 1440

gttgtaggtg tatgcctcat cgtcagtagc gacaagacag gccgcctctt catgcccaag 1500

gtcgtgcagc accagcagcc gtatatgccc atccagcaac gtgtggtttc ccgactgctg 1560

atcaaccgcg gtcacggaaa gtggctcgat caggcccacc tcttcgatcg aggagcggat 1620

ctgcttgaac tttctggtgc ccgtgatgcc ggagggagtc ctgcgagagg ggagaatgca 1680

cgtcagagga acagcaagtg gctcaggcac aaagcccaga gtaacgcccg tcatggcgaa 1740

ctcccagtgg accaaacacg ctcagccaga tatttgggaa gtgtcgccaa agattccgct 1800

cgcagaaggt tgacgaaatt ctcgtcggca aatagctgcc gcaacgctcc gacgacgaac 1860

agcaaccgtt gctgagtgaa ctccgccttg cggaccatgg ccttctggcg ctccacctct 1920

ccctcatacg tacgcaccaa gcttgacgtg gtcacgtctg cgcgactccc ggtcatatag 1980

cgggaggcag atctccctaa ggtatgtcgc cgctcgatga gccgtctcgc gcttatcagc 2040

tgcttgccgc gcagcttccc cgactcatat gcctcctgga gggcggcttg gatagccttg 2100

tcgtcatcgc cagccccaac gatcgcgagc gccgccgtca gcggaatcgt gcccttttcg 2160

acggcaatca gcagacgctc ctccccatga tggagcaagg tcagaacacc ctgcacatag 2220

gcggcggtca agcccgtctt ttctgcgatg atcttcggcg agtacccgct atcacgcaac 2280

tggcggatgc cggctaacag ctccagagga cggcattgcc ggcgcgcgat gttctcggca 2340

aggctcatga tgaacgcatc ctcgtcgctg acgtgaacca ctattgccgg tatcgtggtc 2400

tcgcccagcg agcggaatgc cttgagccgt ccttcgccgc agacgagaag aaacttctca 2460

ctgccatccg ctgcggccct cggcgtgacg gtgattggct tctttagccc aatggtcttg 2520

atgtttccaa cgatctcgtc aaagaccttg ctgttccgct cacgtgggtt gatcacgtcg 2580

atacgatcga tcggaatcat ccggatctca ccaggttgta tccgcgtatt catgctgccc 2640

ttctcagctt tgcacgctcg gccatgtgat acaggtagtc cagcgtgtcg aagcgatagc 2700

tctcatactc gatggcattg cgctcggcca agcgaatgcc agggcgtgcg aagtcgagac 2760

gcgggagcag gtagtagtcc aaaggcgcgt cgttcgcctt gttcagacga atggccacag 2820

tgacgtccgg caagaggctt gtatcgaacc gaaccttcca gtgatacccg ccgttcggtc 2880

gcggctgaca gcgcgacaag acaagcgaca cggtgaactc ctgattaacg tccaggatgt 2940

ccgtggccgg gtctcgaaga accgtaccgc cgagatcggc aatagcttgc tccgcttgcg 3000

acacgatctt cggatgcagt tgccgcagga atcgattgat ctccaggtaa cgatagtccc 3060

gatccggcgt aaagcccacc gcctggtagg cgcgtatcag gctgccgaag cgataggcgt 3120

acgccgcaga ggagggcatc ccctcggtct cgtcgatgat gaggccggac agaaagccgc 3180

gattccgata gaggttgcgt agccgctcga taagctcttc actgctgtac cgacgcgcgc 3240

gagcccggat gatgccctga gcggtgtaga acatgtccgg cgctacgatg ggctcgaagg 3300

caccatcctt gcggatccac atttcaggtg cgttcacgac gcgagcccgc gcgagcttgt 3360

acgacacacg gttatagatg ttgttgccga tgtatttctc gttggtgagc acctccctca 3420

cggttgcgcg agtccagtcc cggtccaagt ccgtgcgcgc atgcatgctg ttcagtctcg 3480

cggcgatttc gaactcgttt agggactcat cgataaacca gctgtagacc agattgacaa 3540

tacgcacctc gctctcggga cccggcacca ggacaacgcg gtcggtctgc aggctcttgc 3600

gctggccgcg gcagagctcg gctttcacga ggccatgctc gtcgaccagc atgcgacgca 3660

ggccgtagcc ggcgggaccg ccttgccgga accccagttc gatcagccgg cactggcctg 3720

cgaagacctt cgctgagagc tcgcggctgt attcaccggc cattgcgcgc ttgacgcctt 3780

tgacgatcgt tgacaccggc gagccatcgt tgtcgaactg ctcggcacag tagatgacct 3840

ggatgccggc acggcggcag atgtactcgt agtacgcgct ctcgtcggca tcctggaagc 3900

gcccccagcg gctgacgtca tagaccagga tcgcctggaa gtccgcgcga ccgttggtca 3960

catctcgaat gaggctttgc agtgcctctc gcccgtcgat ccgcaggcca ctcttgccct 4020

catcggcata ggtgcggacg acttcgatgc ctcgtgacgc tgcgtactcg cggatcttgt 4080

ctcgttggtt ctgggtcgag tactgctggt gctcagtcga catgcgtacg tattccgccg 4140

cccgaagcgc agaaggcgcg atcggcgtag cgcaggtgta ctgatcgatt gccatggtgg 4200

tcaccggcca acgtgtggcg ccgctctccc gcgtcaggag gtgccgttct gctccccggg 4260

gcagcacaca ggatcacgtt agcaggtggc cgcgcaccgt gcctcacatc atctctttgc 4320

aatcagccct ggatggtgcg cgcaccggcg gcagcattac gatctgcacc ctccagacgt 4380

ccatctttgc aaccccgtca tcggacgcgt gctggagcat gtaagtgcct gaaggcagag 4440

gcgttttcgg agctgacgcg tacatctttg caatcgtcct tctccttagg ttcctggcac 4500

gctgtagatc tcggttgcgc gccgtatagg ccgggtgagc ctcgcgatac cgtcgccagt 4560

agtcgctatg gcactgactc catgcagcgc gggaccgcgc ctggttgtcg cgatagtcgt 4620

catcagagcg caagtgctgc tgttgccacc gtcggcgacg ttggcgctgg cactcggcag 4680

cggagcaata gcgttggcct ggagtttgtg ggcggggaag aaagagggtg ccgcatgctg 4740

cgcaccgccg ccgaatttga ttcatcgcga gctccgggca aggtcactgc gcggggccag 4800

caagtagatg tggcgttcgg ggcattcaac ccgaaatgga cagctcgcac atgagtcgag 4860

cactcttcca ccgggcgcga gccgctacgg attgcaaatg atgtatgcac atacatcagg 4920

aaaacagtgc ccgacgcgct cgattttgcc acggctcaag ggttgctcta ccatgggcca 4980

aaaagtagga gctcgacggc cgctcgtctt gcaagtgatt gatttttaag gtatcttttg 5040

gccacttgag gtggcgtcaa gattttgcac ttttcttggc acgtgtgccg agtcttttca 5100

gtaattgcgg caagggacgc gcgactcagt gtctcaattt atgaaaaaac gcttttaaat 5160

catagtgcga tttttcagga attgtgacac tcaacaggtt gttgcagacc ccagaaaagt 5220

tgatgtaatt cacaaataag tgaatcgatc caaggcggcc gcattgccgt ggcgcaccag 5280

atctacccat taacatttcg tgcagcccca caaaaagacc gggcctctca agaggtccgg 5340

tcttttttta ggtgtcggta gccacctcgc cagtaggaat cgctctgaca gagaaaggtg 5400

atgtcggtcg agctgcgcgc aatcgctaca ggggtatagc tgggacttgc cagtcaaccc 5460

cgctcacgag aaagcgtcct ccgaaattct agcaatgttt gcgggcccga cggtaacacg 5520

ccagaaacta gggaaccgct gatcaatgaa gtcggcgaat ggaatcgcga gtcatgccat 5580

cttggttttt cggattgagg 5600

<210> 2
<211> 5306
<212> DNA
<213> Ralstonia solanacearum

<220>
<221> misc_feature
<222> (3202)..(3728)
<223> n is a, c, g, or t

<400> 2
agggggctgg tggagtcgct acatgcttga agttactaaa attgtggatt ttttgacggc 60

acaaccctag acccagcttg gctctgactg acgatgacaa aaaaatctga gttatggatt 120

tttgggacaa atcaggactg agccggctgg tccggtccat gggtaggggc gtactttgag 180

cggtcagagc ggccgtacgt ggcaggcctg tttcccgcgt gcgttgcgtc gaggccgcgt 240

gaatggcgta ctggggcgcc cgctaccggg ccgaattgct ctgccaccgt tcccgatgac 300

gggatgttgt ggtgtgcgtc agtcagcatg attctcgttg gtagaatatg agcaataatc 360

acaagaaacc aaccgaagtc gtttgactgc ctccgctgcg cggggccaat ggggcgagat 420

acgcataatg acggcagaac gtgccactcc ttcgcgaatt gggactttcc ggttctgata 480

atggcaattt cgctcgcttt cgagaatgag caaattcgcc agacctgcga aagtagccgt 540

tctgctaagc gccgccttgg tgtaacggta gcggctcaac tccaggtgcg gctcgctgac 600

ctcaaggcct tggatgccgc gagtgacgta attgacatgg gctttgctcg gctcgacccg 660

tccgaagata cccggcttgt cattccgctg ggctgtgagt atgttctgag ggtagccgcg 720

catcatcgtc ccgaaccacg ttcgtcaact ggtaagttgg actggaagaa cgtgactcgc 780

atcaaaattc tggctatcga gtgcaccggt gaaagctgac catttgtttt ctcccacttg 840

ggctgtgccg ccagggcgga ccgtagttga cttgctcgct gcgaggggtt ggtcgcggga 900

tgagctggcc aactccattg agctggtgcg agaagatctc gatgacctga ttgacggaac 960

cgcacagcta tcggaaagcg ttgcttcccg gttggggggc gtctttggta cgagcgccgc 1020

cttttggatt cgtcgagagg agcagtaccg cgagcagctc gcgagtttgc atgtcaaagc 1080

tgatgtggcg gatgcgggct acaacgagtg gctgaaggca ctgcctcttg cggacatggc 1140

tcgctttggc tggttcgctc caggcgcgac ctccgctgac aaactggcgg cgtgtcttga 1200

tttctttgat gtcccgaact tggacgtgtg gagtcgggac tacgacgatg tgaagcgtgc 1260

ggttgccttc cgggcatcag ccgcccaccc agtgaatgta cctgcgacca ccgcgtggct 1320

tcgacaaggc gagcgtgaaa gtgagcgtat tgagtgctca gcgtgggacc cggccaagtt 1380

ctcatcggcg ctgaccaaag cgcgtgcgct aacccgtacc aaggacccgc aggtttttgt 1440

gccagctcta caagcgctat gcgctacgtc gggcgttgca gtggtaatcg tgcgagcacc 1500

gtccggttgc agagccagtg gtgcgacgtt cttctcggct ccagataagg cggtcctgct 1560

actgtcgttc agatatctca cagacgacca gttttggttt tccttttttc atgaggctgg 1620

tcatctactg cttcatacgg acagggatag tctcatcctg gagacgtcgg agcgaacgac 1680

gtccaaaatg gaagctgagg cgaacgagtt cgctgcgaaa actcttttgc cagtcgagct 1740

caaccgccgg ctacctgagg tggctggcga cgttcgagcg atacttcgtc ttgctcgaga 1800

ggccggcatt gccccaggca tcgtcgtcgg ccaactgcag catcgcggtt tgatcaaaca 1860

ccagcacctc aacaagctca aggttcgcta tagctggtag tagttctagc cacggaaagc 1920

cacgaacttg tttgggctct tctgccagtt gcatagcgag tcttccagca cttgcattcc 1980

aacgttcaga tggttatcaa gcacaaccag gagttggtca gcttgcgcgc acgagatgtg 2040

cattgcctcg gtcgttccgg cgaatagcaa gcgggcaccc gacagcggtc cagtagcacc 2100

aaccatgtaa gccgaaggtg cacgaaggct cgctaggccg agctttgcga ccatcgtgag 2160

gtagtcgaac ttggcagttc tgccgaaccg gtggacgact ttcatgctct tgtatagata 2220

cccaaacagc gcccttgggt tgtcgccgac ggctgccgtg gcacgttcga gcattgccgc 2280

atgcgaacgt tcggggccta cccattccgc gtagctttca agcaccacat ccactttctc 2340

gatactttcg tacttgcggt ggttgccgaa ttggagctgc tttcgatttt ccagcaactc 2400

cggaaggcgg tcgttgaacc agattgtgaa aatattcggg gcgctgttga actgctccca 2460

cgtccaaata aactggtcat tccatgcgcc atagatcgcg cggataaggt tccatctggt 2520

tcgcaggctt ttcccgaaat ggacgaagag gaacgcgagc cagaacgcct catcaagatt 2580

ttcctgctgc cggtagagag tcgcagcccg caaggggtcg aacaagtctg acttaggatc 2640
ccgtctctgt gggcagatgc cgcgccgtcc gatagtcgtt acgaagtcga cgcggcggat 2700
actctcgaca agctgctcaa cataggcaat gcgattgtcg tcgttcgcga tgcccggcag 2760
cggcttgttg tccgccttaa actcaagcag ttttgcttcg aggtcagctg ccaaggcttg 2820
attcctaaga gtcgctaaca caagtcatcc acgaatcgag cgcagatgat ggcggcagca 2880
agcgagagga gcgcgcaatg gatatcgagc cgtcgttcga aacgaacgcg tatctttcca 2940
aagccggcaa accaagcgtg cgtgcgctca accacccacc ggtaacggcc caggcgctcc 3000
ctgctctcga tgcccttgcg ggcgatccgg gcccttatgc cgtgccgctt aagatactgc 3060
cgacatcggc ggcagtcgta agccttgtcg gcatgcagct tgtcggggcg cttacgcggc 3120
cggccatcca ggccacgtat cgcgggaatc gcatccaagg tggattcgaa tgcgatcgag 3180
tcatgtcgat tcgcccctgt tnnnnnnnnn nnnnnnnnnn nnnnnnnnnn nnnnnnnnnn 3240
nnnnnnnnnn nnnnnnnnnn nnnnnnnnnn nnnnnnnnnn nnnnnnnnnn nnnnnnnnnn 3300
nnnnnnnnnn nnnnnnnnnn nnnnnnnnnn nnnnnnnnnn nnnnnnnnnn nnnnnnnnnn 3360
nnnnnnnnnn nnnnnnnnnn nnnnnnnnnn nnnnnnnnnn nnnnnnnnnn nnnnnnnnnn 3420
nnnnnnnnnn nnnnnnnnnn nnnnnnnnnn nnnnnnnnnn nnnnnnnnnn nnnnnnnnnn 3480
nnnnnnnnnn nnnnnnnnnn nnnnnnnnnn nnnnnnnnnn nnnnnnnnnn nnnnnnnnnn 3540
nnnnnnnnnn nnnnnnnnnn nnnnnnnnnn nnnnnnnnnn nnnnnnnnnn nnnnnnnnnn 3600
nnnnnnnnnn nnnnnnnnnn nnnnnnnnnn nnnnnnnnnn nnnnnnnnnn nnnnnnnnnn 3660
nnnnnnnnnn nnnnnnnnnn nnnnnnnnnn nnnnnnnnnn nnnnnnnnnn nnnnnnnnnn 3720
nnnnnnnnca ccgctaagtc tcatatcttc atccttctga ttggctaaac caacaggggc 3780
gtggctattc gtcggcgggg gtgtccaatg aggccattcc ggcatccagt tcagcaggcg 3840
tgaacagcag accaataggc tcggcgagct ccggcgcagg ctggctcttg cgaacgcgtt 3900
gctgtatgca cgcgtcatag ttcgccgtgc tcatcttctt ggctaacgca cgaattgacc 3960
gcgaactgtt aggagtctta tttttcagat gccgctgaat aagcaacaag cggacgaggt 4020
ctgcccagta gggttcgaac tggttgacgt tcacggcgtg ccccaagcga atttcccgtt 4080
ccgcatctac cagcgcgtta agggccggct gagggtcacc gaccggcatc gatggcatgg 4140
cgatgcgctc ctgcaagccc tcgtcgacat actttgtcgc ctggtccgtg ttcttcgagt 4200
acaaatgtag gcttccgacg gcgtgcttgt atgtgcctag ttccgcgccg acggcccgtg 4260
caactatctc ctgcagcatc gtgaacgcga atatgtcatg aggcaggccg agataggcgt 4320
cgttcgaccg catcgtcacg aacatgtgga gctgacgcgc tctgagcgag aactgcatgg 4380
tgcacgtgca ggggatatct tttctgccta ccgccacatc gcaaccaagg tcttcggcgt 4440

tgaacagttg gatgaccgca cggcgtgtat cgttgttgcc gctcaacttc tcgatgacgt 4500

tgctgacttg gttgacacca cgcatgttga acaagcgagg gccgtatgcc ccgtgaacgg 4560

tgtttccgtc gtccgagaac tcgtcgtacc ggctgatgta gtactggatg aagctgagct 4620

cgttgctgcc ggccaagtac cacaggaact caccaaggca gctgaacaat gtactgcgct 4680

gttcagtgcg gctcacacgg gcacgtgggt tcctgatttg gagcaagacc ccgtgcaggt 4740

cactgcattc gccttttgat ggcgtgatgg acgatttccc gcgcaaaaga cgtctgtaga 4800

ccttgagcaa taggtcatca agcgtttctg cagtcatgaa catgctgggc accccaattc 4860

atgtcacgcg ctccctctgt ggctcagcgt tcgctggcct ccaaagcgag gtcaatcaaa 4920

gagagccagt tgcgctgtct cgcgggcttg ctcgctgagt agctcttgct tcagtcgccg 4980

gccgctcgtt atcaatgtgc gtcggtctac caactttgct cccttgctca gtgcaatttc 5040

ccgagtttcg agggtgatgt cgtagtgggg gagggacgca ttggcttgga accatccgcg 5100

gtgcaatccg attgagcgag cgaatgagtg gagctcgtcg aggttgtcag ctactagatg 5160

gcaccacttg cgcccgcgcc agggaattcg ggcgttgtcg gtatacacag ccatcgtcag 5220

ccccaaaata attctgattt tactgattct gcattcttgc gagtgctgac caagcatagg 5280

ccagcacaat gcattctaga gcgtgt 5306

<210> 3
<211> 18073
<212> DNA
<213> Ralstonia solanacearum


<220>
<221> misc_feature
<222> (9530)..(9679)
<223> n is a, c, g, or t

<220>
<221> misc_feature
<222> (10886)..(11062)
<223> n is a, c, g, or t

<220>
<221> misc_feature
<222> (13381)..(14304)
<223> n is a, c, g, or t

<220>
<221> misc_feature
<222> (16201)..(16358)
<223> n is a, c, g, or t

<220>
<221> misc_feature
<222> (17695)..(18042)

<223>  n is a, c, g, or t

<400>  3
atttgttcgc acaagagcca tctgcaccac tcgctgaagc ccttcgcccc aagaccttgg      60

acgaggtcat tgggcagtcc catctgctgg gtgacggcaa gcctttgagg ctggccttcc     120

agtcaggcaa gccccactcc atgatcttct gggggccgcc cggtgttggt aaaaccacct     180

tggcacggct cacagccacg gccttcaagt gtgagttcat agccctctct gcggtgtttt     240

caggtgtcaa agacatccgt gctgctatgg agcaggcaca gcagaatcta tctatgggca     300

agagcaccat cttgttcgta gacgaaattc acaggttcaa taagtcccaa caagatgctc     360

ttcttcctta tgcagaaagt ggcctcgtaa cctttattgg tgccaccact gagaaccctt     420

cttttgaggt gaactcggct ctgctgtcac gtgctcaggt ctatgtgctg aagtcactga     480

cagaagaaga actgaagctg ctcttgaagc gagttcaaga agaagatgct cttggtgact     540

tggagtttga agataaagca gtagatacca tcattggcta tgcagacggt gatgcaagaa     600

ggttcttgaa cctgcttgag caatgcaaaa cggctgctgg tgctgctggt gttcagcatg     660

ttaatagtga cttcattcag aatgccctaa ctctcaatag caggcgtttt gacaagggtg     720

gagacaactt ctatgaccag atttcagcct tgcacaagtc tgttcgtggc tccaaccctg     780

atgctgccct ttattggctg acacgcatgt tggacggtgg tgctgatcca cgctatctat     840

ccagaagaat cgttcgcatg gcttgggaag acattggctt ggctgatcca cgagccatgc     900

agatagccaa cgatgcagct ttaacctatg agcggctggg tagtccagaa ggtgagctgg     960

ccctgggtca agcggtcatc tatctggctg tagctgccaa gagcaatgcc ggttacaacg    1020

cctacaacca agctagagcc ttcgtgaagc aggataagag ccgtgaggtt cctgttcatc    1080

tgcgtaatgc accaacaaag ctcatgaagg aactcggcta cgggcatgaa taccgttatg    1140

ctcatgacga acccaatgcc tatgctgctg gtgaaaccta ccttcctgaa ggcatagaag    1200

agccgggttg gtatcagcct gttcctagag ggttggagat caagattggt gagaagctgt    1260

ccctgctgaa gaagtgggat gaagaagcca aaaagaactg aataagggga tcagatggag    1320

caaagaacac ttctctacga tgagcggttc cttgaaagct atgctggagc catcatcact    1380

gatcctgcta cggcgattgt ggagcttgtc gccaactgct gggacgctta tgccacagaa    1440

gtgaagatca catggcccga tgttcaactt gaaaagcagt tcaagattgc ggacaacggt    1500

catggcatga cccgtgaaga gtttcaatac atctggcgga ccatagccta caaccggttg    1560

tcgtcaggtg gaaacacaac ggctccccca gcagatgttc aaggcttgcc acggcttgtt    1620

tttggcaaga acggtaaagg ccgttttgcc agcttttgct tcgcttctga atacttgatt    1680

acttcaagga agaacgggca agagttcgtc tgccgtgttc acagaacctc aactgatccg    1740

ctcgttctag aagaaatatc gttcaaggaa aagggtgttg agggtcatgg aacggagatt 1800
gtgggtaacg gcatcattcc ccggctcatg ttctcggaag agaaagctcg tgaattgatc 1860
ggtagccgtt tccttgccaa ccctgctttc aaggtgaagt tgaacggtag tgaaatcacg 1920
ttcaatgaca tccctgactt gttgtccaag agcgaagtgg atgttgaagg gtacggcaag 1980
gcagttattc ttcacattga caccaagaag gctgacaaga caacgaagca acatggactg 2040
gcatggtggg ttcaaggtcg tgctgtcggt gaatgcaagt ggagtcgtag tgactacgaa 2100
agaattcttg acggccgaac ttcagaggcg aagcgattca cattcattgt tgaggctgac 2160
tatctaaatg cccaagatgc agtgctgggt gattggagtg gcttcaagga cgaaaaccag 2220
gcgtgggtga agacccgcga ggccgttcaa gatcgcatcc gccaaatcat ttttgattcc 2280
agcaaggcag agcgtgaaag ccgccgcagt gcagtcattg aaaagattgg cacaacgatg 2340
aacaccctgt cccctgttag caaggaccgg gtggaaagct tcgtcaatga ggtggtagat 2400
acctgcccga actttggtga aaacgagatc gtccagttgt ccagcatcct tgcaaagctg 2460
gagcagtcca agtctcgcta tgggttgctt gatctgctgc acaagtgcga gccaaacgac 2520
tacgacaccc ttcatgacat cctgaaggaa tggaccatag ggatggcaaa gctggttttg 2580
gacgaaattc agaaccgctt gaaattgatt ggggagcttc gcaccaaact gaaggaagtt 2640
ggcgttgatg aagtccatga gttgcagccg ttgtttgagc gtggcctgtg gatgttcggg 2700
gctcaatttg aatccattga gttcacgtcc aacaagggca tgaccaatgt aatcaagacc 2760
atcttcaagg atgaggatgg taagggcact cgtaaccgtc ctgacttcgt agccctgcct 2820
gacagcagtg ttggcttcta tgctcgtgct tcatataacg aagattacga tgaagacggt 2880
gttgaacatc tagttattat tgacttgaaa accactggtc ttgctctcgg tagcaaggag 2940
aaagatcaag tctggaagta tgtgaaggag ctacgagaaa agggctacct gaagaagcat 3000
acccgtgttg atggatttgt cttgggagac aagattgaat cgggtgagaa tgaccctaca 3060
acgcatggtg aagaagtcaa aatttcaccc atgctgtatg acaccatcct cattagggct 3120
gagaagcgcc ttctgaacct gcacggtaag gtcaaggatg ctccgttcct gatagagcag 3180
caggaagtcc tgaagaagtt tttggagcct attgaagtca ttcaggcgga attggtggaa 3240
gctgacccag tggtggcgaa atgacctcgt ccgtgaattg gatcgcagcc tacaactacc 3300
tgtttgcttc tttcaacagt gagaacaagg atctctatgt tggcggttct gtattttgtc 3360
gcatggtgca gcaggtcgat cctggctcac caagctacca acagttgctg ccgctgagac 3420
aaagccaagg aaagtccaat tcgcgcaagg acttttattg ggaccttatc caaggtcttc 3480
cagaggccca gcgatttcaa ctctatcgtg tcttcatcaa ccacattgaa gttcacgaca 3540
agccagcggc agacaacatc aggaacattg tgttcggcgg tggttatgcg gtgcccacta 3600

cggttgttcc cgttgacctc tggaactctg agaagctgaa caacagtctc aatgacattg 3660
accatgccat tgatgctcac cattacaacc gggcaacgac actgagctac acgtgtcttg 3720
aagggctcta caagacatac gttcgcaagc acgttcctgg acaaatggcg ctgacggatc 3780
tcatgccatt gtgcaaggtg gtgaaggagg acatcagcaa gaagcttcag gcacaaggcc 3840
cgtttcctgt cgagattgtc aatgccatgc cgaccttgac caatgccatt gcgaactcaa 3900
gaaacggctt cagtgaatct cactttggtg acgactccca gcggtggctt gctttgtttg 3960
ctcgtgatct gaccaactca atcggtcgtc tcatgctcaa cttcatgtga ccaccggcga 4020
gaggaagatg actacttcta gaacatgcgc actttgccca aacatactgt cagccaaaaa 4080
taaaacaaaa gagcacatca tccccaatgc gattggcggc aggaagaaga ccactgggtt 4140
tatctgcaac gattgcaata acaagttggg tcagagctgg gatgcggagc ttgcaaggca 4200
acttaactgg ttttccttgg cagtcggcat cagtagagag cgaggtgagc cacctaaaca 4260
ggttgtgcaa acaatcgatg gggacaggta ttggcttctc agcgatggct ctttcacgcc 4320
agaaaaatca tcttattcag aaaaggatct gggtggggca atcgagatca acctgaccgc 4380
caagacggtt gaagaggcaa agcagcggat tcaaggtatc gccagaaaat acccgaagtt 4440
tgacgttgaa aaagctctca atgagttgga gataaaaacc agctatttgg attcgcctct 4500
tcatgtcagt ctttctttgg gtgggccaga ggctggaagg tctgtagtca aaactgcgtt 4560
ttcttttgca tctgaatgtg gtgttccaca tgctcaatgc gagaaggctc ttgaatatct 4620
tctagatcct actgctgaaa atttcccctt tggatttgcc tacttgtctg acctggttga 4680
aaaccgacca aaggacaaga tttttcattg tgtgtctttg catggtgatc caaagaagaa 4740
gcggctatgg tcttacattg aatacttcgg aatcttcagg cttgttgttc tcctaagtga 4800
tgtgtatgtg ggtccctaca agaatgaggt ttatgccatc aatccagttg atggagaacg 4860
gcttgccgtg aaggtgacta cccagatcag cgaagaagaa tttacttcgt tgatgagggg 4920
cgaaggtttc gaccaagaaa agcatagagc agcggcggac tatgccctgc ctatcatcct 4980
ggatagaggg cgaagccgaa ccttggagcg cacagtcaag gaaggctttg aacatgctgc 5040
caagcagctt ggaatcagtg aaggcgaaat cattcccaag gaaaaggcca ctgagttcac 5100
tgcctttatg atggagaaaa tctctccata catagagcat ctagttcgta gtgggcacag 5160
aaatgaccaa ccataagcca gcaaacggca ccaagaagct cctgcttatt gaccttgaga 5220
acaagcacaa ggtagacctg tctcccttgg atgagagtta cagagctatc atttttgtgg 5280
gtgccaacca gaacccgcca aaagcttcca agaagccagc aacagcccat aggttcaaga 5340
gggtggactt cctgaagatt tcaggtgctg gcaagaatgc cttggacttc cacatagcct 5400

ttgaactcgg caggaccttc gagactgcac ctgataccca atgcttcgtg ctcagtggtg 5460

acaaaggctt tgacccgctg ctgaaccact tgaatagcaa cggtatgagt tgccgaaggg 5520

tggaaagcat tgatgagctt gtgccagcta cggtggtgcc tgtagcagtt ccatctgatg 5580

tggttgtctg ccctcactgt aagaaggcca gcaccattga gcatcacggc ggccactggt 5640

gttctaactg tggaaggttt gctactccac cagatcccaa gctgctgccg tccaatcaac 5700

ccggctacag gcagtctcaa agagacaggt ctgaagaaga ctatgccatc tattcggtat 5760

gtggctggtg ccatcaaaga actgatatga ctggcggtat ctatgacgat ggggaatgga 5820

tgtgtggagg ctgcattgcc aggtgcgcca gatgaaagca ccttcgggtg ctattttttt 5880

gcctcctgaa gcgtcattgc tttcttgatc gccttggctg tcgccggtcc ccggacctca 5940

caaacaagaa gaacgtcgga gcgttccagc ctcacggcat cctgcaaggg agcatggact 6000

tgaaacctgc atatcaaccc tcatgttgtg gtggtttatc gttgccagag aggaaggccg 6060

tgccaagaac cattgaacag ggtttcaacg attttcttgt aaatcttaga gccactgctg 6120

ctgagactgc cgcaaccgtg tctcatcggg catccatcaa ggcatgcctg gaaaataact 6180

ttggcttgaa acgcttcacc cgaatcggtt cgtttggcaa tggaacaaat gtgtcgggct 6240

acagcgatgt tgactacttg gcttgtctgc cgactacctc attgaaacaa tcatcaacct 6300

attctctcag gttggtgaaa gaggcattag aaaaccgctt ccccagtacg ggtgttgtgg 6360

ttcgttgtcc aacagtggtt tgcccctttg gtcaatacaa atctcagcat acagaagttg 6420

tcgttgcaga ctatgtgaaa gaacaaaatg gctacaaggt ctatgaaata gctaatggca 6480

atgatgggtg gatgcagatt tgccctgatg ctcacaatgc ttacgttgaa catgtggatg 6540

gtaagcacgg aggtaaagtc aggcagttga ttagactgat caaggcatgg aagttctatc 6600

agaatgtacc tatcaaatcg ttctatctag agatgaaggt ggcgaaatat gcgagcaatg 6660

agcaagccat catccatcac ctagatgtga aatattttct tgaccgtctc aataatgaag 6720

gtctgtccgc catgcaagac cctatgggga tcgctggcta tatctatcct tgcaacagtg 6780

acgttcagaa gattgacgca ctctcaaaac taaacacggc agctacacgg gccaacaagg 6840

ctgaagctgc tagagcattg gggaatataa aagaagcttt tgattggtgg cgactgcttt 6900

ataacgacaa attccccacc tactacctat gagtatcatt caaagacagg acctgccaga 6960

gaacattgag cttctggctg ctcaaaggaa cctctattca agggccaaga acatcattgg 7020

acttcaaatg gtgctcagtg gcccggttgc catttgtgct gcgatcacaa cgattgtgcg 7080

accagattta aaggggtatg tggcactctg gggcatcttg gctgtggtgt ttgatctctt 7140

tgtcttcacg ccttgggtca agaagctaag ggatagtgca gctagggttc aagagctttt 7200

tgacaccagg gttttggggt tagactggaa tgaaatttcc gtgggcaaaa aacctgagcc 7260

tgaactgatt catgaggaag ccaaaaaata cggagtgggt gaagaaaaaa gggccagcct 7320

aaagaattgg tatccactca tcatagacaa agttcctgaa atttttgggg taatcattag 7380

ccaaagatcc aatgtgtggt gggattgcaa gatgcgaagg aaatactcct ttttcgtaag 7440

agtagtcctt gtctccatcg cactaggatt gatcggttat ggcctctatg aaaagaagga 7500

tatgtttgaa tttttggcct acatcgtggc acctcttgcc tctacctatg tctttggcta 7560

ccggcaaatg atggagcata gtgatgccgc agataggcta gataagctga gggacttgtc 7620

tgagaaagtg tggtctgacg cggtttctgg aaaagatata ccgacgctga agattaagtg 7680

caggacattg caggaccaga tttttgatca tagaaagaga aatccaccag tctttgattt 7740

cttgtttaag tggttcaggg atggaaatga agcactgatg aacaaagggg ctgaagctct 7800

tgtggcagaa atcaagaagt aaagaagtgc ctatggaata ttttttattg gctgaattag 7860

ttagttaaat tcatatgaat ttaactaacc gtcaaccaat caagaaaaca gtacgttaga 7920

gtcatacttt ctatgaagtt gggagagagt atgaggaaaa ctttgttgtc gttggccgtg 7980

gtgttgggtg gttgtgcctc ggcacccaaa gagcctccat tggtgcttta tgggcaggtg 8040

ctggctgtac agcctcatca agtggtagag cagaaggtca acatgactgg tgcggtcgtg 8100

ggtggtgtag ctggtggggt cgtcggcaac cagtttggca aaggaaatgg cagaaaggcc 8160

atgactgccc ttggtgtggt cgctggtgct gtggtgggct cccaagtgag taagagcagt 8220

gaagtgaaga atgtcactga cttgcaagtg aggatgcctg atagcaccgt cattgatgtg 8280

actaccaatg atgttgggtt cagggtgggt cagaaggtga agatcaccca gcaaggtaag 8340

caggccacca ttcaagccat gaactgaagg caccatgctc aaaaagaaag caccctgaat 8400

gggtgctttt ttgtttatct atttaacttc ctccatctac acacacgtta taccagtatg 8460

tatagctatt ccactgactc aagcttatga cccagtaggc aagaccattc aagttgtatg 8520

tcctgctagg taatattctt tcttgggatg ctgtttgcca ctcatatctg catctccccc 8580

aaggtccttg gctttctcta gaagatatgt agccttcact tgcagataat ggaacataag 8640

tagaagtgaa tgagccacct tcaccgggta acatactatc gtagtaacga tagatggttt 8700

tgctctgaga tggtggaatt ggtggagcat aggcagcaga gcacattgct gggcttccat 8760

aattgccagc acaaccccat gtgtattgat tgttggcgaa gttgaagccg taggactgac 8820

cagtagcaca aacattagtg ggattgctgg acagagtttt gccattgtca gcaccacatg 8880

caccaacttc tctctggttt gccgtacata attgactacc taaactattg ctacaagtcc 8940

aagagtaagt tgtttctcca tttgcggtgg tagtagaaac tgcactgggt gttcctgcat 9000

cgcacaaaaa gctgttagga gcacctggca agatagatcc agcagaaggg ccgcatttag 9060

gtttaggggt ggggctggct acatatgttg gtgcaacaaa tgtgtttgct ttaggtatta 9120

aagcgagcat tgactgctgc tgtaaaacac aagttgaatc gcaatttaat gatctgtccg 9180

attgctgata ttgagcatgt gcctgtggtg tgaatatggc tgctgccaat aatattccga 9240

atgctaattt ggttatactt ctcatgaatt tcccttcctt taaataatca taagaaatat 9300

attcaattag tcaaatttca cgcataaaaa acatccatgt agggtgcttt tttatttcct 9360

cttgcccatc tggtcgcgtt ttctggattt tgccaaattc aagatatggt tgttctcata 9420

aatcttggcg acctcaatca cctcatggtg caaaccggtc aggctcaggc atttctctcg 9480

gaggggcacc gtgaaatgcg ctacgatttc cttttttttt tttttttttn nnnnnnnnnn 9540

nnnnnnnnnn nnnnnnnnnn nnnnnnnnnn nnnnnnnnnn nnnnnnnnnn nnnnnnnnnn 9600

nnnnnnnnnn nnnnnnnnnn nnnnnnnnnn nnnnnnnnnn nnnnnnnnnn nnnnnnnnnn 9660

nnnnnnnnnn nnnnnnnnnc tctgagatgg tggaataggt ggagcatagg cagcagagca 9720

catgctgggg cttcataaat tgccagcaca accccattgt gtatgatgtg gcgaaaagtt 9780

gaagccgtag gactgaccag tagcacaaac attagtggga ttgctggaca gagttttgcc 9840

attgtcagca ccacatgcac caacttctct ctggtttgcc gtacataatt gactacctaa 9900

actattgcta caagtccaag agtaagttgt ttctccattt gcggtggtag tagaaactgc 9960

actgggtgtt cctgcatcgc acaaaaagct gttaggagca cctggcaaga tagatccagc 10020

agaagggccg catttaggtt taggggtggg gctggctaca tatgttggtg caacaaatgt 10080

gtttgcttta ggtattaaag cgagcattga ctgctgctgt aaaacacaag ttgaatcgca 10140

atttaatgat ctgtccgatt gctgatattg agcatgtgcc tgtggtgtga atatggctgc 10200

tgccaataat attccgaatg ctaatttggt tatacttctc atgaatttcc cttcctttaa 10260

ataatcataa gaaatatatt caattagtca aatttcacgc ataaaaaaca tccatgtagg 10320

gtgctttttt atttcctctt gcccatctgg tcgcgttttc tggattttgc caaattcaag 10380

atatggttgt tctcataaat cttggcgacc tcaatcacct catccaatga ggctccttgc 10440

ttgacctggt ggagcacaat cacttcttgg ttgtgtttga cagcagcctt gataaaacct 10500

ctgtctgtac aattgacacc aaggttctca agctctatga ctcggccaag gttgccgtca 10560

acacaggcaa aggccatctt gtcaaaggtg ttgccatgct catccacaag atccatgacc 10620

ttgccaatgt tgccttcctt tgaatgcttt tccatttcat cttctatttc ttttagggtt 10680

ctttctttca tattgcctcc ggggattgtt tttttcgaaa tttacttata aagtcaaatc 10740

ttgttgatct taggtttggc tgctacctgt tgttctttga gctgaagctt ctccacaagc 10800

ctttggttag taataagctt gctgatttcg tgccccatgt ccggttcatt gagtagattt 10860

gctacataca actcaatcat gttgannnnn nnnnnnnnnn nnnnnnnnnn nnnnnnnnnn 10920

nnnnnnnnnn nnnnnnnnnn nnnnnnnnnn nnnnnnnnnn nnnnnnnnnn nnnnnnnnnn 10980
nnnnnnnnnn nnnnnnnnnn nnnnnnnnnn nnnnnnnnnn nnnnnnnnnn nnnnnnnnnn 11040
nnnnnnnnnn nnnnnnnnnn nngaaaacat gatttaatga tttttgtggt acatgaataa 11100
acccttttgt ttatcacgac attgttttaa ggcacttact tcggtaggtg cctttttatt 11160
tatatttgag ttcattgtct ttggcttcct tgagcttcct cttctcaagc tcaaggttca 11220
atgcgacaag tttttgctca atggcaatga tctgagcctg aagcttcgtg gctcgtgtgc 11280
cggtggcacc agccagcagc aacagcaggt tgtgatactg catggacaaa ccaccgatct 11340
gagcctccaa acctccaaca gcagaaccca cattgggcag gttggtgggg ttctcacgct 11400
cgtgctgcat gcgggccact ctgtgggggg cagagtggtt gatgccttgc tggccaagct 11460
gaacctcctt ggcatgttcc ttgatctcct tgagcttctt ttcaagcttg ctgcggatct 11520
cggtggtgtc acgttcaaga gtagttggca ccttctcttt ggtgggtgtc ttgccatcct 11580
tcctattgcc accagaaggc cctttcttgg ccgcatatgc acgttttgca ccaggtctag 11640
gggtgaggta gcgtttggtg ttgaaggctg ttctggctgc aatgaagtcc accagcttgg 11700
aacgttggtc cgcggactcg gccggggtga ggaacttggg tatcttggac tggtggtgtt 11760
ccttcaccaa ggccgcatag tcactgtcct tgctgaatag cgagcccttc aatctggtgt 11820
tccttccagc accaggtaag cgaacggtga tgaactcgtc acctaccaag gcaatgccaa 11880
tgcctcgctt cttcatgtag ccaatgagct ggttacggtt ggctatggag ccactgacga 11940
tgcgagtgtg aagctcatca gcaaagtcat tcttggcttc cttctcgcgt ttaccgttgg 12000
gggacttggc ttcaaatggc ttgaggctga tcttcaaggg atcgggcacc acctgtgcaa 12060
aaccgaagtt gtcattcatc acggcactga aggcattgaa gaactctatg ttcttctccc 12120
caggcggatg gatgttcagt tgctggccgg tcttcaattc ggtattggca acaaggaagt 12180
gaagttcaag gttgcctttg tctctgtgca tcacccagaa gtcagcgaaa ttctcatcaa 12240
ccttcaagcc tggcatgaag gtggaacgga aggcttcgat caatgtggcg atctgctctg 12300
gtgtgacaga ctgggattca agtccatcac gaaaagcgat gacaccagag gtgtacttgt 12360
gctgcctttt cgtggcattg gcaatctctg tgaaggtggt tggatcgcca aaaagaattt 12420
caggcatgat agatcgcttc ttgccttcat gatcggtgtc actgagcagg tatctaactg 12480
ctgattcgga acctcctttg ccagtgggga aaatggtgac gttcatatct tccctcgttt 12540
tgaagatcct ttctcaccaa ggttggactc aagtttttct ttgagtgctt tcttctctaa 12600
aaagtggcca tactcttctt ctgaatattg atgtccattg acccaccatt ggtgtggctt 12660
gccatcaaga aggacagcag gcccgtcttc tctatgaagt tcgccattga aaaagtgctt 12720

gatgtagacg gcagatacgg tttctactcc ggtggcaaaa tcagttgaaa ccatcttgaa 12780

ttctgctacc ccagaaaaat catctggtaa cgcttttgtt atgctcattt cttccccttg 12840

aataattctg cgatagtgct cactccttcc tgaatcttgg tgagttcctg gaacatccct 12900

ttggtggtgt cgttccacat cttcatgcga ttcaaagaac gagcaatctg atttatgttg 12960

ctgccgatct ttccaagaga agcaagaagc tgaacacgca acttgtgttc tgccttggtt 13020

tcctggttgg cttgctggtg tgatggaagc aggattgaac gggcataaat cgctgtggaa 13080

gatgcacctg cttctacggc tcgctgttgg agagccatcc attcttcagg tgtgaacctg 13140

atctcaaaac gcttggttcg ctttttattg ttggcttctg tgccagcttg acctgccctt 13200

attttgagag tcgggtcttt ttcttttttg tggtatgcca ttggggcctc ctttgttctt 13260

gtttttatcg agcctcgcgc agcagcgggt gtgggtgaaa cccacgatat tcaaaccaag 13320

aatagcccga aagggatata cgtaggattg attgaccttg cttaaactac ttttccccca 13380

nnnnnnnnnn nnnnnnnnnn nnnnnnnnnn nnnnnnnnnn nnnnnnnnnn nnnnnnnnnn 13440

nnnnnnnnnn nnnnnnnnnn nnnnnnnnnn nnnnnnnnnn nnnnnnnnnn nnnnnnnnnn 13500

nnnnnnnnnn nnnnnnnnnn nnnnnnnnnn nnnnnnnnnn nnnnnnnnnn nnnnnnnnnn 13560

nnnnnnnnnn nnnnnnnnnn nnnnnnnnnn nnnnnnnnnn nnnnnnnnnn nnnnnnnnnn 13620

nnnnnnnnnn nnnnnnnnnn nnnnnnnnnn nnnnnnnnnn nnnnnnnnnn nnnnnnnnnn 13680

nnnnnnnnnn nnnnnnnnnn nnnnnnnnnn nnnnnnnnnn nnnnnnnnnn nnnnnnnnnn 13740

nnnnnnnnnn nnnnnnnnnn nnnnnnnnnn nnnnnnnnnn nnnnnnnnnn nnnnnnnnnn 13800

nnnnnnnnnn nnnnnnnnnn nnnnnnnnnn nnnnnnnnnn nnnnnnnnnn nnnnnnnnnn 13860

nnnnnnnnnn nnnnnnnnnn nnnnnnnnnn nnnnnnnnnn nnnnnnnnnn nnnnnnnnnn 13920

nnnnnnnnnn nnnnnnnnnn nnnnnnnnnn nnnnnnnnnn nnnnnnnnnn nnnnnnnnnn 13980

nnnnnnnnnn nnnnnnnnnn nnnnnnnnnn nnnnnnnnnn nnnnnnnnnn nnnnnnnnnn 14040

nnnnnnnnnn nnnnnnnnnn nnnnnnnnnn nnnnnnnnnn nnnnnnnnnn nnnnnnnnnn 14100

nnnnnnnnnn nnnnnnnnnn nnnnnnnnnn nnnnnnnnnn nnnnnnnnnn nnnnnnnnnn 14160

nnnnnnnnnn nnnnnnnnnn nnnnnnnnnn nnnnnnnnnn nnnnnnnnnn nnnnnnnnnn 14220

nnnnnnnnnn nnnnnnnnnn nnnnnnnnnn nnnnnnnnnn nnnnnnnnnn nnnnnnnnnn 14280

nnnnnnnnnn nnnnnnnnnn nnnngcatag ggctgtcggc tcaccaaagg gtcttgtttg 14340

gtttggccaa tctggtgctc gccaggaggc agttgctggc cagtcctggg agcattgcgt 14400

cctgagcgcg caaagcgcgc caagataggt gcgaaagcag cgaaaaaccg tgctgaatcg 14460

aagcatccct tcctcaatcc gaaaaaccaa gatggcatga ctcgcgattc cattcgccga 14520

cttcattgat cagcggttcc ctagcgcatt caaaatctaa gtcaacgatc atcagcagta 14580

tgagcagatg ttttatgact caaaattttt atgaataaaa aacggactgt gtcatcgtca 14640
ataaaatcaa tagaagatga tgtcgccatg agacatggga cagattcatc atatatgtgt 14700
tcatgcaaca taagacaaat tcatcatata cgtagccatg cgacataagt gcaatttgtc 14760
attgtggttg acttcgataa aaaatacgtt tcaatgagtc atgagcgatt tggagaaaat 14820
tttgtcatga ctaagaagag cataaagaat gaaagaacaa cagtaaggat tgggcctcga 14880
atgtccagag gcctttatca gcttgcgaag tatgtttatg gcgtgaaagg cgtcaacttt 14940
gaggatcaac ttgaagagtt ggtggaaaaa gatctggact atcagatgag gcaaaagtgg 15000
tttctcaatt ccacttctgg aaaagagggt cagtattttg agaaattaaa aaaagcaatt 15060
ggtgactcga aagatgatct ttcatctgag gatgtcagtg actttgattc aaacaaccca 15120
aaaaaggatg gaggcgagaa tgagtgatga atacaaagat tgcttccagc cgaaagaagt 15180
gaagatcagt gccaagatgc cttttgaact ccatttggct gcgaagcaac tttactcaat 15240
gcaaggcaca agcattgggg aaagggttca aaaattaatc aggactgacc tgaaaaaaat 15300
gatgagtcgt gattggttca aggagttgcc agaggaagaa gtagccatct ataagatgtt 15360
gcttgaactt agcgagcgag aaagagtgaa atgaaatttc aatacgataa aaccataatc 15420
accactcaat ctgatttgca agtgtcagtg gcagacttga gggatctggt tcaagctttc 15480
actattccaa atgaagccca aagggttcaa gaaattcaat tgattcttga atcagttgtg 15540
aggaagaatg gtctgcaacc aggtgcttta ggtgtggagt aatcaacctg ctgcgcacac 15600
gccttgtggc ggccccggcc tcacgtggtt aaggaacgac gaagcgttct cactagggaa 15660
acgctgattt atcagtctca gccgtcatcg ctgacgcagc tgaggccgtt gtagcaggta 15720
gctcacggaa cggagccata gtgatgaagc ggcagatgag ctttgcagaa gcggaaagcg 15780
caggtaagaa gcgcgtgacc aaacgccagc gcttcctggc agagatggag aaggtcgtgc 15840
cgtggcagcg cttgctgtcg gcgattgggc cccactatcc gagaggcgag cgaggtcgcc 15900
cgccgattgg tctggagagg atgctgcgga tctacttcct gcagcagtgg tacgggctgt 15960
cggacgaagg tctggaagac gcgctgcacg acagtatggc gatgcgagcc ttcgccggca 16020
tcgatctggc ggtcgaggac gtgccggatg cgaccacgct gttgaagttc cgacgcctgc 16080
tcaacgaaca cgacttgacg cgaaagctgt tcgacgagat cggcatcatg ctgtgcgagc 16140
gggggctgat gatgaaggaa ggcaccatcg tggatgcccc cctcattgaa gcgcccccgt 16200
nnnnnnnnnn nnnnnnnnnn nnnnnnnnnn nnnnnnnnnn nnnnnnnnnn nnnnnnnnnn 16260
nnnnnnnnnn nnnnnnnnnn nnnnnnnnnn nnnnnnnnnn nnnnnnnnnn nnnnnnnnnn 16320
nnnnnnnnnn nnnnnnnnnn nnnnnnnnnn nnnnnnnnaa cgcggaggga cagtcctcag 16380

ggatcaaagg gcttggccaa gaacacggcg caactgttca gcctgtttgg tttggccaat 16440

ctggtgctcg ccaggaggca gttgctggcc agtcctggga gcattgcgtc ctgagcgcgc 16500

aaagcgcgcc aagataggtg cgaaagcagc gaaaaaccgt gctgaatcga agcatccctt 16560

cctcaatccg aaaaaccaag atggcatgac tcgcgattcc attcgccgac ttcattgagc 16620

agcgcttccc tagcgtgata gaaatgaagg agtagaacga atgaagaatt gggtgggaat 16680

atttcttatg ttgtggaatg tctgccactt caaacatgaa gaatggcatt taatgagaca 16740

gcgattgaaa ttaaaaaagc acctctaggg tgcttttgtt tttttacatg gcctgctttg 16800

ttggaatgaa tgcgtgtatt cgtttcaatg aagtgggtgc tccttagctg tgaaaaatat 16860

ctagccacct ctttctacgc ttgatgctat ctttatgtgc cagcaggaaa gggaaaaagt 16920

cctcatagtg tggaatcacc attgaggaaa ttccattttc tgcttcatat tcactcgcct 16980

tgtgggccat tcctgtaagt aagaagtgga tgggtcgcag catctcgatc agtcgccaga 17040

gaaccagtgg ggcactcttc tcaatgacat attgacggtg taggcagtca aacaccatca 17100

tggggtgtcc atgctcatca aggtggctgg tgaagccgtt gggtggccct tctctatcca 17160

tgcggtacat gaggttcagt gctgacctct cacgcacctg gtgcggtgtg tagtcttcat 17220

aggtagctct ggcaagatcg agccacttgt agagagggct tctgtccatc ttggcttcgc 17280

agatttcatt cagattgaag tagcgggcac catcaccgta ttcactaatg aagttcaata 17340

gaacttcgtc attggcggtc agagtcggtg ctacagcatt ggggtggctg tagatgggct 17400

tcaacctgat gcactcttca tagagcacat caatcttgtg tccaaagcct ttaagctgct 17460

tggtggtcgg tgtctggtag ttgttcgtca gcatgtggtg tgtcaccaca gccaacttca 17520

gaagcctctc cataccaatg gagagatgaa agaacgccga ataaaagtag ccgtccttgt 17580

cctggaagaa gttggcctta agcagaaggt cgaaacctga cagcaacgtg ttctttgtta 17640

ggtgagcttc ttgtgcaaga aggctgaatt ttttatcaaa acctatctgc ttcannnnnn 17700

nnnnnnnnnn nnnnnnnnnn nnnnnnnnnn nnnnnnnnnn nnnnnnnnnn nnnnnnnnnn 17760

nnnnnnnnnn nnnnnnnnnn nnnnnnnnnn nnnnnnnnnn nnnnnnnnnn nnnnnnnnnn 17820

nnnnnnnnnn nnnnnnnnnn nnnnnnnnnn nnnnnnnnnn nnnnnnnnnn nnnnnnnnnn 17880

nnnnnnnnnn nnnnnnnnnn nnnnnnnnnn nnnnnnnnnn nnnnnnnnnn nnnnnnnnnn 17940

nnnnnnnnnn nnnnnnnnnn nnnnnnnnnn nnnnnnnnnn nnnnnnnnnn nnnnnnnnnn 18000

nnnnnnnnnn nnnnnnnnnn nnnnnnnnnn nnnnnnnnnn nnatcaacgt gtccctagcg 18060

cgccggatgc aga 18073

<210> 4
<211> 4775

<212>  DNA
<213>  Ralstonia solanacearum


<220>
<221>  misc_feature
<222>  (1077)..(1966)
<223>  n is a, c, g, or t

<400>  4
aggggtttgt agacatggag ccatcgcaat cgagcaatgc cgaagggctg agcggagacg      60

atatggaggt tgtctctgca gtatcccggc gatacaggcg ttatcacaag atagccgcaa     120

ggcatctgcc ggaatgggcg ccgtctgggg ttacgctaag cctcatacaa gcgatgttga     180

acctccgaaa cgttcgttgc gcaaagagaa acattcacga gaacgatgac ggcacccagt     240

caacccatgc ggagcatctg agatatgccg aggccgaaat agctcgtctc gtgcgtctgt     300

gtgcctatag caatgaaatg cagaaggctt ggcggacaat atcaactatg ccagctcgtg     360

gtggtggcga tccggcattc tccatatgtt caaaaatctc ctccacgctg gggcaatttg     420

gtgacctccc caagaggctt ccagcacaac gcaagaaatc gctttctcgt gtggcgaagg     480

cagtggacga tctgcttgag gcaatcgaat cggagagcga ggcgcaggac tctcttgaga     540

ttgcactgat tgaatacctg ggaggtcaga acctcaagat gcgctctgac gacggagagg     600

aaataggagt cgatgaatct ctcgatccct tccgagcgct tagtccggcc atcattccat     660

gcgacgatac agaaggcttg gaggactccc ctcattgctc caagccctgg cacgagtggt     720

catctttcga tagatacggt tggcttcacg atgaagtatc catgacggga atggccgagt     780

tgctgatctt ttgtcgcgac agaataaatg ccttggctca ggagccgccc aaaattctgc     840

agcctggaag accagatggt gggctacagc cgtttctgat tcggagtttg tccaactgga     900

tgaagcgcat atacggacag cccttggacg actctgtagc actgcttgtc tctgccacgc     960

tagatctgcc agagcctctg acaagagaag cgatccgccc gtatctgaaa ggcaccggga    1020

agatttcgag caggaatcga tagatttctt cccgtttcaa aatatcagag tccacgnnnn    1080

nnnnnnnnnn nnnnnnnnnn nnnnnnnnnn nnnnnnnnnn nnnnnnnnnn nnnnnnnnnn    1140

nnnnnnnnnn nnnnnnnnnn nnnnnnnnnn nnnnnnnnnn nnnnnnnnnn nnnnnnnnnn    1200

nnnnnnnnnn nnnnnnnnnn nnnnnnnnnn nnnnnnnnnn nnnnnnnnnn nnnnnnnnnn    1260

nnnnnnnnnn nnnnnnnnnn nnnnnnnnnn nnnnnnnnnn nnnnnnnnnn nnnnnnnnnn    1320

nnnnnnnnnn nnnnnnnnnn nnnnnnnnnn nnnnnnnnnn nnnnnnnnnn nnnnnnnnnn    1380

nnnnnnnnnn nnnnnnnnnn nnnnnnnnnn nnnnnnnnnn nnnnnnnnnn nnnnnnnnnn    1440

nnnnnnnnnn nnnnnnnnnn nnnnnnnnnn nnnnnnnnnn nnnnnnnnnn nnnnnnnnnn    1500

nnnnnnnnnn nnnnnnnnnn nnnnnnnnnn nnnnnnnnnn nnnnnnnnnn nnnnnnnnnn    1560

nnnnnnnnnn nnnnnnnnnn nnnnnnnnnn nnnnnnnnnn nnnnnnnnnn nnnnnnnnnn 1620
nnnnnnnnnn nnnnnnnnnn nnnnnnnnnn nnnnnnnnnn nnnnnnnnnn nnnnnnnnnn 1680
nnnnnnnnnn nnnnnnnnnn nnnnnnnnnn nnnnnnnnnn nnnnnnnnnn nnnnnnnnnn 1740
nnnnnnnnnn nnnnnnnnnn nnnnnnnnnn nnnnnnnnnn nnnnnnnnnn nnnnnnnnnn 1800
nnnnnnnnnn nnnnnnnnnn nnnnnnnnnn nnnnnnnnnn nnnnnnnnnn nnnnnnnnnn 1860
nnnnnnnnnn nnnnnnnnnn nnnnnnnnnn nnnnnnnnnn nnnnnnnnnn nnnnnnnnnn 1920
nnnnnnnnnn nnnnnnnnnn nnnnnnnnnn nnnnnnnnnn nnnnnncggg aaaaacgcgc 1980
caacgggtgg ccccggttgg ggtgggcgaa cccttggccc gccaggaggc aatgctggcc 2040
agtcctggga gcattgcgtc ctgagcgcgc aaagcgcgcc aagataggtg cgaaagcagc 2100
gaaaaaccgt gctgaatcga agcatccctt cctcaatccg aaaaaccaag atggcatgac 2160
tcgcgattcc attcgccgac ttcattgatc agcggttccc tagatgaatc gtcgactgct 2220
acgccggcgg tggggcttcg tgaatgaccc ccatgcgcac catctcgtcg atttcggcac 2280
gagcaactgc cacaatgtct tccagtgtgc ggaaacgagg gctttgaggg cccatgacgt 2340
cttttgtgac tggatggccc agcgcgtcgc ggtaccgaat ccggccagtg caagtcgctt 2400
cgctaagctc ttggactagg acgatctgcg cgcctttgta cgcttcaacg ggtagttgag 2460
ccatggctgg cctccggttt tgatttggag cttctgcgtc ggccgttgcc aagcctgtcg 2520
cccatgtcga taacgatgag ctcggttgcg gcaatacgct ggatctcagc catgatgggc 2580
agttcgcgcg ggctactggg ctcgcttgca gccacaccgg tggcgtgctt cgcttttggg 2640
ccaggcgggt agaaaacgaa gagggcggtg gacggcgcat ccggctcggc acgcaggcgc 2700
ttgttgagaa tcttcaagta cttcttcttg gtgaccttct tggttgccac agagcctcct 2760
cagagggttt caggtacctc atgcagctga cagtacgtgt tgaccagttc cttcacctcg 2820
gccgcttcat tgggcgttcc gaggcgctca acgaacgcat tgagcgcgac cagaagcgag 2880
cagatggcca gcaccacggt gtcggcgtac tgcgtgtagt cggggccgat ctgtaagccc 2940
tcaattttcc ctgatgcgtt caggatcaga tgatgttgca acgacgtggc cgaagggtgt 3000
gcatcttgag acagttgccg gtagatcacc gtgtagagat ctcccaggtt gagatcttcg 3060
gccaatccac gaaggtctat gtcctggtgc ttgcccggcg cgccaatttc atcgagcaat 3120
gcttcggccg ctgcactctg ttcggtcgtt accccaggca tctcgcgcag gtcggtggcc 3180
acttgtactc ggaaccgttg gtggccgccc tcgaaggcat cgaagaaact ggtggtcggt 3240
tcgtcgccct tcttgggttt gaggcgagcc ccttgcgaaa gcagtaggtc gcgcgaggcc 3300
gcggcgcagt acagcgctgt ctcgaatgcg gatcgcgcca tcgatcgcga ttgcgcccgc 3360

aagccccgtg ccgccagaat caccgccgcc tcgaagtcct gcaccgcacg gctcagcgag 3420

gtcacggcca aaagatcggc gtcgcgttgg aagatgtcgc ggggttgaat tagcagtgca 3480

acagcaaagc ggttgactcg ctccatcagt gttgtatggc atggaaaacc actcggtact 3540

tggtttgcga gggtatcaac gtcaggcgac aggaacccat ctctgctcag catacgggag 3600

tgccctccgc actacacgct agcagttgcg tggctgccag cggcctttcg agccttcgac 3660

ggctagaaat tgtgtgatcc gagcttgacc acatctagcg tcgaattaac ccgtcgcatt 3720

tcgaatgcgg cccggcggat gcgctcagag tgagcctcga agaccgcaag atcgttgtgc 3780

ggctttccct gcgctagcgt ggcaagagct gttcgtgaga tcgacgcggg aatctgcttc 3840

tcgccgtccg tgatcgtaaa ggccagatag tcgccagtta tgactgcctt cggggctgaa 3900

atctgaaagc tctcgtccat atcgcctccc actctcagtc tcggggcatc ctagcatgac 3960

ccatctgacg gaagggaccg tgccggcgag gccggtgcca ctaaacccgc catgaagatg 4020

gggcaaatcc actgtccgtc gctcgggaaa tcaatcagcg acaccgatgc ctgtggtgtg 4080

agccacccga ggtggtggag ttcaggagag ccggaaggtg gggcggctgc tcttcaaagc 4140

cagccggaca cgccaccgtg gcgtgagcac gtcccgcggt gcgactcgct gaagctgtac 4200

gacccgtccc ggcacttggc ggtcgcgccg ttcggcacgc tcccatcctt gctgtgcgct 4260

ggcgagtgga cgtcatgccc ctgccggttg gtgtagtgtc catgctcaac gagcgcggat 4320

tcgttcggag ttggccggta gttggggtga cgtggcgctc tggcgaatga gggctgcgcg 4380

accatcgcgg cgacggccat aacgacggcc gccatcgtgg gtatggattt catgtgtctc 4440

cccctgatgg tgtttgtgcc caccatcctg atccatcccc ggggaaagaa agcccccaca 4500

gaagtggggc tcggctgtcg gtggccacgg gttccacgga ttcaaccatc tgtcggttga 4560

tgcaaccgcg gtgcatgagt ggccggctac tccagagaat tcctgccgcc gaagcgcgat 4620

cagagccggc gtatcgggat gtaggcccgg gcgagaggca ggctgcattg agctcgagcc 4680

agctgcgtgc gggctcttta aatttctgag attcgcagct tgtaggcggt gccgcttgac 4740

tcgccactaa tagctaggga accgctgatc aatga 4775

<210> 5
<211> 3825
<212> DNA
<213> Ralstonia solanacearum

<400> 5
atatcgccgc ctggatgata ggaaaaaaga tcaatggcgt ttcgcattcg tcactatcca 60

cacaatgact tgctgaacct cgcgtcgcat cactgcgaga cgatcaagat gaaggtgaaa 120

aacaaccagc tcgaaggtat aacgctcgat tgcatgagct gtttgatcgc acttgccttt 180

gcggtggagg cgctagtcaa ttttgttggg tctcggaaag tgaccgactg gaacgagatg 240
gccaaggctc cgagcaaggt ggcaaagatc gcgaaagcag tgggactcga catccagcca 300
gagtttgagc ccttttccgc aatctatacg ctccggatca ttcggaacgg attggctcat 360
gggaagccgc ttcacaaaag cgccagcgcc aaatcttctg acgagttggc gattgccatg 420
caggccccgt gggcaccata ttgcacccct gaacaagtta tagccctgta tgagcaggta 480
cgcgaacttc gtagggtgtt gttcaaaagc gcaaacatcc gatgggttga cagcctgacc 540
tctgccattg gctcgcacgg ataggaaaac catggcaaac gttgtgacct tgctcgatca 600
ctggcagacg ctcgtcggcg cgttcatagg cggcctcatg ggcgtagttg gtgcgctaat 660
cgttgcagta ctgaccgtgc gccgccagcg ctgggtgatg gcctctgcgt tgctccccga 720
catgcaacaa ctaagagccg ccgatgatgc gctcgaacga gcgcttcact ccattgatcc 780
gccgctcgga gagtgggttc gatctcagtg gtgtgcagag cgtcttgcac aaacgcaccc 840
cacgttgtcg gcgcttcatg acggtgtagc cgtgacccag gtgtctgacc ttgatgcgcg 900
actaagcgca cacttgatgc tctgtcggat gaggcacaag gacttggacg cacttctgca 960
gcaatttgtc gccgcgctcc atggctcgcg ggcgccgatg cctgcggcca atgttcccgc 1020
agcgatgacc gtggtgcggc gaagtgcaaa acgagtacag caggcgtggg atctatgcgt 1080
cgaacacggg acgcttgcgg aatacctgct ggatcgattg attttcaata ggtggccaaa 1140
catttggcac cggctgcgca tgcgttggtg gccgaacgac cttgaccatc gctcgatgca 1200
tttgctgaat tcggggtcga tcctgggcac acaggactcc catcaggacg acgtggccgg 1260
ataggaaaaa tcaattctcg tactaatgac gagcttggag attcgacatg cacgacatcg 1320
aaacagacgg cttcttggca cccgaggccc tggcgtttcg ggatgcagtg cgagtccaat 1380
ttgtcgagca aatccgtgag tgcgaggatg tttcgcgccg cgccacgacg caagtatttg 1440
agcaacacat cgcgaacgtg acggttgcaa atgcgacggc agcctcgctg tgggcgagat 1500
gtttgagtgc aagccaggcc gccatcttgc tggctgagcg cggcatgggt gtggaggcgc 1560
tggcactttt gcgtacggct tatgagaact tgttcttctg cgctgcgctg gtcaagcagc 1620
cggccgtgat agtgcgcctc gccggtgaag atacgggtca gcgtctcaag caggcgcgtg 1680
aaatgctcaa agacaaacag atcttcgcgg caatcacgga tgcagatcgg gcgctgcttg 1740
aggcgttcgt ccgcgataca ccggctgcca cgaaagccat cagtgcgtac gaagccgctg 1800
agatagctgg catgaccggc atctatcaag gtgcgtggcg gacattctca ttgattgcga 1860
cccatgccac gctgaccggg gccgggcacg catttggcaa tgacctgtac gatctacagt 1920
tcggcccctc gttcgcaaac gtcgactcgg cattaggctt ggcccgtgac tgcattcggc 1980
tcgggcttga tgcggttgcc ccgattttta ctgcgtgaaa gacgtgggaa gacaagcatg 2040

gcggaattcc acctcgaact ttaggttcca atcggtcctt tgctcgaacg aaggagccgg 2100

tagcgatttt gtcctacagt ggggtaggaa caaattcgtg cgaaaacagg gaacaaattt 2160

atgcacatga acggttgctg ctaatcgtgc gtctgcggcg accattttat ttaacataag 2220

ataaattatg cgaagtccaa cttgtagggg ccaaaccaga atgtccgctt ttagccagat 2280

cagaatgtcc tctggcgagt cctatactgg tagcgcctga gtcggaactg gagcgcacca 2340

tgaaggatcg agggttgatc accatgagtc tgcgcgaagt tgaccgcttc aaggtcattc 2400

aagccgccgc cgaaggtctt ttgccgcaat ggcgcgcggc tgaacggctg aatctgacga 2460

cgcgtcaagt ccggcggttg gtccaacgct ggcgtgtaga cggcccgacg gggctgctat 2520

cgcgtcagcg cggccagcct ggccatcgac agctgccccg catgctggaa gcgcaggcgc 2580

gcgccctgat ccaggctcaa tacgctgatt acggcccgac cctggcggcc gagaagctac 2640

gcgagcgcca tggcgtcgac ctggccaagg aaacggtacg acggagtcga cgtttgctta 2700

ctttgccgcg acccgcgctt atctggaaag gcacggcaag cctgtggcct tctacagcga 2760

caagtccagc gtgttccgag tcaatgccag gggcgccacc gcgggtcgcg actatacgca 2820

gttcggccga gcgctttatg aactcaatat cgagagcatc tgtgccaatt ccagccaggc 2880

caagggtcgt gtcgaacgta tgaatggcac gctgcaggat cgcctggtca aggcgttgcg 2940

cctgcacaat atcagcacgc tggaggccgc caatgccttt gcgccggtct tcctggcgga 3000

cttcaacttg cgctttgcca agattccccg cagcgatttc aacgcgcatc gcccggtaca 3060

acctgaagag gatctcgaac gcatttttac ctggcgggaa tggcgcaagg tctcgaatcg 3120

actgaccctg caatacgatc gtacgctgta cctcatcacc gatcagcccg agcatcgtcc 3180

gctggtccac cgctatgtcg aggtggcgga atatcccgac ggccacatcg aactgtgggc 3240

cgacggcatc tccctgccct atgtcctcta tgaccggctt tgcgagatcg atcagggggc 3300

gatcgtcgag aacaagcggc tcggacacgt actgcagatt gcccaacgcg tgcaagcgct 3360

gcgtgataat cggcgcaacc tcgaccggcc gtcacgcacc ctcattggcc agccaccacg 3420

gcaccctgag ttgcgtgcgc caggcaccaa gtcaccgcga cagttgagtt cagaagactt 3480

gcggcgcgcc atcgaggagc tggcaccacc gccccaccct gcggatgccg caccgcctca 3540

ggccaactcg actgccctac ggaaacatcc cggaaaggca ccaagaaaga agagcaccac 3600

actgcactaa ccggacattc tgatctggac agagatagga cattctgatt tagccacaac 3660

aaaaatttgt cagggccaaa tagaattgtc gtatctgagc caaatagaaa tgtcgcgcct 3720

ctggccaagc caggcatgct gccggtttcc cgtcagtcgt gactggaggc tgagactacg 3780

gagctgtatg aggacaaacc ggtccggcaa gcaacccgga ccgct 3825

<210>  6
<211>  7305
<212>  DNA
<213>  Ralstonia solanacearum


<220>
<221>  misc_feature
<222>  (1325)..(1594)
<223>  n is a, c, g, or t

<400>  6
gtcttggccc aggaggcagt tgctggccat ttcttgggag catatggttc ttgggccgcc      60

aaagcgcgcc aatgataggt gcgaaagcag cgaaaaaccg tgctgaatcg aagcatccct     120

tcctcaatcc gaaaaaccaa gatggcatga ctcgcgattc cattcgccga cttcattgat     180

cagcggttcc atagcaatgc cctccgaatc ctgcagtaga ccgcatcgat ctttaaacca     240

tcctgtgcag cggctacggt ggtcgaaaac tgtcgctcaa tcatcacaag ctccatcaaa     300

gtttttttct gtgacgcagt cagctatggt tcttgcagac aggacaagtc cactggccga     360

agtgtgcgtg tatgccgacg gagagaggca agcgaatctt tcttcccgaa gtctcgtagc     420

aacttgcgca ataagagccg ctaacataac tggccctacg gcaagaagcg ggttatcgta     480

cgaggcatgt cgagaagaaa agtcagcaaa gagttgtggt tggcgctgga gccgctgatc     540

ccggagtttg tcgcctcgcc caaaggtggc cggcggcgct cggtcgatga tcgtgcagcg     600

ttgagcggca tcctgtatgt gttgcatacg ggtattccgt gggaagacct tccccaggaa     660

cttggctttg gcagcggcat gacctgtcac ctgaaacgcg ttcactgcag ccgatacttc     720

gattgcctgt tcttgaatgc gggctcggtg ccttcgagaa tctggacaac tcggcccttg     780

atctccggtt gctcaatcgc acccgcttca tactcaaatc gaacaccctc ggtgttacga     840

gacgcgtaga caatttgctc ggcgtcacac accaccgcca agttcggatt gtgtgtaacc     900

atgatgacct gccgcctctc cttcgccttc ttgatgcact tgactaggat cttgtagatg     960

gtctggttgt cgaggttctc ctccggctgg tcgatcacga tcggaatgtc gtccttgtcg    1020

accagaaggt agaaaaccaa gagcaataat ccgcgctcgc caggcgagag ttggccaatg    1080

tcttgccccc cgaaggtcaa cgagtagcgc ggcgaaaggt attcaaggcc aaagatgtag    1140

tcgagaactt cggtagcttc gactcccttg cgaagctgat cggccaaccg cgtctcgcgg    1200

ctgtcctgcc cttcgcgctg atcggagcgc atggtttggt caatccgctg tgcgaaggca    1260

acggcgtctt cgggcgccgc gaagctagcc tcttggagca tgcgcctaac gagctgattg    1320

ctttnnnnnn nnnnnnnnnn nnnnnnnnnn nnnnnnnnnn nnnnnnnnnn nnnnnnnnnn    1380

nnnnnnnnnn nnnnnnnnnn nnnnnnnnnn nnnnnnnnnn nnnnnnnnnn nnnnnnnnnn    1440

nnnnnnnnnn nnnnnnnnnn nnnnnnnnnn nnnnnnnnnn nnnnnnnnnn nnnnnnnnnn 1500

nnnnnnnnnn nnnnnnnnnn nnnnnnnnnn nnnnnnnnnn nnnnnnnnnn nnnnnnnnnn 1560

nnnnnnnnnn nnnnnnnnnn nnnnnnnnnn nnnnatacag ttggcatcac cttcagtagt 1620

gcgccgacta ttacccgcgg gtctcctcaa tgcggacgtg aaattcaaga ggaagattca 1680

tgtccatctg cccagccgac tgcacgaacg cctggacagg caggtagagc cgttggtatt 1740

cgtcgaccgt gccgcgaatc aacccatgga tctctcgtga tatcccggag cgttgctcaa 1800

tgagcttctg gagctcatca ggaagcgcgg caagcgccgc aatctcggat tccaactgcg 1860

cgatagttcc cggcttgtcg gcaggcccca tcacgtcggc ctttgatttt tcccacttga 1920

ccaactcctc cttgtagagc acgaagagac gctgacgctc gccgagcttg ctcttggcct 1980

cagcgaggga agcgaaaagc tcctcgcgtc gcttcaagat gctgtcgggc tctgcacttt 2040

gaagctttgc gtcgattgaa tcgatctccc cttgcatcgc tttggcgatt ccctcaacag 2100

gcccggcatt gatccgggtc tcgaacaatg agtcgatggg aatctcaacc ccgagctctg 2160

ctaagagctg tttcaactcg ctttcgaaag cttcgctctg cttcttctgg ttagccaaag 2220

cctgggcgat gcggcgcgcg actgcttgcc cctttgcagt agtggacttc gcctcccgaa 2280

gccgtgcttc ctccttgccg acatcctgca gttgctgctc aagaccgtcg atcttggttg 2340

cggccgcttt ggactcttcc aatgcttcag gggaagcatt cggatcctca acctgtctag 2400

gcttggcttc ttcgagggag gcgagctcag ccttctttgc cgcaagctgc ttgaccaagc 2460

tctccttgaa ttccgggaaa agcctgcgct ccgtaccaag aatctcgacg ttgagcgcag 2520

caatctggcc ccgcagcccc cccatggcct tgttgatctc ggtcaccttg aaattgagca 2580

gcgcgtccat cgatgactgg ccaagttggt cctccagcgg gacgtgtgag tagatgattt 2640

ttcgcaactc ggcatcgaaa gtcgccgatc cgcccgcacc cagctcattg cagagcgtct 2700

caaggtaact ctgcggcaga tacttcacgc gctccacact actgggctcg ggatcttggt 2760

ccaaatcccg cgtagtcggc gagccgtcgc gccagaccaa ggttcccacg aagttttgtg 2820

caagccgtgt tcgcggatcc cggaagcgca ccgcgttcaa aaacgagaag ctacgatagt 2880

ttttggtgtt ccccgccaac gcgataatgt cagcaagagc gctctttccg ctccccttgt 2940

tcccgatgat tgccaccaaa tctgaattca gcggcagttc catgtcgaac catgtgtcag 3000

cgagctttga gccttccttc ttggcgaccc taactgagcg gatgaacttc gttcgattga 3060

gctcaacgta ctggcgcttg gccgggttat cgccaacgca gacgcgatca tcgaactctt 3120

cgatagcctg ctgcagaccc ctgaaggtgc agtccgcctt gatccaggta aagcagttac 3180

cgatgcggtc cttgtcggtc gatgcgctca gccaatgtgc atcggaacag tcaagcaagt 3240

tggagttaac accggactcc ttcaacttgg cgcgcgcctt ggcgtgatcc tctggggatt 3300

ccgcagcggt aaagacaaga tctgcactgt taatgagcgt ctttttctcc gcgatcgagt 3360
ggtcatcaca tttgaggttc tcccactcgg tcttgccgac tgcaaggatg aacctcccct 3420
tgaggacctc gttctccagg gccttgcgca agccatccgc gctgacgttg aggttgttga 3480
aaccctcaat gagagggctc ccatacccca cccgcttgtc tgctggggcc gagtcaatga 3540
tggctgcacc aagagcttca atgctttgcc gggagatcac tgagttccat tttccttcgc 3600
cagtcgaccc aggaagaaga cggtagctcg gggcgattgc ggaaataaac tgctggcgga 3660
tgaattcagg atccacctga tcgaagatga tgtggatgtt gatcctgctc cagcttgatg 3720
gtgccttgcc gctttttccc ccttggacaa cgccgccgaa cttgtccaac cgcagttcca 3780
cgaccggcag aatcagatcg atgttcttca gccttccctg ctcgcgcttc gccttcagca 3840
ccttttcgta gccatcgacg agcacatagt cgttgatacc gattactttg aactcctctg 3900
gcagcttttc aacgtcagcg agaaacgctt cccaggcttc atcttggctc cccgggtagt 3960
tgtgatagaa ggattcgggc gtgtggacgt gtaaatccca tttcttccaa gtggagccgg 4020
ccagggcaag gggcatgttc ttgtctcgct gaagttgttg ttcacaagcg ggcattgaat 4080
caaataaccc ggctcacgat ggaggccaaa cgcacgggat cactctctac cagtctctta 4140
acggcggaga ggctcgacac cccaatcagc tcttggatca gcttggtcga tcggccttgc 4200
cgatgaagcc tcaccgcgaa tgtccggcgc gcggaactgg cattgccgtt ggcgatgcca 4260
gcctgctggt gtagccgtct gaatatctct gccagggact cgcacgaata gctagtggca 4320
ccggccgcag tcacgcgacg cgtaaagccg aaaggctcac cctcgccagt cagaaagagg 4380
ggactccgac aatccaatcc acggtacgcg gctggacttg tggtgatgcc atgtcgaaac 4440
gccagccggt gttcgagata gacgtcgatc gcagcgcgca cctttgcact ggcccatagc 4500
agcggtcgca ctctgccgtt gaaggcaatc tccgctctca cgtgcgattc gatgcgtgtc 4560
tccccattcg cctgcagata gtcggacagg cgcagcttgg cgatttcgtt gggcgtaagg 4620
ccagtgccat aggcaaccaa taacagggcg acatcgcgct tgctgttctg cccagtgacg 4680
gctgcaacct tcagtacgtg gcgaacctgg cgctcttcga tgactgatgg ctttggcatc 4740
ccgtaatgct cccgcttgtg gttgcgttac gagacagtat ggaaaaattt acatccgagt 4800
gcaaaacgag gccgccccat ccggtcaacc ctcggaaaat tgtgcgcagt ggcagcgcgc 4860
cgatctccgg caaaatcggg cgatgagtcc cctactcagc ctcgggtcgc cgggcagccg 4920
ttcattttct ggtaccaaag gcgtccccga gctgccgcgc gcgcttggcc tcatcgctgt 4980
gcaggtagat cgaggtggtc gagactgatg cgtgtcgcag gttgtcgcgc acggtggtca 5040
attccgcacc gcgtgcgagc gcgtgggtgg catgcgtatg gcgcatccaa tgtggactgg 5100

ccttgcgcag cttctcagcc agcaaagggt gatcgctgtc gataatttcg gcggccgacc 5160

agaaaaaacg ccgcaccaca ctccacaacc gcgttcccgt aatgctgcca ttgggatcga 5220

gccccaaccc accaatcatc ggcgtcttcg gatcccaacg cgcgcgcgta atcggtagcc 5280

cgcgttcaac caggtagcgg tcgattgccg cccgagccag cggcggcagc gcaaccttac 5340

cagcgcgact gcccttcccc accaggttca gccagtgatc gtcctgccgg tccgtttcga 5400

tatcaccgag cttcgcagaa acgagttcgc aggccggtcg catagccgaa gtccagcacg 5460

aagcgcagtc gctgcgcggc ggacacctcc cagccgtacg accactcaag gccatcggcg 5520

atggtgcgga ctagcgccca ttccccctcg ctgaaagcat gcgaggcatc gagcgcagtc 5580

gttcgcccac ctccgcgcac cttgattcca gcaaaggggt tagccagcac gtagcgctgc 5640

tggatcagcc agcggaacat ggcccccaat acggacaacg cataggcgat cgagcgcgct 5700

gacaatccgt ctgcaaacgg ccgccattcg acggacgtgc gcggccgcgc cgggccgacc 5760

caccgctcgc gtggcgtcgg tcggcgcagg aagctccggt aggcaatcgc gtcctcggtg 5820

gtgagcgacg acagtggccg accgcgttcg acgatcgccc acaagatcag gcgttcggcc 5880

tctttgcggt acgcccgctg ggtggcgggc gattcgtgca gcgatagcca cgcttggacc 5940

gcctcatagt cgttgtcggc agtgaggacg caggtctcgc gcggcgcacg gaattgcccc 6000

cgcgagccgt cgacctcgtt cggcaggtgc aattgctccc acggcaccac cacgccgcgg 6060

acctgttcag caatcagggc ccgggcgcgc tcggtcaatc gaggatgttc ggcgaagaac 6120

ggccggcgcg gcgcgcggac ggtcagatcc gccagggtgg tgatgccggc ggcccgcagc 6180

gcggcgactg tacgcgcatc caaccacagc gccacgtcat cactgatgac cggctctggc 6240

agtcgcaacg ttcggagcaa ctcgatcgcc tggtcggctg ccctgccctg tttggcgcgt 6300

tcggccgctg gtatctcgaa cagcaccgcg aggtcttcgc gctgacgctg ccgtgccagt 6360

accgcgagct ggcgccggat gcgaccaagc acgccgcgtg aggactgccc cggcgtaagg 6420

cgctcgccca gatagcgctc aacggcatcg tgggtcgaca tgcctgcatg ccaggcgcgc 6480

aatgccgcca gttcggcggc atcggggaac tccggcgaag tgctgacagc gggattgacg 6540

ggaagcagac gcgttttcat tgaagccagc ttagcgcttc aacgttgcta tttcgataag 6600

tattcttatc gcaatagatc aattagacag cgaaacttcc ttccgtaaaa acgcatcaga 6660

catggcgaca ccagcttcgc ttgatgctcc ggacaacaac ggccgaccag cgctccacaa 6720

ggcgcctttt cgcgtcagcc aggatcggca aggttttctg ccgtgagagc gatggcgcat 6780

cacgtcacgt cgttcgcgaa gtacgccgct gcccttttta gaaccgggct caccggcttc 6840

gcacccgcat cggtcaaccg cctctcccgg tcagttttga ccccgttgca tgttgtcggg 6900

tccgctacgc ccggcgtggc ccctgctgcc gccatgcccc gcccggcttg accgcttgcc 6960

gcttgccgct tgccacttgc cacttgccac ttgccacttg ccgcttgcca cttgccactt 7020

gccgcttgaa tccgagcatg tgccgtgccg ctttgccttg cttgtctttg ttcttctcat 7080

cgctcgggct gatcggctct ttggtcagca ccctggtttc aataaattat ttcaatcaat 7140

ccaattaaac attcctatgc gcatcccggt ccggtgtttt gtagcatgca attgattcat 7200

gttgatgacc atcaaaccgt ccgattttgg atttaggtag agtcatcgga atttcattgc 7260

agtcattgct tttcaacttc tccatcttga aaccgggatc cgtta 7305

<210> 7
<211> 6751
<212> DNA
<213> Ralstonia solanacearum

<400> 7
tgagtttgaa cccgcattgg cggtggacgg gttctgctgc tgcaactggc gctcacggtt 60

gattgacggt cgccagtgaa ggtctcgtct acaagtgaag tactccccag gactcagcgg 120

gaaactgagc tgttcgagag cgagcttgcg aatgtgatcg ccttcgggtt cgcgtgattg 180

ctctcgatac ctcagcgtga tttcctgctt aacaggatca atgacgaacc accgggcccc 240

cctccgctca taaaatgccg cgcgcggccc ttcatccgcc ccacctcctg cataaatatt 300

gtccccgacc acgacaacgt cgccactacc gctaacctgt gggcgaagtg acttgaacgt 360

tcgcccttgg tcgaaggata cgtagactgc ggcatcatcc cgagcgacca catagggcga 420

attgaccacc ttgaacatcc ccgtcggatt gcgcttgcca ttaccgccaa actgcgtccg 480

gatgcccaac tgctcgtcta catagaacaa ttccgcctga cacggcaggt tatatcccgg 540

ccccggatcg ttctcgatgt agcggtgatc gtcgaagcgg tacacaacct gtttaggtgg 600

acgcgccata ttactcagcg tcttctcgct gatcgtgtcg ccgaaaccga ccgacgctgc 660

agcccggtcc gcctgggaaa gaccgcagcc agtcagcgat agcagcgtga cggttgcggc 720

aaccagcaac atgcggcgca tggcgacggg atgtttctcc tgtttcaacg agagctcctt 780

aagtagttct gaatggatgc gcacgctctc agcctcgacg ggggcgccag cggcggatca 840

tatggtggtc aagcgacacg tcggtgccaa cctgcccacc cctgatgacc gtacgcgttt 900

cgttctgacc gggccgcaca atggcgtagc tgtcaggcat ggtgttgcct agcttgtcca 960

ccacgggcag gggttcatcg ggtttcagtg ctgacgtgac cgcgtgcggc accaccggcc 1020

ggctatccgg ccccgcgaag atatgcccgc tggcatgcac ttgatattgg ccgttggtgc 1080

cactcgtaat ccccgactgg ctcacgtcga tatagcttcc gcccccgttg atgcgcaccc 1140

cgtccacccc ggtgatatcc acccgcttgc cctccatctt gatatccaga ttcgccagga 1200

tgttgatgcc gttcttgagc gcctgcatgt cgatatcccc gctggcggcc accagcttca 1260

ggccgagccg gtaggcaaac aaacgaatcg cgtccttggc gctggcgagg aagctgcgcc 1320

ccgtggcaat gctggcgtgc ccgccgctgg tgatcgcatg gtgcgcgttg ctctgctggt 1380

gcgtgctgcc cgccgtactg ctctggatgc cggccgggct ggccagcatc aggtgcggtt 1440

gctggaactc ggggaattgc ccctgcgccg ggtcgccgcc gttgcctttc aggtcggcat 1500

tctgctggtg caattgcttg gcgacctcgc cctggtcgtc cgcttgctgc acccctgccg 1560

tgcgtgccgc ttccgacaat cgttcgtgca gttcgtgggc caccgtcagc cgcgcgaccg 1620

gcgcgcccgc gtccatcatg tgcccccgcc caccgggcag ggcttcggtg gtgagctgca 1680

tcccggcagc ggcacggttc acgccgtggc cctcggtatc cagtgcgaaa ccttggccgc 1740

gcgggttctg ccggccggtc gtgccgtcga tccgcgtgat gtgccccagg ctcaatgcac 1800

tatccagatg atcgctcttg agttgcacct gaatcttgcc cgtggtgtcg tccaagacca 1860

aggcgttgct gcggcctccg ggatcgttgc cgccgtccgg ggtcagttcc cggctgcgga 1920

tgcccatcag cgccttctgc gtcggcaact gccacggcgg catgcggtgg gcgttatgga 1980

cgacgctgat tacgaggaaa tgatcgggat tgccatccag tgcgaccacc acctcgctgc 2040

ccacgcgcgg ccacgacacc atgccggttt cgccgcccgc ccaattgctc gccacacgca 2100

cgcgtgcact gaattttcct tcccggtccc agtggaaccg gatcaggatg cggccgtatt 2160

cgtccacatc cagagacccg aggccgtcgc ggccgaccac cagcgccgtc tggtgtccgg 2220

ccaccttcca ttcgatgctg tttcgtcccc ggccggggcg ccagggcacc gtgcgcttga 2280

tcgcggtaaa gcggttgcgg tattcgccct tgcggccggc aagcggctgc gccttctgcc 2340

ccgcctgctg accaagttca gcaaggtagt tgttgctcgc gctatggctg gcctccacga 2400

tcagatattg gctgtcctcg tcgcgctccg tgttgctgaa gtgcccggac aactcgaacc 2460

ataagccgct cgccacgctg gcgttgttgc cctcggcttg gtactgctgc gcgtgtgctt 2520

cgatttcttc acggcgcaag ttgagctgcg caacgcccct ggggtcctct ttcgggaaac 2580

cgtagtgccc cgcgtattcg tgcacctcca gctcgggaac gcgcccctgc ttgcgctgcg 2640

tcgattccgt atggtgctgc gctctcgggc gcttgaaatc gaaggcgctc cccgcgaaca 2700

acgcggccac cgctgtctgt atcggtgtcc agcgctggat ggcgttttct tcctgcggcc 2760

cgccctcggc ctggaagggt atccggggag cgctctcctc caccggcggg cattggctat 2820

tgctgtcgaa gatgacgagc cggtggccgg tcgcatcgtg ttcatcgcgg caggtcttgc 2880

cctgcgcttc caaccgtcga tagatgtagt tgtgatcgct ttcgccctgg ccgccgccct 2940

gcacggccat ggtgaacggt gcatcctctc ccgccacgtc ccagcgccat tgcgcccaag 3000

ccccgtagtc ttgcaggatt tcgctgatct gctggtacag gctctggtcc cggaatacgc 3060

ggttgttcac ccgatgccgc gcgaaccaca gccacggccg gagtacggcc cgataggtcg 3120
ctatgccggg gtcactgccc accaacgaga attccgccac atgtccggtg aaccagcgcg 3180
gcgtgccgtc cggcttgacc agcgacactg ccagcagttt gcccaacatg tccgccaaca 3240
cgagacccgc atggtctgcc aacagcgtca actcgaatcg gaagtcttgg cccaggccct 3300
cctgggcttc caggcgctcg acaaccatct gcacgggaaa ggagcggccc cggtggtcgg 3360
cggccgaagg gggatcgtcg tccgggaacg acaggcccat caagcgctgg ttctgctgga 3420
aaaagaagag gtcttgcaaa tgtgccgttc gatcgaattc ggccatgctc tcacttcttg 3480
gaatgttggc gacaaacaga aacagccacg gccaaaacgg gcgcggctgg atacgctgag 3540
ctcagaggaa gcgaagaaag gaacgggctg atattcagcg aatgaagatc agacacccaa 3600
caagccgaat atcggccctg actatgaagt gaacatcctt tctttattat cggaaactct 3660
ccttcccttg ttatcggcgg gcagattacc gatacaggaa aacccgcgca agggcacttt 3720
agtgccgatt ttaaggatca aaaaccggca cttttgagtt ttgttaacaa atcataacaa 3780
atgtgccgcc atgaggtgca ggtaggcgat tcgtcgggca gggagggaag cagcgtgcgc 3840
gtagtgccac cgcacatcat gagcccgggg ctgcgggtac gccgggggcg tttcgaccct 3900
gtgcgcgcgg ttgggacccc gcctcacctg cgcgcttcat tgagttgttt ttatgcaggt 3960
gcataagccg ctgttgaccg cctgtgacaa aggattgccc gcgccaaaca cagtagcaaa 4020
tgcatgcaaa tctatgcacc aagcctatgc aacaccggtc agcgggctgc tatgccgttg 4080
acaaggtata tgtccagcga acgtccctgt ccgggtgcgg gcctgcatag agcaccacca 4140
cgtatgtcag cccttgatgc gtgaaaactt ggtagtagag cctggccgct ccctgcgggg 4200
ttgtcttcct cccctggctc aaatgcgtgt tggacgtccc ccggtggata gtgtttgtgc 4260
cgtccagcac ctccagggtg atggtgatac caccagtctg ggcatgatcg aatcctcgag 4320
cgcggaagaa tgcatcgatc cgcccgccag ctccgtccat cgcaaattca accagcgcat 4380
caactgcact cctcaaaaag tcgtagcggt ggtctgcgac ggtatcgcaa gtggcaagca 4440
ctccagtggc aaaaataatc tgaggattcg cttcagcgac aaggcggaag tactccgacg 4500
gaggcggcgt cccttgctgt tggctgacag gcagctcttg ccgaaggcgc gcgtacgcgt 4560
cattgatgat cgtggtgtca ggcttctgct tctttcctaa gagcggccat ccgtcctgac 4620
cggcggcgac acgaataatg actcctccca tttccgcaac cgtccccagt tcaacctcta 4680
ggtcagccgg gatgggcgca tacggaagga caatcacatg ggcgtcagtg tagtggtcaa 4740
ggctgtcgat gtatgcacga aacgcataca cagcatcctt ttcaatcggc agaagtgtga 4800
gcgtaccctc cgctccgatg ccgaccagaa tgctccgccc ttcaatgcca ttacgtccgc 4860
agtcggcatc gaattttcgc tgaagcgact taaacagacc gatgacgaac actgcaggca 4920

ttctatgggc ccatccagta tcacagacgt gatctgacca agatggtgct caacagggtc 4980

ggcaaaacga ggcgggcaat atcctcgtcg ctcagcaact ccaagagctt caacgcttgc 5040

tgccgcgccg gaatctgact cgctgcggca cgcggtgcag tagggataga tgggccggcc 5100

aagtcaggca ctggagacgt gggtacgaac gcggctggct ctttggctgc cgcgtgtagc 5160

cggttcaatg caatgccatt tctttcagcc cactcgctga cgacctcaac gacttttgct 5220

gtacggtgtc ggcgccatcc ccaggcatag ttcccaagtg catccgtgaa tgccttattc 5280

actgtgctcg agtatgcctc agcagccaac ggcccgagcg cctccttttg cgccgctgga 5340

agtggcgctt cctgaagata ctctcgcatc cacgcaccct gctgatccct gtcgatcggc 5400

aaaatagcaa tgaaggaatc tggcgcggat tcgacttcgg cgcgctcccg actctgtcgt 5460

tcgcgcagat agtgcaccac cttatcagtg gccttcttga agtagcgcaa gcgatcttcg 5520

tctgggttgg caaatgcctg ccagatatcg ctccgaagca ccggcgtcgc cacaagcctc 5580

ggctgaggca acgcaggctc gggggtagcg cgggtagcgc cacgcaagga aactaagata 5640

tcgcccttac cctgggggcg atcgacactc acgagatcgc cgtggacgcg cagaaggtag 5700

tcagaaaacc tcttgaaccc tagagacttt tcatcgaact caccaaagcc gctcacggca 5760

aatgcccgtt tgagccgttg cttaaggcgt gcgccaagtt cgggggattg ttcggaaaga 5820

agctgcgtaa gcagttgtgt ggacgcagcg tgcgaatcgg acatagcggt ctcagcaggg 5880

tcagaggcaa aaggagatag cccgccacag agtgtggcgg gtatttccaa tactgaaatc 5940

atcaattgct tctgagttcc ctgacttgaa gccacgtcgt ctaaccggca gaactcggca 6000

cccggtagca catcggcgct gctcaagctt tcaagatggc cctggatact ttgatccggc 6060

gaatctcgaa atacttccaa ggcggaaggt tctggaattc tactgccggt cgaccggttg 6120

tcaagcaggc ctcaactcgt cacaacgacc agcgcacacc ttgcacgcca caattatgac 6180

tcatagagca ctttatgtga tgtactacag cgtagctaga cttcaccctg cgtagccgtg 6240

atttgcattg gatgaccttg ttcgacctta gttttgcagt ttgtgagttg cacaaggtct 6300

tgatttatga aaggtactga gtcatcccaa aaactccgaa agcaggtatc gccgcgaccg 6360

ccctcgacga tagcgcagat aggacaggag gcagtttggc gctcggtcca gcatggtcgg 6420

gttggagcaa cctagactct ggccgccccc cctgatcgtg gcaaatagcc agccttccgt 6480

tgctttggca caacgtccga atggccacgt ggcactactt ttgagaaggc cgaaaaaccg 6540

ggggcatcaa tgggggcatc aaccccaccc acccagaaca aatccgttta taaacaacca 6600

gctatagttc gtatgtgata cctgtcggcg gaccaccgga tacgacaaaa agccaggcaa 6660

ccgcctagct tttccgtttc cgcagcagca taacaacgag gcccgccatc catgcgcggg 6720

cctatggctg cggatgcgcc ggggcctcct t 6751

<210> 8
<211> 11740
<212> DNA
<213> Ralstonia solanacearum

<220>
<221> misc_feature
<222> (5055)..(5154)
<223> n is a, c, g, or t

<400> 8
   tccgacaggc tcttgagcac atacaccgcc gcccgcgaca gcaatgcgcc gttcacctcg 60
   aacgacgggt tctcggtggt cgcgccgatg aaggtgaaca ggccgctttc cacgtgcggc 120
   aggaaggcat cctgctggct cttgttgaag acctgtacga tgcaatgcac ctgttgaaaa 180
   tcaataggtt ggcgtgcatg catccggatt tttcgcacaa acaacccgtt ttcatgcaca 240
   attttttgca ccaacgattt tgattgtgca atggatcggc cccttggctt gtgccggtcc 300
   ccaaggacct cactgaacaa gcgccccctt ggcctgcaca ctgccccaaa gttaggtgct 360
   tgagactcaa aaaaacagga aggagtcact gtgtcggtct acccaatcaa gtacaaggtt 420
   ctgaacaagg agactggccg caaggtcagc gtcaccaggt ggcgtgttca gatccgcaaa 480
   ggtggcgaga gcatcagcaa gctctttgat gatgaaagca ccgccaagtc ttgggaagaa 540
   caagaacttc accgcatcaa cactggtgtt cctcagaatc tcatttttga tatgaaatat 600
   caacttgaga tgcccgacat gcgcaggctt ctcaaggact actttgatca atacatgagc 660
   aagcaggcag cttcttcact gaagacgaac cagaaccgtt gcctgagtgc cattccctca 720
   atccctatct accttaaaga tttgggtcca aagattgaca actatcgtta taaaagcacg 780
   attgttgatg ccatgatggg caggaccttc aacaaagacc acgttgaatt tggtaacttc 840
   aaaattgata cggtagattt ctggctcttg attgcctaca tggattctcg taggaaatct 900
   ggcatcaagg acaacaccat ccttcgtgaa atctccacta tcagttctgc ctttgagaag 960
   gtctacaagc tctatccaga acaattcccc aacagcatca tgaacccagt caagatgctt 1020
   cctaaaggtg aaaagcctaa agcctatctt ggcaggaaac gggttctctc tgatgatgaa 1080
   gctgttcaga ttgcagaatg gatgaagctt aaagccaatc aggagcctta ctacctgttc 1140
   gttacttgct tggagtctgg agcacggaaa agtgaagtgc tgggtgctca gtgggaaaac 1200
   gtcaatctta agctttggtc tatccacatt ccgaaaacca agaatggcaa gccaagggac 1260
   atcatgattc ctgaagatga tgatttccgc gaatggttga aggccaatag gcttgctaaa 1320
   ggacctatct ttaaactgac agcttggaac ttccgtcaat actgggtgga tgctttgaag 1380
   gcacttggtc tttatgatga tccagacaca agacttcact tccatgacac ccgccgcact 1440
   gcattgacca agctcattcg tcagaagcga tctaacaact ttcagattgc caaagagatg 1500
   ggagtgtcgc ctcagactgt tgagcagacc atccagaaca tgcctgatag gttgtcagaa 1560
   gtgtttgcga agcttcgttc tggtcaggca ctcaatgaag aagaaatcat gctgctggct 1620
   ggtcacagtt cttcatccat gaccaacgcc tactttggcg acaggaacta acaagcagca 1680
   agggataaaa acggatagaa atgacagata aaataatcat ttccaatgaa gagaaaatgg 1740
   ctgatgttca gcttgaaatt ctcttctctg gcccattgag ccttcttgat cctagacatc 1800
   cagcaatcag tgaagagctt cagaagcaga ttctgtatga caggtaccac aacaaactga 1860
   ttgcagatgg cctggaaaca ggccattttt tctacgatta caacaacgat ttaaggtttc 1920
   aaaccaagca accgctctat acctttgcag ggttcatctt tgaagccttt gccgtcaatg 1980
   ttttcaataa caacatgaga accattggca agaaggcttt ttcttggtgt acaaaccgag 2040
   agcagtgcaa ggacgattac attgatcaat tcaaggtaat tggaactgga ttcattacaa 2100
   caaagtcact gtttccgaac ttctacgggc cacaaagcac ttttgacctc atttttatca 2160
   agaaaaatat caggcaagat gtgcatgagc cagcaaccgt tttgggaaca acaaaccaag 2220
   ctggaattca aatcaaagcc atcactggct cagaagaaac tgaaataatt gagccattaa 2280
   gaactggcag atactcccat gttctgactt ttcttcgtca ctcagatgga gtgcatagct 2340
   acacccattg catggaagtt ttaaactcca tgcatagaag aggtgttttg gggctgccag 2400
   aaaaatatgc ccttgagcga cgtatcagcc acccagaaat gtttgggatc gaccaacgtg 2460
   aggttgatga ttactaccaa tacatacttc attggtacac aggtcatgcc aatcccgatc 2520
   aatttattac tgaaggtgct ggtttggaaa tcaaaggcta taaatattca aacggcttgc 2580
   ttgtgccaac cgattaagtt caaccgtttt acacccaacc taaaatgcta gttctagcga 2640
   ctagcttttt catattgaga aacgggatca acatagatac aactacccag ttcttgaagt 2700
   aaaaaatatt caagaggata gttgacgcac cttgttcttg cacatagagt acagtcatca 2760
   acaagcaaga agaactgtag aagcttgttg agtgtgttat ccttcaagta tccatagttc 2820
   tatctttttt aacggagaat ttttcatggc cgaaaccaag aagctgaccc ttgctgagat 2880
   ccttgccaag cgcaaagagt ttgatgacat ggtggcggct cagttggaag cggaacgtcc 2940
   cgctgctctg gcagccatgc tggaacaaat caagatgttc aacttcacgg ctgatgaact 3000
   tggtttccat gctgctcctg atgcacgtgc cgctgctggt ggtgccaagg ccggtgctac 3060
   ccgcaacatg agcaagccgc tgaagtccac caagaccgac gaaaccagtg tgtggctggc 3120
   tcacccgccc aagttcttgg aagctgaagg tgctttcacc acctacaaga gcggcaaacc 3180
   cgttgatgca tggctggtga ctcccaccga caagaaggcc aagaccaact tcctgaagaa 3240
   gctggcaagc cgtgaaggca aggttcccac caaggaacaa ctgggtgaaa tcaccgaggc 3300
   agaattcaag gcagcctaag ctatagcagc ttaagataaa agcacccttc ggggtgcttt 3360
   ttgccgttga cgattaagaa tattgagtga agtgacgtct tatgatcatt gcccaagtag 3420
   aagaaaaagt taaagccctt gtgggcaata ttgataaggc tgaatttctc tatggccttt 3480
   tggaatgcta tagcaagcct aaagcctcca ttactcgcct gaaaatggtt ggcaaaggta 3540
   gctataacct ctccaagaac gaaaatgaag ttttatggaa gaagcaggtc tacttcaaag 3600
   ccactgacag cgatcagctt ctatccgtca tcgatgaaat gaagaatgca gaggtggtta 3660
   caaaacacca acctcgcttc attattgcca tcaatgatac tgacctcttg gcgattgata 3720
   ccaagacggc agacacgctt gatacatctc ttgccgaact gaataagaag tttgactttt 3780
   tcctgccttg ggcaaatatg gaaaaagccc agtttcagcc tgaaagtgct gctgatgtta 3840
   aagcagccga aaaaatggcg aagctgtttg accttctccg agataacaac ccggcaacca 3900
   ctgcacaaga aattcactgg cagaacgtct ttctgtccag aatcttgttc tgctactttg 3960
   cagaagacac aggcatcttt gaccccaagc tgtttaccaa ccacatagcc tcacacacgg 4020
   ctgctgacgg atcagacttg gacgactact tgagtcgtgc ttttgatgtc atgaaccagg 4080
   aagaccgtaa tggtgttcct gactatcttg cagacttccc ttatgtgaat ggtggtttgt 4140
   tcgctgacaa gctgcctgtt ccgaaattca atcggaagtc tcgtgccatg ctcattgagt 4200
   gtggggccga tctggactgg tctgaaatca atcctgacat cttcggctcc atgattcagg 4260
   ccgtagttga ccccagccaa cggggcaaca tgggcatgca ctacacctcc gtgcagaaca 4320
   tcatgaaggt ggttgaacct ctcttcctca acgaattgaa ggaagagttt gaaaagcact 4380
   ctgctagcaa gaccaagctg gaacaactgc ttcttcgctt ggaacacctg aagatttttg 4440
   accctgcttg cggatcaggc aacttcctca tcattgcata caaagaactt cgccagcttg 4500
   aaatggaaat tttcaagcga attcaggaac tgacacctga aggaattttc ccgctttcca 4560
   gaatcaagct ctcccagttc tacggcattg aattggacga tttcgctcat gagatcgcca 4620
   tcctttctct gtggctggca gaacaccaaa tgaaccaaaa gttcaaagag acttttggac 4680
   ataccaatcc agcccttcct ttgaagagca gcggcaacgt cgtcagcgat aacgcaatcc 4740
   atgtcgattg ggacgagttt cttgctccca aagcagagca tgagacatac cttcttggga 4800
   atccacccta ccttggagca cgtaaccagg aaccagagca gaaagcagac cttgaactga 4860
   tttttgaagg ccacgaggaa tacaaggact cagactatgt ctgctgctgg ttcatcaagg 4920
   ccgcaaagtt cattgctggc aaaaatgcca agtatgcgtt cgtggcgacc aactcaatct 4980
   gccaaggtga gcaggttgcc tacctatggc cggtggaagt ttggtcaatt cccgcgtcat 5040
   gctatctacg aatcnnnnnn nnnnnnnnnn nnnnnnnnnn nnnnnnnnnn nnnnnnnnnn 5100
   nnnnnnnnnn nnnnnnnnnn nnnnnnnnnn nnnnnnnnnn nnnnnnnnnn nnnnccaact 5160
   ggtagtgcgc ggcacttcgc acatgccgcg caaaagagtg tcacggcaat aaaaaaagca 5220
   cccgaaggtg ctttttcttt aatcggtagc gatcctacgt ccgcctgtag tagccaacaa 5280
   tctcttccct cacggcctca gcaagctcca gcttcatatg acctgtcagc ttcatttgct 5340
   gacccagtgc atcggcgcaa acataggcag tttggacata tccattggca gaaccactaa 5400
   ctgattctgt aacaacagag aatttcttaa tgtcagagcg agcaagttgc ttcttgcccc 5460
   caccaaactg aaccccatca ggaagaagac ggatgcggta tttacgacct gcaaacacaa 5520
   acttcaagag tagcaacgga gcagccaatg cgaccagtgc cgcctttccc atgtgattct 5580
   caatgaatcc aatggaaatg tattcaccga gaaaaaccag accgatccaa gccgccacat 5640
   agataaacag cagaatcacg gtcagcccta ctggattaga tgcggctccg aatgcgcctg 5700
   cacggctacc tttgaccaca acttcagccg agccatcaga ttgctgagtc acctggtatt 5760
   gcttcacaaa cgagcttttc atatgtaggg gatcaatcat gttcaaggtt gttctggtaa 5820
   gtcgctggca tcaacaagct gtttttccag ctactcatcc tcaacccgac ctttcgggtt 5880
   gatactcaac tctaacgcat gttcatcttt ttgggttgac aggcaactat ttgggttgtc 5940
   aaagatcgtg gagagaacat gccgccagag aacaaccaac acggagaact attcaagagc 6000
   attgggcgca cgttggccca gcggcgggag gctaagggcc tcacccagga tcaggtggct 6060
   gaagcccttc acatcggcac agaggctgta tcccgcatgg agcgaggcat caccatgcct 6120
   accgtccaac gcctggcaga gcttgccgag gtgtacgagt gcggcatcga tgagctactg 6180
   atagcaagta gcaccaggac cggcgaccag gccgaactga tctctcagat cctccacacg 6240
   cttcccgaag cagacagagc catgatcgtt gaggtggtgc agcggattgc cgcacgtttg 6300
   aaggatcggc tttaaatcta agaaggctat ggatttatgg acgatatgcg ttgcctaacg 6360
   gcatgcttgc cgtggacaac tcttacgagt tccccaccgc acataccttc gcccacaagc 6420
   tccacatcct gcgatcactt tttttgattg atctttcttc catgaagcca aacaaagaag 6480
   ccgcagagca acaagaagct tgtcgctcat acttacgatc tttttcttga tttttatgat 6540
   gtgatgcgca aaagcttgat gttgtcagag atctgcttat agtgtacgta tcgattggag 6600
   aaatcatgcc ccctcacctc gaaccaggag catcacaaag cagcatcgcc catgactcag 6660
   aacaacaagt tctgtctcac tggattgaag gtcaaccagc tttcaaggaa gtcctctcca 6720
   agttgcccga cacggatcaa tgtttctccc ccaaccagat tgatgccatc cttgccattc 6780
   ggcgcaggat cacagaagcg aagcacgccc atgactgagc agtcaccctt gccaaccgac 6840
   gaggatattt gggaagaact tttgcactcg gaagccagca agcagttctt agataagctg 6900
   atcaaaaaag tccaagagtt tgtagagcaa caaacgcatt gaagcacctt cgggcgcctt 6960
   tttattgcct accatcttgg cttgactccc tttgacgtac taccttcttc aaagaacttc 7020
   ttcctgattg ccaatattcg attctcaacg gctttcacag gtttgtcgta gacacttctc 7080
   acctcgacca ccttcttctt gttgaaagaa ggatgttggc ggttgaattc ttcctctgag 7140
   agcttgatgg cacgaagatg ccactctcta gtgccgtctg agccttcaac ggcagggccg 7200
   tcttctctgt gtagctggcc tttctgatac cacgccgttg caccactggc agcttctact 7260
   gccggaccat ctgtgcggtg cagcttgcct ctgagatacc aagagcaact gccatcggca 7320
   ttgagaacag cgggaccgtc ttcgcggtgc ctcgcaccat caatccacca ctcctgactt 7380
   ccgtctcccc agataactgc tgggccatcg gtgcggtgaa gttttccgtg gatataccag 7440
   gactgggttc catctggctc aacacaagcc gggccgtctt cacgatgtag gtgatcgttg 7500
   aagtaccaaa cgtctatgcc gtcctcttgg tagcgtccgt tttcatgtta cgcacctccc 7560
   ccagtctatt gattctcaat ctagcaagat caatcttaaa aacaagagtc gtaagttatc 7620
   gactatggag cactttcggg tgcttttttt acgaccctac atgcctagta ggaccaccac 7680
   ccataaggag tggccctttc ttctttacag gcgtacagtt cgttctattg attgatcgac 7740
   attaattaat caataaatca actaacaaaa tggagaattt tatgaaaaaa gaaaacaacg 7800
   gccaagcacg aataatagaa acaaacatag gtcacttcca atactgcgac tttgcaatca 7860
   aggtcatatg agttgtcaag aagtgattct ttctgactct agcacctgaa tatcaggtgc 7920
   atttatttga atttaattta gaacacccac ttcataccta ccaagtgaat gactcacatg 7980
   gaaaggaggg ttctaacaat gacttaacta caaggatatt tattatgaaa acttttatcg 8040
   cggttccaaa tggcgtgttt cctcttaaag aagttcttgc tgcacctacc accaagatca 8100
   tcatcaaaaa tcttagtgct gatccacgta cccacggtag gaagttgagg attttccttt 8160
   ccttctatca gacctttgac atggttgccc agcttcaaga ccaaggtgtt gatccttgca 8220
   cagtggatgt ggaagacatt gccacggctg cttcatttct gattgaaaag ggctttgaca 8280
   agattattga aacctttgaa ccagaagacc gtgccttgct ggattctttg tttcaagaaa 8340
   gtgaggtgag ccatgtctaa gacagaaacc acaccagaac ttgatcttcc aggcattcca 8400
   tttggcattc tgactttgga acagttcatt gcttcttctg aaggtcagga agtcattcag 8460
   accatgaaga ctatgagcag tgacccatat tttcttatgg tgccttttct tacctcatat 8520
   caatactatg acatgctggc acaattgaat gacagggcta ttccaatgaa ggaagaacga 8580
   gagaatgatg aagttctctt gaatgttgca acaaggtatg tggttgaaca tcttccaaaa 8640
   atcgttattg atattggtca agagaaaatg aaaagtctga ttaagatgct ttcaccagag 8700
   ccacgccagt gagaaggagg ctgcatcgtc agcctgccag gtgattcaaa aagactgtat 8760
   tgagcaatag agcacccttg aggtgctttt tgcattcttg atttccctcc tggtctattg 8820
   acatagtttt tttattttta tatctcccac ataaacacaa cattttttag gagataacta 8880
   tgtccaatga caacaaaaaa gaacaatcag tagagagcca tccacttgat atgctttatc 8940
   attccttcca tgatagaacc aatgaagatg aatattttct tgttgtgatt gctccagaaa 9000
   aaccattgca acaaaccgtt caaatcgatt taaaaacttt catttcaaac ttaaatttag 9060
   gatataaaga acaaaagata gatattttaa aaattgaaat ttcagacact tcttgcgtgg 9120
   tatatttaga tccgaaaagt gaatttatag acatgattga aaaaagaata accggagcca 9180
   ctgtttatgg tgcattgcat atactagcca tgaagatttg ttattttttg agtgaacatg 9240
   gacatgagaa atattacaat attgatttgg gctataaaac aaaaagaaag aaccccacca 9300
   tcttcttaca agatttggtc gtaatggact agaagataaa taaaccaaag caccttcggg 9360
   tgcttttttg ttgccccaac aaaaactact ccaccctacg tgcctagtag gaccacttga 9420
   ttcccagtcc tgtacctttt tcttcaaaaa gcgcaaacta cgtttattga ttgaagacca 9480
   gaagaaatca gtcaataact acagcgatca actatttagc gactacccac ttaacaccta 9540
   ccagtgaaat ggttcatctg gaaaggaggg attaaatatg acttaaaaca caaaaggaat 9600
   tcatcatgaa agaacaaaaa tattttgaag gtgttgccag caactacttc agcaacgtgg 9660
   atggacaggc tggccttgcc gcagtccctt ccaacttcgt tgacttggta ctgaccgacc 9720
   caccctatgg cattgcagac ggtgccaaac tcaccaagaa gggcagcaag atcgtcacca 9780
   ctcagcaggc atggggtagt gactttaagg atagctgggc gaccattgat cagtattacc 9840
   agtggctcaa gcccttcatt gctgaattcg ttcgggtgat gaaggacaac ggctccatga 9900
   ttctcttctt ggatcgcaag tacaccgggt tgattgccca ctacttggaa cgagactttg 9960
   acttgaattt caagaacaag gtctacttca agaagaagaa ccctgttccc agcattcgca 10020
   agaacaacta ccgcagcacc attgaagagg ctgtttggtt caccaaggga aaggagtaca 10080
   ccttcaactt cggtgcccag agcgacatgc ttcaggtcta tgagggtccc attgggaaga 10140
   agaagaccca gcaccctacc gaaaagtaca catggatggt ggagcctttg atcaggaacc 10200
   acagcaagcc cggtgacgtg gtgttggacg cctttgcagg ctctgcaacc accctcgtag 10260
   ttgcgaagca gcaggatcgg cacgccattg gctttgagaa gagcaccgcc ctgtatgaga 10320
   tggccaaggc ccgcatcgag gcagagcagc ttgagtttgc attccatgca ccatgtgacg 10380
   aagcagccat cgtgcgcgag ctggatcagc tcatggacgc cgagcttgca gacatctcca 10440
   gcagccgcag cagtgccgcg gatctggctg acctggtcac agcgtaagga gggcagcagc 10500
   catgaaagaa atgaactgcc tccaatactg catcaacggc atgagtgacc gattgttcag 10560
   cttcgctaag accaaggaag gcaaggcact gcttgctgcc ttcaagaagc tcatcgtcat 10620
   tcgtgagaac cagatcaaag agcttctgat tgcttacaac agctacttca tggtggctgc 10680
   tgccatgcag ctcaagggca tgcctcagca cccacgggcc atgattgagt tcatggcatc 10740
   agaggaattc agtgccctgc atgctgaact ggtgaagacc gttgaggaca actacccctt 10800
   gctcatgtcc tgcttggata ggaagcagaa gcgaaaactt gattccctct ttgaataaac 10860
   aaaagcaccc tacgcggtgc ttttttgttg gcatcgactc aatccgcgag atttacacca 10920
   acaagaaaga gaataggcac aagcctactc tctgagcaga aaaaaacacc ctgtggggtg 10980
   atttttattt acgaacacaa tctatcttga tgaccttcct gctcttcaga atttcatcaa 11040
   cttcaggtgt cagtgttttg tgaagctcaa gcatggctgc tgacaccatg cagccttcaa 11100
   accaatactc cttcacttca cctttgacga ctgctgggcc atcttcacgg tgcagcagcc 11160
   cgtgacgata ccaagcagca tagtttctac ccacgaccgc aggcccatct tcacggtgca 11220
   gcacaccgtt cttgacccat tgcttcactt ggttgtctac aacacattgc agcttggtct 11280
   tcatttcagt cggcaataga taacgagatt tttcattcta gcgtgctttt tgttgacttc 11340
   catttatcat ctgcaagaat aaaaaaaagg agacaccatg aagaggtcat caattccaat 11400
   attaaccgca atgcttatgt cattaatgac agtccaatca tttgcaggaa ctacagacac 11460
   cgccacaaaa accacggcaa cacttgccgc cacttgcact atttcagcaa gttcaattgg 11520
   ttttggaatg cttatgcttc ctttaactac tcaaagtgca aacagcacca tgaatgtgtt 11580
   atgcaataaa ggagccccat acaaaattga tttggcttat ggtggagttt atggaggcgg 11640
   tagcaattct tatatgaact actcagcctg ggagagcggt agttatgtta atgtctacaa 11700
   tttttatatt acaaaacgga tgcgccccgt cgctttgacg 11740

<210> 9
<211> 27090
<212> DNA
<213> Ralstonia solanacearum

<220>
<221> misc_feature
<222> (11955)..(12104)
<223> n is a, c, g, or t

<220>
<221> misc_feature
<222> (22351)..(22367)
<223> n is a, c, g, or t

<400> 9
cgcacagcat gatgccgatc tcgtcgaaca gctttcgcgt caagtcgtgt tcgttgagca 60

ggcgtcggaa cttcaacagc gtggtcgcat ccggcacgtc ctcgaccgcc agatcgatgc 120
cggcgaaggc tcgcatcgcc atactgtcgt gcagcgcgtc ttccagacct tcgtccgaca 180
gcccgtacca ctgctgcagg aagtagatcc gcagcatcct ctccagacca atcggcgggc 240
gacctcgctc gcctctcgga tagtggggcc caatcgccga cagcaagcgc tgccacggca 300
cgaccttctc catctctgcc aggaagcgct ggcgtttggt cacgcgcttc ttacctgcgc 360
tttccgcttc tgcaaagctc atctgccgct tcatcactat ggctccgttc cgtgagctac 420
ctgctacaac ggcctcagct gcgtcagcga tgacggctga gactgataaa tcagcgtttc 480
cctagcggct gagttgagct gaccgatcag gccagcatac ttctgccgca gtgtgtcgcg 540
gaaagcaacc caatccacgt tgacctgccg cacctgggca gcgtcatgcg acagaaaaag 600
ccattgccct tcgtcactca ggcaccacag cggcacggcc cagtcgggat cacgccactg 660
cgcagcaatc gcagagccca ggaggttgcg ctggtctgtt tccgacgagg gatacgtatg 720
aacggcgccc agcgctgagg aggcgaagcc tgccacgatg gatgcctcgc acgcggcttt 780
gatggcggcg agcttcgccg ccatcacctg cgcgagggtt tgagaaggga atggcgccgc 840
cgagttcccg ttttccaccc atgccaggta cgcgtgatag tccacattcc tggcatcctg 900
cggaatgatt gcgccgtcct cctcacggag gatggcgcca ttgatccatt gcttgtatcc 960
catgacttac agcctcgcat tcaggatgta gttaacccgg caccatgttc cgccgtacac 1020
gccccaagtt gcatccatat cgatcgtgga tgcgctgcac ccgatgccgc cgctcgtcat 1080
cccctggttg tggattgtcg cgccgttgta gacggtgtac gccccagaat tgccagccat 1140
atccttaaac gtcatcgatg gccccgcgcg catttgaacc ggaagagtct ttgtcccctg 1200
caaactggtc gagttgacag cgattgctgc actcccactg ccacttgcga ttggcgcgcc 1260
gccgcagtgg aatgcatccg cctggtagta gcgctggcac gccaccagct caattggaat 1320
gggccgaaac tccacggccg ttgcggtagc tccttcttcc aggctccact cggtgaacca 1380
gaaatctttg gccacgacag cgccacacgt agcgtccacc ccgatctcca ggccgttcga 1440
gcagtcaccg agcgccactg tgaacgtcag taactgcgac gttccggacg ccacagccac 1500
cgtcttgctg ttgattgcgt tgaccgatgc gaaattgtcg gctgccgtgg gcgttctcag 1560
taccaccgtg tagttgatgg ccgcgccaac ggtgtgccgc actttaatct ggaacgtaac 1620
ggtctgattc ttgaggtttg ctgcgtcagt agcctccatg cggtaacgcc atgaaagttg 1680
ccccgccccg gtgagcgtgc acccctgaaa gcggacggcc ttgccgctgc ggcccacagg 1740
ggccgccgtg tcctgcacta gcgtccctgc cgtaatcgcg ccaccagaag cccagccagc 1800
aatcatctca accggcccat actgaggcga cgtagaaagc gacggcgatg tcgcctgaat 1860
gacaacctgc gccccgctgt tgatgagctt gttgcggtac gacgtggagc ccgcagagcc 1920

ggtagcggcc tgcaatagcc ccacaatcgc ttggcgaaac tgggtgttat cagccttgtt 1980

cagcacaagg ccgcaccact cgatgaacga cgccacttcc tcttggatgg cgttcatgaa 2040

cggtgccgta atctcagtcg gcggccggtt ggtcccggcg tcttccgcca cccacgtgcc 2100

gttgacgttt ccggccccat caatccggtg catagttacc tccaactccc gtaatgtctt 2160

caaaccaaag aaacacgtgc gcttgctgga atgccgccag ggcttgccag agcgcagccg 2220

gatcgaccac cgagcggtag tagcgcacgc gcagcacgta gcgggatcgg gtgccccata 2280

ggcggttgcc aatgtggctg cccacacgcg caggccccac caggtggtcc acctgcacca 2340

gatcaatggg gaacatcagc gttggcgcgt catggctcca caaccggttg ccgaccgtgc 2400

tgccaatggc ggctatccgg cgcggcatcg gctcgcccac gccggcaatg gcctcggccg 2460

caacgcggcg gtactcgctg atgtgccagc ttccgaactt gggccggtgc agctcaatgg 2520

cggcgtccag aaccgcttgc gtgtcggtgc ccaggcgctc cagctcggcc gcagtgcccg 2580

ccagcatggc gtcgcccatg ccgccgtccg gccattccca cgcggcgcca ggcggcaaca 2640

gcgccttgag cgcgtcggtg tagtcgcgtg tggtgtgcgc cgtgagcttc atgcccacac 2700

cacaggattc atcaccagca cttcgcccgc tgcgacggcg atatcggcca gcggcgcgag 2760

gcgggtgtat tgggtggtga cggtggcaac ggcagcatcc acctcggcca tggccagcac 2820

cgatgtttcg ctgacctcgg ccagcacggc ggccatcagc gccgaagaca cggcggtgcg 2880

gttggcggcg gtgtcactgc cggccagcag atgaatagac gggttcacgt cgtgccgcag 2940

tggtgccacc gcgcgccaat ccgccgtggc gggggcaatg tcttgcagat gcgcagctac 3000

tgcggccagt acggcagcag tcggcatgcg gtcggccagc gtgttgcaga tcggccggac 3060

gatgattgtt cccatgccca gcacgtggcg ctgaacgagg gccgtgctaa cggatgggtg 3120

ggcgcttctg gcccaaaacc tgtagtcgtc gtccttgccg gaacgagcgc cacgggtggt 3180

gatggtggtc cactcgtcgg ccacccgtgc gcgccaatcg tccacgctct cgtcttcagc 3240

gccgcccgtg attcccgcgt cgcctaccgt caaggtgcca ttgacgccag gaatcgggtc 3300

caccaccgtg agtgccagac ccgcagccag gttgctggcc aggccggtgg tggtgcagcg 3360

aatggaaaca ggggtagtgg ggccagcgcc gatatcgact gccgccgcca caatgtaatc 3420

cagcccgttg gggccgcgca gcggcgtgcc agcaaggatc ggcatgcctg ccacaccggt 3480

ggccagcgcg ttgcccgtgc tagcggtggc catgaggcgg tcaacaccgt agagcgccgc 3540

ccagtcgtag aggcgttcca gctcgcacgt gagcggcgag cactgggcat gaagccattc 3600

gagatggcca tgcatgccgt gcgcggcacg tgcccacatg gcagccaggg gaccacgcag 3660

gaccgcaggc attgcagcca gatcggcggc aatgcgtgct ttcaggtcgg tataaagcgg 3720

tcgaacgtag gcggtcacaa aggggctcgc acaatgaact ttcgtccatt gtgaaagccg 3780

ctgacctcta gaactacgct ggaaacgttt cctgccgcct cggtggtgac ctcttccacg 3840

gtcacgtctg tgagggcggg cgcctgggct gtaaatgccc gacgcaccat gttgatagct 3900

tcgcggctgg cgacggacgt accaaaggcc ggttccggcc tcggtatctt cataccagcc 3960

gcgccggtcg taggtgtctg ccacgcggcc ggcgggcgcc tctacgtccg tgaacaacac 4020

ggcgtagacc aacgtggccg ccgcggcatc cgcgtcgccc agcgccgggt catcaagcgc 4080

cacgtcgaac ttcccccagt cgctttgaat gagcttcagc atgtcaattc acgtccgaag 4140

tgggcgtgcc aggggccgtg ctcgtgtggg tgtgcttgtc gctcacgtcc ttaccattca 4200

ccctgagttc tccggtcaca tcagcaccgc ccttcatctg cgcagcgccg ccgccgtcgc 4260

cgaagtagcc acccaccgag ctggtttggc ccgccactgt aaggttaccg ccgatcttgg 4320

cgtctttcgt cgtctcgaac atgggcgtat cggccagcac cttcgtgctc gccttgacct 4380

cgatgatgcc gccgcgcttg atgtgcaccc agttctgttc gtcgtcgtga atggcgattt 4440

cgccctccac cagctccatt tggtagcgct tgtccccgat gatgagcgcc acgccatagg 4500

agcggtcacc agacgggaaa gccaggtacg tttggcagcc ggccttcggc cggtaggaaa 4560

agccataggg ctccacacgg ttgatgttgt ccaacgtctc ttcgtccagc acgcgcacct 4620

gcaccttgct cgcgccgact acaaggccaa cgccctgcgc gaacagcaac tggagccgtg 4680

cccaaacctg ttgcccgatc atttcttgtg ccccttgccg cgcacgcctg cgccgcgctt 4740

ggatttcttc ttttcttcgc ccaggaacgc accccggttc atcacctgta gcagcgtgac 4800

gcggccgtgc ttgtcatcca tacggaatgt ccgttcaccg atcaggaaca caccatcaat 4860

ttcttcttcc gggatgatga cgcgcacctg ggtgttgagt gcccacgggt gccacttgcc 4920

gtcggtgtcc tgatagcgcc agcctggcac ctccagttcg atgcggtggg cacgcgccag 4980

gcgccggttc cgctccagga gtgcgcgccg ctcgcagccg cccaggccgt tgctgtgtcg 5040

gtccgccaca acgtgcatgg gccggaaata cgtgatgcca ttgtccttca cggcaccctt 5100

gagtgcggaa tcactgctgt agtcgaacgt cttcacaacg tagtccgaaa accgcagctt 5160

gtattcgtcg atgacctcgt agaccttgaa gtgcatgccg tattgcagcg tggccacggg 5220

cgcagcgctg gtaggcgacg tgagcaccag gccaccgtcc ggcaacggat acagcagcag 5280

gtttgccgcc cgcacagcgt tgatgagcgc gttggcaggc acctcgcact gcatcgaaaa 5340

actgggcacc acgggcgttt cagcgtcgat cttcacagac actttgaacg tggcgcaaag 5400

gcgcttcacg atctcgctca gtttcaggcc ggatagcgtc ttggaatact ggcagtccac 5460

aagctcacga ccaagcgacc gggcgtgaaa gttgattgtg tggctggtgg gatcgacgcg 5520

gcggcggttg acatccggtc gcaccttcgt caccagctcg tcgccgatca gcacgtcgat 5580

gacggtgttc gccgtcatgc ccatgccgct gccgccgttc gcgccgctgc ccggccgcgc 5640
gatggcgagc tgtacggcgg cgcacagatc gtccaccgac tcccgaattt ccaccttctg 5700
ccagtagccg tagcgcgtgc cgtcaaagcg gatttccgcc aatgcctcat ccatacacac 5760
gccccttcac aaacagcgga tggcgcgccg cattgcgcgc caggaacacc gattcatcca 5820
cgcccagccg gtgcgcaagc acgatggccg gcagcgggtt ggtcacgtcg cgcaacgcgg 5880
tcggcttcag gtcttgggcc agcagcgctt caatgaccgc cgcccgcgcg gccacggccg 5940
cgtcaaacac agggtcgggc atgctgggca gcagcgcatc cagggcagtc accacggcgc 6000
ccagggctgc atctctgtct gcctcggcgc ggtagtccgc catggcgatt tcgatggcgg 6060
ccgtcaccag cagccggccg cgcaacgctt cttcgcgcag cagattgcga cgcacggcac 6120
catccgttga ggcaacacca gacagcgcca gcggcgcagc cgtggtggcc acggatgcga 6180
tgcgagccgc cacacgcacg cgcgacgtat ccgggtagtc cgtcccatcg gcaccaccgc 6240
ccaggctgtt ggtgaggctg cgcaatgcgt tggaatacgc gctgggtatg gccatcagcg 6300
tggccagatc gcccttggcc ccggcaatca ggttcatgac ctggctcgac caggtgagcg 6360
gcagcgtggc cagggaaatg acctggcgca gcgcttcgag ccgctgactc acggccgcaa 6420
cgaatgcagt catgccgtca aagctcatcg actccagcgt gaagtcgtcc atggcggcgt 6480
cggccaagct gctgttgcgg tcataggcca cgtccaccgc atcggtttcc ccagcggcta 6540
ccgtgccgcc gcccggcacg aagtcaatcg tgatggagca atagccgccc ttgtcgttgc 6600
tctcgtgaac agaccaatca tgtgcacgga cccacttgtc acccagccac gggtgcgtca 6660
gccaatcggc gcccggctgg ttgagcagtt ccaagaacgc atcgcgctcc agatcgtagt 6720
ccggcccgat gaaataggcg ttcagcttcc agtcccacgc cttcgcgccc aggtcttcca 6780
cctcgggctc ttccgcgccc ggcagctcgt gcacagcgag gcgacggccg ccgcgtgctt 6840
cgtggctctc ggtcagaaaa tcgacgccgc gaaagcttgc gccgaccaaa cggtcttccc 6900
agctcatcag ggcgctccgt tccaaacatt accggtgttc atctgcacgt tgccgcccgt 6960
ggcctgcaca gactggcttt gcagcacaag gccgggggca aggcccactg tcatccgggc 7020
gttcacgtcc accggcttct gctcggctgg cttcagcagg tctttgagac cttcccaggc 7080
taagcccaga ccgccaccga cagcggcacc aacaccggtg cctaggacgg gcaccacgct 7140
acccaccatg gcgcccatag cagcgccatt gagcgcactg gagccgtagc gggaaatagc 7200
cgactcttca ccaaaagctt tctcaagggc gtagtcaccc accagagcgc ccacgccagc 7260
gatgccgccg accttgccca ttttcatcgc gccccggccg accgcgctac ctgtcgccca 7320
agcagcggca ttggtgatgg cctgtccgcc tggcagacct ttcccgccca tcgcaatcga 7380

tgccaggcca gccacacccg ccagtgcgcc gagtgcggcc gtggcgagcg tggtagtgcc 7440

gaccagaagc ggatgcttct tcgccagatc gccaaaggct tcagctgcct tgccaattgt 7500

acgtagaagg ttgtccatgg cggacttttg cgccacggcg gcatcttcct tggcctgctc 7560

catcttgaag tcggccgtat ctgcaatgcc cttgaaggag gtggcgattt ccgagcgctc 7620

gggggccaag ccatactggc cccgcacctt gcccaacacg tctttggtgt attcctgatt 7680

gttcatcacg cccaggaacg ccatcagcga ctgctggttg tggaacacct tgccgaccgc 7740

cgtaccctgc acctgggcgc tcatggcttc cagaatctgc cgctggcctt ccttgtcgtt 7800

cttgtcggtg gcacgcagct tggcttgcag cgcttgaaac ttggcatcct tggagaaaat 7860

cgactgcatc atgtcgatgg tggcgcccac cttgtcaatg cctcggctct ggtagtccag 7920

gaacacgtca tcaacaccct ttaggcgctt ttccttctcg ccgcgtttca gcttctggcc 7980

gttggccagg tactgctccg ccatgtactt gcggaagtgc ggtgtgttca gctcgttcag 8040

caggtcgcgc aggttgttgc ctgcctcgtc gcgggtgcct gcggtaatga ccgatgcctg 8100

attccaggcg gccagcttcg caaagccttc cttgcctgat aagcccaggt tgctggccat 8160

tgccatctgc tgcggcagcc acttcgccat atctcgcagc tcgaagccgc ccgcttggcc 8220

ggccaccatg gcagcgctca ggatttgcgg catgtcgtcc gcgctgatct tgaatgactg 8280

tttcgcgcgg atggcgatgg tggccagtgc gttggcgtcg gtgccggatg cagtggcggc 8340

cttcatgatg cccggcagca tcttcattgc atccgtatca gacacggtgc cggacgcgat 8400

catggtgtca agtgcttccg ctgcctgctc gcgcgtgcca ccgccttcgc gacgcgcctt 8460

gttcacagca tcctccagcg acttggtgcc gaccttgcgg cccgcggcgt cgcgttcgga 8520

ataggcggtg ttggccatgt tggccagacg ccggtcatag ctcatggagc gttccgcagg 8580

ccccctgagc gtgtaggcgg cagcaccgat gccggcgatg gcagcaccag tgaacttcag 8640

gcccgcagcg gtcttctgcg cagcagtcag ccgccccatt tcgttggtca gctcagtgac 8700

cttctggcgc atctggccga ccgctgcggc ttgctcgcgc catgacatgg tgccggagcg 8760

cgccagccgg ttgtatgccg cttcggtttg ctggatttcg cgctgaatgg cgcgctcaga 8820

gcgcacgccc aaggtttcgc gcgcctgggc aagccgttca taggagctgc gttggcgcgc 8880

ggcgcttttc tcggctgcat cggcagcctt ctgcgccgcc ttctcggtgg ccgtggccgt 8940

cttggcagca gtctgctctg ccgttttggc agtcttctga atcgcggcat tcgcgctgcc 9000

atcatcaaca atcttgatct tgacggcgac atcaacagca ggcgacgaca tgaaaaaagc 9060

tccagtaccg gaaggtatct ggagcttatc gggggtgctt ctttcctggc aggcaggaaa 9120

tgtttcctac cagtctgtgc gcgctactcg aatgtcttgc cgtccatctg tgctgcaatc 9180

tttgcccagg tgaacagctc gggcaacggc atggctttaa cgacgggcag cggctggtgc 9240

aaaccgcgcg ccaccaatgc cgcagcgagc agcacgcgcg ttactttttt cgcgctgcgg 9300
cggcggcttc atcctcgggc ggcgcttcgc cgctttcggc tgccggttcc tcggcttcgt 9360
cttcgtcggc ttcgatgata gcgtcggcca tcttctcggc ccggcgatag tcaatgccgt 9420
gcagcttctt caccagctct tcgtcggtgc ccgtcaggct ggcgatcagc gcaatgcgtt 9480
gggctacgcc gccgcgcttg tcgaacgaca gatagtcttc ggcagtggcg tagtcacgga 9540
acgtcaggtg gctgaccgtg actttgccga acgccagcgg gcgtttcagg ttcagtttct 9600
gtgccatgtt gttggtctct tcagtggcgg gtaaaggtca agttgcggcg tgcagtgcgt 9660
cgcgcaaggc gtagcccatc agcggccaca ccttttgcgc agcgttctgg cgcgcaatct 9720
tgcggccgat ttcggcattg aagttctcag ggcttgcgca ggccgattca ccggtcacgg 9780
tgaaaccatt gcgcaatacc agcacacaga aggtcagcag gtgcagggac gggtggcgat 9840
cccgaaagtg gccgcggccg tcccgcccgg ccatcatgcc gtcttcggca gtgaagtaat 9900
gctcgcttgc gatgttcgct tcgatgtcgg ccggcgtaac acgcggcgca gtcaggccca 9960
tggactgaat ttccttctcg atgttgtcca tagcctgctc ttaggaaatc cgctcggacg 10020
tgttggcgat gatggtcacc ttgccatcac catcgcccac gggcacgggt tccgacgtga 10080
atgcctggct catgaggtac acagtgccat cagccaggcg cacagtcaca tcttcgtcct 10140
tgatgtcgtt gatcgcgtca acgtccagcc ctggcgtcag attcaggttc agctccagct 10200
tggccggcgc gccggactcg gtgtagccac cgtcttgcgg caggcggcct ggcttgtgct 10260
cgcgcttctt gccactcggc gtgaacgtac cgggcttgtc ggccagcggc agcttgccga 10320
tggacggcac cgacacggta cggatgttgt tgagttggga cacgttgaac tcccttcaaa 10380
ttgcgttgga aaccaagact tcgtgcgcgt cgatcagacc accggtacag ccttccggaa 10440
gcgcgagcgg cccgcgagga tgtagaacgg cgagttgacc accgggtcat ctaggaagtt 10500
gaagcgactg gggttgtccg ggtcttgctc caccaccaga gtgctcttgt agtagtcata 10560
cgcctgcacc cagccgtatc gcgacatcag cacgttcttg tagagcgaca gcaggaacgc 10620
tttcacgcca tcttccgtgg tgatgggcag gcccggccgg tagccttcgt ccgtcttggc 10680
ggcaacggtg ccccggaact tcttgatggc gcccgtgcgc tgttcgtagc ggatgcgctc 10740
cgtaacctcg gccacgttga tatccagata ggcgtcgtcg gtgctgccat cggagcgatg 10800
caggtacatg gaaatcagcc gcttgatgta gcaggaaccg tccgtgccta cctccatgag 10860
gctcatgccc ttgaagagca ggctgttccc gctggtccac tcgtgatagc tcgtggccac 10920
aagcccaggc agcgatgtac cttccagcga gataaccggg ttgttgtaca gcttcggcgc 10980
agcggcggcg gccagcgtcg cggcggcttc ccaggtagtg ggcgggttga tgcccagcga 11040

caggtccgtg atgtgctcgt agttcttcgt ctcgccaaag ctggctgctg ctgcgtagtc 11100

cccacgatag gcagcgaatg cgcggaagcc ggcctgcacg ggcacccggt agcggcgttg 11160

gctctctgca tgccatgccg ccagcgtggc ggcatcgttg atgcccagca cgacgtagcg 11220

gaaccacttc tgaccgatca gcgttgccag gtcgcccggc agcgggtcgc ccgtaccgcc 11280

tgccatggcg gtcagcgcga gcgcgagccc tgcgggcatg tcttcgccgt acaggttcaa 11340

ccgcaggtcg atggcgttgc cgcaggtgcc tttatggcgc gcgctgagcg tcaccgcagc 11400

accagcggca acggcagtca ccggaatgtc cgcacctgcg tccgtgatgg cttgcgcaat 11460

ggccgtcgca atctgcgcag ccgtcttgcc cgcagccacg cccacggtga tggcgcgttg 11520

tgcgatgtac agcgcgagcg tgccgtcacc ggtggccgca ctgttgacgt tgatggaacc 11580

ggttgctgct acgccggccg cgttgtccgc gtagggcagc atgtacaggt cgagcgtctg 11640

atcgacattg cgatagcgcg cggccatctg cgccagcatc gaaccatcgc ctgccagcgt 11700

cttggcatcc gctacgctgg cgacacgcac cagttcgccg gtcggtgccg tgccgctggc 11760

caatttctgg cctaccagca gtacgacagg caaatcactg ctcaggcccg cctggctccc 11820

atcaatctcg atgtaagcgc ccggatagcg cagcgccgaa ggcacttcac tgaaggtaat 11880

ggtcacagct tttctcccgt caacagctcc cggtctcttg tggtagggcc cttgtcatcg 11940

tcagcctgaa atctnnnnnn nnnnnnnnnn nnnnnnnnnn nnnnnnnnnn nnnnnnnnnn 12000

nnnnnnnnnn nnnnnnnnnn nnnnnnnnnn nnnnnnnnnn nnnnnnnnnn nnnnnnnnnn 12060

nnnnnnnnnn nnnnnnnnnn nnnnnnnnnn nnnnnnnnnn nnnnaggggg gggggggttt 12120

acccgcaagc ccccccaggg ggaaaagggc agtttccccc ggaaatgttc cggcaccggg 12180

gttcccggga ggggttgggc aaaagggcgg ttcccatttt ggcccagcgt ttttgcccgg 12240

cacccacgcc ccccggggga gggggcgttg tgcgatgtcc agcgcgagcg ggccgtcccc 12300

ggtgggcccc actgtggacg ttgatgaaac cggttgctgt tacgcccggc cgcgttgtcc 12360

gcgtagggca gcatgtacag gtcgagggtc tgatcgacat tgcgatagcg cgcggccatt 12420

tgcgccagca tcgaaccatc gcctgccagc gtcttggcat ccgctacgct ggcgacacgc 12480

accagttcgc cggtcggtgc cgtgccgctg gccaatttct ggcctaccag cagtacgaca 12540

ggcaaatcac tgctcaggcc cgcctggctc ccatcaatct cgatgtaagc gcccggatag 12600

cgcagcgccg aaggcacttc actgaaggta atggtcacag cttttctccc gtgtaaatga 12660

gcctgtcaaa tgcaggcggg tccggtaggt agttggtaat caaggcgtcg aactcgtagc 12720

gatcgcgcca gtacaaatcg ccgtcggtgt actccaggac ttggccaccg acgaacttga 12780

tggggcgcac gccaggctga agctgccagc ccagcagcag cgttttcacg gcaatgcggt 12840

acttcagcaa ctcatcgtcc gtatcgcctt gcttgtgcgt gcggcggttt tcaatggcaa 12900

tcaccacatc gaaggccagc gtcacgttct cagcccgctc gcctgcgtgc tgcaccttgt 12960

ctgctgcccg gacgatccac gcggccggca gcggcaacgc ttcagggcga acctgggcga 13020

actcggcagc accagctacc tgccgaaacc agatgcctgc gaactcggca ggcttcaact 13080

tcacgtgttc aatcagtggc gtcagcgaaa tcaccgcccg acctcatcca cataggtgcg 13140

gccaaagcgg cgcggtgcgc tttcgatgat ggccagatca tcagaaggaa ccggatcatc 13200

ggatgcgggc gggacgaggc tgatatcgcc acgggcgtgc gccttgagcg tgtccagcac 13260

accttcatag gcgcggcgca cgtcgtcggt catggcctcc gcaccttgca ggtagtacag 13320

cgccacggtg gatgccagcc gcgccagcag cgtggtccgc acccgctcgg gaatgctgta 13380

cgacagcagc agcgcatcgg cgtctgccaa tgcgcggtcg atagcatcca gggcaagcgc 13440

caccgcctct tgctcgcgcg gcgtgaggtc gcccaggtcg cctccctcaa tggccgtgcg 13500

cagcgcgtct tccggcacca tgtccatgtc ggcgggaacc gccaactggg ctagccgcct 13560

agcgttgctg cgcgcaagca gatcagagcg ggaggcgaag cccattactt gcccgccttc 13620

ttggcggccg ccttggtctc gggcgagtcg tcttgcgatt gcgtggtcga gtcttcagcc 13680

tcttcgggca tatctgccgg gcgcgtttcc aacacctcca gcatctgctc ttcgtccagg 13740

cgcttggcgg ttgctttgtc cacttccact tttgccatgg ccttgctgaa cttgaagccg 13800

cagcggtgga acgtctcgct gctcttcttc ggttgcacgc gcacaaagag tgtcgtcacg 13860

ttgattctcc taaatcggtt cctgtaccca acccggcgga acaattcccg ccggggacgc 13920

acctgctctc gctacggggt atgtgttgtt acagccaggg cgtaacgacc agttccacct 13980

tgttgtagtt ggtgttgctg gagccgttgg cgttctgcgc ggccttcagc agcgcttcgg 14040

catcggccat gttgtccggg ccgcagagca gcgtgtccgg cacgatgccc agcttgcgct 14100

gaccgtcacc cctgaacttc atcatggcct cgtaggccgc gttgaagttg gcagcgttga 14160

gcgcagcctt ggagccgaac gcgcattgcc agaagccgta agcggcctcg ccgcgccaac 14220

ggccgccgaa gctgtacacg tccaggtcga agacgttggt gttctgcgtg gaggtgatgc 14280

tgttgaactc cgcgggcatg cgctgttgca ggtagatcgg agacgctgcg cgcttcgtgc 14340

acagcagcac ccaaggtgcg cccgcaccat cctgcatgtt gctcacgttg gtgacggcac 14400

cggtgccgtc ttcgttctcg tacaccgaat gatcggtgtc gaagaaatac tggccgtcgt 14460

agcagacgga ggcgaagcca gcgcccagcg cttgaaacac cagatcgttc ttcagatcgg 14520

tggccgactg gccggccgac tcggcgatgg tgccgtactg gccgatttcg tcgtcttcga 14580

tgtccgtacg ctgcacgtcc accgtgcact cgaacttgcg gttgaccacc tgataggcgg 14640

tttccttgaa cttcttgtgc aggcgtgcgc cgacccattc ccggaaggcc gggaactggc 14700

tcagccattc gtaggtgttg gatttgccgg tactggtgac tagcttggcg atcttcttcc 14760

agtcttccgg cgtcagcacc aagccagcat tccagcgggc aatcagcgtg gtcttcagtt 14820

ggtccaactg ggcttgcgtg agtgcaccca tcgttttctc ctgttgggtt ttgggatgat 14880

tttgtcgtgt ggactagcgg cgcgagctag gccgcttttc agcgtttggc ggcgacgtac 14940

tgctcggggg tgacccccat cttcttgcac atcgcaagct cttcgtgggt caggccatgg 15000

gcaccttcgc tcttgccatc ggcctgcttg tccaggatgg ccagcggctt gctggcgtcc 15060

aggtactctg tcagcgcggc cagcgattgc ttttcagccc acggcttctg cgccggggtc 15120

aggcggccat cggtcagcgc ggctttcagc agatcgccgt gttgtttgcg ctctgcggcc 15180

agcgcggctt cggcttcttc cttctcgatg ccttccacct tggccttcag cgtgtcgcgc 15240

tctgccgtaa gggcagcaac gttggtcttc aggccatcgc gctcagtggt cagcgctgcg 15300

agctgcgatt tcaggccgtc acgctcggtc gtgagcgcgg cgatttcttg ctgcgacatg 15360

tcgaactccc cttctcgaaa attgaattgc ttgcgggcca gagcggccac ggcttctagc 15420

ccatccaggg cgggtgtgtt ggtgagtgcg accgaaagga tttcgagcac ttcacctgtg 15480

acggggctgt agaagaagac agtgctgatg tagcggtact tcttctgcgc gatgagctgg 15540

gcggcgtcgc cgacccatgc gatgttggtg gcgtagagcc ctttgccttc gcgccattcg 15600

agcgtgcggg gaatccagcc agccgcatcg gcacgctggc cattccgttc cttgttcagg 15660

ctctggtggt cgtagtcgac caggatgtcg ttcttctgct gctcggccag cgcgatcacg 15720

cgcgcggcga tggcggcgtc gagctgccaa gccgcgcagt cgaacgggcg accatccgtt 15780

gcccggaacg ggccgagcgg cagcaaatgg gcttcagtcg gaacggcacc atcgctgccg 15840

ggcaccaact cgaacgctag cgcggctatt gcggtttgtg cgatgggggg attctgcgtc 15900

acagcgatgc tcctaatcga tgaggagcat tcttgacttc agcgagactt tggtcaggta 15960

gtgaaatgtt tcatgcgcgc ggtaatctgg gttaccgtgt ttgttatgct ttcgcgaaac 16020

ccttgataat ccaggagatt tgatagatat gccgactgca tcctgcacat ttacgctcaa 16080

cgggcaggca accagcacgc taacgtgcga cggccgcgtt tatgtggctt tctctggcaa 16140

taaagggcat gagaacaagc ccggcgatac cggcatcgct ggacaagggc cattaccaac 16200

cgggcgttat tacatcgtgg accgtgagag cggcgggcgc ctggggccga tgatggactc 16260

gatccggtca gcggtcagca actccaatca cgccgagtgg ttcgcgctct accgcgatga 16320

tggcgcgatt gacgactgga cgtttgtgag tggcgtgcgg cgcggtaatt tccggctgca 16380

cccagttggc ccgcgaggca ttagcgaggg ctgcgtgaca ttggtcagca aacagcaatt 16440

cgcggctctg cgcacctacc tcaagtctcg tcagaccggg gttgtccccg gcacgaacac 16500

gaagtattac ggtgtgctgg aggtcaaata gccatgcggt ttgccctcaa gaccgtcgct 16560

gtcgtgttac tggcgttcgc atatacgcgg gccatttatc tctggtcttg gcttgaccca 16620

ttctttgcca gttcggcatg gcagcgaatc tatggcttcc tcgcgcacct gtttggcgtg 16680

agcggtgcgg agcaaggcga agacttgcag ctcgccatgt tccttgtggt ggcactgctg 16740

ctggcgatct tgtcagtttg gctgttcgac cgctacgtgt gtcggccggc ctgccacaca 16800

ttagcgcggc agagaaccgc gaaaaggccc taacgatgcc gcctccgggc ggcattcttg 16860

tatgtatggg gcggaattca ggcgaggtct ggtgggcgac cgtctaaaac gcgctcagag 16920

ccgttttagg ctgctgatgg accgtgggag gtccacgccc cggaaaggcg tttttaaacg 16980

gctttcgaac gcgaatggag gcatcccgca aggcggcgct ccccaaaggg gcaccgtgcg 17040

gggtcagaag aggtctagct gtcgatcgtc gtcctcacgg cagatgttga caatctgacg 17100

ggacgaaagg tgatggcgcc gagccaaagc gttgatgctg gcgttgtgcc gatctttgcg 17160

aatctgcgcg tcacgcacgc ggataaacag cttgtcggcc ttgggcagcc aacaacggcc 17220

atccgaatcc agatgcggag ccagcgtcgt ccgcaaacgg gccagttcgt cggcctccaa 17280

gcccagtgcg tgagtacggt actgcgggat gctgacattg acgccaccat ggtgggtcag 17340

ccagtcccgc gcccgaccgt aaccaagcgc gcggaccacc gcacgcagca cgggtggcaa 17400

ggttctcagc aaatcttcat cgacagccgg gtaggcgtct accacgcgct cgatttccga 17460

cgcggcatgc gcagcgggat ggtgatagtg cggaatgacc atggcttacc cgcgcgccag 17520

ccaactcttg agagcttcgg caatcgtttg gcattcagct acggccaagc tgtccaagtc 17580

cggcacgtga cggcccactt gacgcgcaca gaactcaagc agcgccgggc gcgttgcctt 17640

agccaccttg cccgcctgcc cgagcttgaa ccacatgcgc acgaggtgag cgatgcgggg 17700

tgggacttcc ttggtcacgc cagcctgacg gaacacgcgc cgatagcgcg accacccccg 17760

acgctcgtag tcgtccagca cagcgccgag ctgcgcgagg ttgagggtac tggcggacac 17820

gcggccgtca accacggctg cgccatggcg tgtaagcagg tcgcggtggg tctcttcaga 17880

ccagccaggc aggctgacca tggcccagcc tttggcgatg ccgacaagct gccgatgatg 17940

cttgatgagg aacgtagaca ttttcatgcc cccagcattt ggggtgcatc cacccctgtt 18000

ccaaccccac gatggagggt ggcgccgcgc ccagcgcgaa cgccttgcac gcagtcgccc 18060

acctgggaaa catggcgccc aggttcgcgg caccgcgtct caacttgctc ggtcacggca 18120

tagttcgcgc gcatgtaggc atccaccgcg ctgcggtggg cttcgggcgg cacggtggca 18180

cccaccagct cgcgcacggc aaacacccat ccttcgcaga acatgtctgc gcgggccacc 18240

ttgttcttgg agcggcagcg cttgagcgcg gtcttgatgt agtcggcccg cgcgcggcga 18300

agctggcgga acagaacccg atagctgtaa ccggcgaccg tcgaagcgct ggccataccg 18360

ataaatacgt agccgccttg gatggtggtt cgaccggaca ggcggttgga aaacaccagc 18420
tcgcaaccaa aggcggatgc gatcatgctg gccagccaca cctcatagcg ggccggcgtc 18480
cgggtcgcac cgctcttcac ccacagctcg gccacgccgg ccgccagcat gtcgtcttcg 18540
ctgacgccat aggcggtcat catcttctgc gcctgacgca tggcggcagc ggcctcatgc 18600
gggttgctgg attgcccaag cgccaaacac ttcttgatct tgtcgattgc ggtttcgcgg 18660
tccataactc ccctctggct gctcatcagt gtccggccac cacgccgggc agacacccgc 18720
ttgcgcgggc gtttcgcggg tgggccggct aggccgctgc tgcatccttc agggccttgg 18780
cagccgagaa cttcggcgcg cgcttggccg caatggcaat aacgtcgcca gtcttcgggt 18840
tgcggccggt cttggcggca cgctccacgg agccgaactt gcccaggtcg gtgatcgtca 18900
gctcgttacc ggcgcgcacg gtgtcaagaa tggtttcggt cagggcgttg agcaggtctt 18960
cagccttgcc cttggttact tcggccttgt cggccagatg cttgatgagt tcttgcttgg 19020
tcattacaaa agttctccta tggtttgccg cgatacgctc gcggcgggcg ttactgaaat 19080
cgaaccgcca gggcgttaca gagcggcaaa gtccagggtg acggctgtgg gcttcttggc 19140
accctcggcg cgctcgtaga agcggatgta ggccgtggtg cccaccacat ccaccgactc 19200
gccgatggcc tgcatggcgc acgtccactt gtcgtcgctg atggccaggc ggcgcagcgc 19260
gagcacgcgg ccggtgttga tctttccctc cttgtccgtt tcgaaggctt gctgcaccag 19320
cacctgaatc ttcgggtcgc tgcccttggc ccattccttg atgcactcgt cgatgagcac 19380
cttggcggct tgcaggcgtt catcgaaagc gatgttctcg gcctgggaca cgactacctg 19440
ctggcgaccg tcgaacgtgc tcaaggtgac gttgcccttt gcaccgccca gcttcacttc 19500
gtagcgctcg gccgagagcg atacaaatgc gtcggtgtcg ttaaaggctt ggcgtttgaa 19560
atcggccatg gctgcttgca gtgccttggc tttggccacc agctcgcgca ccagcttgtc 19620
gcgctggagg tcgatggggt ggatggtcga ttcgggcacc aagtggcccc gcgcgttggc 19680
gcgatagcca gccggaatct cggtggggtg ttgcgttact gtggtcattg gcgtcctttc 19740
ttcaggttgt tgagggcttc gcgcacttct tgaggcatct cggttttcgg cagttgctcg 19800
cttaccgctg ccggccctac ggtggtcgtg gccgcttggc ggcgggcttc cggtgtgcca 19860
gccccagcaa tgccgcgccg ctgcgcctct tggtcggctt ccgcttgact cgctgcgcgg 19920
gcggcctgcc ccgcgattac agagcgcagc cacccgtgcg acttcagggg gagttctagg 19980
cggccgtggt tcgcttggtc cactgcgtag ctgaaagccg acgcccacag cgccatgggc 20040
gccgcgtaca cggcaccgtc gcgcttaacc tgggccgctc gcatctccgg ctccagctcg 20100
cggatgaggc ttgccatgcg tccgttggcc atctgcgtct tcttcggtcc aaataggccg 20160
atgtagcgca ggagcggctt gatgaacgag tcgcctgccg ggtgcacgtc caccagcgcc 20220

agcagcatgt cgcgcacgcc gtcatcggct agcaccacat ccaggctcat ccgcacgcgg 20280

cagttcgggc agacgacttc aggcagcggc attgccacct ccaaccagat cggggttcgt 20340

ttcgcgcaga cggcggtaca gcacgcggcg gcgcccggcg tacttcgctt gaaggcgcgt 20400

gaggttgtcc cgagcacggg ccatttcttc ctgccgccac tccacatcgc gcaccgcgtg 20460

gatctcttcg cggtcgatgg cgcgaagctg gcgctgcacg cccacccgca cgcgcaaccg 20520

gcgccactcc gcgcagattc ggcgcaagcc cgccagcagt tcggctgcaa caggtccctg 20580

ggtgatgcct gctagctgtt tcatgtcttc accttccatt tcacttcgtc ggggatttgg 20640

catgtgcctt cgatccaggc gccaaacgcg gctgcctctg ctgaaggtgc gccgttggcg 20700

atggcatcca agaagcgcac ggccgctgga ccggtggcat agatgcgctt ccggcgccgt 20760

gtatgggtaa tcgtgatgcc cggtgggtca aactgttccg ccatggctgc ctccgctcag 20820

gccagcgatt gcaggcacag cgcctgtttg gccaccgcat ccaccagctt cacgtccagc 20880

ggacgcccac ggcggaactc cttgatgcca gcgaccaacc cctccaccag catccgcgcg 20940

ctgcccttgc agtattggta aaggcgttgc accacctctt caggtacgtc ttccgtgccg 21000

aagccggctt gcaccagggc ggcggcgtct tccaggttga tggcgcgcac cgtctcgggc 21060

cagaagcccg tgcgcgagcg gatttggtca aactggccat gcagcggctt gatgaggccc 21120

gacaggtgtt cggtgccgca gagcacgatg ccgacgttgg ctagatcacg gaggcggcgc 21180

agcgtgtgga gctggtgcgg cgtgagcgtt tcggcctcat ccacgatgag cagactgtcg 21240

gtgttgcgaa gcgccgtcac cacggagcgg aacttgtcat caatgctgcc cttgccgtca 21300

tagccggcca ccacgcgggc cagcagacgc acaaggctct gcggcgtcat ggtgggcgtg 21360

gcctcaatca ggtgcgtgtt cggatgcgat gccacgtatt gcttgatggc aaaggtcttg 21420

cccgtgccca cgtaaccggt gaacaccgcg aagttgcgat agcggcgggc catgctgcac 21480

gcggcgtgcg cgagcttgaa gacgctggtt tcgaccgggg ccaccacatc ggccttcgtc 21540

tcttcagcgt ggcgcatggc cgagtccacc gacgccagca gcttgcccgg actggtggca 21600

tagctgcctt tgagaatctg gttcaagctg cttgcgctga tgcgcgccag ccgcgccaga 21660

gcggcttggg tgtaaccacg ctcttccatc caggggccaa tgcggccgat aagcgcaacg 21720

tctgcggcgg cgtaatgggc gggccaggtc gtttgcttgt ccatggggtt ctcctgttat 21780

tcgtcgttca tcgtcaggtc gagcagcagc ggcgcgtcgt cgctgtcgtc caccaggcgg 21840

gcttccacct cgattgcagg cagcacgccg tcggccacgc tgtccacatc gagcaccatg 21900

cccgcacggg ccttctgctc gtccatcttt tgttgcaggc gcttgatggc atcggcggcg 21960

cgcgcctggc gcttttcttc caggcggttg ggcgcgatgg cgtcaatggc cgtgacgagg 22020

tgtgcgtcgc aaatccaccg gccttcctgc gtgcgcatga cggcgacacg gtcatccatc 22080
aggtcgtatt ccatgaccac cctgtggccg ttgaaggcgt gcagctcggg gtgcgtgtag 22140
gcacgcttgc cgtgcttgat ggctgcgcga ttcaccgtca gcaccaccgc ctggcgcttc 22200
agttcggtca cgttggcgtg cggcggaagc ggtgccagtt gcgaccacag cgcggcgcgt 22260
gtcgtgttct ggtcttctgg gtgcgggcgg ttggcgtagc ggtccaccca ggcgtgaagg 22320
cgtcggcaaa ctggggcaat gtgggcagca nnnnnnnnnn nnnnnnngtg ttcttgtagc 22380
cggacccgtt gtcgatgtag agcatcggcg gcacgtggtt ccagcgggcg aagcactctg 22440
cccacatgtt ctgcacggca tacgtgcctt catgttcgtc ggcccgccat cccacgggga 22500
agcggctgcg catgtcgatt gccaccgtca gctccgggcg ccagatatcg cccgtgacgg 22560
ggtgcgccag gtacacgtcc gcgcggtagc cgtcggccac atacacatcg ccgggcaagg 22620
cgttttcggt cgagcggcgg atgtatgcct tctcggtcag gcggtacagg ttgcggccaa 22680
tgcgtgcggg gctgttgcgg ccgagcatgg caggaacccc cgtcaggtag ttgcgtactt 22740
ggtcgtagct gaccgcgaag ccgtccactt ccgccaggcg gcgatgtaca gcggccatat 22800
ctggtttgcc gggcgcgttg aaatactcca gcgccggccc ccaccagccg gcggcctcca 22860
ccacgcggcc cttgtggtcg ggcagcagcg cagttgcgcc gccttcgcga tactgagcgc 22920
accattcgca gatggcggag cgcgatggcg tcttccggcc tgccttggcg gcgccagcca 22980
gcgccatggc gaagtggctg ggcaggctgc cggcctcgcc gcgctccagc agcagcgcca 23040
ccgcgttgtt ctgcgtcaca ccgtcttccg tcatggcgcg cacgtacgcc accaccgtct 23100
cacgccacgt cgccacttgc cgggccctgt cggttgcctc gcgccacggg tcgcgagcac 23160
gaagcgccag cacctttgct gtgggcatga ccgctaccgc gccacttgcg ccgcctgcgt 23220
ccacgcgctg aaccagttgt gcccgtgcca tgacttactc ccccttagcc ttgttggtgg 23280
aacccttggg acggccggcg ccgcgcggtt tggctgcatc acgcttctct tgccggtttg 23340
ctgcttcggc ggcgtggcgg ttttcgatca tcgggtagtc gatcagccat tggcgggcct 23400
cgtcagggct gaggatgtgg gtgccttgga tgcgttcggg catgtcgtct tcgcgcacga 23460
ggctggccat gtggtcgatc aaatccaggc cgcgcgaagc catgacgtgt gccgtcaccc 23520
aaatctgttc catctggagg cgccattcgg gcgactcgtc gccgttgacc tcatcgaaca 23580
gctttttcag gctggaagtg ttgagttcaa cgccaagttg cagggccatg cactcttcgc 23640
ggatttcctc tgtgcgcaag aggaattcgg tggtgcgatt tacttgcgac aggcgtttga 23700
cctgcgagtg cgcacgctcc agctcagctt cgtagttctt ttcacgccgc ttcagcgcgc 23760
gaatctcttc cttgagttcg gccaccgtca tcaggtcggc tttgccgatc atgtcgttgc 23820
cggactcggc ggcttggtcg atgacttcct gcggcagcga ggccaagagc atcaccttga 23880

ccttgccgag cgccagcacg ccttccagtt cgtcttgcgg cagttgagcc acaaacttcg 23940
cgatgcgcat ggcttcgtac acgcgctggt ttgcaagtcc atgcgcgttg atccaggtca 24000
gaaagccgtc cttttcaact cccgaacgtt cgggagtcga tctgcgatcc ccaccatgcc 24060
caagcgaaat caactcttgg gcgcgcagaa atgccatccc agcacgcgta atttcgaaca 24120
tggcacggcc gatagagtca gcgccgatgc gggccagctc gccgaggtca tcggtggtca 24180
cgttgaaatg cgacgcgact gcggcgagat tcgccactgg tgcgagcgcg atgggcagca 24240
ccgccgcagc ggttgcaata tccactggct gcgcgtcgtc cgtctgcgtg gcgtgaacta 24300
gagcgccttc cgtgatgccc aggtcgatat cttcggttgc ttgtacggct ttggttttct 24360
tcgtcatggt cggcttcacg cagctttctt gagacccagt gccacggcga ctttgtgagc 24420
ctcgccgtaa cgccccttgt tcacgccgtt gatgaccgcg ataaccgtgc ggtattcaaa 24480
gcccttctcc tttgcccact ttgcaaaggt cttgccctcg cgctcaaagc gcgcctttac 24540
ttgctccggg gtcattgcta cgctccatcc gttacgatta agaatgactc attatgggaa 24600
agaaaacttt cccagtcaat acattccagg gaaatttttt atgaccatcg gagagcgttt 24660
gaaggaagag cgcgagcgat tgcgcctcac ccagccggcc attgcctcgg ccgccgacac 24720
gacgaagcaa acacagcacg catacgaaac cgaccgcacg ccgccgaaag ccagctatct 24780
ggccgcaata gcgatcctgg gcgttgatgt ggcttatgtg ataacggggg agcgggcgct 24840
taatacggcg cgcacgccga tggaagttgc actgctggag aactaccggc acagcccagc 24900
agaggtgcag cggggggtta gcatgcttct cgcacaaacc tccggagccg cagattcagt 24960
cgccggaaag ggaaagaaaa atcgatcctc ggaggaagtg aagaagtgaa gcgcacaatg 25020
ctcattcttg cgatagcaac tattgctagc gcaccagcca tggctaaagg accgatttcg 25080
ccagaggtct tcagagacag ctataacgcg catgcgaaag acctacttcc cgcgttgcag 25140
acaacacagt gcaggaccat cagcgctgcg aaggagccgg gcaaggcgat cttggaatgt 25200
cagaccgtgg caaacaacac gctgttgagc atcaacggca tgaacaagcg tttcgacggt 25260
gcttggctga tgctggacgc gagtgcattg ccacatcctt ccgacttgac gcgggcgggc 25320
gggttgttgc tccgcgttgc gaagggctac cactacggca accatctggc ccttgctcag 25380
gaggcgatta ttggtgcgca aagaagcaag gggaagcaat cctgcattga cgatcccgca 25440
tcaggtagcc gcctctgcgt ctcgaccgat aacggcacga tctacaacat gatcttggag 25500
caagccgagc ccaagaaggg ctgatacatg cactaagtgg atacccgccc ccaggaaacg 25560
tttcacgcct aatcgcgctc cgcgcgcgtg cgtaacctct ggtcattcca atgacccgga 25620
ggttacgcat gaaactccct cgccttacca cgtggatcat cgccaccatt gcgctggcga 25680

tcatcatcgg tctgctgtct ccgcagcaac tacccgtcag tctttacaag ctctcgttgg 25740

tcaccatggc tgccgtggtg gcgtactggc tcgaccgcgc gctgttcccc tatgcccggc 25800

cggccggcta cctgtccagt gcggactggc gcaaagacgg gcctatgtgc gatgacgccg 25860

accacgccat tgttaccggc tacgagctgg tgtttgccgc cgccatgctt cgccgcgccg 25920

tcatcgtggc cggtgcgatg ctcgccatcg gtctgggggc gtaaccatgc gccgccagtc 25980

gatcctcttg agcgtgttcg ccgcgctcca gctcgcggcg gcgctgctgc tcgcatcggc 26040

agatgcattc ggtgctgatg ccgccatccc gcacgtcgcg agacgctacc gtgcagacct 26100

gacccgcatt gcacgcgagg catggggact tgacgcaccc gtggctgtgt tcgccgcaca 26160

gctccaccag gaaagcggct ggaaccccgc agcggtctcg cgtgttggtg cgcaaggtat 26220

ggcgcagttc atgcccgcca cggcgaaatg gtggtgtgtg ctgaatggcc ttgccaacga 26280

tgcgtgccag cccaccaacc ctgtgtgggc tatgcgtgca ttggtgggct atgaccgctg 26340

gctgtatgag cgcgtgcgcg gtgcatccga gtttgaccgg ctgtgggctg cactacgtgc 26400

gtacaacggc ggactgggcc actggcaaca ggaagcggcc acggtgcggc ctgcgctgaa 26460

ccgcgcaacc atcgatagcg catgtggcac agcgcgccgc catcgcagct tctgccctga 26520

gaacctgggc tatccgcatc gcatcttgaa cgtcttgcag ccgcgctatc tggcgtgggg 26580

gcgtggggta cggacatgac ccgcacgctg attgcaacgg cggtagccgt agcgtgcggt 26640

gcccttagcg cgtggagcta cacacgcaat cactacgtgg ccgagattgc tggcatgacg 26700

gccgatcaag ccacggcccg agagaaagcc gagaagatcg cgcgtgagct gctcgaagcc 26760

gagcaaaagc gcggcaacgc gctgtcggac acgttggcca agaaagaaac cgcgatcacc 26820

gaaaagaccc aggagcttgc caatgcacta tctcgcctca ctactggccg caagtgtctc 26880

gatgctcgcg ttgtgcgcgt gctcaacgac tccagcaccg gcaccgctac tgacaacgtg 26940

cgcgccacca ccggcacatc tgatgcagcg gatggacccg ctgccaccga taccgacgtc 27000

gctagctgga tcaaccacgc caaaggccaa tacgaaatct gccgggctcg actcggcgca 27060

ctgatcgact ttgaaaaagg gcgagttcaa 27090

<210> 10
<211> 10384
<212> DNA
<213> Ralstonia solanacearum

<220>
<221> misc_feature
<222> (8360)..(9288)
<223> n is a, c, g, or t

<400> 10

tgtctagacc gcgctcatca tgagtgataa ctcattttac tatgctcata gttgagcaat 60

agcatatgga cgtgcgtagc aattaacaaa gaggaaccac aacttcgtgg tgacacacat 120

gacgacatcg agcacttcca tgccgctggc acctacgccg cgaccttcaa gcgagccgcc 180

agcgctcagc ccgatgctga gcgtcaagct gatcacggtc gccttgttct gggggggaac 240

cttcattgct ggcagagttc tggcccaggc tctgcctctg atggtggccg cattcggcag 300

gttcctcgtg gcggcaatac tgctggtctg ggccgcattc cacatcgaag gaaggccgcc 360

tcgtttgaac cggtcgcagt tgctcacgac tgctgcgatg ggactgaccg gcatctgtct 420

ctacaacgtc tgcttcttcg gtgcgttggc gcgccttcca gccggccgga ctgccttatt 480

tgtcagcctg acgccgatcg tcaccgcatt gatggcgagc ttggtattcc gggagcggct 540

cgaatcgaag cgctgggtgg gcatcggtat tgccttgatg ggcgccttga ttgtgatcac 600

ccgaggtgat cttgccgggg caatgcaaga cattggcgca tcgatcggac ttggggaaat 660

gctgatgtcg ctggccgtct tgaactgggc cgcgtacacg ctgattgcca gaaaagccac 720

cgagtcgatg tcctccattg ccgccaccac ctacgccgct ttatgggggc tcgcctttct 780

ttccgtggga gccgtgggcg aacttggagc ggttcaatgg ttgacccttg gctggcaagt 840

ctgggcgtcg gttctttact tgggggccat tggcaccgtc gtcgctttca tctggtactc 900

ccaggggatt cgcgcggttg gcccctctcg gacggctgtg ttcactaacc tggtacccgc 960

gttcggtgtt gtgctctccg ctggcctgct cggcgaggcg gttctggcct ccatgctggt 1020

cggaggtgcc ctgtctgcta tcggtgtttc cttggcgaat cggaaatagg cgccgctgaa 1080

gggtatggat ccgggcgttc tcgggccagc cccatcgcgt cgtggccatg gcgaatggtc 1140

agttcgagta caacggcaag ccttacaaca gcctcaccgc gactgcctgc gaaatcgccg 1200

gcacccgctg gtcgggtccg gcgttctttg ggctgcgcga tggagccaag aagcagcgga 1260

agggggcggg cccatgagcg tggattccag caagaaacgc cgccgttgcg cggtctacac 1320

gcgcaaatcc atgggcgagg ggctcaatca ggaattcaac ggacgactat gacgacccgc 1380

gtattccgac ggcaacatgg agcggccggg cctgaagcgt ttgctggcgg acatcgagcg 1440

cggcctgatc gagatcgttg tggtctacaa gatcgagcct ggtggacttc tccaggatgg 1500

tggtggactg cgggagcgaa tacagaaaga ctcttgcaaa ggttgcgggg ttttgcgtgc 1560

caatgcctct cgctgcagaa agcgttagaa ccggctgatt tgtcgtacaa cgcccatcac 1620

cagccgccta attgtctaag gacctcctgc ctaggtggtc cttcgagcgc cgaccgactc 1680

gcgtgacagg cctctaaatc acgctgagta cggaggcaat tcacatttgt ctgataccgg 1740

tgtatcggta actgagaaat atcaggggcc tgatgaatat gagcggagtg agtaatcgtg 1800

tggccgttcc ggatttatca agaagcaggc tgagcgggtc tggcttgatt gagtatatct 1860

aatacatgtt caacgcgccc cagtcgttcc ttgctctcct cggatcgcag tgccgcgatt 1920

aaaaatctaa tcggagatat ctccaattca tacgagccaa ttttatcaat caaccctcga 1980

agttcagtag ctaggtcggc aaggttttta tccgataaac cagccaattc gtaggcaatg 2040

atgcgcaggg cgagatgatt tacttcgacg aaggagccgg gatgaggggt gatactatcc 2100

aagaacgatt ccagatcatt aatttttctt tctaaacatg cgcacatggc aaaattgaaa 2160

gtgctgtgca cctttccaag tccaagtttt tcggcttcta aatagccgga cttggcttcg 2220

gcgatattcc ccaaggccag actgtgatgg ccctgcgcgg cgtactgtgt ggccacggtg 2280

gatgaactcc ttccaaatag gcgtccctcc tcaatccgtt gccggaccgc tgctatctgt 2340

gagttgtccg gagatatttc ttcggccgcg tccagatctc gaagcgctcc ggatcgatct 2400

cctagcagaa atcttgcctt tgctcgcccg acgtaatgtc catactgacc tggctcggta 2460

tcgatgagat cattgtatcg ttccaaagct cgcactatgc ggtcatcaga ggatccgctc 2520

ttctccgatg gagtcatgac ctcgctggca cttactgtac tggtcgacga aacaaatccg 2580

ctaatctcca gctgcgacaa tgcttgcgtt agctcgatac gaagctcggc tctcaggcgc 2640

tcatcttctt gctggatgct ctggaattca cggtcggcta tttcaagtaa cccggccagt 2700

gcaacaaaac gcctcttaag atcgtttgga atttgctgat ccgatttgta ttggatgtcg 2760

tgttccattt cggcccaggc atgctgaaga atggttcgga cttgtatttc ggcaacaagc 2820

ggagtaaatc tagagtattc cgtcgcctgg atacgtggat cttttaggcg aacaagaaag 2880

tgaatgcttt tgtagccgat ctggccggag cccagtaggg ccgcgtcctt gtcgagcctc 2940

tcctgcacgt taaactcgcg gcgaattaca tcctcaacgt ctctgacagt ttgcggtacg 3000

tatgtgatta ctcggacgcc ggcaaggtct gttatatctt gaagcggttg agggtatttt 3060

agttgccctg tatctgggtc gatcttcgat actttttctg aaaaactgtc aaaatccttt 3120

gctcgccact gaactgtgtg tattgagttg ctaatcaggg ctgtatccag aatgttttta 3180

attgcttctg cgaaagaggc atacaaatcg cgtacgggga gatatgccag aagacttttg 3240

ttggcgtgcg actcgactgc ttgcggatca atgctcataa taattttagt atcagcggtg 3300

cgtttcgtgg cgagtcttcc gtgcaatgta tccccggtcg tctgcagcgg agaagcaggc 3360

ggctagatgg cctccaacgc agtcatcagg tgtaaagtga ccatcgggaa tggccgtgtc 3420

tcgccaccaa atttggcctt gctcatcaac tacttggttg tgcatttgac ccctccgacc 3480

gtgtgctttc tttaatatat agtacacgca gtcgaacggt atagtgacgc attcgttcat 3540

cgcataaatg ccatgtctcg cttgggaatc gagtatcaga acggtctgat ttatgagggc 3600

agggacaacc cgagctcctt agccgttccg acgccaatca tctcgcagtg cacactcatt 3660

gagtccccct ctgatcttga gaggctgcct cgtgggatag actcggatcc gtttcgctgg 3720

attttcaggg aggactcgtt cgaccccgtt tccagggttc gacggggccg actatttcag 3780

cctttcagta actcgaacaa ggagttggtc tccgttgacg cgcatccctt tctgccttcg 3840

gatttgggcc ggcgtggcgt cgatggtcaa ttgcgcaagg aaatgaccgt gttcattcac 3900

tgcacgcaac ttgtggtgcg ccgggagcgc ggggaaggtc tgcagcttgc aatcggccac 3960

gaaggggcac actcgctctg gcgcattctg cagactgagc aaacggttac ccaggacgtg 4020

ctggtcaccc ttcgagcgga gtctgtattc ggggtactgc cacccctcga tctggcgcaa 4080

atcccagagg acggccgaac ggcagtagct gcggcctacg accgggtcat gcatgttgca 4140

tatcgagact ccccgacatc ggttgtggac caatgcagga acctctgcgc agttctcatg 4200

gctcgctggt tgcgccagcg gacgagcaac gataggttgc ttcacgacga tctcggcgcg 4260

tgtatatcgg cagtcaagaa gttcgccgaa ggagagcacc aactcgtccg cgccgcactt 4320

gagacagtga atcgtctcca tccacgaggg aaggagaacg agcgtgagcg gtacagtttg 4380

cgggaggtgt caaatgagga cgcagagctt gcattgcacg caaccgggtt cgtgattagg 4440

gagcttggct gggggcgctg atgaacccca cctttcattt cttaggagtt cctttttttg 4500

tggggcgcgt gaacgctttg tgagaattca ccccgcatcg ccgctctaga atgccccgcc 4560

gtattggagc aagcgagtgg gtcggtagcg ccacccgccg ccggtcggcg tatagaacag 4620

gtacaggtgg ggtagttgcc gtgaaggttt tcaatgcccg gcattttctg aggcatatcg 4680

ctgccagcgt gctgcgcgag ttcacgcaag cgcatgcgct ggccacgcgt ttggtggtgg 4740

actggtccgg accagccgac atgttgtcgg gactgctctg cgatgcggtt gatgcattgg 4800

agcagcaggt tgctgccgcc gaccttcccg aacgcgaccg cgaagcgctg gagcacgacc 4860

tgctgctgtg gactgacgac ctgcggcgtg cttgtctgat ggccaacagc cttgctgtag 4920

ccgagttccg gagcgcatgc caggacgatc cagatgccct gggggcgttc gcctcccgtg 4980

acgagcgaga gatcgcactg tggatgctgg tcttccgcga caagatcttc cgcgacgtcg 5040

aactgcatct ggccttccaa tccaagaccc acggcaagtt ttggaagagg caccgcatcc 5100

agcccggcct cgaactgacg cgcgagcggg tccggctcga acagttctgc catgccgtgg 5160

cgcagcttta caagaagtcc ggcggcggcg atggtgtgca catcgaactc tccgagcgac 5220

gcagcacggc aggcctggtg gatgccatgt ccagtttcca gctgaccctc tatgtcgagg 5280

gaccagtgac ggcgctcacg cacttcacgc agagccattt cactcgcgtc accacccgcg 5340

tggcgctgga gtccgcgttg gtctaccacc cagcgaccgg cgaagtggaa acagtcgtca 5400

agggtggcgc caagaaccat ggtgcgatgc tggagttgtt cggcgagcat gtcgtccagc 5460

aggatctggc ccccgagcgg attgagccgc aacgctacca cctcaacgcc ttgcgcgatg 5520

acctgcagcc ctatgaggat tggtcgatcc acggcgtgga tagggtccgt ctgcgccgcg 5580
cacgcctagc gccggcggcg ggatcaggtg tcagtttcac tgtcgaagcc tcgcccgaca 5640
aggaccagga cgacgccatc cggatcgccc gcaaggcgct caaggtcgag catatgtttg 5700
aggcggaata tcacctcgag gctgcgaccg tcatcgtata cacgcaggcc gccgacggcg 5760
ggcgggcggg ccatttcagc ttcaatatcc gcgcctcggg agcctcgacc atcaagaacc 5820
tgtcgctgag gaaccaggtg ctcgcgcgca aggtcctgca ggcgctgatg gtgatcgacg 5880
ccgaggacga tctcatcgcg accatgtcca ttcccaggga ggctatcgca gcgtgagtca 5940
ggctcaggtt gacgctaccg cgctgctctg ccgattgctg gagcgcgaca aacccgaaat 6000
caacggccag gccctgttcg atggggaccc gcaggcagcg gctcacctgc tgcgtgagcg 6060
gttgctggtg gtcggccgcc cgctcgaatg ggtcacatgc ccggactgcc gtgtggaaat 6120
cgcgcgggtc gtgcgtgatg tctcggccga caggatcgcc ctgcgttgcc cggaatgcga 6180
ggacatcgac gcctcgcgtc gtctgcggga aacgtacacg gcggtgccgg ctcgggccgt 6240
cgctgcctta ctgagcgggc tcgggatgac agccggtggt atgaaggtca tcgaacccga 6300
ccgcgtctgg cggctgggca cgacggagcc gacgcgaggg aagccgctga cttggtactt 6360
cgcacgccag cttgggcgac cggagatcgg cgcccggctg cgcgagcaga tccagctcga 6420
gcgcacggcc ggttcctgcg tggcgctgac caccagcgag ttgccgttgc cgattggctc 6480
gccgctggtg ggcttcgatg tgcgcatgct gtccagcgcg gcgcggataa gtcagagccg 6540
cttcgaattc ttcgcggacc ggcaggctgc gccgggggcg cagcaggtgg gggaggcaga 6600
gccgcggttg accgcgcaga ccaccctgcg ctacgtgcgt tcgctgggca aagccttcat 6660
cgaaggaacg gaatatcctc tggagccgcg ccagcaggcc atgctgcttg ctttgctcaa 6720
cgatctcgat cacgagatgg gtaaggccgc gctcaagatc gcctgcggct cgcaggcgca 6780
acggttctcg ccgagcaagg agttcgaccg caacccggtc gtctacaaga ccttcattcg 6840
ctacctgcgc gacgacgaac gctacgcgct tatcgttcct gacgaggatc gggactggtt 6900
ggggtaggtg ccgcaataca cccattcgcc ggcccagccg gccaaatgcc cgatactcat 6960
gtgctcgacg ctattataag agcgggggca tgtcatcggc gcgaacgagg atttcgagca 7020
aatcgaccct gcaggtttga cagccggtgc ttgcgtttag aacgtggtta gggctgcaga 7080
agcttccgga gctcaggcaa gggaccgttt ctgtcaaaaa tttgcgaagc aagaaaggct 7140
tcgtcaaggg cgccaagtga gcccgaaggg gaccgacgca actgagcaag gacaagcttc 7200
aaagcggcaa tggggaacgc gagcatcgcg tggtctgggt caaaagtgta aaggtgtatt 7260
ttgtcgagaa cataggcttg gaccaactgc cgcactccga cctgttctac gaaagcaaaa 7320

tgagcggggg ctgtaggctc acagaacttc gggattttgt agttggctgc ctctcgctta 7380

tccaccagcc agtcgaacgg tttatcggcc ccaatatcct gtgaggcgag gacgctgctc 7440

gtgtaatgat gggagaacgt gtctaacacg actttgtgcg tagtgcccgc acgctttgtc 7500

ggagtagcgc cggcggttgc ccgccacgaa tagggcgtag ttcttgaata gaagatcccg 7560

acgccaccga gggcaagcga cgcccgcgtc aaatagaagg cgctgtaata gagcttcacg 7620

gtcgcccacg tgtacaactt cctgtctagc ccctgtattg cttcagcaat ggacaaggtt 7680

ccggagtaaa ttgcatcgcg tgcgtcctca ttcaaggagg cagcaagctg gacagcctcc 7740

ctggcttgga gctgcctgga aactatggca gccccggtaa tcccagatag aatggagtcc 7800

gcaacgcgtt gcgcacgatg cataagatta gaatttgaac ttcgttgatg ccttgacttt 7860

ggtggggctg aaggcctctt gcagtcgtgc gtatatcttc ttctgcgccc cgctaccacc 7920

ctgcccctta acttccgcct ggatgataag tgactcgttg aacttcagag ctgcctgaat 7980

tgttttcacc tcaaatgagg cctgattctt acagaaggga atcaattgaa gcctgaggaa 8040

atccattgcg gcaagaaatc ctgatgcgta gagaaagacg ttctctttgt cttcccaacg 8100

atcgccatag atgccctgga gcgcgatgaa ccagttgaga atgatctgct tttgcaactc 8160

tagggaaccg ctgatcaatg aagtcggcga atggaatcgc gagtcatgcc atcttggttt 8220

ttcggattga ggaagggatg cttcgattca gcacggtttt tcgctgcttt cgcacctatc 8280

ttggcgcgct ttgcgcgctc aggacgcaat gctcccaagt agcattacgc accgctccac 8340

catcgcacat cttctcagan nnnnnnnnnn nnnnnnnnnn nnnnnnnnnn nnnnnnnnnn 8400

nnnnnnnnnn nnnnnnnnnn nnnnnnnnnn nnnnnnnnnn nnnnnnnnnn nnnnnnnnnn 8460

nnnnnnnnnn nnnnnnnnnn nnnnnnnnnn nnnnnnnnnn nnnnnnnnnn nnnnnnnnnn 8520

nnnnnnnnnn nnnnnnnnnn nnnnnnnnnn nnnnnnnnnn nnnnnnnnnn nnnnnnnnnn 8580

nnnnnnnnnn nnnnnnnnnn nnnnnnnnnn nnnnnnnnnn nnnnnnnnnn nnnnnnnnnn 8640

nnnnnnnnnn nnnnnnnnnn nnnnnnnnnn nnnnnnnnnn nnnnnnnnnn nnnnnnnnnn 8700

nnnnnnnnnn nnnnnnnnnn nnnnnnnnnn nnnnnnnnnn nnnnnnnnnn nnnnnnnnnn 8760

nnnnnnnnnn nnnnnnnnnn nnnnnnnnnn nnnnnnnnnn nnnnnnnnnn nnnnnnnnnn 8820

nnnnnnnnnn nnnnnnnnnn nnnnnnnnnn nnnnnnnnnn nnnnnnnnnn nnnnnnnnnn 8880

nnnnnnnnnn nnnnnnnnnn nnnnnnnnnn nnnnnnnnnn nnnnnnnnnn nnnnnnnnnn 8940

nnnnnnnnnn nnnnnnnnnn nnnnnnnnnn nnnnnnnnnn nnnnnnnnnn nnnnnnnnnn 9000

nnnnnnnnnn nnnnnnnnnn nnnnnnnnnn nnnnnnnnnn nnnnnnnnnn nnnnnnnnnn 9060

nnnnnnnnnn nnnnnnnnnn nnnnnnnnnn nnnnnnnnnn nnnnnnnnnn nnnnnnnnnn 9120

nnnnnnnnnn nnnnnnnnnn nnnnnnnnnn nnnnnnnnnn nnnnnnnnnn nnnnnnnnnn 9180

nnnnnnnnnn nnnnnnnnnn nnnnnnnnnn nnnnnnnnnn nnnnnnnnnn nnnnnnnnnn 9240

nnnnnnnnnn nnnnnnnnnn nnnnnnnnnn nnnnnnnnnn nnnnnnnngg atgcgattcc 9300

cgcgatacgt ggcctggatg gccggccgcg taagcgcccc gacaagctgc atgccgacaa 9360

ggcttacgac tgccgccgat gtcggcagta tcttaagcgg cacggcataa gggcccggat 9420

cgcccgcaag ggcatcgaga gcagggagcg cctgggccgt taccggtggg tggttgagcg 9480

cacgcacgct tggtttgccg gctttggaaa gatacgcgtt cgtttcgaac gacggctcga 9540

tatccattgc gcgctcctct cgcttgctgc cgccatcatc tgcgctcgat tcgtggatga 9600

cttgtgttag cgactctaag tacgtttgta ccggctctag tggaaagatt gtgtggggcg 9660

gtggcggatt ggaggggcgc tcagagtttg ggccgaataa gtcggatttt atggcggcgc 9720

tttatttgga tgagcaaggc aatttgattg tggaggatcg tattcaggtt cacatgagct 9780

tgcgacttct gcatattcca acggggacgg cgcagcatta cacggtttac agatttaggc 9840

gattgaaata agaatctgtt atggaaccac gcgaaagtag agggccgcgc agaatttatt 9900

tgcgctcaat gtgaatcgtg agtgttttta ttcaccccac aaggccagaa cgcagagtcg 9960

agccaggctt ttgctgggca gcgttgcttt tcgggagcat ttgggcttac tcggaaggat 10020

tgatcgttca tggcggacga ctggctgccg ccgatggggc tgtcgggttg ttgtacctct 10080

acggcgagca aaccggtcat ccatcagcgg ctggtggcgc tctactgctg tttctcgcca 10140

agaacgttta ttgcgccgtg cgaggcaggt tttggctgca gtgcttgctg ctcagtcagg 10200

gctatcgcgt ggtcagtcag tagaagcccg agcatcgggc cactgatatg caggcggcat 10260

gagcgacgtg ccaccgctac aagtacacat caagcagttt ttggggaagt ttctgtgcaa 10320

tcaccacacc gcccggtcca actggccgta ctcctcggcc tatgcgccgt cttcatccca 10380

ccag 10384

<210> 11
<211> 7663
<212> DNA
<213> Ralstonia solanacearum

<220>
<221> misc_feature
<222> (2532)..(2681)
<223> n is a, c, g, or t

<400> 11
tttttattct tctccattat tatttttatt gtgagtttgt ctccctgtat taattctatt 60

aaatcaatta atcattgcaa taccaaaatc aacttacaaa gatatccaat gttgtcaagc 120

aaaaaatgga taaataaaaa aagcaccctt gttgggtgct ttcttgacat tcatggtgtt 180

atttgttaat taataggcaa ataaggagaa ttcaatatga atgtgagcta atggaaaggg 240
atgacctggt gtccaaaatt cacagtcaaa gatttaagat catgcagcac tacatgtttt 300
tgtacgccga tagattggag ggtaagccgt gccgtttatg acagatacaa cgagggcaag 360
ttgattgggt caagagaagg tgatgcatgc cacaattcat tgcttgtgat acaccaggag 420
gaattgaagc tcggttcaat gttggatcgc ttggaggatt tatcgaaaga gcttcaatct 480
cattgaagaa aagtagaatt agaaaaggca ccgaaaggtg cctttgttgt atgctctggt 540
gcaaaaaagt tccgccgcca atttttcttc atcttctttg ctttggtcct tcggacattc 600
atactgaagg atgaacccta tcgggatcat gatttttgat gaagaagtcc ttcggaaacc 660
tctttgcttt atcaacccat aaatgggtat atatctgata tacacctaca cataccatag 720
taggtaaaat caactaagta catcatcaag aatatgaatt aatagtacat ctgcaaagaa 780
gcgctatttg cgaaagcaaa taaaattccc tgagcgaaaa atcaggcacg ttttttatac 840
aacccaagct ggctatccac ttcattccaa tactttttca cagaaggaat ggagacatca 900
gcacgaatgg caacagactg ctgggtgact ggtgttcccg ctgctttcaa tgcaatggca 960
gcttcaagga tggcttcctt ggtgttcttt gtcctagtca cagcactgaa gccagctcca 1020
gcagcttgct tggccttcat ggacatctct ggtggaagct ccatgacacc acggttcttc 1080
tggttgccta tggtgtcctt gtgtctccat gtccatgtgc ctacagatct cgcagtggaa 1140
agcacttcct tggctggtag aacgccgttt aagcactcta ggaaggtctg gttgatagac 1200
agtgcttcag agtccgcagc ctgctggaaa tcagcatagg agccgcactt cctgacctcg 1260
atgtaggccc agtgtctcag ggtgtcaaag agggtggtgt ttcggccctc tagagagtcc 1320
aagagcttgc gtttcttctt gtagcctacc aggtcaaggc cccaatcctt gaagtcttcc 1380
aagtcatatg tggcatggtg gcagatgaca cgccagtggg ggtgcagcgg gttcttgatg 1440
aagtggccta caaagccaag gtcagcccct agcatgcagg tcagagcatt gtctgtagcc 1500
tcaaagaact tctgtggtcc gcggcgtgca tgctcggtgt agtagaccgg ggtcttcagt 1560
tcataaaggt agtggcactt ggcattctca ggattgacca tcacataggt gggtggtggc 1620
aacccaaggt ggtaccaaag atcaaaagag cctgggatgt ctatgtccag ggctatgaac 1680
ttcttgtaga gctggggaag ctctacatag agatgctgca aagctttctc tttcttcctg 1740
aacttgcaac catattccaa atcatttgag cttcttacct tgtctggtag tgcctctata 1800
aaactgttga acataaaaaa ttccttgtat ttgtgcgaaa taaaggaact tcttgttatt 1860
agtcttggca atttagatta aatacgtttt aatatcaaat atcaggtcaa gccgacacag 1920
cttcttctaa agttcctgaa tttctaaatg cctctagctt ccaactgggg gcatttttca 1980

tttcttctcc ttcttttacg aaataaataa ttaaaagtca aatcctatag atgctattga 2040
ctttaaattg atttatatta ttcttgtact gttgcctggc ttcttgttgt tgattattgt 2100
tgttgagtct tggcaatggt ttgtatttat ataaatcatg atcatccttt tgcaaatcag 2160
aacaaggcgc tcttcggagt gcttttttct ttgtgtgctt gacttctgca ctcaattttg 2220
taatttagat aaatgcaaaa ggagtgctat gaagaagtat ggagttggag ttgtcttggc 2280
tggtttaatg atctgtggta atgcgcaaga acccaagtat gggttcgtga aagtagccga 2340
gtctgaagaa atggtcttgt tcgtgaatgt tgattcatat aggtatcgtt acctggcaga 2400
cattcaaagc ttcggggcgg tggttcagat tttcaggaag atgggtggtg gctcgtcatt 2460
cagaaaagaa tatgtactag agcctgactg tcgtagaggt tatggaaggc tatacagcca 2520
cggccttgat gnnnnnnnnn nnnnnnnnnn nnnnnnnnnn nnnnnnnnnn nnnnnnnnnn 2580
nnnnnnnnnn nnnnnnnnnn nnnnnnnnnn nnnnnnnnnn nnnnnnnnnn nnnnnnnnnn 2640
nnnnnnnnnn nnnnnnnnnn nnnnnnnnnn nnnnnnnnnn ntcgaagatt ggtcaatccg 2700
gcggcaagaa acacagaatg ttgattcata taggtatcgt tacctggcag acattcaaag 2760
cttcggggcg gtggttcaga ttttcaggaa gatgggtggt ggctcgtcat tcagaaaaga 2820
atatgtacta gagcctgact gtcgtagagg ttatggaagg ctatacagcc acggccttga 2880
tgacagaaat cttcaatcat atgagtggct gaaggatagt ggaaccgttg caagtattca 2940
tgcagaggtt ctttgccatg tgtcttcttg ggataagccg aagaaataaa aagcaccccg 3000
tagggtgctt ttatctttgc tggtctatct tcttcggcct gtcccgtagc tgcctggtag 3060
catgaaccta gaatccatca ggtcatgact tacctttgct tcagatttct tcttagcagg 3120
tttaggctga gtgcttttga cggcaacagg tttgctcaag gcttgcttcg ctgcctttgc 3180
tacctgttct ggtgtggcct tcttgaactt tcggggcttc gtatcagtga caatgaagtt 3240
gagcttggga cgtagagagg caatgaaccc gctggcatga gtgctgccgt ccagcagagt 3300
caggattttc tccagatagc cgaagtcatg gtgtttggca gcatgttccg tggccatctg 3360
aaccgcaagg ttgtaaatgc ccgactggca caacttcttc agatcaatga aggcactgat 3420
cttcgggtca ctgatagtgc ccaaaaactt ccgcagttct gcaatctgct tctcatccat 3480
ccactgttcc ccctcaatcg ttatcgttct tctatgctct gggcatcagc gtcataccga 3540
tagttgacga tgtctccgtt gatcagcatg ttggcagccg cctcaatgat gtgcagaggg 3600
gcaatgaacc actcacgggg tgaaaaccgt ttgccctgct ggtcatagac atccacgttg 3660
aggcatgcct tgccaaagaa ggtgtgcagg agcagttcaa atttctgtgg attgaggttg 3720
tagcactcaa agacttctac gattttgacc ttggccatca ggtaggtggg ttcctcggcc 3780
gcattcttga tgcgttcggg cactggcacc gtggagtagc caatcttgta gaggttcttg 3840

gtgttggcaa tctcaggcac cgtgctgagg gactgaagca catagaggta gcctgacttc 3900
ttgtcctcgg cttgaatcag ttccagtgcc tctagagctt tctccgtggg gtccaggatg 3960
cggcggcccg tttcgtcctt gtatagctcc gttgcaaggg agcgaagcaa catgtcagac 4020
tcggtgccgt tctcaaagat acagcgcaac cgtgcgttga ccttgccgtt cttgacttcc 4080
ttctcaccga cttcagccac ataggcagta atgccgtgga ggatgaagaa gtgaccaggc 4140
tgaatttgct gctcaccagt gaagccacgt acctcacgaa cgccggcggc aagctctgca 4200
tggcactgct tgaacaaggg ttcaaactgc tcaaagtcct tgcagcgttt gcgctgggcg 4260
atcttgtcgg gcatgtcctt caccaaagga acatgcttga gttggaagat gctggcagct 4320
tcgtcgtcca gcaggcccag agagtcatcc ttgaagatgt cagctacaga ggtgattggc 4380
ttggctggct ccacataggt gagcaacttg aactcatcca cgtcctgcaa agcttcgcat 4440
ttctccttgc tggcacgaag ccccttgagc ctgttgaata gctggaactc aagaatgttg 4500
cccggattgg actgcggctc cttgccgtgc tgacggtaga aatcgttgat ctgctggaat 4560
gaagccagca gccgttcatc cacactgata gcagcacgct tgggcttcac attgagcaag 4620
cccatttcat catcttgcag aatctttaga aggtcgtcct tctccacggt cagactcctt 4680
gctgacggcg ttgtgccttc agatagacca cagcatcagc catacgccgc tccaacggat 4740
cgagcgaagc aatgttcggc tccttgccgt gggtcttcac gaagtcctta attttgggcc 4800
aaagcaggat ggcttcctcg tcactcatct gcacacgagt agcttcaatg gtgtcttgaa 4860
tcaccttgaa gattttcgtg gtcagcgact tggacaggat ttcaaacgcc ttctggaagg 4920
ggttcacccg gtcgattagg tcaatgtgca ggtcgtcaat gttgatgaac ttgtcggcca 4980
tgcgaacaaa gcgtttgtca ccttcctgct tgatctcgcc attcttgatg accgaatcca 5040
ccaccacgtg ctgacgaact tcttcaacct cttcgtcact gagttcagga tacttgacct 5100
tgatgatttt ggggatcatg accttgttaa tcacttcggg atcaacattg ccaggcagtg 5160
ccttcacaat cttgtcgtca gacaggattg ccgccttcag gtcattgagg tcagattcaa 5220
tgatgtcctt gactcgcttg gaggtaggct ccttcaagcc tcgcaccttg atttcagtat 5280
cggtgctctt gtcgtcgtca ctgaccttgg tcttgaactt gaagttgggt gcaagcacct 5340
gctccatgag caaggaacag gtgatggcct tgagcatgtt gttgaccgac agcttcacca 5400
ggtcgtcagt ggcatcaggc tgggcaatga gatttgtgaa ctgggcatgg ctcttgttgt 5460
tgctatcgcg ggtgcagcgg ccaatgattt ggatgatctc agtcagcgat ccgcggtagc 5520
ctacggtcaa ggcatgctca cagaatttcc agtcaaagcc ttccttcgcc atgcccaagg 5580
caatgatgag atccatgtct tctggcttct caatgcggcg taggtattca acgatacgtt 5640

cgcgggcttt gggctcgtca tgcacgagat cggcaacctt caaatccttg ccgtcaccat 5700

gccgcttgac gtgaatgaca caggtttcag ggtcccggtg ggtgacggtg ccaatgatgt 5760

ctagaatgcg atccacctcg tcatacttgt ccttggtgga ttcaccagac tgcacgcttg 5820

gtatgtgcag aatgttcttc tgatcggtgt tcagaatttc atcgatggca ctggtgtact 5880

tgccttggta gaagtggtag ccaatgccca gagacttgag gaaggtatag ccgtttaact 5940

gctcgtagta gttgtaggtg actttggtga actgagcctc gtcttcaggc agcaggaccg 6000

gcacgctgtc accacggaag taggagccag tcattgccac gatatgggca tccgacttcg 6060

ccatgacgga acgtagtagc tcaccaagtc ggttctcacc atcagcagag acatggtgga 6120

actcgtcaat ggcaagcagc acattatcaa agtccgattc ctccaatgcc tcaaaggcaa 6180

agcgaagagt ggcgtgggtg cagatgagta tcttggctgg acccttgatg aactccacga 6240

aagcttgaac cttgctggag tcaccaccag gtgtgcagag gttgtttttg ggctctacga 6300

tccagtcaga aaagaagcca aacttgctca gttccaccgt gtcaaaggaa ccaccaatgg 6360

accgttcagg cacggcaatg attgccttgg acagcccttg gttgatgagc ttgtccagtg 6420

caatgaacat gagggcacga gacttaccag aagctggcgg tgctttcagc agcaggtatt 6480

gagcattgcg agcttcaaag gcacgctcct gcatctcacg catgccatat tggttagtgc 6540

tggtgctgtg accagtctgg ccgtaggtga cttcaacaat gttaggcatg ctcggcttcc 6600

atcatttttt cgtatagttt gaagaggcat tccaagcgtt cggtatcgct gtagaacggc 6660

ttaatctggt agcactgctc aaccaccaca tccagttcgt ggtgtgcctg cttcaagtca 6720

gcaggcatag tgtcagggtc atacaggtcc tcgatgctca gttcaggata cgcttcacgg 6780

atggcgacaa tcgccatagc cttctcagcc actgcttgcc gtttcttctc gctggcatca 6840

gggaagggga aggtgttgta gcaaatctca gcagagtagc gaagtcgcgt ttcaagctgc 6900

ccccccaccg tgcgaaccca gaggatgtgc agtttggaag caatgatccc aaagagaaca 6960

ggatcttgct tgtaaatgac atttgcagag tttgtgataa ctacattggc atcaaggtag 7020

ccaataggca catactctct tcgttccgaa gaggttgcag gaacaatgat agagccgccc 7080

ttgatttgaa ctcgctgggc aaacttatga gcaatcttgg catagccatt ggtggtcttg 7140

gccttggact tcacccggaa gtcatagcag gcatcaatgc gtgtcttgat aggtgggatt 7200

gagcgagcaa gttcaagctg ttcgtcctcg atccaaaggc actgacgtgg attgccatcg 7260

atgaattctt gggtgccgta gagagttctg aatagtggct tggacttcgg atactgctga 7320

acgatggcat cgcgttcttc aggcttcaga atgaggttgc caccgtcacg agccatgcaa 7380

ccgatcacca ttgccgggaa ccctgacaga acttcagagg tccgacgcac gaaaacatcc 7440

ttaccttcaa tcaggtaagg attgatgttc ttcacaatct tgtagctgtc ggctccaaac 7500

agcagcttcg gcttgtcagc cgtgttcctc accccaacaa tcacacacgt aacaccagca 7560

ttgttctttg cgaagttgga ccacttgaat gaggtgtagg caaagtcaat ttcaagacca 7620

ggctggaaga ctctgcatgt tagtagagca ggtggaaaag acg 7663

<210> 12
<211> 1398
<212> DNA
<213> Ralstonia solanacearum

<400> 12
agcgcgcaaa gcgcgccaag ataggtgcga aagcagcgaa aaaccgtgct gaatcgaagc 60

atcccttcct caatccgaaa aaccaagatg gcatgactcg cgattccatt cgccgacttc 120

attgatcagc ggttccctag cttcatcatg agcatagatc ggatcgccca cgattctgtc 180

tgtggcactt ttacgctcgc ccgactcacc cttgataagg atgttcaacg acactgggca 240

aacctgcccg ataggcagct tgacgtcaat cagaccctgg caggcgactg acattgttgc 300

caaaaatgac gttgcgatga gcgcgtccgg cgccttgacc ttgttcatca cctcgtatat 360

cacctcggca atttctattg gaaacgcggc gattggatat ggaggtggcg catgattgta 420

accattgttc atctttcaat ctcaaaaaag aggggcgccg aagcgcccca tgatttatta 480

aacctgttcg ttgtgccttt tcagcagttc gtcccatttc ttccgaaatg ttattggcga 540

gtactttgca cctagttttt catagtgcct acgaatcttc gccagactga caccggcgtt 600

gaaacgctcc agggctttca cgaagtgctc ttccaggatg atctccttct gagccttgcc 660

ctcagcatcc aactcgtccc aaccatctgc agaagcgtcg agttggctcg tgttgctgcc 720

atgctcatcg caagcattcg cctcttcggg cattctccca atagctggag atatacccga 780

catcatcttt ttatcgccgt gcatgggcag tcctatttag agagaggcct tcctcttcag 840

gtagttggcg gtcgcctcac cgagaacacg tgttgcgcta ccaattctga agggttttgg 900

aagatcgcta cccagaacgt caatccacgc agccccgagc caatactcca gacacaacca 960

agaagttcta tgggaaaaca gcggacctgc cgtgcagacc cacacactgc ccgcgccccc 1020

gaatactgat cgccttgtca cacgatccga cgtaccggag ccagtctatc cttcaccccg 1080

atagtcggca cgcacagaac agccttacag ggcacaggcc taacagcaga acggacccgt 1140

gactcatccc ccgaggctga cgacctcatg gccctcgcag agcacttcag gacctatcgc 1200

catcgacctt actggaatgc ccacccagcg acgcgggaga agcgagcgac agaagaaaac 1260

tacccatggc tggatgatgg gccgtttgat gggccagaaa aacaaaaacc ccgtaagtcc 1320

ttgaacttac ggggttttgt ctttgcaact ggcggagagg gtccttttcg aactcctccc 1380

gtgcttaggg cgacgggt 1398

<210> 13
<211> 2525
<212> DNA
<213> Ralstonia solanacearum

<400> 13
tggcggaact cctgtgatgg tgaggaatgg tgcaacgtcc tgaacgttgc gcagtgatgc 60

caagcggccg gctcagccgg tgcgatacgc cgaccctgcc ggtaacgagc atctaccaag 120

ggttgcccgg gtgcaaacag ggcgcgtgcc gggtcgttgc cgcgtgtcgg aaagtcgggg 180

aggaacgcac ttcttgcgta gcgcttgagg ggctccggcc gcgcaggttt ggggtggcgg 240

gcggtgccct tggttaaggc aggggccgcg aacttgcgag tgtttggggt ctgggatgct 300

tcggctgtca ggggcaatgg cacccgagac gtctgtgcat ttgttgggcc gtttagccgt 360

ttaaacagcg gcgttgaggg ttgagccagg gatggttttc aaacctacgt atcctgcttg 420

caactcattg aattcattgg aggcattggt gccggcaagc gttttggatc gggcgtctgc 480

ggcgaccatt ttatttaaca taagataaac aggtcggcct tctgaatggc tcaacggcgc 540

ggctttggcg gccgagggtg tcacaaaatt tgtgacacct gcgaccacca ccaggaggag 600

ggctgccgtc gcactacctg ccatcctcgt cgaaaaaacc tcccacagag accgttgtcc 660

gtgacgctcg gcgcgatcca tatcggctgc catgatcacc ggcaaggggt tttccagctc 720

gagcagttgc gccaggcgca ggcacgcttc gttgtccatc ttgcggtcgc ccttgcggta 780

ttgcgtcacc gcgttgccgg ctacgcctag cagttcccct acttggttgt cagacgtcaa 840

gcccctttga gctcggagct ggtcgaagta tttgccgagt tgcattgcac ctcctcttgc 900

gtgctcacgc tttgtgagta cactcacgat ccgtgagcct cacggcttgt gagactgagc 960

gaaatataac cggttaaccc gggcgagggg taaacgatgc agcgtcgaga catcaagcct 1020

gtggcgtcag ctaccgcagg tacgcgtatg ccggcctggc tacgccaggt gctggagcag 1080

agggccgccg tgcgccgcgt gcgcgccgtc gagctggcat accgccatgc atcgggcggc 1140

cgtgtggccg ctccccgccg ttccctttcc cttgacgagg tgaatgcagc actttttgac 1200

gctgctgacg ccgagttttt ggcctcgcgc ggcgaggctg ctttttgtca ggcgggcgtg 1260

gtagcaccgc ctgacagtct cacggatgag actccgcctg attctggcga ggggttctga 1320

gcgcatggcc tacgacaccg tccgtctgcg ttccccgtac cttggcaaca ccgttgttga 1380

gcgcatcaaa cgtcagtgcc tgctgcgctc gggcctggat tgcgccagcg gcgaaatcct 1440

ctatgagttg ttcacgggcg aactgcttgg ctcgtgggat tcgcggatct cggtgatccc 1500

gaagaacgag gaatgggtgg tcaacaagaa cggccgcccc gagctggtgc cgtgcgagcc 1560

gtacgtgctg attgaggcgt ccgtgcacaa gatcaagcac ggccacaacg tgtacggcgg 1620

gccgaccagt ttccaccagg cgtgccgcga cttcgtgcat ctggtcgagg atctgttgga 1680

gaccgatctt ccggccgccg actggtggac cgttcatcgc gtcgacgtgg cgtcggtcta 1740

tcggctctcg aaggctgcgt gcaaggagtt cttcgacggg ctgcagctca tgagcttccc 1800

gcgtcggaag aagggcgcgg ccaagtattc gatggcggtg tacttcgccg gcaagaccac 1860

gacggtcaag ttctatcaca agggcaccga gttccaggtg cacgaccgtg gtcgcctgcg 1920

gggcttcttc tcccaggttt tcaagcattc gcacggacac gacgacgagc ggagtcgcgt 1980

gttggcggaa cgcaagatcg cagcactcca gcgcctggcg gacggccgct tgcgcgccga 2040

ggtggaggtg cacagcgaca agctccagta cgactttggc aagcatccgc gagtggatga 2100

ggtgacggac gcctatttgg agggcgtctt tgacaccgag atagagaagc tcctgcgcga 2160

ggggaaacag gcgatggcga cggtacgaga gagcagggcg gtgctgcaca ggttgaaggg 2220

cgtctatggg gacgcgctcg gcatgcgctt gtacggcttc tggggctcgc tcaccacgtt 2280

gggcgacgag gtgacgcgtg agcatttcgc gaaggcgacg ttctatcgct gccgtaagca 2340

gttggaggac gcgggcgtgt cgtggcgcgg cacggacgtg cacgtcatcg caaacgatgc 2400

cgtgcttcca cacgatttca gtccggtacg gacggaccca cggttgtgcc atctcccggc 2460

gcgcaaccgc gaggaattcc aggtgagccg ggaaacgttg cggcttgccg cgtaaggaga 2520

agtga 2525

<210> 14
<211> 1277
<212> DNA
<213> Ralstonia solanacearum

<400> 14
catcaggatc cttggtcggg ttcagatctc tttgctctgc cgttgcagct cgccccgagc 60

ctgccgtcaa aacaagacag gccagcacga ctgccatctc aggtcgcatg gttcacctcc 120

cagtcgatgc gctcgggtaa gacgaagctg actactggcg atgcagggcc cgatatcggc 180

ggatcgccgg ccagcgtcgc cggaattttc cttgccgccc atgcagctca ggggaccggg 240

acggctggtc cttcaagacc agttgcccct tgaccagcca agcgccggca aaagacatca 300

cggcaagcgt ttccggccaa aaaatggacg cctcgccccc caggagccag agcatggcaa 360

tccagccgaa cgcgaatccc atcaggcacc cacagatcac gtatacccaa ttgcgccgcc 420

gcttttgtgg cgtggggttc gccggcgtat tgacgcgaaa accgaaggtc acaagataga 480

acgccagcag aaagaacgcc cccacgaagt agaagtgata cccgagcggc ccatgcggcc 540

cgtgctgcag ggcgtcaatg agcctggcct cggcatcggg tgttgcggcc tgctgcgggg 600

tgatcgcgct tggtatgatg cccatcggga acagcccgat tccgatcgct gacacgcccg 660

cgctgatcgt cacccagcga tcccgcggat ggccggtccg aaagaacgcg agcaccaggc 720

cgccgacggc cagagaaccg acgaacacat cgcgcaaact ggtgtagtag taagcgctga 780

tcgaattcgt cgtccatatg tggtcgaagg ccagcgcgaa gacccgcaca gtgaccggca 840

tcagcagcgc gatccagccc acggtctgct gaagagcatg atccgaaatc agcaatggat 900

gacgggtgct ggcagtcatg atgccccctg ctttcccgaa cgctttcaca ttcatatggc 960

ttgtcgggcc ccgttcatcg ccggcgcctc tgccgttcgt tgtcccgtga tgccaagtct 1020

cccaccatcg ctcgtgttct tgcgcggtgc ctgcgggcag tgcattcgcc tggcactcgg 1080

atgctctgca cattccccgc cggcgtttca cgcattttcg atgggcggtc tgcgctgaga 1140

caggtatagg ttcgaaacac gtagcgcgcc accggcatga aggattaacg agaactgggt 1200

agcgcttgcg atgcagccga aaaaaacaaa gggccgcgat gaagcggccc tctcggcaga 1260

tcggaactga cggcgct 1277

<210> 15
<211> 2308
<212> DNA
<213> Ralstonia solanacearum

<400> 15
tcacttcctg attgctgctg ctagtcataa acacggtcgt atgcctaccc gtagtttaag 60

tgggtgactg tgtccggaga ttcagggggc cgctccagga ggtgagcatt agacatccgc 120

cacgtaacgt ttcgagacga cttgtaatgg tattcgcgag cgagttcacc ctcggcaact 180

gcgaactctg gaacgcccag atcatcgtaa acgacgcgtt gctgctcgtt acccaggcac 240

cttggcgata gtccgagtgc catccagacc ttttcagcga gggcatacgt gtagtgcgat 300

ccgtctcccc aggacgcgac aatgtagtca taaccggaca cgctgggcga aaactccgcg 360

ctcaagcctt tgttgccgag aatggggaca gagtaggaca ccacgccact tgcctctgcg 420

tccaggatag gaacgggact ttcaaccgtc tccgggacga ctgccattag catgtgcacg 480

tattcgctgt tgggcccgtg attgtcaaac tcaaggtcat tggtaacgtc cacctgctcc 540

aggggatcca gccctacagg actgagaagc gtgcgcaggc gtgccggtag aacagctccc 600

tgaataggga acgtggcgag atggtcaatg gtaggcatcg gcttagcggc tcaattgcat 660

ccgagtcttc gattccgcta tggtagccgc gaccattctc gttgcgtgcc ttgccgtaag 720

cggtccgctg gaatgagcta ttgagcaaaa ctgcggattg agctcggcta cttgaggccg 780

taatacttga gcaacgcttc tttgacgacg tccgtggcaa cagcaacgcc catctttcaa 840

gtgcctgacg cggccttctt gagattctca actagtcagt tacgcacttt aggtcccatg 900

ggctccgcgt tgctctccgg cttttctgac gcagcgagat gcgccaactc ctgggcaaac 960

gggcagcctc atggctgctc aaaatgctgt gatactcgtc attactggcg tcatgctgat 1020

acacatagcc agccttgggg ccgaaatgat cgtctggttg gtcagggaaa tcttcccggc 1080

caacgccgtc caaatacgtg tgccagtcgg ctaccgcgct gttgttgcct gtcgtgctca 1140

tggcttcccc tgattctgtg ggtgtccgca tttcccctgt tgcaggctgc aatgcaagag 1200

ccgtgcagcc ggaactacga ctgcacggcg gtactgccgc aatggaagcg cgacggcgat 1260

ccgatgcgtt gcttgttcat gggctgatcg aaggagtcgc ctgctacgcg gcttgtagcg 1320

cagtttcgct gttacgtagg caggcacgag aatgagcgcg aggtacgctc ccagataggt 1380

ggctatcgag ctccacatcg gcgcaaacac gtaatagcga tccatccagt agaaataaag 1440

ttggaagaga atggctggca gcgcactgaa cagaacccac cggatcggcg cattgcagaa 1500

cgcgatgcct agcgcaacac cctgcaggat agcggccacc gcttcagtag gcgaccagta 1560

cgtcctaatc aggtcgtctc ggcctcgtat tagcagcagg ctgatcgtac cggaaaaaat 1620

cggccacacc agaagtacct tcgataataa ggaaagttca actggccttc tccctccgag 1680

ccatttcttg aacccgtcaa tgtttccata gccttggttg cgaagtccgg cgtaaggcaa 1740

gcaaaaaaca atgatcactc tcagtgccaa ccctagcccc agcccacacc acgcaacgag 1800

caggaacaga taccaacccc cgagcgttat atcgtcaggg taggttttcg catcgatctg 1860

gcatgcgtca tatcaggcgg cgactctcag gacgaccgat gcggctacga acaaccagaa 1920

actcacctca agctgcctgc cccatctcaa cagaagaggc gggatggcaa acggtccaag 1980

aaaaggcaga caaaacagaa gaagacccaa gacagccaac acgcaatccc ccttttttgg 2040

attcccattc tttgccccgc gcatggggcc ttaacgcgag tggtacggca acgaccaaga 2100

cctgggactg aacacctagg gagcaatgcc aatgtcggca tcgccaccgg acctgcgtca 2160

ggattattcg tgctggacgt agacccaggc catggtggag acgagagttt taaacatctc 2220

tgccaggagc acggcgagct acgggacatc aattcattga cgtacacatg ctcagcgaaa 2280

caaccttgac gatcaagacc gtgcactt 2308

<210> 16
<211> 1693
<212> DNA
<213> Ralstonia solanacearum

<400> 16
ctcgctcgcc tctcggatag tggggcccaa tcgccgacag caagcgctgc cacggcacga 60

ccttctccat ctctgccagg aagcgctggc gtttggtcac gcgcttctta cctgcgcttt 120

ccgcttctgc aaagctcatc tgccgcttca tcactatggc tccgttccgt gagctacctg 180

ctacaacggc ctcagctgcg tcagcgatga cggctgagac tgataaatca gcgtttccct 240

agcgtgacat tctgcatgat ggcctccgca aagaaaaaca ccctaccagc gttacccgct 300

cgtaggatgg gggtgaccat ctcattagac ccggccgagg ccgagttttg tcagacgatg 360

cccaccgcgt gggttggcgc ctcgggacat ctacgacgcg aggcctggat tacgttgagc 420

ccattgtttt gatgaagcag caaacccatc ggcatcacca agagacgcct agtccgcctc 480

tttcattact ctcgctctca cacgccttca cgcctaatct cgttcttgag aatttgaagt 540

ccttcatcag ccagccctcg aacattggag ggctttcgat cctccagacc acgcagatcc 600

tcaagcagct ttcgctcatc tacatgtggg aagttccggg ccacaaaatc gcagaccgcc 660

aaacacaagt ccgaatcttc gtacggggca tagtgccccc tgaagattgc tccgagttgc 720

tcaaagtggt cgggcctcaa ccgatgtcgt ccggcgctca gagcctgttt ggaaatgcgc 780

ggatatcagc aagaacaggc tccgcaacga cgggaaaggt ccgttcaggc gccgaatttg 840

cttacatttt aggcaaatca agcgccgatg cggtagctgt gcgtccccgt cgggcaattt 900

ccaaacgggc tctcagcacc atcaggaaat tccatacgcc aggttgccga taaagcgttg 960

gttctgtgag caaccgctcg atgaactcga aaggaccttg tggtagcccc tccaaatcca 1020

tcaactcatc ctcgaactca tcagcaatct tggtgcagat gctttcactt ttcgatgtcg 1080

ccgattggct aatcgccatc gcaaagttgt tcagtttctc gttgctcaag gacatggctt 1140

gcaccccctg ccgtaattgc cttcgagctc gttcaatcgt ccccactgcc gattgataag 1200

cttttcgtgc ccacggactg tgtcgcgcag tttctcactt ggcccaatcc ggtaaggaat 1260

ccgggaggcg tccacatgag cggctcctaa tcatcagcca ccgcactgct tgggcgccat 1320

gtcacgcgga aatccgtagc aaaaccagat gagcctccta tcatcaggct tagcgacaat 1380

aataacggat acccgccgtt gatagtttat agccataata atattttcac cagccaaaaa 1440

ataatccgta tctcgtgttt ttaacgttga tataaagttt ttagtttccg agcaagaaat 1500

tagagatgtg cgtttatcca gccagcatcg ctcagcggca tctctcgccg gggacatcgc 1560

ctgcagcata gtcataatcg cagctctctt ctcaggaatg gaatcatacc cccgcagcga 1620

caaccaccca atcaaggcca gcccaaggaa aaatgcaatc caaattgaca atcttttcga 1680

cataacaaac tcc 1693

<210> 17
<211> 3733
<212> DNA
<213> Ralstonia solanacearum

<400> 17
gtcgatgccc gaggcgttcc cctggccatc accgtaacag gggcgaatcg acatgactcg 60

atcgcattcg aatccacctt ggatgcgatt cccgcgatac gtggcctgga tggccggtcg 120

cgtaagcgcc ccgacaagct gcatgccgac aaggcttacg actgccgccg atgccggcag 180

tatttgaagc ggcgcggcat aagggcccgg atcgcccgca agggcatcga gagcagggag 240

cgcctgggcc gttaccggtg ggtggttgag cgcacgcacg cttggtttgc cggctttgga 300

aagatacgcg ttcgtttcga acgacggctc gatatccatt gcgcgctcct ctcgcttgct 360

gcctccatca tctgcgctcg attcgtggat gacttgtgtt agcgactctt agtcctcggt 420

actacgcctg actcgctcga ataactcctg ccaagtctcg ccgggtacct tggggcccat 480

ccaccagtcc atctcgtccc actgacgatg aactcgaacg cgcgaggcgg tcttgccgtg 540

gaccttcgct agtgcgagtt tctcaacgga atcgtagccc tcacggacgc agcttccaag 600

cttctcgagt tcgaccgagg caagctcgtc catgtccaag gacagttctt ccgccgcaaa 660

atgccgacgc acaagttccg gaagggagaa gatgtaagcg agctccggga gaacccgcag 720

gacgaaacgg cgcgcttgct cgcagcgttt ctgctgttgc aacgtgggct tcagcttctg 780

aatctccgct aacgtctgac cgctaaccca cgccctcagc gctgcaatca caacgtcgac 840

ggcgaactct cctcgctccg cgtcgtcctc aagcgacttg aacggctcgc ccatgaatcc 900

gtccatgctc tcacgcctgg tcatctgcga gatgaggatg ggccgctcgg tcagccacgt 960

ggccatccac gtgaaccagt ccagcgtcgt gcgcccggca ggaggcgacg tcttgaggcg 1020

aaccttcagc tcgcgcagca cgttcgacgg cgcgccgtag ttggctgcca tcttgtcggc 1080

ccaatcgggc tcctcatcca gttcttgaag cttcctgcgt gcgcgcagtg catggttaat 1140

gcaatcgttc agttgctctt cccgcgcctg cttcctcagc ttgaacgccg cgaacgactt 1200

cttcaatagc gtcctggccg ggccatcgac gtcctcggcc tgcgtcgaga tgggaagccg 1260

gcgcaaaagg tagtccgcat cgccaccggc acgctcgcca agttcttgaa tacggtcgag 1320

gatagcttcc agcggatctt cgatcgccaa gcattggtcg ctttgcgcaa agatgccttg 1380

aagatccttc caatgcttgc caatcctatt attcgaatcg tcgatgtcca caacgttgct 1440

gggaatcacc agcaccagac catacgaact ctgcccggcc cgcccagcgc gaccggcagc 1500

gttgagcaga tcatgcgcct tcatctgcgc gagctgcttg ttctcctcat cgaacttact 1560

gtcgcccgcg atgatgacca cgtgactcgg caggttcatt ccctgcgcca gcgtcgacgt 1620

ggcaaccagc acggacacgc catcgggacg cttgaagagc gactcgtgca gttgccgctc 1680

ggtcggcaga agaagcccat gatgacagag gctcggccac cgcgcgaact tcccttcgac 1740

ggcatcgatg tagagcgccg actcgtcgcc cagctcatca agcgtcaggt tgtacagatg 1800

ctgctcatct tccgtgaagg caatgtttgc ctccccaaga tgctcacctg cggtctcctg 1860

agcgctcata gcgtacggga cggtctgcgc gaagatgagc gatttgattc ccttgttgcg 1920

ggatgcgagc gacgcaagct cagctgccac gttgttcaca ttcggcgtca gcttccacgt 1980

ggttgcattc accgaaagcg gcatctctct gtcagaaagc gggagtaacg cgtaatcatc 2040

tcggcgcatc gagtcccacc ggaacttcag tccaagcagg cccagcggcc gtaccttgag 2100

ttcacgcttc agcgcgaccg gcggcccagg cttgcgcttg ccggcggcca acaattcttg 2160

gcgcttcgcc gccagtcgga tgcgcaggtc ggcaatttgg tcgctcggaa agaccacaca 2220

gccgcgcacc tgccgtgtcg gcttccacgc catgtttagc ggcaagcact tgcgaccggt 2280

aagctcagcc aaccaagccg cgacctcctc agtgttgctc atcatggccg agagcagcaa 2340

gtagtctgcc tcaggtgcat gcgtggagag gttcagaatg caaagcatcg catcgacggc 2400

gcgacggcta cgatcgttca cgcccgcatg cagcagatgg cactcatcga acacgagcag 2460

gccaacgtcc ttgaagagct ccggctgaaa ccccatcaac gccaagcagc gctccggcgt 2520

catcaccgac atcggtggca gtcgagtgcc caggtcaaaa tcgaacgggt tgtcgctcgc 2580

ccgctctcga ctcagccgcg cctcaggaaa ggccttctgg agcgcgcgcg cggtctggtc 2640

gaccagcgcc agagtgggcg ccagaaagat ggtgtccttc ccttgacaca ggttcgaaag 2700

aatcttgagc tcagatagag tcgacttccc cgccccagtg ggaaagccaa tggcagctga 2760

aaccccaggg gtcaggtagc cctgctccac tgcattccgg tgatttgccc aaagatacgg 2820

ccgagtcttc gcaatgagcg ccaggcccga aatccaaagg ggtgtgccga cactaggcgg 2880

tgccggcagg caagccaagc tatcgatcgg cagttcgcga cccacggcct tcaacaaaga 2940

cgccaagtgg cgaggcccag ggaagccgct gttgccaagt cggtcatggg acaagacgac 3000

cggctgtgcg cacatgtctt cgacacgctc cagttcgggt aacgacggct gataacctgg 3060

gcgtccaagc acctcagcaa cgatgctttg aacgcaacga tgcagcatca ggtatagcgc 3120

gctcgctccg acttcggcga tcgtcaggtc gccatccaac gatggcggtt ccgggacggt 3180

cgagtcagca agcagtgcat caagatcgcc ggtcgccaac cgcttgatgt cctgcaagag 3240

cctgcgctcc acctgtggca agccatcaga gagctgaatc tgctgcgaca tctctgcggc 3300

atccgggcta gcgcctgcgg caaggaacag gagcgtcgca gagacctctg gcgcaatccc 3360

gccggccgtg agcggagcaa ggtcatcccg gtcagccggg tccacaagcc cccgcgcctg 3420

cagggtcaca tagtgcgctg tcgccgcaag gaaggccgcg ccgcgccgct gagggccttc 3480

agggttcacg gaggcgatgg cctcctgggt gaacgcaatc ttcctaagcc tctcaagttc 3540

gggccgcagg gtgtcaagca gatcagtggc gtcggacgtt ccattcgcca tccttcgaac 3600

gcgcagccgc actccaacta tttcggcgta ggctttagtc agcgccttgg gcagtgagtt 3660

ggggtcaaga ccagacagtt ctggcgcaga gcgaataagc tctgaggtgg agtcatcaaa 3720

cacgcgtcgc tct 3733

<210> 18
<211> 1813
<212> DNA
<213> Ralstonia solanacearum

<400> 18
cgggaaggcc caatcaagga cttggtgatc gcgggggagc gaaccaaggc acagattcgg 60

gctcgggtcg aacatccgtt ccaagtcatc aagaatctgt tggttcatcg caagtttcgt 120

tacaagggtt tggccaagaa cacggcgcaa ctgttcaccc tgtttggttt ggccaatctg 180

gtgctcgcca ggaggcagtt gctggccagt cctgggagca ttgcgtcctg agcgcgcaaa 240

gcgcgccaag ataggtgcga aagcagcgaa aaaccgtgct gaatcgaagc atcccttcct 300

caatccgaaa aaccaagatg gcatgactcg cgattccatt cgccgacttc attgatcagc 360

ggttccctaa agtcatccgc cagcctctcg atctccgtca gcaaccggca gccggagacc 420

gacaggccag ggcacgacgt cgaacggagc ccgtcgtttt cgcctcggtc tcggatgatg 480

tccccggatg cactgctggg cgaatattcg cagcgcagcg acagcagcct gagccagctg 540

cgccgggcac ccgactccat gcaggtgtcg tcggcccgat ccagtgcatt ggattcgctc 600

gggggaaacc cgccaccgcc cacggtccac acgccccatc ccgggcaaga cagccatggt 660

gtcgacgttc ccaaccggat gcagatcgcc gagcagttgc gcgacatctt cagaccgcac 720

gtcaacagtg ccaacgatgc gcaattcgaa gccctgatcc agcagcgagc cgagcggctc 780

gacgagatgg gagaaacgcc ggcgagcctc gccgaggtgc tgtccaaggg tgccaaccgg 840

gaccggctgg cgcaggccac ggtgggcttc gtgcgctcgg tgccgttcgg cgttgcctcg 900

cgcctcttcg atgtgaagca gcaactcacg gccttcgcca agacgccggc gcaggtgggc 960

gcggtggttg gcgccggatc gggcgtggcg gacgccttcg gcggcagcct ggtgaccaag 1020

gccaccagca acacgcaatg gctggcggcc tcggccgcgc acctcgaacc cgtcatggcg 1080

caagcgcacc aggccgtgca gcccagcctg cgccggctgg cggtggaggt gagcggggcc 1140

ttccaggcct attcgctgcg caacgtggtc cgcaccggcg tcgcgccgct ggcaacccat 1200

gtcctggggg cggcaacggc ggccaacgtg gattcgtgga tcgccgcggt gggcgggccg 1260

gtggccggcg cggcggccta catggcgatg cagcacatga acgagaccca gcaccgcacg 1320

ggcgcggaat acctgctggg ccgaacggac tgggaaacgc agttcacgct gctcaagcag 1380

tcgacgtgga cggatccgct gaagggggcc gcccagcgcg cggcaaaact gcccgtcgat 1440

ctcctgaccg agacgctggc cgccacgcgc agcctgttca ccgccaccaa catcatcaag 1500

aatgcgggcg cgctggccgg cggctttgcc ggtgtgctga ccgcgcagac cgccgccggc 1560

cggatggcga cgaacgccgg tgccagcgag gaagccgtcg tggccatcaa gcgggccgtc 1620

agcacggtgc tgtcggcacc ggtctatgcc acgtggacga cggccgatgt cgcggccggc 1680

cccatcgtcg atgcggcggc cgggcgcatc cagcaagcgt tcgcacccag gccgcaagcg 1740

gtgccccccg cgcataccgc catcgacatc gtgtgatgtg tgaagcgtga ggtttgacgc 1800

cggcggggcc ggc 1813

<210> 19
<211> 2459
<212> DNA
<213> Ralstonia solanacearum

<400> 19
gccccgtcct tgttgctgct cggatgttgg gtatccagcg accaaagtct caccgggctt 60

tggttacggt taagcaggag aggtggaatg aaattcctgt cgcgtacacc ggtcttgttg 120

gcggtgatga tcgggctgac tcgcagcgcc tatgccgacg ttctgcccca acctgcaacc 180

gcaaagggag agccgacaat cgacgtccgg ggcggcgact ccattgtcga aagcatcgac 240

atgtctccac aggccactcg cttctggcgc aatctccgtg agacggccaa caacatcgac 300

ctgaacgacc atgagaaggt cgcatctcag ctcgctctca atggacacga tcgtcgggcg 360

ctgcaacgcc cgatgcagtt cagttatcca agtctgccgc tgctccgcga atccgtgcgg 420

gtgacgacct atcgtgtccg gtcgggtggg tccgtaacac gatggacctt cttgcctgac 480

ctgaggctcc tatgcctgtc acgtagagag gtcgagcgaa acttcggagc cggcgagaac 540

gattcacaca ttcccccaca ggttgtcgac gcaggatatg gcggaacctt cgagacggta 600

acgagccgac ttggttacgt ccattcgatg cggtacaaag cgaagggcgg ggagcgatac 660

gttgaggtgt cgctctcccc tggaggatgt gccgacttca tcgggttgat tcaattcaac 720

gccaatgatt ttgggttgcc tgagccgccg aacgcaatct cgatagagta gcgttcgcga 780

aggtctggcg aatatcctca cagcgttcgt ccgctcctgc caattcttgg cggttcatat 840

tcacaggtgg atgtagttgt aggcggtttg gagcccattc gcgggcatca atccacagtc 900

gacaacctgt ccgcccggac aatgacaggc gccacaaaaa taaccttgcg tagcgatgat 960

tgtggcccgt acggctgcat gcggaaatgt ccgcgccgcc aatgcgccgg cagttcctgg 1020

cccggcgtca agcgttcacc atgctccccg ccatggacgc cttcgagcga tgccggccca 1080

acgatatagc gatcgtacag ttggtccacc tgcgccaact tctcctcacg cttgcgccgg 1140

ccaagccccg gaaacgcctt gggtgcggcg ctgtacggct gaaggtggcg cagctcggcg 1200

ccatccgagc gtaggtacag caaggtcttc gtcacataat ccaggcgttg tcgccagcga 1260

tcacgcgaag cctcgatttg atgcgattcc caggcgcttc cagcctcact gcactgccga 1320

tccaattcct gatcgatgac cggttgcagc ggtgcattct cgtcgtcgat ggtcacacag 1380

aagccttcgg ctacgacgcc ccccttctcc gacgccccga aactcgacat cgccagcgcc 1440

cgccccgtcg acgctgccgc tgacgcactg gggaactcga acaggaaaat ggcatgcgag 1500

ccgccgcaat agtgacggtg ttgccagggc gggacgatgc acagcgtgtg cagcggtggc 1560

gtcagcaact gtgcaggcag gtccgaggcg atatcacttc cgctcagcag ggtccccaac 1620

gacagcgtgg gctcatacag cgcgccacct cgccaccaaa aggtcagcgg catcatgatc 1680

gcgctcatct tgccgcggag gtcctctgcg gcaccttcgc ggtccacctt cgaaaggacg 1740

atgctcggtg tgatcgcatc gatcagcgcc atgaatgctt cgtgggaccc tgtatcgggg 1800

tcgctgacga ttctgcgggc cttcgccacc cgcgcgtcac gttcgctcag aagatcccat 1860

gcacgcagcg ctccgctggc ccgaagccgc accggcgccg gcaaatgctt gaacgccggt 1920

gacaacgccg caaccgactt ggcgacggtt gcattgacca acggatcctg aaaaaaccaa 1980

ggttcgcgac gaatggttat tgtctgcatc gtgcaatctt caccgtatcg gatcaataga 2040

caggcgccgg ccacgacgcc ggtgcactac atcgcccagt ccgcttgaag cagacgtccg 2100

cgaatgcctg gatcggcgca gccgctctgg gccgctcctc cggccaacgc acgatctatc 2160

gctgtgactc cagattccag cacatgcgcg tgcgtgagcc tgtcgccttc gtaggcgaac 2220

agcatcggca ggcccggcgg cagatacgcg ccgacgaatt cggcgtggtc ttcgaggtcg 2280

agccacagcc acacctcgtg cggcccggcc tcgcttgccc agcgatcaaa caccgggcgc 2340

aaggcacggc acttctcgca ccagctttcg gcacccaggg caacgacgag gcgagctgag 2400

ggatggccaa gccgctcggc gatctcacgt gcatgggtcc aagggtcgag ttgggcagt 2459

<210> 20
<211> 1114
<212> DNA
<213> Ralstonia solanacearum

<400> 20
   ataaccaccg ctaaacgaac gccatgaaga gcttcatcgc agtatcggca gcaaccgtat 60
   ccatcgccgc gaacgcatat accgtcacga ctacgacgac gaccacaacg cgctccgacg 120
   ggactgtaac cacgcagaca tcaaggaccg ttccctacga tgccgcccga tgcgcgcgac 180
   taaccgtcga cctcagcatg ctcaacgacc agctacgcag aacgggatgg aaactggaac 240
   aggacaacgt gcgggcacag atcatgaccg ttcagcagga aatgagcgat cggggctgct 300
   aggccgcgag cagcacagcc ttagacgcac tagcgaaata cgacgagcca tcgagggcga 360
   cgaagcatct tatgtccgcg atatcgaact cggcgcacag cgtcgtgagc acgtccttca 420
   gtgcgtccgc acagtctgag cgactgttct cgatcgcata gaccacggcc tcaggaacgt 480
   tgatgccgcg actggtgtag tactttttct tgttgaaccg agtctcagca atgtccttgc 540
   cgatgtactt ggcgacatag ctggccagcg catccaactt ccattgcttg ccgaagtgcg 600
   ccccttgagg gttgcgaatg tggcagtacc ccttgccacg accgcccacg accttcagcc 660
   atacccgccg cgcgaggttg agcgcgaccc gacctgagac ggcgacgtgc atatgccatg 720
   cgccgcgttt ctgcagctca ggaaccgcga tgtagtggaa ttgactgtag cgagccatcg 780
   cacgccgaaa agcgtcccag agccccagga accagtccag atcggtaatg cactcgcgcg 840
   tagtcagcgt aatcatgtgc gtgacgcgag cagtcttgca gcgcaagcga acgttctgtt 900
   tagctcgctt cgcagcagat ttgacgctag cggcccgtgc ctctttatcg tcttcatttt 960
   caggcttggc gccgcgtgca cggcggggaa gatcacgcag atcgttcagg cgctgcacca 1020
   cggtacgacg ataaccgctg aactcgacac tgccatcttc gaacgcccga accttggcaa 1080
   ccatgtcatg acgcgccggc ccctccgccc acaa 1114

<210> 21
<211> 2428
<212> DNA
<213> Ralstonia solanacearum

<400> 21
ccctgctcag atgtgcgatc tatccggttc accccagtcc attgcaggcc gatgtcgacc 60

cgcttcagcg acggtaaagc tcgtcagtag tgcctcgccg ccgatgcccc aggtgcccca 120

ggtgccttgc agttcttcgc gctgacatca ggaattcgaa attctcacag aagctcacct 180

actgtcgctc cagccagatg ctcgcttaaa gtcacatatc tcacaaaaat aagcgacttt 240

ggggttggga atgaagcgat ttgtcaaacg gcagctttgg gttgccgtat tgggcctgag 300

tgcagcatcc ggtgcatggg cgggttcgtc cgactctggt tttgacgggt tcatgtggat 360

caacagtggc ggcatcggtc aaactcttac tccacctgag cgcagccctt cagagcagtg 420

catggtatcg gtcgctgccc acgatgccat atatggaacc cgctacaaat atgttggcct 480

cgacacgacg aatagtaaga acatccaatg catccttgat attggtagcg gtaccccagc 540

ggggtatgcg actgtgcgcc gcatcaatac atgcgtacct gggtatgggt tgcaacctga 600

tggttcatgt aagccactgt ctccgcgtac ccccgtgcaa gtctgtacgg ccagcagctc 660

tgtcattccc ggtacaggca ccaagacctt catcgagggc gacgaggggg gcagtccaga 720

cctgtctgtc acacttgcct accagcagtc ttacaacgac tacggatcga atgtcggcat 780

cggtcaatgg acaacaaact ggcagcgcag cctggacact ggttccgcca gcgagtcgac 840

tccccaagtg tcggctcagc gtgaagatgg ttcagtagtc accttcagcg gaaacagcgc 900

ggcctggagt gccataggca gccaggacac gctgcggcca atcgcggatg caaatggcaa 960

tacaaccggg tggcagtaca ccgtggttga tacgggcgtg gttgagacct acgatgtcac 1020

cggcaaactg caatcggttc gggaacgcaa cggccgcacc acgacactgg cctataacgt 1080

ggtagggcag ttgaccgctg tcacgacgcc aagcggccgc agtgtgacgt tcgcgtacga 1140

cgcgtcaggc cggacgacta gtactatggc tcccgacggc gccgtcactc gctacggcta 1200

caacagcgct ggcatgctgt cgacgatcac gcggccggat ggcgccgtac gccagtatgt 1260

ctacgaagac agccgcttcc cgaccgcact gacggggatc gtggatgaaa acggcagccg 1320

ctacgccacc tatgcgtatg acgaccaggg ccgggccatc agcagcacgt tggcgggcgg 1380

tgccaatacg taccagttcc agtatggcga caactaccag accactgtca ccgaccccac 1440

cggcaagacc agcgtctaca gcttcctgaa acagaacggc gtgctgctgc cgacttcgat 1500

cagtgctcca tgtggcctgt gtggcagcac ccgccagagc agcagctacg acgccaacaa 1560

caacctgacc caggaaaccg actacaacgg caccgtcacc tcccacacct acgacagcca 1620

gaaccgcgaa actcagcgcg tggacggctc gggtacgacc agcgcccgca ccacgacaac 1680

ggtatggcac tcgcaattct ggaacctgcc gactcaaatc gcctcgccga ccaagctgga 1740

aacgtacagc tacgatagca acggcaatct gacccgctac agcgaaacgc cgaccgcgga 1800

cagtgacggc agccagggct tcagcgcagc ggcgaccggc ccggctcgga ccaccaactg 1860

gacctacacc gccgacggcc tggtggcgac cagcagcggc ccgcgaaccg atgtggcgac 1920

cagcaccacc tacgtttacc gcaccgccga cgacaccgcc acgccgccgc aataccgcaa 1980

gggcgacctg taccagatcg ttgacccgct gggccacgcc accacgatca accagtacga 2040

cgccaacggt cggccgctgc agatgacgga tgccaacggc gcggtcaccg cgttcaccta 2100

ttcgaaccgt ggctggctga ccagccagac gatcaccccg gcgagcgggg cggggcagac 2160

caccaactac agctatgacg ccgtggggca attgaccaag gcgacgttgc cggacggcag 2220

cacggtcagc ttcacctatg acggtgcaca tcgtctgacg ggcgcagccg acagccaggg 2280

caacagcatt gcttactcgc tcgatgcgat gggcaaccgc acgcaggagc aggcgaagga 2340

tccgagcggc aacttggccc gccaagtcac gcgtgtcttc gattcgatga accggccgct 2400

caaggtcacg gtcggcactg cgtcttaa 2428

<210> 22
<211> 1818
<212> DNA
<213> Ralstonia solanacearum

<400> 22
agcgctcgcg cagcctggcc agatatgtcg ttggcctcgt ttcaggagcc gccattggtc 60

atctcgaaat ggatacctgt caaccgtggt ctggccttgt agccttggct cacctggaac 120

tgacgcggca tttcagccgg ccagaataga acgacgccca ccgcgtgagt gagcgtcgtt 180

ggcaatagca ttgcgactta gcttgacgaa gtgtcttctt tcagcggaag cccgagcgca 240

tcgctaattt tgttgtcgat agacccgata ccgctcactc catcaacacc gacgggctcc 300

gccgcttcag atagacgatt cccatgctca tcaaagaacc acccgcgaaa atctcggaaa 360

caatgctgcg gcagcaaatc aaccgctgtg ttggggccag acctcacaac ctcaaagaat 420

ccccgtatcc aagcgcgatc attggtgccg atagggggca ccagcaaatc ggacggacgg 480

aggtcgggga ttttcatctt gtcgcccgag gtggccctgt acaagtaaat caagatcacg 540

cccccaccgt ggacgttacc gatcttggtg tccgtgtcca cgactcgccc aaagaagtac 600

cgatccggaa actgctccaa ctggaacgca aagatgtctc cggcctcggg cttttgccgc 660

gttttcttca gaacgactaa attggttggt ggtgttttca tgtttagaag ccgcgttctt 720

cgagccagca gtcggcccaa ctcttcgcgt cttggtagcg ccatcagatc ccgtgtgtca 780

ggtttgattt acccacgtgt gcttggtcgc aatccccaaa tatattgttg tgcgcacggg 840

ttttgcagac ctatgcccct tcgttgctct ttagcttaga tgcgcaatac ccaaccatta 900

ccgaaagaac gaacagcagc acagctaaga gagatctggt gtagaacctc atcgaaagat 960

tgtgtatctg cgcgtcagtt gcgaacgtag ttagccaggc agcttgaatt gcccatattg 1020

caaggacaat tgatgagagt aaaaaaataa ccatcaatat ccacagcaag aatcttttca 1080

tgtcgcctca cttaccaatt tatctattat ttaagattta ctgctttttt ggaccaccgc 1140

atttatcgat aacatcctgc acaaacacgt gacagtcgtt ggcgaacggg acaaatggcc 1200

ccgtcttcct gcctggtttg agcaaatcat taacgcactg ctcgttaagc gaacgcccag 1260

agtatggaac ctggaatgtc ccgggctttt ccgattcccc agaatgattg accacttccg 1320

tcggagtaag tggcatatca ggtgcggcct gaccaggaac atctccacca gctggcccca 1380

ttcctccctc tgctgtatcg gttttgatcc accaatgatc aatgcctgga actttgttca 1440

atacgtcgag tgggccatca atgtgaatgt ggcgaccata tattttcact tcgaggccac 1500

gagagtccgt attccgagtc ggctgcccgc caacatagct gtaagtcgac cactgtccac 1560

ccttcaggcc aatgggatca gactggatgt agcgccctgt caccggatcg tagtcccggt 1620

tcgcgttgta gtgcttgccc gtctcctgat cgtagatttg accggggaag cgcaggttgt 1680

agggaaccgc tgatcaatga agtcggcgaa tggaatcgcg agtcatgcca tcttggtttt 1740

tcggattgag gaagggatgc ttcgattcag cacggttttt cgctgctttc gcacctatct 1800

tggcgcgctt tgcgcgct 1818

<210> 23
<211> 382
<212> DNA

<213>  Ralstonia solanacearum

<400>  23
tatgcctttt ccaacggttg ccagactcct cgacgtcgaa gaagagcagc gaaagatcct      60

ggcggcggat gcggtgatcc tgctgttccc actatggtgg ttcagcatgc ccgcaatcat     120

gaacgggtgg atagatcgcg tgtgggcctt cggtttggct tacggctaca aaggggctgg     180

caatgcctat cgctacggtg agagtggctt tgccgtaaag cgcgcactgc tggcggtatc     240

agttggtgga cctgcggacg actattcttt ccgtggcatc aatgggccgt tggagcagat     300

gctctttcat atcacacacg gcactttgtt ctttccgggc atgcaggtga tatccacatt     360

cgcggtatat gagactgttt ga                                              382

<210>  24
<211>  1434
<212>  DNA
<213>  Ralstonia solanacearum

<400>  24
tcaatccgaa aaaccaagat ggcatgactc gcgattccat tcgccgactt cattgatcag      60

cggttcccta ggattcagaa gctgttgtcg acggaacaat tcaattttct ttcttaacgc     120

caaaccggct ggagtcagat tttccttcgg gatagatcga acgatccatt tgtggctcat     180

ctgcacaccc ttgttttctg tgtcccccaa ccccttcttc agttccgatt ctccaccatc     240

ctgattgacc ggcgccttgg ccgaactcac ggcatcacga ccttcactgt gaaagatatt     300

gggctgaccg cttggcgtcg gattctcgac ctcgcccttg ccaagcgctt gcagttgctc     360

ctggatgtcc ttgggcaacg cagccaggaa acggaacgcg ccaaacaagc tgctcttggt     420

ctcgccaatc acgccggggc ggatcgggtt gcctgtctgc atccagacat ggtcgatccc     480

ctgtcgcccc gccggatgcc actcttccca gagtttcccg aaattgttgg ccttcttgag     540

gttgcgcttg ttgcgggcgc accagtaatc cgtgatgtgc tcggcgggca caccggtttg     600

ccatttctgc ttcttcttgg ttgcccggcg cgtatcgctg cgcgctgcat ccgtgggagc     660

ccgctccgtg gccgtatggt cacgctcaac tttctccgtc ttgcgcgact ccggtgcctt     720

ggctttcggc gcttcgccgg ccgagggggc atgcggcgca tgttcggatg gcgcgtgcgg     780

tgccgaaggg gcatgcggcg tcgcgtgggc agcggtgctc ttgggcacgg catcgctcat     840

ggacttcttc cacagcgcct gcagttcttc gactgcctca tcgagcatct tgggggcgcg     900

ctgcctcaag tccttgatct gttcgatcag cagcgcgact tccttcttgc cgatgaccgt     960

ctggacgatc cagccatcca ggccatggat gaatgcaccc atgacgtcgt cgaagttccg    1020

tttgaggctg ccggctatgg acccccagtc gaccgtccgg aaccagtgtt cgaggttgcc    1080

tttgagcgag ggggagaccg catcgagcac gcgtggcaat gcgtggcccg acttgatcat    1140

gcggaacgag gtcttcaacg catcgcccag cgcagggacg cagccgacca gcgtaatgga 1200

catatcgagc cagccggaat ggctggtcgg gctcttgctg atgacgatga cgccgacgat 1260

gatgtcgcgc acgtccatcg cctggcccag gccgggaatc acgctcaaga cacccgagac 1320

aatgatttgc gatggcgagc tgccttcgcc ggttaccagc cctttcagcc agccgctcag 1380

atcgtcatcc gctgccttga ccgcgccgcg cagcgctatg gcttccgcgt tcaa 1434

<210> 25
<211> 3126
<212> DNA
<213> Ralstonia solanacearum

<400> 25
tcactgcagc cgatacttcg attgcctgtt cttgaatgcg ggctcggtgc cttcgagaat 60

ctggacaact cggcccttga tctccggttg ctcaatcgca cccgcttcat actcaaatcg 120

aacaccctcg gtgttacgag acgcgtagac aatttgctcg gcgtcacaca ccaccgccaa 180

gttcggattg tgtgtaacca tgatgacctg ccgcctctcc ttcgccttct tgatgcactt 240

gactaggatc ttgtagatgg tctggttgtc gaggttctcc tccggctggt cgatcacgat 300

cggaatgtcg tccttgtcga ccagaaggta gaaaaccaag agcaataatc cgcgctcgcc 360

aggcgagagt tggccaatgt cttgcccccc gaaggtcaac gagtagcgcg gcgaaaggta 420

ttcaaggcca aagatgtagt cgagaacttc ggtagcttcg actcccttgc gaagctgatc 480

ggccaaccgc gtctcgcggc tgtcctgccc ttcgcgctga tcggagcgca tggtttggtc 540

aatccgctgt gcgaaggcaa cggcgtcttc gggcgccgcg aagctagcct cttggagcat 600

gcgcctaacg agctgattgc tttcgtcgac gccagaaaaa gttccccgca cttggcgatt 660

gagcttgccg agaaactgat cctggaatcc agtctcctca atgcggacgt gaaattcaag 720

aggaagattc atgtccatct gcccagccga ctgcacgaac gcctggacag gcaggtagag 780

ccgttggtat tcgtcgaccg tgccgcgaat caacccatgg atctctcgtg atatcccgga 840

gcgttgctca atgagcttct ggagctcatc aggaagcgcg gcaagcgccg caatctcgga 900

ttccaactgc gcgatagttc ccggcttgtc ggcaggcccc atcacgtcgg cctttgattt 960

ttcccacttg accaactcct ccttgtagag cacgaagaga cgctgacgct cgccgagctt 1020

gctcttggcc tcagcgaggg aagcgaaaag ctcctcgcgt cgcttcaaga tgctgtcggg 1080

ctctgcactt tgaagctttg cgtcgattga atcgatctcc ccttgcatcg ctttggcgat 1140

tccctcaaca ggcccggcat tgatccgggt ctcgaacaat gagtcgatgg gaatctcaac 1200

cccgagctct gctaagagct gtttcaactc gctttcgaaa gcttcgctct gcttcttctg 1260

gttagccaaa gcctgggcga tgcggcgcgc gactgcttgc ccctttgcag tagtggactt 1320

cgcctcccga agccgtgctt cctccttgcc gacatcctgc agttgctgct caagaccgtc 1380
gatcttggtt gcggccgctt tggactcttc caatgcttca ggggaagcat tcggatcctc 1440
aacctgtcta ggcttggctt cttcgaggga ggcgagctca gccttctttg ccgcaagctg 1500
cttgaccaag ctctccttga attccgggaa aagcctgcgc tccgtaccaa gaatctcgac 1560
gttgagcgca gcaatctggc cccgcagccc ccccatggcc ttgttgatct cggtcacctt 1620
gaaattgagc agcgcgtcca tcgatgactg gccaagttgg tcctccagcg ggacgtgtga 1680
gtagatgatt tttcgcaact cggcatcgaa agtcgccgat ccgcccgcac ccagctcatt 1740
gcagagcgtc tcaaggtaac tctgcggcag atacttcacg cgctccacac tactgggctc 1800
gggatcttgg tccaaatccc gcgtagtcgg cgagccgtcg cgccagacca aggttcccac 1860
gaagttttgt gcaagccgtg ttcgcggatc ccggaagcgc accgcgttca aaaacgagaa 1920
gctacgatag tttttggtgt tccccgccaa cgcgataatg tcagcaagag cgctctttcc 1980
gctccccttg ttcccgatga ttgccaccaa atctgaattc agcggcagtt ccatgtcgaa 2040
ccatgtgtca gcgagctttg agccttcctt cttggcgacc ctaactgagc ggatgaactt 2100
cgttcgattg agctcaacgt actggcgctt ggccgggtta tcgccaacgc agacgcgatc 2160
atcgaactct tcgatagcct gctgcagacc cctgaaggtg cagtccgcct tgatccaggt 2220
aaagcagtta ccgatgcggt ccttgtcggt cgatgcgctc agccaatgtg catcggaaca 2280
gtcaagcaag ttggagttaa caccggactc cttcaacttg gcgcgcgcct tggcgtgatc 2340
ctctggggat tccgcagcgg taaagacaag atctgcactg ttaatgagcg tctttttctc 2400
cgcgatcgag tggtcatcac atttgaggtt ctcccactcg gtcttgccga ctgcaaggat 2460
gaacctcccc ttgaggacct cgttctccag ggccttgcgc aagccatccg cgctgacgtt 2520
gaggttgttg aaaccctcaa tgagagggct cccatacccc acccgcttgt ctgctggggc 2580
cgagtcaatg atggctgcac caagagcttc aatgctttgc cgggagatca ctgagttcca 2640
ttttccttcg ccagtcgacc caggaagaag acggtagctc ggggcgattg cggaaataaa 2700
ctgctggcgg atgaattcag gatccacctg atcgaagatg atgtggatgt tgatcctgct 2760
ccagcttgat ggtgccttgc cgctttttcc cccttggaca acgccgccga acttgtccaa 2820
ccgcagttcc acgaccggca gaatcagatc gatgttcttc agccttccct gctcgcgctt 2880
cgccttcagc accttttcgt agccatcgac gagcacatag tcgttgatac cgattacttt 2940
gaactcctct ggcagctttt caacgtcagc gagaaacgct tcccaggctt catcttggct 3000
ccccgggtag ttgtgataga aggattcggg cgtgtggacg tgtaaatccc atttcttcca 3060
agtggagccg gccagggcaa ggggcatgtt cttgtctcgc tgaagttgtt gttcacaagc 3120

gggcat                                                            3126

<210>  26
<211>  372
<212>  DNA
<213>  Ralstonia solanacearum

<400>  26
ttgtgttggt caagccaacg ccggccttgt tgcgggtccg ctggcaattc cggtggcgtg      60

ggcatcggtg cgttggacat cgcaacctgt gtggggatga aatcctggcg accgcccggg     120

tgggtcgtct gggtcccact gggcaatatc cggaccatcg ttccgtcggc cttgcgctgt     180

tggacatatg gatggcccgc ttcatcaaag ccgcggctca ccgacccgct ggcatcaatc     240

gtcggtgcag gccccggcac tacgggctta ggcggcacga cgacggccgt gccggcgagg     300

aggctggtct tcgcgttcag cttgctgatg gcccgcagcg gcggtgctgc gccttcgacc     360

aacacctcgt ag                                                         372

<210>  27
<211>  174
<212>  DNA
<213>  Ralstonia solanacearum

<400>  27
gtgcggccag gatgcggcgg cggcgctggg cgtcctcgac catctggccg ccggcatgcg      60

gctcggcact cgctccttcg ccccgcccga cggcagcggc cctgcgctga cacggcaggc     120

gtccgacagc gacagcgtcg acgacttttt cgacgccctc aacaaattcc atga           174

<210>  28
<211>  546
<212>  DNA
<213>  Ralstonia solanacearum

<400>  28
ttattgcttg gcgggttcgg cggccttcgg agccgaggcg gcatcggtct tggtgtccga      60

cttcgcttca gccttctcgg ccttcttgtg gtggtgatgc ttcttcgcgt gcttggcttc     120

ggccttcggg gtcgaagcgg cttccgtcgt ggtcgctgca gccggcgccg cagcgggggc     180

ggcagcggcc ggtgcggagg cggtgccagc ggcgaacacg ggggctgcga aggcaccagc      240

aacgatcagg gcggccaggg attgagcgac tttcatgcga gactccttaa cggatatgag     300

cgggactcca gatacatgga tctcgtccca tgtgcgatcc atgtctgcaa aaacgcctgg     360

catcgcaagc gcgtggaccg ccgtttgtaa gggattcgcc cacggtgtaa gcaggcgtaa     420

cacgggcctg ccggacccgg cgacgggcgt gttgcctgct tgaggccggc cgcgtgcagg     480

ccggttgagg cacacgttaa ccggcccgcg ccggcccgca ccggcgccgg cacgggccga     540

tcgcac                                                              546

<210>  29
<211>  111
<212>  DNA
<213>  Ralstonia solanacearum

<400>  29
tcatcgccgc cagcaggctc tggccgccgc cgaggtactt gctgtccgga tgcgtcttga      60

gggcggtgac ggcggtctgg gcgtcggtgg ctttgtggta ctcgaaggca t              111

<210>  30
<211>  807
<212>  DNA
<213>  Ralstonia solanacearum

<400>  30
atgagccccc gcaagcctta cccgacagac gtatccgatg aagaatggag cttcgcagcg      60

ccatacctga cgctgatgcg cgaggacgcg ccgcaacgca cacatgacct acgcgagatg     120

ttcaacgcac tgcgctggat ggcgcgtgcc ggagctgcgt ggcgcatgct gccgacgaac     180

tttcctccgt gggagctggt gtaccagcaa acacaacgct ggctcaatgc gggttgcttt     240

gaggccatgg tcaatgatct gcgttcggtg atacgggtcg cccaagagag gcaaggtcaa     300

cccagtgccg tcattctgga tggtcgaacc ttgcagtcga cctgcgagag tggcccgcgc     360

gcgggctacg acggctacaa acgcaaacgg ggcagcaagg ttcacatggc agtggatacg     420

ctgggccatc tgctggcggt gcatgtcacg ccggccaatg aacaagagcg tgcgcaggta     480

gcggaattgg ctcgtcaggt tcagcaggcc acaggccaaa ccgtgaaggt agcgtttgcc     540

gaccaggggt ataccggtga agcgcctgca caagccgcgc tggatgaagg aatcgacctt     600

caagtcatca agctatcaga ggccaagaaa ggctttgtgc tgctgcccag gcgctgggtc     660

gttgagcgta gctttggctg gctcaaccgc ttcaggcgac tcgcccgtga ctatgaacgc     720

cttccagaaa cccttgcggg tctgcacttt gttgtcttcg ccatgatcat gttggttcat     780

gccgtgccga tcatgcaaag tgcctaa                                         807

<210>  31
<211>  234
<212>  DNA
<213>  Ralstonia solanacearum

<400>  31
atgaaaaagt ctctcctgat cgcttcgctg atggctgccg tggctctggc tgcttgcggt      60

aagaaggaag aagccgctgc tccggctgcc gccccggaag cttcggctcc ggccgccgca     120

ccggctgctg ctgccccggc cgcttcggaa gctgctgctg ccgcttcggc tccggccgct     180

gctgctccgg ctgcttcgga cgctgctgct tcggctccgg ctgccaagca ataa 234

<210> 32
<211> 102
<212> DNA
<213> Ralstonia solanacearum

<400> 32
ttgccgccgg gcgggctggc ccctggccgc ctccccggaa gggttcaccc tgctcggcgg 60

gggtgcccgt tcgtcgcgcc gcctgcggtg cgatcgcgct ga 102

<210> 33
<211> 807
<212> DNA
<213> Ralstonia solanacearum

<400> 33
atgagccccc gcaagcctta cccgacagac gtatccgatg aagaatggag cttcgcagcg 60

ccatacctga cgctgatgcg cgaggacgcg ccgcaacgca cacatgacct acgcgagatg 120

ttcaacgcac tgcgctggat ggcgcgtgcc ggagctgcgt ggcgcatgct gccgacgaac 180

tttcctccgt gggagctggt gtaccagcaa acacaacgct ggctcaatgc gggttgcttt 240

gaggccatgg tcaatgatct gcgttcggtg atacgggtcg cccaagagag gcaaggtcaa 300

cccagtgccg tcattctgga tggtcgaacc ttgcagtcga cctgcgagag tggcccgcgc 360

gcgggctacg acggctacaa acgcaaacgg ggcagcaagg ttcacatggc agtggatacg 420

ctgggccatc tgctggcggt gcatgtcacg ccggccaatg aacaagagcg tgcgcaggta 480

gcggaattgg ctcgtcaggt tcagcaggcc acaggccaaa ccgtgaaggt agcgtttgcc 540

gaccaggggt ataccggtga agcgcctgca caagccgcgc tggatgaagg aatcgacctt 600

caagtcatca agctatcaga ggccaagaaa ggctttgtgc tgctgcccag gcgctgggtc 660

gttgagcgta gctttggctg gctcaaccgc ttcaggcgac tcgcccgtga ctatgaacgc 720

cttccagaaa cccttgcggg tctgcacttt gttgtcttcg ccatgatcat gttggttcat 780

gccgtgccga tcatgcaaag tgcctaa 807

<210> 34
<211> 318
<212> DNA
<213> Ralstonia solanacearum

<400> 34
atgcagaagc tatttgcgct gtgcgcgctg gccgccgccg cattgccgtc tcaatccgcc 60

ctggccggcg tcaacgtggg aatcggcatc ggcatgcccg tgccggtcta tgtcgccccc 120

gccccggtct atgcgccgcc gcccgtgatc gtcgcgccgc agccgatcat cgttccgccc 180

gcgcccggct actacgagga cgactggcaa gcccgcgcat ggcgggagca gcaatggcgc 240

gaacagcgct ggcgcgagca cgaatggcgg cggcaccacc gccatgaccg ccacgatgat 300

gacgacgatg acgagtga 318

<210> 35
<211> 180
<212> DNA
<213> Ralstonia solanacearum

<400> 35
atgaccccgg agcaagtaaa ggcgcgcttt gagcgcgagg gcaagacctt tgcaaagtgg 60

gcaaaggaga agggctttga ataccgcacg gttatcgcgg tcatcaacgg cgtgaacaag 120

gggcgttacg gcgaggctca caaagtcgcc gtggcactgg gtctcaagaa agctgcgtga 180

<210> 36
<211> 1683
<212> DNA
<213> Ralstonia solanacearum

<400> 36
atggcacggg cacaactggt tcagcgcgtg gacgcaggcg gcgcaagtgg cgcggtagcg 60

gtcatgccca cagcaaaggt gctggcgctt cgtgctcgcg acccgtggcg cgaggcaacc 120

gacagggccc ggcaagtggc gacgtggcgt gagacggtgg tggcgtacgt gcgcgccatg 180

acggaagacg gtgtgacgca gaacaacgcg gtggcgctgc tgctggagcg cggcgaggcc 240

ggcagcctgc ccagccactt cgccatggcg ctggctggcg ccgccaaggc aggccggaag 300

acgccatcgc gctccgccat ctgcgaatgg tgcgctcagt atcgcgaagg cggcgcaact 360

gcgctgctgc ccgaccacaa gggccgcgtg gtggaggccg ccggctggtg ggggccggcg 420

ctggagtatt tcaacgcgcc cggcaaacca gatatggccg ctgtacatcg ccgcctggcg 480

gaagtggacg gcttcgcggt cagctacgac caagtacgca actacctgac gggggttcct 540

gccatgctcg gccgcaacag ccccgcacgc attggccgca acctgtaccg cctgaccgag 600

aaggcataca tccgccgctc gaccgaaaac gccttgcccg gcgatgtgta tgtggccgac 660

ggctaccgcg cggacgtgta cctggcgcac cccgtcacgg gcgatatctg gcgcccggag 720

ctgacggtgg caatcgacat gcgcagccgc ttccccgtgg gatggcgggc cgacgaacat 780

gaaggcacgt atgccgtgca gaacatgtgg gcagagtgct tcgcccgctg gaaccacgtg 840

ccgccgatgc tctacatcga caacgggtcc ggctacaaga acaagctgat gagcgacgag 900

atgacaggtt tctacgcccg cgctggcgtg cagcaaatca tccacgccat cccaggtaac 960

ccgcacggca agggctgggt ggagcgcttc ttccgcaccg tcaaagacga cttcctgaag 1020

ctgtggcagc cggctttcta ctgcggcgag gacatggcgc cggaagtgct gaaccgcacc 1080

gtgcgcgagg tcaaggcggg ccggcttgtg ctgcccacat tggcccagtt tgccgacgcc 1140

ttcaacgcct ggttggaccg ctacgccaac cgcccgcacc cagaagacca gaacacgaca 1200

cgcgccgcgc tgtggtcgca actggcaccg cttccgccgc acgccaacgt gaccgaactg 1260

aagcgccagg cggtggtgct gacggtgaat cgcgcagcca tcaagcacgg caagcgtgcc 1320

tacacgcacc ccgagctgca cgccttcaac ggccacaggg tggtcatgga atacgacctg 1380

atggatgacc gtgtcgccgt catgcgcacg caggaaggcc ggtggatttg cgacgcacac 1440

ctcgtcacgg ccattgacgc catcgcgccc aaccgcctgg aagaaaagcg ccaggcgcgc 1500

gccgccgatg ccatcaagcg cctgcaacaa aagatggacg agcagaaggc ccgtgcgggc 1560

atggtgctcg atgtggacag cgtggccgac ggcgtgctgc ctgcaatcga ggtggaagcc 1620

cgcctggtgg acgacagcga cgacgcgccg ctgctgctcg acctgacgat gaacgacgaa 1680

taa 1683

<210> 37
<211> 327
<212> DNA
<213> Ralstonia solanacearum

<400> 37
atggcacaga aactgaacct gaaacgcccg ctggcgttcg gcaaagtcac ggtcagccac 60

ctgacgttcc gtgactacgc cactgccgaa gactatctgt cgttcgacaa gcgcggcggc 120

gtagcccaac gcattgcgct gatcgccagc ctgacgggca ccgacgaaga gctggtgaag 180

aagctgcacg gcattgacta tcgccgggcc gagaagatgg ccgacgctat catcgaagcc 240

gacgaagacg aagccgagga accggcagcc gaaagcggcg aagcgccgcc cgaggatgaa 300

gccgccgccg cagcgcgaaa aaagtaa 327

<210> 38
<211> 807
<212> DNA
<213> Ralstonia solanacearum

<400> 38
atgagccccc gcaagcctta cccgacagac gtatccgatg aagaatggag cttcgcagcg 60

ccatacctga cgctgatgcg cgaggacgcg ccgcaacgca cacatgacct acgcgagatg 120

ttcaacgcac tgcgctggat ggcgcgtgcc ggagctgcgt ggcgcatgct gccgacgaac 180

tttcctccgt gggagctggt gtaccagcaa acacaacgct ggctcaatgc gggttgcttt 240

gaggccatgg tcaatgatct gcgttcggtg atacgggtcg cccaagagag gcaaggtcaa 300

cccagtgccg tcattctgga tggtcgaacc ttgcagtcga cctgcgagag tggcccgcgc 360

gcgggctacg acggctacaa acgcaaacgg ggcagcaagg ttcacatggc agtggatacg 420

ctgggccatc tgctggcggt gcatgtcacg ccggccaatg aacaagagcg tgcgcaggta 480

gcggaattgg ctcgtcaggt tcagcaggcc acaggccaaa ccgtgaaggt agcgtttgcc 540

gaccaggggt ataccggtga agcgcctgca caagccgcgc tggatgaagg aatcgacctt 600

caagtcatca agctatcaga ggccaagaaa ggctttgtgc tgctgcccag gcgctgggtc 660

gttgagcgta gctttggctg gctcaaccgc ttcaggcgac tcgcccgtga ctatgaacgc 720

cttccagaaa cccttgcggg tctgcacttt gttgtcttcg ccatgatcat gttggttcat 780

gccgtgccga tcatgcaaag tgcctaa 807

<210> 39
<211> 807
<212> DNA
<213> Ralstonia solanacearum

<400> 39
atgagccccc gcaagcctta cccgacagac gtatccgatg aagaatggag cttcgcagcg 60

ccatacctga cgctgatgcg cgaggacgcg ccgcaacgca cacatgacct acgcgagatg 120

ttcaacgcac tgcgctggat ggcgcgtgcc ggagctgcgt ggcgcatgct gccgacgaac 180

tttcctccgt gggagctggt gtaccagcaa acacaacgct ggctcaatgc gggttgcttt 240

gaggccatgg tcaatgatct gcgttcggtg atacgggtcg cccaagagag gcaaggtcaa 300

cccagtgccg tcattctgga tggtcgaacc ttgcagtcga cctgcgagag tggcccgcgc 360

gcgggctacg acggctacaa acgcaaacgg ggcagcaagg ttcacatggc agtggatacg 420

ctgggccatc tgctggcggt gcatgtcacg ccggccaatg aacaagagcg tgcgcaggta 480

gcggaattgg ctcgtcaggt tcagcaggcc acaggccaaa ccgtgaaggt agcgtttgcc 540

gaccaggggt ataccggtga agcgcctgca caagccgcgc tggatgaagg aatcgacctt 600

caagtcatca agctatcaga ggccaagaaa ggctttgtgc tgctgcccag gcgctgggtc 660

gttgagcgta gctttggctg gctcaaccgc ttcaggcgac tcgcccgtga ctatgaacgc 720

cttccagaaa cccttgcggg tctgcacttt gttgtcttcg ccatgatcat gttggttcat 780

gccgtgccga tcatgcaaag tgcctaa 807

<210> 40
<211> 210
<212> DNA
<213> Ralstonia solanacearum

<400> 40
atgtcaaccc ttgctgagat cgaagctcag atcgctgagc tggaagaggc caacgagaac 60

ttgcacgact cgttggatgc cgctgaggcg atggacgacg ctgaggctat cgagttcttc 120

ggcagcctgg atgagcgtga tgaggcgatt gaggcctggg aggcggcgat ccagtcgaac 180

ctggagcaga ttgccgagct ggaaggctga 210

<210> 41
<211> 369
<212> DNA
<213> Ralstonia solanacearum

<400> 41
ctagtgaaaa atggtcgcgc ggatctcgcc ggccattttt cgtctagcgt caggagtgtc 60

cgcgcggatg tcgaagccgc tagggggccc ctttggggat gtgggagtgt ggatgagggc 120

aggacggttg tcgccggagg cgtctggcgg gaccgagtag cgggtaggac tggggcggtg 180

aagacagggt gagggttggg aagacctccc tcttgcaagc cccctctgct gcgtttggcg 240

ggccgctgcg ccgttttccg gaggtggtag gtacgtgggg aggggtgacg gtcgcgcgcc 300

ccgcccgccc gcagcgaagc gaggacggac ggggcgcgcg cagcgcgccc ctacttgtag 360

taataacac 369

<210> 42
<211> 1563
<212> DNA
<213> Ralstonia solanacearum

<400> 42
atggaaattt tcaagcgaat tcaggaactg gcacctgaag gaattttccc gctttccaga 60

atcaagctct cccagtttta cggcatgaaa ttggacgatt tcgctcatga gatcgccatc 120

ctttctctgt ggctggcaga acaccaaatg aaccaaaagt tcaaagagac ttttggacat 180

accaatccag cccttccttt gaagagcagc ggcaacgtcg tcagcgataa cgcaatccat 240

gtcgattggg acgagtttct tgctcccaaa gcagagcatg agacatacct tcttgggaat 300

ccaccctacc ttggagcacg taaccaggaa ccagagcaga aagcagacct tgaactgatt 360

tttgaaggcc acgaggaata caaggactca gactatgtct gctgctggtt catcaaggcc 420

gcaaagttca ttgctggcaa aaatgccaag tatgcgttcg tggcgaccaa ctcaatctgc 480

caaggtgagc aggttgccta cctatggcct agagtcttcc agcctggtct tgaaattgac 540

tttgcctaca cctcattcaa gtggtccaac ttcgcaaaga acaatgctgg tgttacgtgt 600

gtgattgttg gggtgaggaa cacggctgac aagccgaagc tgctgtttgg agccgacagc 660

tacaagattg tgaagaacat caatccttac ctgattgaag gtaaggatgt tttcgtgcgt 720

cggacctctg aagttctgtc agggttcccg gcaatggtga tcggttgcat ggctcgtgac 780

ggtggcaacc tcattctgaa gcctgaagaa cgcgatgcca tcgttcagca gtatccgaag 840

tccaagccac tattcagaac tctctacggc acccaagaat tcatcgatgg caatccacgt 900

cagtgccttt ggatcgagga cgaacagctt gaacttgctc gctcaatccc acctatcaag 960

acacgcattg atgcctgcta tgacttccgg gtgaagtcca aggccaagac caccaatggc 1020

tatgccaaga ttgctcataa gtttgcccag cgagttcaaa tcaagggcgg ctctatcatt 1080

gttcctgcaa cctcttcgga acgaagagag tatgtgccta ttggctacct tgatgccaat 1140

gtagttatca caaactctgc aaatgtcatt tacaagcaag atcctgttct ctttgggatc 1200

attgcttcca aactgcacat cctctgggtt cgcacggtgg gggggcagct tgaaacgcga 1260

cttcgctact ctgctgagat ttgctacaac accttcccct tccctgatgc cagcgagaag 1320

aaacggcaag cagtggctga gaaggctatg gcgattgtcg ccatccgtga agcgtatcct 1380

gaactgagca tcgaggacct gtatgaccct gacactatgc ctgctgactt gaagcaggca 1440

caccacgaac tggatgtggt ggttgagcag tgctaccaga ttaagccgtt ctacagcgat 1500

accgaacgct tggaatgcct cttcaaacta tacgaaaaaa tgatggaagc cgagcatgcc 1560

taa 1563

<210> 43
<211> 111
<212> DNA
<213> Ralstonia solanacearum

<400> 43
ctactggtga tgttgacgat gcgaccccag ccgcattcga tcatcgcatc gaccgtcgcc 60

tgaatcagtt cgatgggcgt gagcatgttg gcgttgagcg cggcgagcca c 111

<210> 44
<211> 138
<212> DNA
<213> Ralstonia solanacearum

<400> 44
ttgcgctgta caacggcggc agctactaca tcgacagcgg caagccgacg ccgcaggcgt 60

tcgagcagac gctgcgcaac ctgcacgacg tacagccgac ggtgtacttc aacgtgccca 120

agggctggga gatgctga 138

<210> 45
<211> 312
<212> DNA
<213> Ralstonia solanacearum

<400> 45
tcacggtgcg ggagcaggag gcactgctgt cgccggcgtt cgagcggatc gccgatgtgc 60

cgatcgccga gggcgaatcg attcccgtgt ttgccgggcg cgagcgctgg caggtctggc 120

ggcggcgcgc tgactgatcc ggtggcgtcg gcgtcgcagc ggcgcgttcc ggcgaccgtt 180

gtgttggtgc gaagcaacaa gggcggcgac cggaaaaaaa gcctgcggac gcccattacg 240

gtccatcgct gaaaaaagtg gcagacgggg ccgcgttcgt ggtcgcaccg atgccccgcg 300

ggggtgtccc at 312

<210> 46
<211> 174
<212> DNA
<213> Ralstonia solanacearum

<400> 46
tcagcgcacc aactgattga tctcgatgat cggcatccgc actgccagca tgatcagcaa 60

cagcagcacg cccatcgtca ggatcagcgt aggctcgagc cgcccggtca ggagcagcgt 120

gcgccgctcc agctcctgcg cctcgcgctc cggcatggtg gaaggatcaa tcaa 174

<210> 47
<211> 375
<212> DNA
<213> Ralstonia solanacearum

<400> 47
gtgctgctgg tgctgctcgt gctggatgtg gacgacgaac tggaactgct gctggagctg 60

ctggcgctct gcagggcgga gaacagatcg tgcgtcagct tctgcatgtc ctggccgatg 120

gtcgaactgc tgctgctgtc gtcgctgtcg ccgtcgtcac caccgtccgg gggcggcatg 180

ccgccgggcg gcggcggggg cggcgcggag gcgctcgtcg tgctgctggt gcccgtggtg 240

ctgctagtgc tgctggtgct gtcggtcgac gacgtggacg atgccgacga cgtggatgac 300

gtggacgaag tcgacagacc gagctggctc agtgcctgct cgatcgcctg catgaagccg 360

ccgccaccgc catga 375

<210> 48
<211> 1026
<212> DNA
<213> Ralstonia solanacearum

<400> 48
atgaccttgc cggtcgaatc gcccatgccc ccgccggccg gcgtcggcca aacggcaccc 60

gcgccgctgt ccgtacggcg catgggcttt gccatggccg tctctatgct cgaagcatcg 120

accgacccgc gcgcgccagc atgccgcgca tgcggccggc ccatcgacaa agccgccacc 180

caggcggcag tgagccaatc cgcgcccaga cggaaggctc gacggggagg tcaccatgcg 240

tcaacgcttc atggacagca ttccgtgggc cacgctgctg ctcggcgtgg gagtcggcgc 300

agcactcgcc atcacgccca gcacggcgca cgccgatcgc cggcagtcct gctatgccgg 360

cctcgtactc gtgttcgacg gcacatggca cccgggcggc gtagtcggct gccgcaccgt 420

caacgtctcc gacagcaaca acgtggtggg cgcggaggtc aacctgctga tcgcgcccgg 480

cacgatccgg ccgcgcgtgc aggccgtggc cggcagcacc agcgtgcagg cgctggtcgg 540

cggcggctac gacttcgcca gcggcaaggc gctggtcggc gtgggcgtca ccggcgatca 600

ctggctgggc ggcgccgact acgagatcgc cggccgctgg accgcatacg ccggcgtcag 660

cacgctcaac cgctatcacg ccaacgcgcg tccggacccc atcgccccga cgccggtccc 720

gtcgaacaat gtcacggccc ccccgtcgac gaacaacaca gcgccagccc ccacaccggc 780

tccgacaccg ggcgccaccc ctgcgcccac accaggtccc ggcccgacac ccagtccggc 840

accgactccc acgccgagcc caagcccaag cccaagccca agcccaagtc cgagtccgag 900

tccgagtccg agtccgagtc cgacaccgac accgacaccg acgccaagcc cgacgcccgc 960

tccggcgcca gcacccagcc caacgccgac cccggcaccc tgcggctgcc acggcggcga 1020

ccatga 1026

<210> 49
<211> 384
<212> DNA
<213> Ralstonia solanacearum

<400> 49
atgaatgcga acaaattcaa tacccttcac caacaagcac tggccgtctt cgcggccgac 60

atggtcgggg agtacgtcgc ggaagtcccg ggcgtccctt ggtttgccgt gaccctcggg 120

gacggtgctc tcatcgcgga gcactacgag gccgaggaca tgtacgcatt gaagacgcat 180

gaaagcgact acctcatcca gcgcgaaggt gaggccgggg cggatgaaat ttgggcggcc 240

ctacagtgtg tgggtcgggc gacaggctac cttgacatgc acgcggtgac cggaagcatg 300

caccgcctta ccagttcgga ggaatcgcgg cttctgaaga agctggagcg gcgtggcttg 360

ctccttacgg agcagatcgc ataa 384

<210> 50
<211> 639
<212> DNA
<213> Ralstonia solanacearum

<400> 50
atgcttcact cgattcaaaa tccgggtcac cccatgcgca ttcttgccct tcttgctgct 60

gttttcgcga gcattggatt cgctgctcac gctgtcgccg acgagatgtc tacacagcgc 120

gttgacggtg tgtcactgtc cgtgcctacg gcctggcgcg tagacacggc tgaattcgaa 180

aaggatagag cacaggctga tgccgctcac accaggcgaa gccgcacgct gttggccgtc 240

tcgggaccag gtgacgatac cgctgttgcg tctctgtcgt ttgttgtcca agagggcttc 300

aaaccagata cgttctccgc aatgccggac caaacgaaaa accgccttgc tcgtcagttg 360

caagagtttg tcgagcgctc aacggggctg cccgcaaagc gcagcgtcat catcgaatgg 420

acgccttacg agacagtcga tatctcgggg cgacaaacgc tgcacaacct cgcgactgtc 480

catttgcgaa accagccggg tgctcaagac ctgttctata tcccaacgat gggcggcatg 540

tacctgctca gcgtttcagt ctttgaccgg cctgtggagg ctgggcacca gctggcaaag 600

gaaatcgcca gcagcatccg ggttgatggg aagttctga 639

<210> 51
<211> 882
<212> DNA
<213> Ralstonia solanacearum

<400> 51
atgacgggcg ttactctggg ctttgtgcct gagccacttg ctgttcctct gacgtgcatt 60

ctcccctctc gcaggactcc ctccggcatc acgggcacca gaaagttcaa gcagatccgc 120

tcctcgatcg aagaggtggg cctgatcgag ccactttccg tgaccgcggt tgatcagcag 180

tcgggaaacc acacgttgct ggatgggcat atacggctgc tggtgctgca cgaccttggg 240

catgaagagg cggcctgtct tgtcgctact gacgatgagg catacaccta caacaaccgc 300

atcaaccgcc tgtcgagcgt ccaggagcac ttcatgatcc ggcgtgccat tgagcgtggc 360

gtttcaccgg aacgccttgc caaggtgctg tccgtagaca tctcccagat cgtgaaaaaa 420

ttgaacctgt tggaaggcat ttgccctgag gccgtcgacc tgttgagaga ccggcagttt 480

tcgccagagg tagcccgcgc gattcgcaag atgcggccaa cgcgacaggt cgagtgcgtg 540

gaattgatga tcgccgcgaa caatgtcacc gtcgcctatg cagaagcgtt gcttgttgcc 600

acgcctgcct ctcgtcttgt tgatggaaag aagccggcca agctaacagg cgtgacgcac 660

gagcagatgg cgaagatgga gcgggagatg agcaacctgc aggagcagta caaaatcgtg 720

gaacagacct acggccaaga cgtactcaat cttgtgctcg ctaaagggta cctcgtgagg 780

cttctcgagt gtgagccggt gcaagcctat ctacaccaac accagcctga cctcgtgcgc 840

gagttcgaat cgattcgcga cgtcgtttcg ctggagcagt ag 882

<210> 52
<211> 870
<212> DNA
<213> Ralstonia solanacearum

<400> 52
atgattccga tcgatcgtat cgacgtgatc aacccacgtg agcggaacag caaggtcttt 60

gacgagatcg ttggaaacat caagaccatt gggctaaaga agccaatcac cgtcacgccg 120

agggccgcag cggatggcag tgagaagttt cttctcgtct gcggcgaagg acggctcaag 180

gcattccgct cgctgggcga gaccacgata ccggcaatag tggttcacgt cagcgacgag 240

gatgcgttca tcatgagcct tgccgagaac atcgcgcgcc ggcaatgccg tcctctggag 300

ctgttagccg gcatccgcca gttgcgtgat agcgggtact cgccgaagat catcgcagaa 360

aagacgggct tgaccgccgc ctatgtgcag ggtgttctga ccttgctcca tcatggggag 420

gagcgtctgc tgattgccgt cgaaaagggc acgattccgc tgacggcggc gctcgcgatc 480

gttggggctg gcgatgacga caaggctatc caagccgccc tccaggaggc atatgagtcg 540

gggaagctgc gcggcaagca gctgataagc gcgagacggc tcatcgagcg gcgacatacc 600

ttagggagat ctgcctcccg ctatatgacc gggagtcgcg cagacgtgac cacgtcaagc 660

ttggtgcgta cgtatgaggg agaggtggag cgccagaagg ccatggtccg caaggcggag 720

ttcactcagc aacggttgct gttcgtcgtc ggagcgttgc ggcagctatt tgccgacgag 780

aatttcgtca accttctgcg agcggaatct ttggcgacac ttcccaaata tctggctgag 840

cgtgtttggt ccactgggag ttcgccatga 870

<210> 53
<211> 1566
<212> DNA
<213> Ralstonia solanacearum

<400> 53
atggcaatcg atcagtacac ctgcgctacg ccgatcgcgc cttctgcgct tcgggcggcg 60

gaatacgtac gcatgtcgac tgagcaccag cagtactcga cccagaacca acgagacaag 120

atccgcgagt acgcagcgtc acgaggcatc gaagtcgtcc gcacctatgc cgatgagggc 180

aagagtggcc tgcggatcga cgggcgagag gcactgcaaa gcctcattcg agatgtgacc 240

aacggtcgcg cggacttcca ggcgatcctg gtctatgacg tcagccgctg ggggcgcttc 300

caggatgccg acgagagcgc gtactacgag tacatctgcc gccgtgccgg catccaggtc 360

atctactgtg ccgagcagtt cgacaacgat ggctcgccgg tgtcaacgat cgtcaaaggc 420

gtcaagcgcg caatggccgg tgaatacagc cgcgagctct cagcgaaggt cttcgcaggc 480

cagtgccggc tgatcgaact ggggttccgg caaggcggtc ccgccggcta cggcctgcgt 540

cgcatgctgg tcgacgagca tggcctcgtg aaagccgagc tctgccgcgg ccagcgcaag 600

agcctgcaga ccgaccgcgt tgtcctggtg ccgggtcccg agagcgaggt gcgtattgtc 660

aatctggtct acagctggtt tatcgatgag tccctaaacg agttcgaaat cgccgcgaga 720

ctgaacagca tgcatgcgcg cacggacttg gaccgggact ggactcgcgc aaccgtgagg 780

gaggtgctca ccaacgagaa atacatcggc aacaacatct ataaccgtgt gtcgtacaag 840

ctcgcgcggg ctcgcgtcgt gaacgcacct gaaatgtgga tccgcaagga tggtgccttc 900

gagcccatcg tagcgccgga catgttctac accgctcagg gcatcatccg ggctcgcgcg 960

cgtcggtaca gcagtgaaga gcttatcgag cggctacgca acctctatcg gaatcgcggc 1020

tttctgtccg gcctcatcat cgacgagacc gaggggatgc cctcctctgc ggcgtacgcc 1080

tatcgcttcg gcagcctgat acgcgcctac caggcggtgg gctttacgcc ggatcgggac 1140

tatcgttacc tggagatcaa tcgattcctg cggcaactgc atccgaagat cgtgtcgcaa 1200

gcggagcaag ctattgccga tctcggcggt acggttcttc gagacccggc cacggacatc 1260

ctggacgtta atcaggagtt caccgtgtcg cttgtcttgt cgcgctgtca gccgcgaccg 1320

aacggcgggt atcactggaa ggttcggttc gatacaagcc tcttgccgga cgtcactgtg 1380

gccattcgtc tgaacaaggc gaacgacgcg cctttggact actacctgct cccgcgtctc 1440

gacttcgcac gccctggcat tcgcttggcc gagcgcaatg ccatcgagta tgagagctat 1500

cgcttcgaca cgctggacta cctgtatcac atggccgagc gtgcaaagct gagaagggca 1560

gcatga 1566

<210> 54
<211> 1406
<212> DNA
<213> Ralstonia solanacearum

<400> 54
tggtggtcac cggccaacgt gtggcgccgc tctcccgcgt caggaggtgc cgttctgctc 60

cccggggcag cacacaggat cacgttagca ggtggccgcg caccgtgcct cacatcatct 120

ctttgcaatc agccctggat ggtgcgcgca ccggcggcag cattacgatc tgcaccctcc 180

agacgtccat ctttgcaacc ccgtcatcgg acgcgtgctg gagcatgtaa gtgcctgaag 240

gcagaggcgt tttcggagct gacgcgtaca tctttgcaat cgtccttctc cttaggttcc 300

tggcacgctg tagatctcgg ttgcgcgccg tataggccgg gtgagcctcg cgataccgtc 360

gccagtagtc gctatggcac tgactccatg cagcgcggga ccgcgcctgg ttgtcgcgat 420

agtcgtcatc agagcgcaag tgctgctgtt gccaccgtcg gcgacgttgg cgctggcact 480

cggcagcgga gcaatagcgt tggcctggag tttgtgggcg gggaagaaag agggtgccgc 540

atgctgcgca ccgccgccga atttgattca tcgcgagctc cgggcaaggt cactgcgcgg 600

ggccagcaag tagatgtggc gttcggggca ttcaacccga aatggacagc tcgcacatga 660

gtcgagcact cttccaccgg gcgcgagccg ctacggattg caaatgatgt atgcacatac 720

atcaggaaaa cagtgcccga cgcgctcgat tttgccacgg ctcaagggtt gctctaccat 780

gggccaaaaa gtaggagctc gacggccgct cgtcttgcaa gtgattgatt tttaaggtat 840

cttttggcca cttgaggtgg cgtcaagatt ttgcactttt cttggcacgt gtgccgagtc 900

ttttcagtaa ttgcggcaag ggacgcgcga ctcagtgtct caatttatga aaaaacgctt 960

ttaaatcata gtgcgatttt tcaggaattg tgacactcaa caggttgttg cagaccccag 1020

aaaagttgat gtaattcaca aataagtgaa tcgatccaag gcggccgcat tgccgtggcg 1080

caccagatct acccattaac atttcgtgca gccccacaaa aagaccgggc ctctcaagag 1140

gtccggtctt tttttaggtg tcggtagcca cctcgccagt aggaatcgct ctgacagaga 1200

aaggtgatgt cggtcgagct gcgcgcaatc gctacagggg tatagctggg acttgccagt 1260

caaccccgct cacgagaaag cgtcctccga aattctagca atgtttgcgg gcccgacggt 1320

aacacgccag aaactaggga accgctgatc aatgaagtcg gcgaatggaa tcgcgagtca 1380

tgccatcttg gtttttcgga ttgagg 1406

<210> 55
<211> 1906
<212> DNA
<213> Ralstonia solanacearum

<400> 55
   agggggctgg tggagtcgct acatgcttga agttactaaa attgtggatt ttttgacggc 60
   acaaccctag acccagcttg gctctgactg acgatgacaa aaaaatctga gttatggatt 120
   tttgggacaa atcaggactg agccggctgg tccggtccat gggtaggggc gtactttgag 180
   cggtcagagc ggccgtacgt ggcaggcctg tttcccgcgt gcgttgcgtc gaggccgcgt 240
   gaatggcgta ctggggcgcc cgctaccggg ccgaattgct ctgccaccgt tcccgatgac 300
   gggatgttgt ggtgtgcgtc agtcagcatg attctcgttg gtagaatatg agcaataatc 360
   acaagaaacc aaccgaagtc gtttgactgc ctccgctgcg cggggccaat ggggcgagat 420
   acgcataatg acggcagaac gtgccactcc ttcgcgaatt gggactttcc ggttctgata 480
   atggcaattt cgctcgcttt cgagaatgag caaattcgcc agacctgcga aagtagccgt 540
   tctgctaagc gccgccttgg tgtaacggta gcggctcaac tccaggtgcg gctcgctgac 600
   ctcaaggcct tggatgccgc gagtgacgta attgacatgg gctttgctcg gctcgacccg 660
   tccgaagata cccggcttgt cattccgctg ggctgtgagt atgttctgag ggtagccgcg 720
   catcatcgtc ccgaaccacg ttcgtcaact ggtaagttgg actggaagaa cgtgactcgc 780
   atcaaaattc tggctatcga gtgcaccggt gaaagctgac catttgtttt ctcccacttg 840
   ggctgtgccg ccagggcgga ccgtagttga cttgctcgct gcgaggggtt ggtcgcggga 900
   tgagctggcc aactccattg agctggtgcg agaagatctc gatgacctga ttgacggaac 960
   cgcacagcta tcggaaagcg ttgcttcccg gttggggggc gtctttggta cgagcgccgc 1020
   cttttggatt cgtcgagagg agcagtaccg cgagcagctc gcgagtttgc atgtcaaagc 1080
   tgatgtggcg gatgcgggct acaacgagtg gctgaaggca ctgcctcttg cggacatggc 1140
   tcgctttggc tggttcgctc caggcgcgac ctccgctgac aaactggcgg cgtgtcttga 1200
   tttctttgat gtcccgaact tggacgtgtg gagtcgggac tacgacgatg tgaagcgtgc 1260
   ggttgccttc cgggcatcag ccgcccaccc agtgaatgta cctgcgacca ccgcgtggct 1320
   tcgacaaggc gagcgtgaaa gtgagcgtat tgagtgctca gcgtgggacc cggccaagtt 1380
   ctcatcggcg ctgaccaaag cgcgtgcgct aacccgtacc aaggacccgc aggtttttgt 1440
   gccagctcta caagcgctat gcgctacgtc gggcgttgca gtggtaatcg tgcgagcacc 1500
   gtccggttgc agagccagtg gtgcgacgtt cttctcggct ccagataagg cggtcctgct 1560
   actgtcgttc agatatctca cagacgacca gttttggttt tccttttttc atgaggctgg 1620
   tcatctactg cttcatacgg acagggatag tctcatcctg gagacgtcgg agcgaacgac 1680
   gtccaaaatg gaagctgagg cgaacgagtt cgctgcgaaa actcttttgc cagtcgagct 1740
   caaccgccgg ctacctgagg tggctggcga cgttcgagcg atacttcgtc ttgctcgaga 1800
   ggccggcatt gccccaggca tcgtcgtcgg ccaactgcag catcgcggtt tgatcaaaca 1860
   ccagcacctc aacaagctca aggttcgcta tagctggtag tagttc 1906

<210> 56
<211> 909
<212> DNA
<213> Ralstonia solanacearum

<400> 56
ttggcagctg acctcgaagc aaaactgctt gagtttaagg cggacaacaa gccgctgccg 60

ggcatcgcga acgacgacaa tcgcattgcc tatgttgagc agcttgtcga gagtatccgc 120

cgcgtcgact tcgtaacgac tatcggacgg cgcggcatct gcccacagag acgggatcct 180

aagtcagact tgttcgaccc cttgcgggct gcgactctct accggcagca ggaaaatctt 240

gatgaggcgt tctggctcgc gttcctcttc gtccatttcg ggaaaagcct gcgaaccaga 300

tggaacctta tccgcgcgat ctatggcgca tggaatgacc agtttatttg gacgtgggag 360

cagttcaaca gcgccccgaa tattttcaca atctggttca acgaccgcct tccggagttg 420

ctggaaaatc gaaagcagct ccaattcggc aaccaccgca agtacgaaag tatcgagaaa 480

gtggatgtgg tgcttgaaag ctacgcggaa tgggtaggcc ccgaacgttc gcatgcggca 540

atgctcgaac gtgccacggc agccgtcggc gacaacccaa gggcgctgtt tgggtatcta 600

tacaagagca tgaaagtcgt ccaccggttc ggcagaactg ccaagttcga ctacctcacg 660

atggtcgcaa agctcggcct agcgagcctt cgtgcacctt cggcttacat ggttggtgct 720

actggaccgc tgtcgggtgc ccgcttgcta ttcgccggaa cgaccgaggc aatgcacatc 780

tcgtgcgcgc aagctgacca actcctggtt gtgcttgata accatctgaa cgttggaatg 840

caagtgctgg aagactcgct atgcaactgg cagaagagcc caaacaagtt cgtggctttc 900

cgtggctag 909

<210> 57
<211> 852
<212> DNA
<213> Ralstonia solanacearum

<400> 57
ttggccggca gcaacgagct cagcttcatc cagtactaca tcagccggta cgacgagttc 60

tcggacgacg gaaacaccgt tcacggggca tacggccctc gcttgttcaa catgcgtggt 120

gtcaaccaag tcagcaacgt catcgagaag ttgagcggca acaacgatac acgccgtgcg 180

gtcatccaac tgttcaacgc cgaagacctt ggttgcgatg tggcggtagg cagaaaagat 240

atcccctgca cgtgcaccat gcagttctcg ctcagagcgc gtcagctcca catgttcgtg 300

acgatgcggt cgaacgacgc ctatctcggc ctgcctcatg acatattcgc gttcacgatg 360

ctgcaggaga tagttgcacg ggccgtcggc gcggaactag gcacatacaa gcacgccgtc 420

ggaagcctac atttgtactc gaagaacacg gaccaggcga caaagtatgt cgacgagggc 480

ttgcaggagc gcatcgccat gccatcgatg ccggtcggtg accctcagcc ggcccttaac 540

gcgctggtag atgcggaacg ggaaattcgc ttggggcacg ccgtgaacgt caaccagttc 600

gaaccctact gggcagacct cgtccgcttg ttgcttattc agcggcatct gaaaaataag 660

actcctaaca gttcgcggtc aattcgtgcg ttagccaaga agatgagcac ggcgaactat 720

gacgcgtgca tacagcaacg cgttcgcaag agccagcctg cgccggagct cgccgagcct 780

attggtctgc tgttcacgcc tgctgaactg gatgccggaa tggcctcatt ggacaccccc 840

gccgacgaat ag 852

<210> 58
<211> 1315
<212> DNA
<213> Ralstonia solanacearum

<400> 58
atttgttcgc acaagagcca tctgcaccac tcgctgaagc ccttcgcccc aagaccttgg 60

acgaggtcat tgggcagtcc catctgctgg gtgacggcaa gcctttgagg ctggccttcc 120

agtcaggcaa gccccactcc atgatcttct gggggccgcc cggtgttggt aaaaccacct 180

tggcacggct cacagccacg gccttcaagt gtgagttcat agccctctct gcggtgtttt 240

caggtgtcaa agacatccgt gctgctatgg agcaggcaca gcagaatcta tctatgggca 300

agagcaccat cttgttcgta gacgaaattc acaggttcaa taagtcccaa caagatgctc 360

ttcttcctta tgcagaaagt ggcctcgtaa cctttattgg tgccaccact gagaaccctt 420

cttttgaggt gaactcggct ctgctgtcac gtgctcaggt ctatgtgctg aagtcactga 480

cagaagaaga actgaagctg ctcttgaagc gagttcaaga agaagatgct cttggtgact 540

tggagtttga agataaagca gtagatacca tcattggcta tgcagacggt gatgcaagaa 600

ggttcttgaa cctgcttgag caatgcaaaa cggctgctgg tgctgctggt gttcagcatg 660

ttaatagtga cttcattcag aatgccctaa ctctcaatag caggcgtttt gacaagggtg 720

gagacaactt ctatgaccag atttcagcct tgcacaagtc tgttcgtggc tccaaccctg 780

atgctgccct ttattggctg acacgcatgt tggacggtgg tgctgatcca cgctatctat 840

ccagaagaat cgttcgcatg gcttgggaag acattggctt ggctgatcca cgagccatgc 900

agatagccaa cgatgcagct ttaacctatg agcggctggg tagtccagaa ggtgagctgg 960

ccctgggtca agcggtcatc tatctggctg tagctgccaa gagcaatgcc ggttacaacg 1020

cctacaacca agctagagcc ttcgtgaagc aggataagag ccgtgaggtt cctgttcatc 1080

tgcgtaatgc accaacaaag ctcatgaagg aactcggcta cgggcatgaa taccgttatg 1140

ctcatgacga acccaatgcc tatgctgctg gtgaaaccta ccttcctgaa ggcatagaag 1200

agccgggttg gtatcagcct gttcctagag ggttggagat caagattggt gagaagctgt 1260

ccctgctgaa gaagtgggat gaagaagcca aaaagaactg aataagggga tcaga 1315

<210> 59
<211> 1950
<212> DNA
<213> Ralstonia solanacearum

<400> 59
atggagcaaa gaacacttct ctacgatgag cggttccttg aaagctatgc tggagccatc 60

atcactgatc ctgctacggc gattgtggag cttgtcgcca actgctggga cgcttatgcc 120

acagaagtga agatcacatg gcccgatgtt caacttgaaa agcagttcaa gattgcggac 180

aacggtcatg gcatgacccg tgaagagttt caatacatct ggcggaccat agcctacaac 240

cggttgtcgt caggtggaaa cacaacggct cccccagcag atgttcaagg cttgccacgg 300

cttgtttttg gcaagaacgg taaaggccgt tttgccagct tttgcttcgc ttctgaatac 360

ttgattactt caaggaagaa cgggcaagag ttcgtctgcc gtgttcacag aacctcaact 420

gatccgctcg ttctagaaga aatatcgttc aaggaaaagg gtgttgaggg tcatggaacg 480

gagattgtgg gtaacggcat cattccccgg ctcatgttct cggaagagaa agctcgtgaa 540

ttgatcggta gccgtttcct tgccaaccct gctttcaagg tgaagttgaa cggtagtgaa 600

atcacgttca atgacatccc tgacttgttg tccaagagcg aagtggatgt tgaagggtac 660

ggcaaggcag ttattcttca cattgacacc aagaaggctg acaagacaac gaagcaacat 720

ggactggcat ggtgggttca aggtcgtgct gtcggtgaat gcaagtggag tcgtagtgac 780

tacgaaagaa ttcttgacgg ccgaacttca gaggcgaagc gattcacatt cattgttgag 840

gctgactatc taaatgccca agatgcagtg ctgggtgatt ggagtggctt caaggacgaa 900

aaccaggcgt gggtgaagac ccgcgaggcc gttcaagatc gcatccgcca aatcattttt 960

gattccagca aggcagagcg tgaaagccgc cgcagtgcag tcattgaaaa gattggcaca 1020

acgatgaaca ccctgtcccc tgttagcaag gaccgggtgg aaagcttcgt caatgaggtg 1080

gtagatacct gcccgaactt tggtgaaaac gagatcgtcc agttgtccag catccttgca 1140

aagctggagc agtccaagtc tcgctatggg ttgcttgatc tgctgcacaa gtgcgagcca 1200

aacgactacg acacccttca tgacatcctg aaggaatgga ccatagggat ggcaaagctg 1260

gttttggacg aaattcagaa ccgcttgaaa ttgattgggg agcttcgcac caaactgaag 1320

gaagttggcg ttgatgaagt ccatgagttg cagccgttgt ttgagcgtgg cctgtggatg 1380

ttcggggctc aatttgaatc cattgagttc acgtccaaca agggcatgac caatgtaatc 1440

aagaccatct tcaaggatga ggatggtaag ggcactcgta accgtcctga cttcgtagcc 1500

ctgcctgaca gcagtgttgg cttctatgct cgtgcttcat ataacgaaga ttacgatgaa 1560

gacggtgttg aacatctagt tattattgac ttgaaaacca ctggtcttgc tctcggtagc 1620

aaggagaaag atcaagtctg gaagtatgtg aaggagctac gagaaaaggg ctacctgaag 1680

aagcataccc gtgttgatgg atttgtcttg ggagacaaga ttgaatcggg tgagaatgac 1740

cctacaacgc atggtgaaga agtcaaaatt tcacccatgc tgtatgacac catcctcatt 1800

agggctgaga agcgccttct gaacctgcac ggtaaggtca aggatgctcc gttcctgata 1860

gagcagcagg aagtcctgaa gaagtttttg gagcctattg aagtcattca ggcggaattg 1920

gtggaagctg acccagtggt ggcgaaatga 1950

<210> 60
<211> 765
<212> DNA
<213> Ralstonia solanacearum

<400> 60
acctcgtccg tgaattggat cgcagcctac aactacctgt ttgcttcttt caacagtgag 60

aacaaggatc tctatgttgg cggttctgta ttttgtcgca tggtgcagca ggtcgatcct 120

ggctcaccaa gctaccaaca gttgctgccg ctgagacaaa gccaaggaaa gtccaattcg 180

cgcaaggact tttattggga ccttatccaa ggtcttccag aggcccagcg atttcaactc 240

tatcgtgtct tcatcaacca cattgaagtt cacgacaagc cagcggcaga caacatcagg 300

aacattgtgt tcggcggtgg ttatgcggtg cccactacgg ttgttcccgt tgacctctgg 360

aactctgaga agctgaacaa cagtctcaat gacattgacc atgccattga tgctcaccat 420

tacaaccggg caacgacact gagctacacg tgtcttgaag ggctctacaa gacatacgtt 480

cgcaagcacg ttcctggaca aatggcgctg acggatctca tgccattgtg caaggtggtg 540

aaggaggaca tcagcaagaa gcttcaggca caaggcccgt ttcctgtcga gattgtcaat 600

gccatgccga ccttgaccaa tgccattgcg aactcaagaa acggcttcag tgaatctcac 660

tttggtgacg actcccagcg gtggcttgct ttgtttgctc gtgatctgac caactcaatc 720

ggtcgtctca tgctcaactt catgtgacca ccggcgagag gaaga 765

<210> 61
<211> 1149
<212> DNA
<213> Ralstonia solanacearum

<400> 61
atgactactt ctagaacatg cgcactttgc ccaaacatac tgtcagccaa aaataaaaca 60

aaagagcaca tcatccccaa tgcgattggc ggcaggaaga agaccactgg gtttatctgc 120

aacgattgca ataacaagtt gggtcagagc tgggatgcgg agcttgcaag gcaacttaac 180

tggttttcct tggcagtcgg catcagtaga gagcgaggtg agccacctaa acaggttgtg 240

caaacaatcg atggggacag gtattggctt ctcagcgatg gctctttcac gccagaaaaa 300

tcatcttatt cagaaaagga tctgggtggg gcaatcgaga tcaacctgac cgccaagacg 360

gttgaagagg caaagcagcg gattcaaggt atcgccagaa aatacccgaa gtttgacgtt 420

gaaaaagctc tcaatgagtt ggagataaaa accagctatt tggattcgcc tcttcatgtc 480

agtctttctt tgggtgggcc agaggctgga aggtctgtag tcaaaactgc gttttctttt 540

gcatctgaat gtggtgttcc acatgctcaa tgcgagaagg ctcttgaata tcttctagat 600

cctactgctg aaaatttccc ctttggattt gcctacttgt ctgacctggt tgaaaaccga 660

ccaaaggaca agatttttca ttgtgtgtct ttgcatggtg atccaaagaa gaagcggcta 720

tggtcttaca ttgaatactt cggaatcttc aggcttgttg ttctcctaag tgatgtgtat 780

gtgggtccct acaagaatga ggtttatgcc atcaatccag ttgatggaga acggcttgcc 840

gtgaaggtga ctacccagat cagcgaagaa gaatttactt cgttgatgag gggcgaaggt 900

ttcgaccaag aaaagcatag agcagcggcg gactatgccc tgcctatcat cctggataga 960

gggcgaagcc gaaccttgga gcgcacagtc aaggaaggct ttgaacatgc tgccaagcag 1020

cttggaatca gtgaaggcga aatcattccc aaggaaaagg ccactgagtt cactgccttt 1080

atgatggaga aaatctctcc atacatagag catctagttc gtagtgggca cagaaatgac 1140

caaccataa 1149

<210> 62
<211> 693
<212> DNA
<213> Ralstonia solanacearum

<400> 62
atgaccaacc ataagccagc aaacggcacc aagaagctcc tgcttattga ccttgagaac 60

aagcacaagg tagacctgtc tcccttggat gagagttaca gagctatcat ttttgtgggt 120

gccaaccaga acccgccaaa agcttccaag aagccagcaa cagcccatag gttcaagagg 180

gtggacttcc tgaagatttc aggtgctggc aagaatgcct tggacttcca catagccttt 240

gaactcggca ggaccttcga gactgcacct gatacccaat gcttcgtgct cagtggtgac 300

aaaggctttg acccgctgct gaaccacttg aatagcaacg gtatgagttg ccgaagggtg 360

gaaagcattg atgagcttgt gccagctacg gtggtgcctg tagcagttcc atctgatgtg 420

gttgtctgcc ctcactgtaa gaaggccagc accattgagc atcacggcgg ccactggtgt 480

tctaactgtg gaaggtttgc tactccacca gatcccaagc tgctgccgtc caatcaaccc 540

ggctacaggc agtctcaaag agacaggtct gaagaagact atgccatcta ttcggtatgt 600

ggctggtgcc atcaaagaac tgatatgact ggcggtatct atgacgatgg ggaatggatg 660

tgtggaggct gcattgccag gtgcgccaga tga 693

<210> 63
<211> 939
<212> DNA
<213> Ralstonia solanacearum

<400> 63
atggacttga aacctgcata tcaaccctca tgttgtggtg gtttatcgtt gccagagagg 60

aaggccgtgc caagaaccat tgaacagggt ttcaacgatt ttcttgtaaa tcttagagcc 120

actgctgctg agactgccgc aaccgtgtct catcgggcat ccatcaaggc atgcctggaa 180

aataactttg gcttgaaacg cttcacccga atcggttcgt ttggcaatgg aacaaatgtg 240

tcgggctaca gcgatgttga ctacttggct tgtctgccga ctacctcatt gaaacaatca 300

tcaacctatt ctctcaggtt ggtgaaagag gcattagaaa accgcttccc cagtacgggt 360

gttgtggttc gttgtccaac agtggtttgc ccctttggtc aatacaaatc tcagcataca 420

gaagttgtcg ttgcagacta tgtgaaagaa caaaatggct acaaggtcta tgaaatagct 480

aatggcaatg atgggtggat gcagatttgc cctgatgctc acaatgctta cgttgaacat 540

gtggatggta agcacggagg taaagtcagg cagttgatta gactgatcaa ggcatggaag 600

ttctatcaga atgtacctat caaatcgttc tatctagaga tgaaggtggc gaaatatgcg 660

agcaatgagc aagccatcat ccatcaccta gatgtgaaat attttcttga ccgtctcaat 720

aatgaaggtc tgtccgccat gcaagaccct atggggatcg ctggctatat ctatccttgc 780

aacagtgacg ttcagaagat tgacgcactc tcaaaactaa acacggcagc tacacgggcc 840

aacaaggctg aagctgctag agcattgggg aatataaaag aagcttttga ttggtggcga 900

ctgctttata acgacaaatt ccccacctac tacctatga 939

<210> 64
<211> 843
<212> DNA
<213> Ralstonia solanacearum

<400> 64
atgagaagta taaccaaatt agcattcgga atattattgg cagcagccat attcacacca 60

caggcacatg ctcaatatca gcaatcggac agatcattaa attgcgattc aacttgtgtt 120

ttacagcagc agtcaatgct cgctttaata cctaaagcaa acacatttgt tgcaccaaca 180

tatgtagcca gccccacccc taaacctaaa tgcggccctt ctgctggatc tatcttgcca 240

ggtgctccta acagcttttt gtgcgatgca ggaacaccca gtgcagtttc tactaccacc 300

gcaaatggag aaacaactta ctcttggact tgtagcaata gtttaggtag tcaattatgt 360

acggcaaacc agagagaagt tggtgcatgt ggtgctgaca atggcaaaac tctgtccagc 420

aatcccacta atgtttgtgc tactggtcag tcctacggct tcaacttcgc caacaatcaa 480

tacacatggg gttgtgctgg caattatgga agcccagcaa tgtgctctgc tgcctatgct 540

ccaccaattc caccatctca gagcaaaacc atctatcgtt actacgatag tatgttaccc 600

ggtgaaggtg gctcattcac ttctacttat gttccattat ctgcaagtga aggctacata 660

tcttctagag aaagccaagg accttggggg agatgcagat atgagtggca aacagcatcc 720

caagaaagaa tattacctag caggacatac aacttgaatg gtcttgccta ctgggtcata 780

agcttgagtc agtggaatag ctatacatac tggtataacg tgtgtgtaga tggaggaagt 840

taa 843

<210> 65
<211> 178
<212> DNA
<213> Ralstonia solanacearum

<400> 65
aaaaaaaaaa aaaaaaaagg aaatcgtagc gcatttcacg gtgcccctcc gagagaaatg 60

cctgagcctg accggtttgc accatgaggt gattgaggtc gccaagattt atgagaacaa 120

ccatatcttg aatttggcaa aatccagaaa acgcgaccag atgggcaaga ggaaataa 178

<210> 66
<211> 1580
<212> DNA
<213> Ralstonia solanacearum

<220>
<221> misc_feature
<222> (1202)..(1549)
<223> n is a, c, g, or t

<400> 66
agcgcgcaaa gcgcgccaag ataggtgcga aagcagcgaa aaaccgtgct gaatcgaagc 60

atcccttcct caatccgaaa aaccaagatg gcatgactcg cgattccatt cgccgacttc 120

attgagcagc gcttccctag cgtgatagaa atgaaggagt agaacgaatg aagaattggg 180

tgggaatatt tcttatgttg tggaatgtct gccacttcaa acatgaagaa tggcatttaa 240

tgagacagcg attgaaatta aaaaagcacc tctagggtgc ttttgttttt ttacatggcc 300

tgctttgttg gaatgaatgc gtgtattcgt ttcaatgaag tgggtgctcc ttagctgtga 360

aaaatatcta gccacctctt tctacgcttg atgctatctt tatgtgccag caggaaaggg 420

aaaaagtcct catagtgtgg aatcaccatt gaggaaattc cattttctgc ttcatattca 480

ctcgccttgt gggccattcc tgtaagtaag aagtggatgg gtcgcagcat ctcgatcagt 540

cgccagagaa ccagtggggc actcttctca atgacatatt gacggtgtag gcagtcaaac 600

accatcatgg ggtgtccatg ctcatcaagg tggctggtga agccgttggg tggcccttct 660

ctatccatgc ggtacatgag gttcagtgct gacctctcac gcacctggtg cggtgtgtag 720

tcttcatagg tagctctggc aagatcgagc cacttgtaga gagggcttct gtccatcttg 780

gcttcgcaga tttcattcag attgaagtag cgggcaccat caccgtattc actaatgaag 840

ttcaatagaa cttcgtcatt ggcggtcaga gtcggtgcta cagcattggg gtggctgtag 900

atgggcttca acctgatgca ctcttcatag agcacatcaa tcttgtgtcc aaagccttta 960

agctgcttgg tggtcggtgt ctggtagttg ttcgtcagca tgtggtgtgt caccacagcc 1020

aacttcagaa gcctctccat accaatggag agatgaaaga acgccgaata aaagtagccg 1080

tccttgtcct ggaagaagtt ggccttaagc agaaggtcga aacctgacag caacgtgttc 1140

tttgttaggt gagcttcttg tgcaagaagg ctgaattttt tatcaaaacc tatctgcttc 1200

annnnnnnnn nnnnnnnnnn nnnnnnnnnn nnnnnnnnnn nnnnnnnnnn nnnnnnnnnn 1260

nnnnnnnnnn nnnnnnnnnn nnnnnnnnnn nnnnnnnnnn nnnnnnnnnn nnnnnnnnnn 1320

nnnnnnnnnn nnnnnnnnnn nnnnnnnnnn nnnnnnnnnn nnnnnnnnnn nnnnnnnnnn 1380

nnnnnnnnnn nnnnnnnnnn nnnnnnnnnn nnnnnnnnnn nnnnnnnnnn nnnnnnnnnn 1440

nnnnnnnnnn nnnnnnnnnn nnnnnnnnnn nnnnnnnnnn nnnnnnnnnn nnnnnnnnnn 1500

nnnnnnnnnn nnnnnnnnnn nnnnnnnnnn nnnnnnnnnn nnnnnnnnna tcaacgtgtc 1560

cctagcgcgc cggatgcaga 1580

<210> 67
<211> 2445
<212> DNA
<213> Ralstonia solanacearum

<220>
<221> misc_feature
<222> (1077)..(1966)
<223> n is a, c, g, or t

<400> 67
aggggtttgt agacatggag ccatcgcaat cgagcaatgc cgaagggctg agcggagacg 60

atatggaggt tgtctctgca gtatcccggc gatacaggcg ttatcacaag atagccgcaa 120

ggcatctgcc ggaatgggcg ccgtctgggg ttacgctaag cctcatacaa gcgatgttga 180

acctccgaaa cgttcgttgc gcaaagagaa acattcacga gaacgatgac ggcacccagt 240

caacccatgc ggagcatctg agatatgccg aggccgaaat agctcgtctc gtgcgtctgt 300

gtgcctatag caatgaaatg cagaaggctt ggcggacaat atcaactatg ccagctcgtg 360

gtggtggcga tccggcattc tccatatgtt caaaaatctc ctccacgctg gggcaatttg 420

gtgacctccc caagaggctt ccagcacaac gcaagaaatc gctttctcgt gtggcgaagg 480

cagtggacga tctgcttgag gcaatcgaat cggagagcga ggcgcaggac tctcttgaga 540

ttgcactgat tgaatacctg ggaggtcaga acctcaagat gcgctctgac gacggagagg 600

aaataggagt cgatgaatct ctcgatccct tccgagcgct tagtccggcc atcattccat 660

gcgacgatac agaaggcttg gaggactccc ctcattgctc caagccctgg cacgagtggt 720

catctttcga tagatacggt tggcttcacg atgaagtatc catgacggga atggccgagt 780

tgctgatctt ttgtcgcgac agaataaatg ccttggctca ggagccgccc aaaattctgc 840

agcctggaag accagatggt gggctacagc cgtttctgat tcggagtttg tccaactgga 900

tgaagcgcat atacggacag cccttggacg actctgtagc actgcttgtc tctgccacgc 960

tagatctgcc agagcctctg acaagagaag cgatccgccc gtatctgaaa ggcaccggga 1020

agatttcgag caggaatcga tagatttctt cccgtttcaa aatatcagag tccacgnnnn 1080

nnnnnnnnnn nnnnnnnnnn nnnnnnnnnn nnnnnnnnnn nnnnnnnnnn nnnnnnnnnn 1140

nnnnnnnnnn nnnnnnnnnn nnnnnnnnnn nnnnnnnnnn nnnnnnnnnn nnnnnnnnnn 1200

nnnnnnnnnn nnnnnnnnnn nnnnnnnnnn nnnnnnnnnn nnnnnnnnnn nnnnnnnnnn 1260

nnnnnnnnnn nnnnnnnnnn nnnnnnnnnn nnnnnnnnnn nnnnnnnnnn nnnnnnnnnn 1320

nnnnnnnnnn nnnnnnnnnn nnnnnnnnnn nnnnnnnnnn nnnnnnnnnn nnnnnnnnnn 1380

nnnnnnnnnn nnnnnnnnnn nnnnnnnnnn nnnnnnnnnn nnnnnnnnnn nnnnnnnnnn 1440

nnnnnnnnnn nnnnnnnnnn nnnnnnnnnn nnnnnnnnnn nnnnnnnnnn nnnnnnnnnn 1500

nnnnnnnnnn nnnnnnnnnn nnnnnnnnnn nnnnnnnnnn nnnnnnnnnn nnnnnnnnnn 1560

nnnnnnnnnn nnnnnnnnnn nnnnnnnnnn nnnnnnnnnn nnnnnnnnnn nnnnnnnnnn 1620

nnnnnnnnnn nnnnnnnnnn nnnnnnnnnn nnnnnnnnnn nnnnnnnnnn nnnnnnnnnn 1680

nnnnnnnnnn nnnnnnnnnn nnnnnnnnnn nnnnnnnnnn nnnnnnnnnn nnnnnnnnnn 1740

nnnnnnnnnn nnnnnnnnnn nnnnnnnnnn nnnnnnnnnn nnnnnnnnnn nnnnnnnnnn 1800

nnnnnnnnnn nnnnnnnnnn nnnnnnnnnn nnnnnnnnnn nnnnnnnnnn nnnnnnnnnn 1860

nnnnnnnnnn nnnnnnnnnn nnnnnnnnnn nnnnnnnnnn nnnnnnnnnn nnnnnnnnnn 1920

nnnnnnnnnn nnnnnnnnnn nnnnnnnnnn nnnnnnnnnn nnnnnncggg aaaaacgcgc 1980

caacgggtgg ccccggttgg ggtgggcgaa cccttggccc gccaggaggc aatgctggcc 2040

agtcctggga gcattgcgtc ctgagcgcgc aaagcgcgcc aagataggtg cgaaagcagc 2100

gaaaaaccgt gctgaatcga agcatccctt cctcaatccg aaaaaccaag atggcatgac 2160

tcgcgattcc attcgccgac ttcattgatc agcggttccc tagatgaatc gtcgactgct 2220

acgccggcgg tggggcttcg tgaatgaccc ccatgcgcac catctcgtcg atttcggcac 2280

gagcaactgc cacaatgtct tccagtgtgc ggaaacgagg gctttgaggg cccatgacgt 2340

cttttgtgac tggatggccc agcgcgtcgc ggtaccgaat ccggccagtg caagtcgctt 2400

cgctaagctc ttggactagg acgatctgcg cgcctttgta cgctt 2445

<210> 68
<211> 306
<212> DNA
<213> Ralstonia solanacearum

<400> 68
gtggcaacca agaaggtcac caagaagaag tacttgaaga ttctcaacaa gcgcctgcgt 60

gccgagccgg atgcgccgtc caccgccctc ttcgttttct acccgcctgg cccaaaagcg 120

aagcacgcca ccggtgtggc tgcaagcgag cccagtagcc cgcgcgaact gcccatcatg 180

gctgagatcc agcgtattgc cgcaaccgag ctcatcgtta tcgacatggg cgacaggctt 240

ggcaacggcc gacgcagaag ctccaaatca aaaccggagg ccagccatgg ctcaactacc 300

cgttga 306

<210> 69
<211> 1308
<212> DNA
<213> Ralstonia solanacearum

<400> 69
atatcgccgc ctggatgata ggaaaaaaga tcaatggcgt ttcgcattcg tcactatcca 60

cacaatgact tgctgaacct cgcgtcgcat cactgcgaga cgatcaagat gaaggtgaaa 120

aacaaccagc tcgaaggtat aacgctcgat tgcatgagct gtttgatcgc acttgccttt 180

gcggtggagg cgctagtcaa ttttgttggg tctcggaaag tgaccgactg gaacgagatg 240

gccaaggctc cgagcaaggt ggcaaagatc gcgaaagcag tgggactcga catccagcca 300

gagtttgagc ccttttccgc aatctatacg ctccggatca ttcggaacgg attggctcat 360

gggaagccgc ttcacaaaag cgccagcgcc aaatcttctg acgagttggc gattgccatg 420

caggccccgt gggcaccata ttgcacccct gaacaagtta tagccctgta tgagcaggta 480

cgcgaacttc gtagggtgtt gttcaaaagc gcaaacatcc gatgggttga cagcctgacc 540

tctgccattg gctcgcacgg ataggaaaac catggcaaac gttgtgacct tgctcgatca 600

ctggcagacg ctcgtcggcg cgttcatagg cggcctcatg ggcgtagttg gtgcgctaat 660

cgttgcagta ctgaccgtgc gccgccagcg ctgggtgatg gcctctgcgt tgctccccga 720

catgcaacaa ctaagagccg ccgatgatgc gctcgaacga gcgcttcact ccattgatcc 780

gccgctcgga gagtgggttc gatctcagtg gtgtgcagag cgtcttgcac aaacgcaccc 840

cacgttgtcg gcgcttcatg acggtgtagc cgtgacccag gtgtctgacc ttgatgcgcg 900

actaagcgca cacttgatgc tctgtcggat gaggcacaag gacttggacg cacttctgca 960

gcaatttgtc gccgcgctcc atggctcgcg ggcgccgatg cctgcggcca atgttcccgc 1020

agcgatgacc gtggtgcggc gaagtgcaaa acgagtacag caggcgtggg atctatgcgt 1080

cgaacacggg acgcttgcgg aatacctgct ggatcgattg attttcaata ggtggccaaa 1140

catttggcac cggctgcgca tgcgttggtg gccgaacgac cttgaccatc gctcgatgca 1200

tttgctgaat tcggggtcga tcctgggcac acaggactcc catcaggacg acgtggccgg 1260

ataggaaaaa tcaattctcg tactaatgac gagcttggag attcgaca 1308

<210> 70
<211> 711
<212> DNA
<213> Ralstonia solanacearum

<400> 70
atgcacgaca tcgaaacaga cggcttcttg gcacccgagg ccctggcgtt tcgggatgca 60

gtgcgagtcc aatttgtcga gcaaatccgt gagtgcgagg atgtttcgcg ccgcgccacg 120

acgcaagtat ttgagcaaca catcgcgaac gtgacggttg caaatgcgac ggcagcctcg 180

ctgtgggcga gatgtttgag tgcaagccag gccgccatct tgctggctga gcgcggcatg 240

ggtgtggagg cgctggcact tttgcgtacg gcttatgaga acttgttctt ctgcgctgcg 300

ctggtcaagc agccggccgt gatagtgcgc ctcgccggtg aagatacggg tcagcgtctc 360

aagcaggcgc gtgaaatgct caaagacaaa cagatcttcg cggcaatcac ggatgcagat 420

cgggcgctgc ttgaggcgtt cgtccgcgat acaccggctg ccacgaaagc catcagtgcg 480

tacgaagccg ctgagatagc tggcatgacc ggcatctatc aaggtgcgtg gcggacattc 540

tcattgattg cgacccatgc cacgctgacc ggggccgggc acgcatttgg caatgacctg 600

tacgatctac agttcggccc ctcgttcgca aacgtcgact cggcattagg cttggcccgt 660

gactgcattc ggctcgggct tgatgcggtt gccccgattt ttactgcgtg a 711

<210> 71
<211> 531
<212> DNA
<213> Ralstonia solanacearum

<400> 71
atgcgctccg atcagcgcga agggcaggac agccgcgaga cgcggttggc cgatcagctt 60

cgcaagggag tcgaagctac cgaagttctc gactacatct ttggccttga atacctttcg 120

ccgcgctact cgttgacctt cggggggcaa gacattggcc aactctcgcc tggcgagcgc 180

ggattattgc tcttggtttt ctaccttctg gtcgacaagg acgacattcc gatcgtgatc 240

gaccagccgg aggagaacct cgacaaccag accatctaca agatcctagt caagtgcatc 300

aagaaggcga aggagaggcg gcaggtcatc atggttacac acaatccgaa cttggcggtg 360

gtgtgtgacg ccgagcaaat tgtctacgcg tctcgtaaca ccgagggtgt tcgatttgag 420

tatgaagcgg gtgcgattga gcaaccggag atcaagggcc gagttgtcca gattctcgaa 480

ggcaccgagc ccgcattcaa gaacaggcaa tcgaagtatc ggctgcagtg a 531

<210> 72
<211> 2405
<212> DNA
<213> Ralstonia solanacearum

<400> 72
tgccccttgc cctggccggc tccacttgga agaaatggga tttacacgtc cacacgcccg 60

aatccttcta tcacaactac ccggggagcc aagatgaagc ctgggaagcg tttctcgctg 120

acgttgaaaa gctgccagag gagttcaaag taatcggtat caacgactat gtgctcgtcg 180

atggctacga aaaggtgctg aaggcgaagc gcgagcaggg aaggctgaag aacatcgatc 240

tgattctgcc ggtcgtggaa ctgcggttgg acaagttcgg cggcgttgtc caagggggaa 300

aaagcggcaa ggcaccatca agctggagca ggatcaacat ccacatcatc ttcgatcagg 360

tggatcctga attcatccgc cagcagttta tttccgcaat cgccccgagc taccgtcttc 420

ttcctgggtc gactggcgaa ggaaaatgga actcagtgat ctcccggcaa agcattgaag 480

ctcttggtgc agccatcatt gactcggccc cagcagacaa gcgggtgggg tatgggagcc 540

ctctcattga gggtttcaac aacctcaacg tcagcgcgga tggcttgcgc aaggccctgg 600

agaacgaggt cctcaagggg aggttcatcc ttgcagtcgg caagaccgag tgggagaacc 660

tcaaatgtga tgaccactcg atcgcggaga aaaagacgct cattaacagt gcagatcttg 720

tctttaccgc tgcggaatcc ccagaggatc acgccaaggc gcgcgccaag ttgaaggagt 780

ccggtgttaa ctccaacttg cttgactgtt ccgatgcaca ttggctgagc gcatcgaccg 840

acaaggaccg catcggtaac tgctttacct ggatcaaggc ggactgcacc ttcaggggtc 900

tgcagcaggc tatcgaagag ttcgatgatc gcgtctgcgt tggcgataac ccggccaagc 960

gccagtacgt tgagctcaat cgaacgaagt tcatccgctc agttagggtc gccaagaagg 1020

aaggctcaaa gctcgctgac acatggttcg acatggaact gccgctgaat tcagatttgg 1080

tggcaatcat cgggaacaag gggagcggaa agagcgctct tgctgacatt atcgcgttgg 1140

cggggaacac caaaaactat cgtagcttct cgtttttgaa cgcggtgcgc ttccgggatc 1200

cgcgaacacg gcttgcacaa aacttcgtgg gaaccttggt ctggcgcgac ggctcgccga 1260

ctacgcggga tttggaccaa gatcccgagc ccagtagtgt ggagcgcgtg aagtatctgc 1320

cgcagagtta ccttgagacg ctctgcaatg agctgggtgc gggcggatcg gcgactttcg 1380

atgccgagtt gcgaaaaatc atctactcac acgtcccgct ggaggaccaa cttggccagt 1440

catcgatgga cgcgctgctc aatttcaagg tgaccgagat caacaaggcc atgggggggc 1500

tgcggggcca gattgctgcg ctcaacgtcg agattcttgg tacggagcgc aggcttttcc 1560

cggaattcaa ggagagcttg gtcaagcagc ttgcggcaaa gaaggctgag ctcgcctccc 1620

tcgaagaagc caagcctaga caggttgagg atccgaatgc ttcccctgaa gcattggaag 1680

agtccaaagc ggccgcaacc aagatcgacg gtcttgagca gcaactgcag gatgtcggca 1740

aggaggaagc acggcttcgg gaggcgaagt ccactactgc aaaggggcaa gcagtcgcgc 1800

gccgcatcgc ccaggctttg gctaaccaga agaagcagag cgaagctttc gaaagcgagt 1860

tgaaacagct cttagcagag ctcggggttg agattcccat cgactcattg ttcgagaccc 1920

ggatcaatgc cgggcctgtt gagggaatcg ccaaagcgat gcaaggggag atcgattcaa 1980

tcgacgcaaa gcttcaaagt gcagagcccg acagcatctt gaagcgacgc gaggagcttt 2040

tcgcttccct cgctgaggcc aagagcaagc tcggcgagcg tcagcgtctc ttcgtgctct 2100

acaaggagga gttggtcaag tgggaaaaat caaaggccga cgtgatgggg cctgccgaca 2160

agccgggaac tatcgcgcag ttggaatccg agattgcggc gcttgccgcg cttcctgatg 2220

agctccagaa gctcattgag caacgctccg ggatatcacg agagatccat gggttgattc 2280

gcggcacggt cgacgaatac caacggctct acctgcctgt ccaggcgttc gtgcagtcgg 2340

ctgggcagat ggacatgaat cttcctcttg aatttcacgt ccgcattgag gagacccgcg 2400

ggtaa 2405

<210> 73
<211> 809
<212> DNA
<213> Ralstonia solanacearum

<400> 73
tgagtttgaa cccgcattgg cggtggacgg gttctgctgc tgcaactggc gctcacggtt 60

gattgacggt cgccagtgaa ggtctcgtct acaagtgaag tactccccag gactcagcgg 120

gaaactgagc tgttcgagag cgagcttgcg aatgtgatcg ccttcgggtt cgcgtgattg 180

ctctcgatac ctcagcgtga tttcctgctt aacaggatca atgacgaacc accgggcccc 240

cctccgctca taaaatgccg cgcgcggccc ttcatccgcc ccacctcctg cataaatatt 300

gtccccgacc acgacaacgt cgccactacc gctaacctgt gggcgaagtg acttgaacgt 360

tcgcccttgg tcgaaggata cgtagactgc ggcatcatcc cgagcgacca catagggcga 420

attgaccacc ttgaacatcc ccgtcggatt gcgcttgcca ttaccgccaa actgcgtccg 480

gatgcccaac tgctcgtcta catagaacaa ttccgcctga cacggcaggt tatatcccgg 540

ccccggatcg ttctcgatgt agcggtgatc gtcgaagcgg tacacaacct gtttaggtgg 600

acgcgccata ttactcagcg tcttctcgct gatcgtgtcg ccgaaaccga ccgacgctgc 660

agcccggtcc gcctgggaaa gaccgcagcc agtcagcgat agcagcgtga cggttgcggc 720

aaccagcaac atgcggcgca tggcgacggg atgtttctcc tgtttcaacg agagctcctt 780

aagtagttct gaatggatgc gcacgctct 809

<210> 74
<211> 2658
<212> DNA
<213> Ralstonia solanacearum

<400> 74
atggccgaat tcgatcgaac ggcacatttg caagacctct tctttttcca gcagaaccag 60

cgcttgatgg gcctgtcgtt cccggacgac gatccccctt cggccgccga ccaccggggc 120

cgctcctttc ccgtgcagat ggttgtcgag cgcctggaag cccaggaggg cctgggccaa 180

gacttccgat tcgagttgac gctgttggca gaccatgcgg gtctcgtgtt ggcggacatg 240
ttgggcaaac tgctggcagt gtcgctggtc aagccggacg gcacgccgcg ctggttcacc 300
ggacatgtgg cggaattctc gttggtgggc agtgaccccg gcatagcgac ctatcgggcc 360
gtactccggc cgtggctgtg gttcgcgcgg catcgggtga acaaccgcgt attccgggac 420
cagagcctgt accagcagat cagcgaaatc ctgcaagact acggggcttg ggcgcaatgg 480
cgctgggacg tggcgggaga ggatgcaccg ttcaccatgg ccgtgcaggg cggcggccag 540
ggcgaaagcg atcacaacta catctatcga cggttggaag cgcagggcaa gacctgccgc 600
gatgaacacg atgcgaccgg ccaccggctc gtcatcttcg acagcaatag ccaatgcccg 660
ccggtggagg agagcgctcc ccggataccc ttccaggccg agggcgggcc gcaggaagaa 720
aacgccatcc agcgctggac accgatacag acagcggtgg ccgcgttgtt cgcggggagc 780
gccttcgatt tcaagcgccc gagagcgcag caccatacgg aatcgacgca gcgcaagcag 840
gggcgcgttc ccgagctgga ggtgcacgaa tacgcggggc actacggttt cccgaaagag 900
gaccccaggg gcgttgcgca gctcaacttg cgccgtgaag aaatcgaagc acacgcgcag 960
cagtaccaag ccgagggcaa caacgccagc gtggcgagcg gcttatggtt cgagttgtcc 1020
gggcacttca gcaacacgga gcgcgacgag gacagccaat atctgatcgt ggaggccagc 1080
catagcgcga gcaacaacta ccttgctgaa cttggtcagc aggcggggca gaaggcgcag 1140
ccgcttgccg gccgcaaggg cgaataccgc aaccgcttta ccgcgatcaa gcgcacggtg 1200
ccctggcgcc ccggccgggg acgaaacagc atcgaatgga aggtggccgg acaccagacg 1260
gcgctggtgg tcggccgcga cggcctcggg tctctggatg tggacgaata cggccgcatc 1320
ctgatccggt tccactggga ccgggaagga aaattcagtg cacgcgtgcg tgtggcgagc 1380
aattgggcgg gcggcgaaac cggcatggtg tcgtggccgc gcgtgggcag cgaggtggtg 1440
gtcgcactgg atggcaatcc cgatcatttc ctcgtaatca gcgtcgtcca taacgcccac 1500
cgcatgccgc cgtggcagtt gccgacgcag aaggcgctga tgggcatccg cagccgggaa 1560
ctgaccccgg acggcggcaa cgatcccgga ggccgcagca acgccttggt cttggacgac 1620
accacgggca agattcaggt gcaactcaag agcgatcatc tggatagtgc attgagcctg 1680
gggcacatca cgcggatcga cggcacgacc ggccggcaga acccgcgcgg ccaaggtttc 1740
gcactggata ccgagggcca cggcgtgaac cgtgccgctg ccgggatgca gctcaccacc 1800
gaagccctgc ccggtgggcg ggggcacatg atggacgcgg gcgcgccggt cgcgcggctg 1860
acggtggccc acgaactgca cgaacgattg tcggaagcgg cacgcacggc aggggtgcag 1920
caagcggacg accaggcgga ggtcgccaag caattgcacc agcagaatgc cgacctgaaa 1980

ggcaacggcg gcgacccggc gcaggggcaa ttccccgagt tccagcaacc gcacctgatg 2040

ctggccagcc cggccggcat ccagagcagt acggcgggca gcacgcacca gcagagcaac 2100

gcgcaccatg cgatcaccag cggcgggcac gccagcattg ccacggggcg cagcttcctc 2160

gccagcgcca aggacgcgat tcgtttgttt gcctaccggc tcggcctgaa gctggtggcc 2220

gccagcgggg atatcgacat gcaggcgctc aagaacggca tcaacatcct ggcgaatctg 2280

gatatcaaga tggagggcaa gcgggtggat atcaccgggg tggacggggt gcgcatcaac 2340

gggggcggaa gctatatcga cgtgagccag tcggggatta cgagtggcac caacggccaa 2400

tatcaagtgc atgccagcgg gcatatcttc gcggggccgg atagccggcc ggtggtgccg 2460

cacgcggtca cgtcagcact gaaacccgat gaacccctgc ccgtggtgga caagctaggc 2520

aacaccatgc ctgacagcta cgccattgtg cggcccggtc agaacgaaac gcgtacggtc 2580

atcaggggtg ggcaggttgg caccgacgtg tcgcttgacc accatatgat ccgccgctgg 2640

cgcccccgtc gaggctga 2658

<210> 75
<211> 3286
<212> DNA
<213> Ralstonia solanacearum

<400> 75
tgctctcact tcttggaatg ttggcgacaa acagaaacag ccacggccaa aacgggcgcg 60

gctggatacg ctgagctcag aggaagcgaa gaaaggaacg ggctgatatt cagcgaatga 120

agatcagaca cccaacaagc cgaatatcgg ccctgactat gaagtgaaca tcctttcttt 180

attatcggaa actctccttc ccttgttatc ggcgggcaga ttaccgatac aggaaaaccc 240

gcgcaagggc actttagtgc cgattttaag gatcaaaaac cggcactttt gagttttgtt 300

aacaaatcat aacaaatgtg ccgccatgag gtgcaggtag gcgattcgtc gggcagggag 360

ggaagcagcg tgcgcgtagt gccaccgcac atcatgagcc cggggctgcg ggtacgccgg 420

gggcgtttcg accctgtgcg cgcggttggg accccgcctc acctgcgcgc ttcattgagt 480

tgtttttatg caggtgcata agccgctgtt gaccgcctgt gacaaaggat tgcccgcgcc 540

aaacacagta gcaaatgcat gcaaatctat gcaccaagcc tatgcaacac cggtcagcgg 600

gctgctatgc cgttgacaag gtatatgtcc agcgaacgtc cctgtccggg tgcgggcctg 660

catagagcac caccacgtat gtcagccctt gatgcgtgaa aacttggtag tagagcctgg 720

ccgctccctg cggggttgtc ttcctcccct ggctcaaatg cgtgttggac gtcccccggt 780

ggatagtgtt tgtgccgtcc agcacctcca gggtgatggt gataccacca gtctgggcat 840

gatcgaatcc tcgagcgcgg aagaatgcat cgatccgccc gccagctccg tccatcgcaa 900

attcaaccag cgcatcaact gcactcctca aaaagtcgta gcggtggtct gcgacggtat 960
cgcaagtggc aagcactcca gtggcaaaaa taatctgagg attcgcttca gcgacaaggc 1020
ggaagtactc cgacggaggc ggcgtccctt gctgttggct gacaggcagc tcttgccgaa 1080
ggcgcgcgta cgcgtcattg atgatcgtgg tgtcaggctt ctgcttcttt cctaagagcg 1140
gccatccgtc ctgaccggcg gcgacacgaa taatgactcc tcccatttcc gcaaccgtcc 1200
ccagttcaac ctctaggtca gccgggatgg gcgcatacgg aaggacaatc acatgggcgt 1260
cagtgtagtg gtcaaggctg tcgatgtatg cacgaaacgc atacacagca tccttttcaa 1320
tcggcagaag tgtgagcgta ccctccgctc cgatgccgac cagaatgctc cgcccttcaa 1380
tgccattacg tccgcagtcg gcatcgaatt ttcgctgaag cgacttaaac agaccgatga 1440
cgaacactgc aggcattcta tgggcccatc cagtatcaca gacgtgatct gaccaagatg 1500
gtgctcaaca gggtcggcaa aacgaggcgg gcaatatcct cgtcgctcag caactccaag 1560
agcttcaacg cttgctgccg cgccggaatc tgactcgctg cggcacgcgg tgcagtaggg 1620
atagatgggc cggccaagtc aggcactgga gacgtgggta cgaacgcggc tggctctttg 1680
gctgccgcgt gtagccggtt caatgcaatg ccatttcttt cagcccactc gctgacgacc 1740
tcaacgactt ttgctgtacg gtgtcggcgc catccccagg catagttccc aagtgcatcc 1800
gtgaatgcct tattcactgt gctcgagtat gcctcagcag ccaacggccc gagcgcctcc 1860
ttttgcgccg ctggaagtgg cgcttcctga agatactctc gcatccacgc accctgctga 1920
tccctgtcga tcggcaaaat agcaatgaag gaatctggcg cggattcgac ttcggcgcgc 1980
tcccgactct gtcgttcgcg cagatagtgc accaccttat cagtggcctt cttgaagtag 2040
cgcaagcgat cttcgtctgg gttggcaaat gcctgccaga tatcgctccg aagcaccggc 2100
gtcgccacaa gcctcggctg aggcaacgca ggctcggggg tagcgcgggt agcgccacgc 2160
aaggaaacta agatatcgcc cttaccctgg gggcgatcga cactcacgag atcgccgtgg 2220
acgcgcagaa ggtagtcaga aaacctcttg aaccctagag acttttcatc gaactcacca 2280
aagccgctca cggcaaatgc ccgtttgagc cgttgcttaa ggcgtgcgcc aagttcgggg 2340
gattgttcgg aaagaagctg cgtaagcagt tgtgtggacg cagcgtgcga atcggacata 2400
gcggtctcag cagggtcaga ggcaaaagga gatagcccgc cacagagtgt ggcgggtatt 2460
tccaatactg aaatcatcaa ttgcttctga gttccctgac ttgaagccac gtcgtctaac 2520
cggcagaact cggcacccgg tagcacatcg gcgctgctca agctttcaag atggccctgg 2580
atactttgat ccggcgaatc tcgaaatact tccaaggcgg aaggttctgg aattctactg 2640
ccggtcgacc ggttgtcaag caggcctcaa ctcgtcacaa cgaccagcgc acaccttgca 2700
cgccacaatt atgactcata gagcacttta tgtgatgtac tacagcgtag ctagacttca 2760

ccctgcgtag ccgtgatttg cattggatga ccttgttcga ccttagtttt gcagtttgtg 2820

agttgcacaa ggtcttgatt tatgaaaggt actgagtcat cccaaaaact ccgaaagcag 2880

gtatcgccgc gaccgccctc gacgatagcg cagataggac aggaggcagt ttggcgctcg 2940

gtccagcatg gtcgggttgg agcaacctag actctggccg ccccccctga tcgtggcaaa 3000

tagccagcct tccgttgctt tggcacaacg tccgaatggc cacgtggcac tacttttgag 3060

aaggccgaaa aaccgggggc atcaatgggg gcatcaaccc cacccaccca gaacaaatcc 3120

gtttataaac aaccagctat agttcgtatg tgatacctgt cggcggacca ccggatacga 3180

caaaaagcca ggcaaccgcc tagcttttcc gtttccgcag cagcataaca acgaggcccg 3240

ccatccatgc gcgggcctat ggctgcggat gcgccggggc ctcctt 3286

<210> 76
<211> 6969
<212> DNA
<213> Ralstonia solanacearum

<220>
<221> misc_feature
<222> (5055)..(5154)
<223> n is a, c, g, or t

<400> 76
tccgacaggc tcttgagcac atacaccgcc gcccgcgaca gcaatgcgcc gttcacctcg 60

aacgacgggt tctcggtggt cgcgccgatg aaggtgaaca ggccgctttc cacgtgcggc 120

aggaaggcat cctgctggct cttgttgaag acctgtacga tgcaatgcac ctgttgaaaa 180

tcaataggtt ggcgtgcatg catccggatt tttcgcacaa acaacccgtt ttcatgcaca 240

attttttgca ccaacgattt tgattgtgca atggatcggc cccttggctt gtgccggtcc 300

ccaaggacct cactgaacaa gcgccccctt ggcctgcaca ctgccccaaa gttaggtgct 360

tgagactcaa aaaaacagga aggagtcact gtgtcggtct acccaatcaa gtacaaggtt 420

ctgaacaagg agactggccg caaggtcagc gtcaccaggt ggcgtgttca gatccgcaaa 480

ggtggcgaga gcatcagcaa gctctttgat gatgaaagca ccgccaagtc ttgggaagaa 540

caagaacttc accgcatcaa cactggtgtt cctcagaatc tcatttttga tatgaaatat 600

caacttgaga tgcccgacat gcgcaggctt ctcaaggact actttgatca atacatgagc 660

aagcaggcag cttcttcact gaagacgaac cagaaccgtt gcctgagtgc cattccctca 720

atccctatct accttaaaga tttgggtcca aagattgaca actatcgtta taaaagcacg 780

attgttgatg ccatgatggg caggaccttc aacaaagacc acgttgaatt tggtaacttc 840

aaaattgata cggtagattt ctggctcttg attgcctaca tggattctcg taggaaatct 900

ggcatcaagg acaacaccat ccttcgtgaa atctccacta tcagttctgc ctttgagaag 960
gtctacaagc tctatccaga acaattcccc aacagcatca tgaacccagt caagatgctt 1020
cctaaaggtg aaaagcctaa agcctatctt ggcaggaaac gggttctctc tgatgatgaa 1080
gctgttcaga ttgcagaatg gatgaagctt aaagccaatc aggagcctta ctacctgttc 1140
gttacttgct tggagtctgg agcacggaaa agtgaagtgc tgggtgctca gtgggaaaac 1200
gtcaatctta agctttggtc tatccacatt ccgaaaacca agaatggcaa gccaagggac 1260
atcatgattc ctgaagatga tgatttccgc gaatggttga aggccaatag gcttgctaaa 1320
ggacctatct ttaaactgac agcttggaac ttccgtcaat actgggtgga tgctttgaag 1380
gcacttggtc tttatgatga tccagacaca agacttcact tccatgacac ccgccgcact 1440
gcattgacca agctcattcg tcagaagcga tctaacaact ttcagattgc caaagagatg 1500
ggagtgtcgc ctcagactgt tgagcagacc atccagaaca tgcctgatag gttgtcagaa 1560
gtgtttgcga agcttcgttc tggtcaggca ctcaatgaag aagaaatcat gctgctggct 1620
ggtcacagtt cttcatccat gaccaacgcc tactttggcg acaggaacta acaagcagca 1680
agggataaaa acggatagaa atgacagata aaataatcat ttccaatgaa gagaaaatgg 1740
ctgatgttca gcttgaaatt ctcttctctg gcccattgag ccttcttgat cctagacatc 1800
cagcaatcag tgaagagctt cagaagcaga ttctgtatga caggtaccac aacaaactga 1860
ttgcagatgg cctggaaaca ggccattttt tctacgatta caacaacgat ttaaggtttc 1920
aaaccaagca accgctctat acctttgcag ggttcatctt tgaagccttt gccgtcaatg 1980
ttttcaataa caacatgaga accattggca agaaggcttt ttcttggtgt acaaaccgag 2040
agcagtgcaa ggacgattac attgatcaat tcaaggtaat tggaactgga ttcattacaa 2100
caaagtcact gtttccgaac ttctacgggc cacaaagcac ttttgacctc atttttatca 2160
agaaaaatat caggcaagat gtgcatgagc cagcaaccgt tttgggaaca acaaaccaag 2220
ctggaattca aatcaaagcc atcactggct cagaagaaac tgaaataatt gagccattaa 2280
gaactggcag atactcccat gttctgactt ttcttcgtca ctcagatgga gtgcatagct 2340
acacccattg catggaagtt ttaaactcca tgcatagaag aggtgttttg gggctgccag 2400
aaaaatatgc ccttgagcga cgtatcagcc acccagaaat gtttgggatc gaccaacgtg 2460
aggttgatga ttactaccaa tacatacttc attggtacac aggtcatgcc aatcccgatc 2520
aatttattac tgaaggtgct ggtttggaaa tcaaaggcta taaatattca aacggcttgc 2580
ttgtgccaac cgattaagtt caaccgtttt acacccaacc taaaatgcta gttctagcga 2640
ctagcttttt catattgaga aacgggatca acatagatac aactacccag ttcttgaagt 2700

aaaaaatatt caagaggata gttgacgcac cttgttcttg cacatagagt acagtcatca 2760

acaagcaaga agaactgtag aagcttgttg agtgtgttat ccttcaagta tccatagttc 2820

tatctttttt aacggagaat ttttcatggc cgaaaccaag aagctgaccc ttgctgagat 2880

ccttgccaag cgcaaagagt ttgatgacat ggtggcggct cagttggaag cggaacgtcc 2940

cgctgctctg gcagccatgc tggaacaaat caagatgttc aacttcacgg ctgatgaact 3000

tggtttccat gctgctcctg atgcacgtgc cgctgctggt ggtgccaagg ccggtgctac 3060

ccgcaacatg agcaagccgc tgaagtccac caagaccgac gaaaccagtg tgtggctggc 3120

tcacccgccc aagttcttgg aagctgaagg tgctttcacc acctacaaga gcggcaaacc 3180

cgttgatgca tggctggtga ctcccaccga caagaaggcc aagaccaact tcctgaagaa 3240

gctggcaagc cgtgaaggca aggttcccac caaggaacaa ctgggtgaaa tcaccgaggc 3300

agaattcaag gcagcctaag ctatagcagc ttaagataaa agcacccttc ggggtgcttt 3360

ttgccgttga cgattaagaa tattgagtga agtgacgtct tatgatcatt gcccaagtag 3420

aagaaaaagt taaagccctt gtgggcaata ttgataaggc tgaatttctc tatggccttt 3480

tggaatgcta tagcaagcct aaagcctcca ttactcgcct gaaaatggtt ggcaaaggta 3540

gctataacct ctccaagaac gaaaatgaag ttttatggaa gaagcaggtc tacttcaaag 3600

ccactgacag cgatcagctt ctatccgtca tcgatgaaat gaagaatgca gaggtggtta 3660

caaaacacca acctcgcttc attattgcca tcaatgatac tgacctcttg gcgattgata 3720

ccaagacggc agacacgctt gatacatctc ttgccgaact gaataagaag tttgactttt 3780

tcctgccttg ggcaaatatg gaaaaagccc agtttcagcc tgaaagtgct gctgatgtta 3840

aagcagccga aaaaatggcg aagctgtttg accttctccg agataacaac ccggcaacca 3900

ctgcacaaga aattcactgg cagaacgtct ttctgtccag aatcttgttc tgctactttg 3960

cagaagacac aggcatcttt gaccccaagc tgtttaccaa ccacatagcc tcacacacgg 4020

ctgctgacgg atcagacttg gacgactact tgagtcgtgc ttttgatgtc atgaaccagg 4080

aagaccgtaa tggtgttcct gactatcttg cagacttccc ttatgtgaat ggtggtttgt 4140

tcgctgacaa gctgcctgtt ccgaaattca atcggaagtc tcgtgccatg ctcattgagt 4200

gtggggccga tctggactgg tctgaaatca atcctgacat cttcggctcc atgattcagg 4260

ccgtagttga ccccagccaa cggggcaaca tgggcatgca ctacacctcc gtgcagaaca 4320

tcatgaaggt ggttgaacct ctcttcctca acgaattgaa ggaagagttt gaaaagcact 4380

ctgctagcaa gaccaagctg gaacaactgc ttcttcgctt ggaacacctg aagatttttg 4440

accctgcttg cggatcaggc aacttcctca tcattgcata caaagaactt cgccagcttg 4500

aaatggaaat tttcaagcga attcaggaac tgacacctga aggaatttct ccgctttcca 4560

gaatcaagct ctcccagttc tacggcattg aattggacga tttcgctcat gagatcgcca 4620
tcctttctct gtggctggca gaacaccaaa tgaaccaaaa gttcaaagag acttttggac 4680
ataccaatcc agcccttcct ttgaagagca gcggcaacgt cgtcagcgat aacgcaatcc 4740
atgtcgattg ggacgagttt cttgctccca aagcagagca tgagacatac cttcttggga 4800
atccacccta ccttggagca cgtaaccagg aaccagagca gaaagcagac cttgaactga 4860
tttttgaagg ccacgaggaa tacaaggact cagactatgt ctgctgctgg ttcatcaagg 4920
ccgcaaagtt cattgctggc aaaaatgcca agtatgcgtt cgtggcgacc aactcaatct 4980
gccaaggtga gcaggttgcc tacctatggc cggtggaagt ttggtcaatt cccgcgtcat 5040
gctatctacg aatcnnnnnn nnnnnnnnnn nnnnnnnnnn nnnnnnnnnn nnnnnnnnnn 5100
nnnnnnnnnn nnnnnnnnnn nnnnnnnnnn nnnnnnnnnn nnnnnnnnnn nnnnccaact 5160
ggtagtgcgc ggcacttcgc acatgccgcg caaaagagtg tcacggcaat aaaaaaagca 5220
cccgaaggtg cttttttctt aatcggtagc gatcctacgt ccgcctgtag tagccaacaa 5280
tctcttccct cacggcctca gcaagctcca gcttcatatg acctgtcagc ttcatttgct 5340
gacccagtgc atcggcgcaa acataggcag tttggacata tccattggca gaaccactaa 5400
ctgattctgt aacaacagag aatttcttaa tgtcagagcg agcaagttgc ttcttgcccc 5460
caccaaactg aaccccatca ggaagaagac ggatgcggta tttacgacct gcaaacacaa 5520
acttcaagag tagcaacgga gcagccaatg cgaccagtgc cgcctttccc atgtgattct 5580
caatgaatcc aatggaaatg tattcaccga gaaaaaccag accgatccaa gccgccacat 5640
agataaacag cagaatcacg gtcagcccta ctggattaga tgcggctccg aatgcgcctg 5700
cacggctacc tttgaccaca acttcagccg agccatcaga ttgctgagtc acctggtatt 5760
gcttcacaaa cgagcttttc atatgtaggg gatcaatcat gttcaaggtt gttctggtaa 5820
gtcgctggca tcaacaagct gtttttccag ctactcatcc tcaacccgac ctttcgggtt 5880
gatactcaac tctaacgcat gttcatcttt ttgggttgac aggcaactat ttgggttgtc 5940
aaagatcgtg gagagaacat gccgccagag aacaaccaac acggagaact attcaagagc 6000
attgggcgca cgttggccca gcggcgggag gctaagggcc tcacccagga tcaggtggct 6060
gaagcccttc acatcggcac agaggctgta tcccgcatgg agcgaggcat caccatgcct 6120
accgtccaac gcctggcaga gcttgccgag gtgtacgagt gcggcatcga tgagctactg 6180
atagcaagta gcaccaggac cggcgaccag gccgaactga tctctcagat cctccacacg 6240
cttcccgaag cagacagagc catgatcgtt gaggtggtgc agcggattgc cgcacgtttg 6300
aaggatcggc tttaaatcta agaaggctat ggatttatgg acgatatgcg ttgcctaacg 6360

gcatgcttgc cgtggacaac tcttacgagt tccccaccgc acataccttc gcccacaagc 6420

tccacatcct gcgatcactt tttttgattg atctttcttc catgaagcca aacaaagaag 6480

ccgcagagca acaagaagct tgtcgctcat acttacgatc tttttcttga tttttatgat 6540

gtgatgcgca aaagcttgat gttgtcagag atctgcttat agtgtacgta tcgattggag 6600

aaatcatgcc ccctcacctc gaaccaggag catcacaaag cagcatcgcc catgactcag 6660

aacaacaagt tctgtctcac tggattgaag gtcaaccagc tttcaaggaa gtcctctcca 6720

agttgcccga cacggatcaa tgtttctccc ccaaccagat tgatgccatc cttgccattc 6780

ggcgcaggat cacagaagcg aagcacgccc atgactgagc agtcaccctt gccaaccgac 6840

gaggatattt gggaagaact tttgcactcg gaagccagca agcagttctt agataagctg 6900

atcaaaaaag tccaagagtt tgtagagcaa caaacgcatt gaagcacctt cgggcgcctt 6960

tttattgcc 6969

<210> 77
<211> 852
<212> DNA
<213> Ralstonia solanacearum

<400> 77
atgacgagct caagcaactg gccgagcgcg cgcggcagga actggggcgg cctggaatgg 60

gtgcccgccg cgctcgacgc cgtggtcgcc tctgccgatg gcgatggccg caagctgctg 120

aacaacctcg agatcgtcac ccgcgccgca cgtgcgcagg ccgaaggcga caccgtgccg 180

accgtcgacg aggcgctgct ggcctccgct ctgtcggaga acctgcgccg cttcgacaag 240

ggcggcgatg ccttctacga ccagatcagc gcgctgcaca agtcggtgcg cggctccgat 300

ccggatgcgg cgctctactg gttctgccgc atgctcgacg gcggcgccga cccgcgctac 360

ttggcgcgcc gcatcgtccg catggcctgg gaagacatcg gcctggccga tccgcgcgcc 420

gcgcgcatca cgctggatgc ggccgagacc tacgagcgcc tgggctcgcc ggagggcgaa 480

ctggcgcagg cgcaggcgct gatctacctg gccgtcgcgc ccaagtccaa tgccggctac 540

aacgcctata acgcggcgcg cgcctttgtc ggccaggaca agtcgcgccc cgtgccggtc 600

cacctgcgca acgcgccgac ccggctgatg aaagagctcg gctacggcca cgcgtaccgc 660

tatgcccacg acgagcccga ggcctacgcc gccggcgaga cctactttcc ggacgacctg 720

ccggcccagg gctggtacgc gccggtgccg cgcggcctgg aaggcaagat cggcgacaag 780

ctgcggcacc tgcgcgagct ggacgcccag tggttggcgc agcagaagaa caaatcctcc 840

cgcgacgact ga 852

<210> 78

<211>  579
<212>  DNA
<213>  Ralstonia solanacearum

<400>  78
atgaaaaacg gacgctacca agaggacggc atagacgttt ggtacttcaa cgatcaccta      60

catcgtgaag acggcccggc ttgtgttgag ccagatggaa cccagtcctg gtatatccac     120

ggaaaacttc accgcaccga tggcccagca gttatctggg gagacggaag tcaggagtgg     180

tggattgatg gtgcgaggca ccgcgaagac ggtcccgctg ttctcaatgc cgatggcagt     240

tgctcttggt atctcagagg caagctgcac cgcacagatg gtccggcagt agaagctgcc     300

agtggtgcaa cggcgtggta tcagaaaggc cagctacaca gagaagacgg ccctgccgtt     360

gaaggctcag acggcactag agagtggcat cttcgtgcca tcaagctctc agaggaagaa     420

ttcaaccgcc aacatccttc tttcaacaag aagaaggtgg tcgaggtgag aagtgtctac     480

gacaaacctg tgaaagccgt tgagaatcga atattggcaa tcaggaagaa gttctttgaa     540

gaaggtagta cgtcaaaggg agtcaagcca agatggtag                            579


<210>  79
<211>  882
<212>  DNA
<213>  Ralstonia solanacearum

<400>  79
atgaaagaac aaaaatattt tgaaggtgtt gccagcaact acttcagcaa cgtggatgga      60

caggctggcc ttgccgcagt cccttccaac ttcgttgact tggtactgac cgacccaccc     120

tatggcattg cagacggtgc caaactcacc aagaagggca gcaagatcgt caccactcag     180

caggcatggg gtagtgactt taaggatagc tgggcgacca ttgatcagta ttaccagtgg     240

ctcaagccct tcattgctga attcgttcgg gtgatgaagg acaacggctc catgattctc     300

ttcttggatc gcaagtacac cgggttgatt gcccactact tggaacgaga ctttgacttg     360

aatttcaaga acaaggtcta cttcaagaag aagaaccctg ttcccagcat tcgcaagaac     420

aactaccgca gcaccattga agaggctgtt tggttcacca agggaaagga gtacaccttc     480

aacttcggtg cccagagcga catgcttcag gtctatgagg gtcccattgg gaagaagaag     540

acccagcacc ctaccgaaaa gtacacatgg atggtggagc ctttgatcag gaaccacagc     600

aagcccggtg acgtggtgtt ggacgccttt gcaggctctg caaccaccct cgtagttgcg     660

aagcagcagg atcggcacgc cattggcttt gagaagagca ccgccctgta tgagatggcc     720

aaggcccgca tcgaggcaga gcagcttgag tttgcattcc atgcaccatg tgacgaagca     780

gccatcgtgc gcgagctgga tcagctcatg gacgccgagc ttgcagacat ctccagcagc     840

cgcagcagtg ccgcggatct ggctgacctg gtcacagcgt aa                        882

<210> 80
<211> 895
<212> DNA
<213> Ralstonia solanacearum

<400> 80
gtaaggaggg cagcagccat gaaagaaatg aactgcctcc aatactgcat caacggcatg 60

agtgaccgat tgttcagctt cgctaagacc aaggaaggca aggcactgct tgctgccttc 120

aagaagctca tcgtcattcg tgagaaccag atcaaagagc ttctgattgc ttacaacagc 180

tacttcatgg tggctgctgc catgcagctc aagggcatgc ctcagcaccc acgggccatg 240

attgagttca tggcatcaga ggaattcagt gccctgcatg ctgaactggt gaagaccgtt 300

gaggacaact accccttgct catgtcctgc ttggatagga agcagaagcg aaaacttgat 360

tccctctttg aataaacaaa agcaccctac gcggtgcttt tttgttggca tcgactcaat 420

ccgcgagatt tacaccaaca agaaagagaa taggcacaag cctactctct gagcagaaaa 480

aaacaccctg tggggtgatt tttatttacg aacacaatct atcttgatga ccttcctgct 540

cttcagaatt tcatcaactt caggtgtcag tgttttgtga agctcaagca tggctgctga 600

caccatgcag ccttcaaacc aatactcctt cacttcacct ttgacgactg ctgggccatc 660

ttcacggtgc agcagcccgt gacgatacca agcagcatag tttctaccca cgaccgcagg 720

cccatcttca cggtgcagca caccgttctt gacccattgc ttcacttggt tgtctacaac 780

acattgcagc ttggtcttca tttcagtcgg caatagataa cgagattttt cattctagcg 840

tgctttttgt tgacttccat ttatcatctg caagaataaa aaaaaggaga cacca 895

<210> 81
<211> 540
<212> DNA
<213> Ralstonia solanacearum

<400> 81
atgggataca agcaatggat caatggcgcc atcctccgtg aggaggacgg cgcaatcatt 60

ccgcaggatg ccaggaatgt ggactatcac gcgtacctgg catgggtgga aaacgggaac 120

tcggcggcgc cattcccttc tcaaaccctc gcgcaggtga tggcggcgaa gctcgccgcc 180

atcaaagccg cgtgcgaggc atccatcgtg gcaggcttcg cctcctcagc gctgggcgcc 240

gttcatacgt atccctcgtc ggaaacagac cagcgcaacc tcctgggctc tgcgattgct 300

gcgcagtggc gtgatcccga ctgggccgtg ccgctgtggt gcctgagtga cgaagggcaa 360

tggctttttc tgtcgcatga cgctgcccag gtgcggcagg tcaacgtgga ttgggttgct 420

ttccgcgaca cactgcggca gaagtatgct ggcctgatcg gtcagctcaa ctcagccgct 480

agggaaacgc tgatttatca gtctcagccg tcatcgctga cgcagctgag gccgttgtag 540

<210> 82
<211> 483
<212> DNA
<213> Ralstonia solanacearum

<400> 82
atgccgactg catcctgcac atttacgctc aacgggcagg caaccagcac gctaacgtgc 60

gacggccgcg tttatgtggc tttctctggc aataaagggc atgagaacaa gcccggcgat 120

accggcatcg ctggacaagg gccattacca accgggcgtt attacatcgt ggaccgtgag 180

agcggcgggc gcctggggcc gatgatggac tcgatccggt cagcggtcag caactccaat 240

cacgccgagt ggttcgcgct ctaccgcgat gatggcgcga ttgacgactg gacgtttgtg 300

agtggcgtgc ggcgcggtaa tttccggctg cacccagttg gcccgcgagg cattagcgag 360

ggctgcgtga cattggtcag caaacagcaa ttcgcggctc tgcgcaccta cctcaagtct 420

cgtcagaccg ggttgtcccc cggcacgaac acgaagtatt acggtgtgct ggaggtcaaa 480

tag 483

<210> 83
<211> 300
<212> DNA
<213> Ralstonia solanacearum

<400> 83
atgcggtttg ccctcaagac cgtcgctgtc gtgttactgg cgttcgcata tacgcgggcc 60

atttatctct ggtcttggct tgacccattc tttgccagtt cggcatggca gcgaatctat 120

ggcttcctcg cgcacctgtt tggcgtgagc ggtgcggagc aaggcgaaga cttgcagctc 180

gccatgttcc ttgtggtggc actgctgctg gcgatcttgt cagtttggct gttcgaccgc 240

tacgtgtgtc ggccggcctg ccacacatta gcgcggcaga gaaccgcgaa aaggccctaa 300

<210> 84
<211> 677
<212> DNA
<213> Ralstonia solanacearum

<400> 84
ctaacgatgc cgcctccggg cggcattctt gtatgtatgg ggcggaattc aggcgaggtc 60

tggtgggcga ccgtctaaaa cgcgctcaga gccgttttag gctgctgatg gaccgtggga 120

ggtccacgcc ccggaaaggc gtttttaaac ggctttcgaa cgcgaatgga ggcatcccgc 180

aaggcggcgc tccccaaagg ggcaccgtgc ggggtcagaa gaggtctagc tgtcgatcgt 240

cgtcctcacg gcagatgttg acaatctgac gggacgaaag gtgatggcgc cgagccaaag 300

cgttgatgct ggcgttgtgc cgatctttgc gaatctgcgc gtcacgcacg cggataaaca 360

gcttgtcggc cttgggcagc caacaacggc catccgaatc cagatgcgga gccagcgtcg 420

tccgcaaacg ggccagttcg tcggcctcca agcccagtgc gtgagtacgg tactgcggga 480

tgctgacatt gacgccacca tggtgggtca gccagtcccg cgcccgaccg taaccaagcg 540

cgcggaccac cgcacgcagc acgggtggca aggttctcag caaatcttca tcgacagccg 600

ggtaggcgtc taccacgcgc tcgatttccg acgcggcatg cgcagcggga tggtgatagt 660

gcggaatgac catggct 677

<210> 85
<211> 456
<212> DNA
<213> Ralstonia solanacearum

<400> 85
atgtctacgt tcctcatcaa gcatcatcgg cagcttgtcg gcatcgccaa aggctgggcc 60

atggtcagcc tgcctggctg gtctgaagag acccaccgcg acctgcttac acgccatggc 120

gcagccgtgg ttgacggccg cgtgtccgcc agtaccctca acctcgcgca gctcggcgct 180

gtgctggacg actacgagcg tcgggggtgg tcgcgctatc ggcgcgtgtt ccgtcaggct 240

ggcgtgacca aggaagtccc accccgcatc gctcacctcg tgcgcatgtg gttcaagctc 300

gggcaggcgg gcaaggtggc taaggcaacg cgcccggcgc tgcttgagtt ctgtgcgcgt 360

caagtgggcc gtcacgtgcc ggacttggac agcttggccg tagctgaatg ccaaacgatt 420

gccgaagctc tcaagagttg gctggcgcgc gggtaa 456

<210> 86
<211> 702
<212> DNA
<213> Ralstonia solanacearum

<400> 86
atggaccgcg aaaccgcaat cgacaagatc aagaagtgtt tggcgcttgg gcaatccagc 60

aacccgcatg aggccgctgc cgccatgcgt caggcgcaga agatgatgac cgcctatggc 120

gtcagcgaag acgacatgct ggcggccggc gtggccgagc tgtgggtgaa gagcggtgcg 180

acccggacgc cggcccgcta tgaggtgtgg ctggccagca tgatcgcatc cgcctttggt 240

tgcgagctgg tgttttccaa ccgcctgtcc ggtcgaacca ccatccaagg cggctacgta 300

tttatcggta tggccagcgc ttcgacggtc gccggttaca gctatcgggt tctgttccgc 360

cagcttcgcc gcgcgcgggc cgactacatc aagaccgcgc tcaagcgctg ccgctccaag 420

aacaaggtgg cccgcgcaga catgttctgc gaaggatggg tgtttgccgt gcgcgagctg 480

gtgggtgcca ccgtgccgcc cgaagcccac cgcagcgcgg tggatgccta catgcgcgcg 540

aactatgccg tgaccgagca agttgagacg cggtgccgcg aacctgggcg ccatgtttcc 600

caggtgggcg actgcgtgca aggcgttcgc gctgggcgcg gcgccaccct ccatcgtggg 660

gttggaacag gggtggatgc accccaaatg ctgggggcat ga 702

<210> 87
<211> 276
<212> DNA
<213> Ralstonia solanacearum

<400> 87
atgaccaagc aagaactcat caagcatctg gccgacaagg ccgaagtaac caagggcaag 60

gctgaagacc tgctcaacgc cctgaccgaa accattcttg acaccgtgcg cgccggtaac 120

gagctgacga tcaccgacct gggcaagttc ggctccgtgg agcgtgccgc caagaccggc 180

cgcaacccga agactggcga cgttattgcc attgcggcca agcgcgcgcc gaagttctcg 240

gctgccaagg ccctgaagga tgcagcagcg gcctag 276

<210> 88
<211> 633
<212> DNA
<213> Ralstonia solanacearum

<400> 88
atgaccacag taacgcaaca ccccaccgag attccggctg gctatcgcgc caacgcgcgg 60

ggccacttgg tgcccgaatc gaccatccac cccatcgacc tccagcgcga caagctggtg 120

cgcgagctgg tggccaaagc caaggcactg caagcagcca tggccgattt caaacgccaa 180

gcctttaacg acaccgacgc atttgtatcg ctctcggccg agcgctacga agtgaagctg 240

ggcggtgcaa agggcaacgt caccttgagc acgttcgacg gtcgccagca ggtagtcgtg 300

tcccaggccg agaacatcgc tttcgatgaa cgcctgcaag ccgccaaggt gctcatcgac 360

gagtgcatca aggaatgggc caagggcagc gacccgaaga ttcaggtgct ggtgcagcaa 420

gccttcgaaa cggacaagga gggaaagatc aacaccggcc gcgtgctcgc gctgcgccgc 480

ctggccatca gcgacgacaa gtggacgtgc gccatgcagg ccatcggcga gtcggtggat 540

gtggtgggca ccacggccta catccgcttc tacgagcgcg ccgagggtgc caagaagccc 600

acagccgtca ccctggactt tgccgctctg taa 633

<210> 89
<211> 588
<212> DNA
<213> Ralstonia solanacearum

<400> 89
atgccgctgc ctgaagtcgt ctgcccgaac tgccgcgtgc ggatgagcct ggatgtggtg 60

ctagccgatg acggcgtgcg cgacatgctg ctggcgctgg tggacgtgca cccggcaggc 120

gactcgttca tcaagccgct cctgcgctac atcggcctat ttggaccgaa gaagacgcag 180

atggccaacg gacgcatggc aagcctcatc cgcgagctgg agccggagat gcgagcggcc 240

caggttaagc gcgacggtgc cgtgtacgcg gcgcccatgg cgctgtgggc gtcggctttc 300

agctacgcag tggaccaagc gaaccacggc cgcctagaac tccccctgaa gtcgcacggg 360

tggctgcgct ctgtaatcgc ggggcaggcc gcccgcgcag cgagtcaagc ggaagccgac 420

caagaggcgc agcggcgcgg cattgctggg gctggcacac cggaagcccg ccgccaagcg 480

gccacgacca ccgtagggcc ggcagcggta agcgagcaac tgccgaaaac cgagatgcct 540

caagaagtgc gcgaagccct caacaacctg aagaaaggac gccaatga 588

<210> 90
<211> 506
<212> DNA
<213> Ralstonia solanacearum

<400> 90
ttgccacctc caaccagatc ggggttcgtt tcgcgcagac ggcggtacag cacgcggcgg 60

cgcccggcgt acttcgcttg aaggcgcgtg aggttgtccc gagcacgggc catttcttcc 120

tgccgccact ccacatcgcg caccgcgtgg atctcttcgc ggtcgatggc gcgaagctgg 180

cgctgcacgc ccacccgcac gcgcaaccgg cgccactccg cgcagattcg gcgcaagccc 240

gccagcagtt cggctgcaac aggtccctgg gtgatgcctg ctagctgttt catgtcttca 300

ccttccattt cacttcgtcg gggatttggc atgtgccttc gatccaggcg ccaaacgcgg 360

ctgcctctgc tgaaggtgcg ccgttggcga tggcatccaa gaagcgcacg gccgctggac 420

cggtggcata gatgcgcttc cggcgccgtg tatgggtaat cgtgatgccc ggtgggtcaa 480

actgttccgc catggctgcc tccgct 506

<210> 91
<211> 909
<212> DNA
<213> Ralstonia solanacearum

<400> 91
atgtctcgct tgggaatcga gtatcagaac ggtctgattt atgagggcag ggacaacccg 60

agctccttag ccgttccgac gccaatcatc tcgcagtgca cactcattga gtccccctct 120

gatcttgaga ggctgcctcg tgggatagac tcggatccgt ttcgctggat tttcagggag 180

gactcgttcg accccgtttc cagggttcga cggggccgac tatttcagcc tttcagtaac 240

tcgaacaagg agttggtctc cgttgacgcg catccctttc tgccttcgga tttgggccgg 300

cgtggcgtcg atggtcaatt gcgcaaggaa atgaccgtgt tcattcactg cacgcaactt 360

gtggtgcgcc gggagcgcgg ggaaggtctg cagcttgcaa tcggccacga aggggcacac 420

tcgctctggc gcattctgca gactgagcaa acggttaccc aggacgtgct ggtcaccctt 480

cgagcggagt ctgtattcgg ggtactgcca cccctcgatc tggcgcaaat cccagaggac 540

ggccgaacgg cagtagctgc ggcctacgac cgggtcatgc atgttgcata tcgagactcc 600

ccgacatcgg ttgtggacca atgcaggaac ctctgcgcag ttctcatggc tcgctggttg 660

cgccagcgga cgagcaacga taggttgctt cacgacgatc tcggcgcgtg tatatcggca 720

gtcaagaagt tcgccgaagg agagcaccaa ctcgtccgcg ccgcacttga gacagtgaat 780

cgtctccatc cacgagggaa ggagaacgag cgtgagcggt acagtttgcg ggaggtgtca 840

aatgaggacg cagagcttgc attgcacgca accgggttcg tgattaggga gcttggctgg 900

gggcgctga 909

<210> 92
<211> 1296
<212> DNA
<213> Ralstonia solanacearum

<400> 92
gtgaaggttt tcaatgcccg gcattttctg aggcatatcg ctgccagcgt gctgcgcgag 60

ttcacgcaag cgcatgcgct ggccacgcgt ttggtggtgg actggtccgg accagccgac 120

atgttgtcgg gactgctctg cgatgcggtt gatgcattgg agcagcaggt tgctgccgcc 180

gaccttcccg aacgcgaccg cgaagcgctg gagcacgacc tgctgctgtg gactgacgac 240

ctgcggcgtg cttgtctgat ggccaacagc cttgctgtag ccgagttccg gagcgcatgc 300

caggacgatc cagatgccct gggggcgttc gcctcccgtg acgagcgaga gatcgcactg 360

tggatgctgg tcttccgcga caagatcttc cgcgacgtcg aactgcatct ggccttccaa 420

tccaagaccc acggcaagtt ttggaagagg caccgcatcc agcccggcct cgaactgacg 480

cgcgagcggg tccggctcga acagttctgc catgccgtgg cgcagcttta caagaagtcc 540

ggcggcggcg atggtgtgca catcgaactc tccgagcgac gcagcacggc aggcctggtg 600

gatgccatgt ccagtttcca gctgaccctc tatgtcgagg gaccagtgac ggcgctcacg 660

cacttcacgc agagccattt cactcgcgtc accacccgcg tggcgctgga gtccgcgttg 720

gtctaccacc cagcgaccgg cgaagtggaa acagtcgtca agggtggcgc caagaaccat 780

ggtgcgatgc tggagttgtt cggcgagcat gtcgtccagc aggatctggc ccccgagcgg 840

attgagccgc aacgctacca cctcaacgcc ttgcgcgatg acctgcagcc ctatgaggat 900

tggtcgatcc acggcgtgga tagggtccgt ctgcgccgcg cacgcctagc gccggcggcg 960

ggatcaggtg tcagtttcac tgtcgaagcc tcgcccgaca aggaccagga cgacgccatc 1020

cggatcgccc gcaaggcgct caaggtcgag catatgtttg aggcggaata tcacctcgag 1080

gctgcgaccg tcatcgtata cacgcaggcc gccgacggcg ggcgggcggg ccatttcagc 1140

ttcaatatcc gcgcctcggg agcctcgacc atcaagaacc tgtcgctgag gaaccaggtg 1200

ctcgcgcgca aggtcctgca ggcgctgatg gtgatcgacg ccgaggacga tctcatcgcg 1260

accatgtcca ttcccaggga ggctatcgca gcgtga 1296

<210> 93
<211> 975
<212> DNA
<213> Ralstonia solanacearum

<400> 93
gtgagtcagg ctcaggttga cgctaccgcg ctgctctgcc gattgctgga gcgcgacaaa 60

cccgaaatca acggccaggc cctgttcgat ggggacccgc aggcagcggc tcacctgctg 120

cgtgagcggt tgctggtggt cggccgcccg ctcgaatggg tcacatgccc ggactgccgt 180

gtggaaatcg cgcgggtcgt gcgtgatgtc tcggccgaca ggatcgccct gcgttgcccg 240

gaatgcgagg acatcgacgc ctcgcgtcgt ctgcgggaaa cgtacacggc ggtgccggct 300

cgggccgtcg ctgccttact gagcgggctc gggatgacag ccggtggtat gaaggtcatc 360

gaacccgacc gcgtctggcg gctgggcacg acggagccga cgcgagggaa gccgctgact 420

tggtacttcg cacgccagct tgggcgaccg gagatcggcg cccggctgcg cgagcagatc 480

cagctcgagc gcacggccgg ttcctgcgtg gcgctgacca ccagcgagtt gccgttgccg 540

attggctcgc cgctggtggg cttcgatgtg cgcatgctgt ccagcgcggc gcggataagt 600

cagagccgct tcgaattctt cgcggaccgg caggctgcgc cgggggcgca gcaggtgggg 660

gaggcagagc cgcggttgac cgcgcagacc accctgcgct acgtgcgttc gctgggcaaa 720

gccttcatcg aaggaacgga atatcctctg gagccgcgcc agcaggccat gctgcttgct 780

ttgctcaacg atctcgatca cgagatgggt aaggccgcgc tcaagatcgc ctgcggctcg 840

caggcgcaac ggttctcgcc gagcaaggag ttcgaccgca acccggtcgt ctacaagacc 900

ttcattcgct acctgcgcga cgacgaacgc tacgcgctta tcgttcctga cgaggatcgg 960

gactggttgg ggtag 975

<210> 94
<211> 2388
<212> DNA
<213> Ralstonia solanacearum

<220>

<221>  misc_feature
<222>  (1457)..(2385)
<223>  n is a, c, g, or t

<400>  94
gtaggtgccg caatacaccc attcgccggc ccagccggcc aaatgcccga tactcatgtg      60

ctcgacgcta ttataagagc gggggcatgt catcggcgcg aacgaggatt tcgagcaaat     120

cgaccctgca ggtttgacag ccggtgcttg cgtttagaac gtggttaggg ctgcagaagc     180

ttccggagct caggcaaggg accgtttctg tcaaaaattt gcgaagcaag aaaggcttcg     240

tcaagggcgc caagtgagcc cgaaggggac cgacgcaact gagcaaggac aagcttcaaa     300

gcggcaatgg ggaacgcgag catcgcgtgg tctgggtcaa aagtgtaaag gtgtattttg     360

tcgagaacat aggcttggac caactgccgc actccgacct gttctacgaa agcaaaatga     420

gcgggggctg taggctcaca gaacttcggg attttgtagt tggctgcctc tcgcttatcc     480

accagccagt cgaacggttt atcggcccca atatcctgtg aggcgaggac gctgctcgtg     540

taatgatggg agaacgtgtc taacacgact ttgtgcgtag tgcccgcacg ctttgtcgga     600

gtagcgccgg cggttgcccg ccacgaatag ggcgtagttc ttgaatagaa gatcccgacg     660

ccaccgaggg caagcgacgc ccgcgtcaaa tagaaggcgc tgtaatagag cttcacggtc     720

gcccacgtgt acaacttcct gtctagcccc tgtattgctt cagcaatgga caaggttccg     780

gagtaaattg catcgcgtgc gtcctcattc aaggaggcag caagctggac agcctccctg     840

gcttggagct gcctggaaac tatggcagcc ccggtaatcc cagatagaat ggagtccgca     900

acgcgttgcg cacgatgcat aagattagaa tttgaacttc gttgatgcct tgactttggt     960

ggggctgaag gcctcttgca gtcgtgcgta tatcttcttc tgcgccccgc taccaccctg    1020

ccccttaact tccgcctgga tgataagtga ctcgttgaac ttcagagctg cctgaattgt    1080

tttcacctca aatgaggcct gattcttaca gaagggaatc aattgaagcc tgaggaaatc    1140

cattgcggca agaaatcctg atgcgtagag aaagacgttc tctttgtctt cccaacgatc    1200

gccatagatg ccctggagcg cgatgaacca gttgagaatg atctgctttt gcaactctag    1260

ggaaccgctg atcaatgaag tcggcgaatg gaatcgcgag tcatgccatc ttggtttttc    1320

ggattgagga agggatgctt cgattcagca cggtttttcg ctgctttcgc acctatcttg    1380

gcgcgctttg cgcgctcagg acgcaatgct cccaagtagc attacgcacc gctccaccat    1440

cgcacatctt ctcagannnn nnnnnnnnnn nnnnnnnnnn nnnnnnnnnn nnnnnnnnnn    1500

nnnnnnnnnn nnnnnnnnnn nnnnnnnnnn nnnnnnnnnn nnnnnnnnnn nnnnnnnnnn    1560

nnnnnnnnnn nnnnnnnnnn nnnnnnnnnn nnnnnnnnnn nnnnnnnnnn nnnnnnnnnn    1620

nnnnnnnnnn nnnnnnnnnn nnnnnnnnnn nnnnnnnnnn nnnnnnnnnn nnnnnnnnnn    1680

nnnnnnnnnn nnnnnnnnnn nnnnnnnnnn nnnnnnnnnn nnnnnnnnnn nnnnnnnnnn 1740

nnnnnnnnnn nnnnnnnnnn nnnnnnnnnn nnnnnnnnnn nnnnnnnnnn nnnnnnnnnn 1800

nnnnnnnnnn nnnnnnnnnn nnnnnnnnnn nnnnnnnnnn nnnnnnnnnn nnnnnnnnnn 1860

nnnnnnnnnn nnnnnnnnnn nnnnnnnnnn nnnnnnnnnn nnnnnnnnnn nnnnnnnnnn 1920

nnnnnnnnnn nnnnnnnnnn nnnnnnnnnn nnnnnnnnnn nnnnnnnnnn nnnnnnnnnn 1980

nnnnnnnnnn nnnnnnnnnn nnnnnnnnnn nnnnnnnnnn nnnnnnnnnn nnnnnnnnnn 2040

nnnnnnnnnn nnnnnnnnnn nnnnnnnnnn nnnnnnnnnn nnnnnnnnnn nnnnnnnnnn 2100

nnnnnnnnnn nnnnnnnnnn nnnnnnnnnn nnnnnnnnnn nnnnnnnnnn nnnnnnnnnn 2160

nnnnnnnnnn nnnnnnnnnn nnnnnnnnnn nnnnnnnnnn nnnnnnnnnn nnnnnnnnnn 2220

nnnnnnnnnn nnnnnnnnnn nnnnnnnnnn nnnnnnnnnn nnnnnnnnnn nnnnnnnnnn 2280

nnnnnnnnnn nnnnnnnnnn nnnnnnnnnn nnnnnnnnnn nnnnnnnnnn nnnnnnnnnn 2340

nnnnnnnnnn nnnnnnnnnn nnnnnnnnnn nnnnnnnnnn nnnnngga 2388

<210> 95
<211> 259
<212> DNA
<213> Ralstonia solanacearum

<400> 95
atggatatcg agccgtcgtt cgaaacgaac gcgtatcttt ccaaagccgg caaaccaagc 60

gtgcgtgcgc tcaaccaccc accggtaacg gcccaggcgc tccctgctct cgatgccctt 120

gcgggcgatc cgggccctta tgccgtgccg cttaagatac tgccgacatc ggcggcagtc 180

gtaagccttg tcggcatgca gcttgtcggg gcgcttacgc ggccggccat ccaggccacg 240

tatcgcggga atcgcatcc 259

<210> 96
<211> 255
<212> DNA
<213> Ralstonia solanacearum

<400> 96
atgacttgtg ttagcgactc taagtacgtt tgtaccggct ctagtggaaa gattgtgtgg 60

ggcggtggcg gattggaggg gcgctcagag tttgggccga ataagtcgga ttttatggcg 120

gcgctttatt tggatgagca aggcaatttg attgtggagg atcgtattca ggttcacatg 180

agcttgcgac ttctgcatat tccaacgggg acggcgcagc attacacggt ttacagattt 240

aggcgattga aataa 255

<210> 97
<211> 534

<212>  DNA
<213>  Ralstonia solanacearum

<400>  97
agaatctgtt atggaaccac gcgaaagtag agggccgcgc agaatttatt tgcgctcaat      60

gtgaatcgtg agtgttttta ttcaccccac aaggccagaa cgcagagtcg agccaggctt     120

ttgctgggca gcgttgcttt tcgggagcat ttgggcttac tcggaaggat tgatcgttca     180

tggcggacga ctggctgccg ccgatggggc tgtcgggttg ttgtacctct acggcgagca     240

aaccggtcat ccatcagcgg ctggtggcgc tctactgctg tttctcgcca agaacgttta     300

ttgcgccgtg cgaggcaggt tttggctgca gtgcttgctg ctcagtcagg gctatcgcgt     360

ggtcagtcag tagaagcccg agcatcgggc cactgatatg caggcggcat gagcgacgtg     420

ccaccgctac aagtacacat caagcagttt ttggggaagt ttctgtgcaa tcaccacacc     480

gcccggtcca actggccgta ctcctcggcc tatgcgccgt cttcatccca ccag           534


<210>  98
<211>  993
<212>  DNA
<213>  Ralstonia solanacearum

<400>  98
atgttcaaca gttttataga ggcactacca gacaaggtaa gaagctcaaa tgatttggaa      60

tatggttgca agttcaggaa gaaagagaaa gctttgcagc atctctatgt agagcttccc     120

cagctctaca agaagttcat agccctggac atagacatcc caggctcttt tgatctttgg     180

taccaccttg ggttgccacc acccacctat gtgatggtca atcctgagaa tgccaagtgc     240

cactaccttt atgaactgaa gaccccggtc tactacaccg agcatgcacg ccgcggacca     300

cagaagttct ttgaggctac agacaatgct ctgacctgca tgctaggggc tgaccttggc     360

tttgtaggcc acttcatcaa gaacccgctg cacccccact ggcgtgtcat ctgccaccat     420

gccacatatg acttggaaga cttcaaggat tggggccttg acctggtagg ctacaagaag     480

aaacgcaagc tcttggactc tctagagggc cgaaacacca ccctctttga caccctgaga     540

cactgggcct acatcgaggt caggaagtgc ggctcctatg ctgatttcca gcaggctgcg     600

gactctgaag cactgtctat caaccagacc ttcctagagt gcttaaacgg cgttctacca     660

gccaaggaag tgctttccac tgcgagatct gtaggcacat ggacatggag acacaaggac     720

accataggca accagaagaa ccgtggtgtc atggagcttc caccagagat gtccatgaag     780

gccaagcaag ctgctggagc tggcttcagt gctgtgacta ggacaaagaa caccaaggaa     840

gccatccttg aagctgccat tgcattgaaa gcagcgggaa caccagtcac ccagcagtct     900

gttgccattc gtgctgatgt ctccattcct tctgtgaaaa agtattggaa tgaagtggat     960

agccagcttg ggttgtataa aaaacgtgcc tga 993

<210> 99
<211> 852
<212> DNA
<213> Ralstonia solanacearum

<220>
<221> misc_feature
<222> (395)..(544)
<223> n is a, c, g, or t

<400> 99
atgatcatcc ttttgcaaat cagaacaagg cgctcttcgg agtgcttttt tctttgtgtg 60

cttgacttct gcactcaatt ttgtaattta gataaatgca aaaggagtgc tatgaagaag 120

tatggagttg gagttgtctt ggctggttta atgatctgtg gtaatgcgca agaacccaag 180

tatgggttcg tgaaagtagc cgagtctgaa gaaatggtct tgttcgtgaa tgttgattca 240

tataggtatc gttacctggc agacattcaa agcttcgggg cggtggttca gattttcagg 300

aagatgggtg gtggctcgtc attcagaaaa gaatatgtac tagagcctga ctgtcgtaga 360

ggttatggaa ggctatacag ccacggcctt gatgnnnnnn nnnnnnnnnn nnnnnnnnnn 420

nnnnnnnnnn nnnnnnnnnn nnnnnnnnnn nnnnnnnnnn nnnnnnnnnn nnnnnnnnnn 480

nnnnnnnnnn nnnnnnnnnn nnnnnnnnnn nnnnnnnnnn nnnnnnnnnn nnnnnnnnnn 540

nnnntcgaag attggtcaat ccggcggcaa gaaacacaga atgttgattc atataggtat 600

cgttacctgg cagacattca aagcttcggg gcggtggttc agattttcag gaagatgggt 660

ggtggctcgt cattcagaaa agaatatgta ctagagcctg actgtcgtag aggttatgga 720

aggctataca gccacggcct tgatgacaga aatcttcaat catatgagtg gctgaaggat 780

agtggaaccg ttgcaagtat tcatgcagag gttctttgcc atgtgtcttc ttgggataag 840

ccgaagaaat aa 852

<210> 100
<211> 456
<212> DNA
<213> Ralstonia solanacearum

<400> 100
atggatgaga agcagattgc agaactgcgg aagtttttgg gcactatcag tgacccgaag 60

atcagtgcct tcattgatct gaagaagttg tgccagtcgg gcatttacaa ccttgcggtt 120

cagatggcca cggaacatgc tgccaaacac catgacttcg gctatctgga gaaaatcctg 180

actctgctgg acggcagcac tcatgccagc gggttcattg cctctctacg tcccaagctc 240

aacttcattg tcactgatac gaagccccga aagttcaaga aggccacacc agaacaggta 300

gcaaaggcag cgaagcaagc cttgagcaaa cctgttgccg tcaaaagcac tcagcctaaa 360

cctgctaaga agaaatctga agcaaaggta agtcatgacc tgatggattc taggttcatg 420

ctaccaggca gctacgggac aggccgaaga agatag 456

<210> 101
<211> 1110
<212> DNA
<213> Ralstonia solanacearum

<400> 101
gtgaagccca agcgtgctgc tatcagtgtg gatgaacggc tgctggcttc attccagcag 60

atcaacgatt tctaccgtca gcacggcaag gagccgcagt ccaatccggg caacattctt 120

gagttccagc tattcaacag gctcaagggg cttcgtgcca gcaaggagaa atgcgaagct 180

ttgcaggacg tggatgagtt caagttgctc acctatgtgg agccagccaa gccaatcacc 240

tctgtagctg acatcttcaa ggatgactct ctgggcctgc tggacgacga agctgccagc 300

atcttccaac tcaagcatgt tcctttggtg aaggacatgc ccgacaagat cgcccagcgc 360

aaacgctgca aggactttga gcagtttgaa cccttgttca agcagtgcca tgcagagctt 420

gccgccggcg ttcgtgaggt acgtggcttc actggtgagc agcaaattca gcctggtcac 480

ttcttcatcc tccacggcat tactgcctat gtggctgaag tcggtgagaa ggaagtcaag 540

aacggcaagg tcaacgcacg gttgcgctgt atctttgaga acggcaccga gtctgacatg 600

ttgcttcgct cccttgcaac ggagctatac aaggacgaaa cgggccgccg catcctggac 660

cccacggaga aagctctaga ggcactggaa ctgattcaag ccgaggacaa gaagtcaggc 720

tacctctatg tgcttcagtc cctcagcacg gtgcctgaga ttgccaacac caagaacctc 780

tacaagattg gctactccac ggtgccagtg cccgaacgca tcaagaatgc ggccgaggaa 840

cccacctacc tgatggccaa ggtcaaaatc gtagaagtct ttgagtgcta caacctcaat 900

ccacagaaat ttgaactgct cctgcacacc ttctttggca aggcatgcct caacgtggat 960

gtctatgacc agcagggcaa acggttttca ccccgtgagt ggttcattgc ccctctgcac 1020

atcattgagg cggctgccaa catgctgatc aacggagaca tcgtcaacta tcggtatgac 1080

gctgatgccc agagcataga agaacgataa 1110

<210> 102
<211> 1371
<212> DNA
<213> Ralstonia solanacearum

<400> 102
gtggcaatga ctggctccta cttccgtggt gacagcgtgc cggtcctgct gcctgaagac 60

gaggctcagt tcaccaaagt cacctacaac tactacgagc agttaaacgg ctataccttc 120

ctcaagtctc tgggcattgg ctaccacttc taccaaggca agtacaccag tgccatcgat 180

gaaattctga acaccgatca gaagaacatt ctgcacatac caagcgtgca gtctggtgaa 240

tccaccaagg acaagtatga cgaggtggat cgcattctag acatcattgg caccgtcacc 300

caccgggacc ctgaaacctg tgtcattcac gtcaagcggc atggtgacgg caaggatttg 360

aaggttgccg atctcgtgca tgacgagccc aaagcccgcg aacgtatcgt tgaataccta 420

cgccgcattg agaagccaga agacatggat ctcatcattg ccttgggcat ggcgaaggaa 480

ggctttgact ggaaattctg tgagcatgcc ttgaccgtag gctaccgcgg atcgctgact 540

gagatcatcc aaatcattgg ccgctgcacc cgcgatagca acaacaagag ccatgcccag 600

ttcacaaatc tcattgccca gcctgatgcc actgacgacc tggtgaagct gtcggtcaac 660

aacatgctca aggccatcac ctgttccttg ctcatggagc aggtgcttgc acccaacttc 720

aagttcaaga ccaaggtcag tgacgacgac aagagcaccg atactgaaat caaggtgcga 780

ggcttgaagg agcctacctc caagcgagtc aaggacatca ttgaatctga cctcaatgac 840

ctgaaggcgg caatcctgtc tgacgacaag attgtgaagg cactgcctgg caatgttgat 900

cccgaagtga ttaacaaggt catgatcccc aaaatcatca aggtcaagta tcctgaactc 960

agtgacgaag aggttgaaga agttcgtcag cacgtggtgg tggattcggt catcaagaat 1020

ggcgagatca agcaggaagg tgacaaacgc tttgttcgca tggccgacaa gttcatcaac 1080

attgacgacc tgcacattga cctaatcgac cgggtgaacc ccttccagaa ggcgtttgaa 1140

atcctgtcca agtcgctgac cacgaaaatc ttcaaggtga ttcaagacac cattgaagct 1200

actcgtgtgc agatgagtga cgaggaagcc atcctgcttt ggcccaaaat taaggacttc 1260

gtgaagaccc acggcaagga gccgaacatt gcttcgctcg atccgttgga gcggcgtatg 1320

gctgatgctg tggtctatct gaaggcacaa cgccgtcagc aaggagtctg a 1371

<210> 103
<211> 216
<212> DNA
<213> Ralstonia solanacearum

<400> 103
atggcgattg tcgccatccg tgaagcgtat cctgaactga gcatcgagga cctgtatgac 60

cctgacacta tgcctgctga cttgaagcag gcacaccacg aactggatgt ggtggttgag 120

cagtgctacc agattaagcc gttctacagc gataccgaac gcttggaatg cctcttcaaa 180

ctatacgaaa aatgatggaa agccgagcat gcctaa 216

<210> 104

<211>  363
<212>  DNA
<213>  Ralstonia solanacearum

<400>  104
atgaacaatg gttacaatca tgcgccacct ccatatccaa tcgccgcgtt tccaatagaa      60

attgccgagg tgatatacga ggtgatgaac aaggtcaagg cgccggacgc gctcatcgca     120

acgtcatttt tggcaacaat gtcagtcgcc tgccagggtc tgattgacgt caagctgcct     180

atcgggcagg tttgcccagt gtcgttgaac atccttatca agggtgagtc gggcgagcgt     240

aaaagtgcca cagacagaat cgtgggcgat ccgatctatg ctcatgatga agctagggaa     300

ccgctgatca atgaagtcgg cgaatggaat cgcgagtcat gccatcttgg tttttcggat     360

tga                                                                   363


<210>  105
<211>  429
<212>  DNA
<213>  Ralstonia solanacearum

<400>  105
gtgacgcaat accgcaaggg cgaccgcaag atggacaacg aagcgtgcct gcgcctggcg      60

caactgctcg agctggaaaa ccccttgccg gtgatcatgg cagccgatat ggatcgcgcc     120

gagcgtcacg gacaacggtc tctgtgggag gttttttcga cgaggatggc aggtagtgcg     180

acggcagccc tcctcctggt ggtggtcgca ggtgtcacaa attttgtgac accctcggcc     240

gccaaagccg cgccgttgag ccattcagaa ggccgacctg tttatcttat gttaaataaa     300

atggtcgccg cagacgcccg atccaaaacg cttgccggca ccaatgcctc caatgaattc     360

aatgagttgc aagcaggata cgtaggtttg aaaaccatcc ctggctcaac cctcaacgcc     420

gctgtttaa                                                             429


<210>  106
<211>  804
<212>  DNA
<213>  Ralstonia solanacearum

<400>  106
atgaatgtga aagcgttcgg gaaagcaggg ggcatcatga ctgccagcac ccgtcatcca      60

ttgctgattt cggatcatgc tcttcagcag accgtgggct ggatcgcgct gctgatgccg     120

gtcactgtgc gggtcttcgc gctggccttc gaccacatat ggacgacgaa ttcgatcagc     180

gcttactact acaccagttt gcgcgatgtg ttcgtcggtt ctctggccgt cggcggcctg     240

gtgctcgcgt tctttcggac cggccatccg cgggatcgct gggtgacgat cagcgcgggc     300

gtgtcagcga tcggaatcgg gctgttcccg atgggcatca taccaagcgc gatcaccccg     360

cagcaggccg caacacccga tgccgaggcc aggctcattg acgccctgca gcacgggccg 420

catgggccgc tcgggtatca cttctacttc gtgggggcgt tctttctgct ggcgttctat 480

cttgtgacct tcggttttcg cgtcaatacg ccggcgaacc ccacgccaca aaagcggcgg 540

cgcaattggg tatacgtgat ctgtgggtgc ctgatgggat tcgcgttcgg ctggattgcc 600

atgctctggc tcctgggggg cgaggcgtcc attttttggc cggaaacgct tgccgtgatg 660

tcttttgccg gcgcttggct ggtcaagggg caactggtct tgaaggacca gccgtcccgg 720

tcccctgagc tgcatgggcg gcaaggaaaa ttccggcgac gctggccggc gatccgccga 780

tatcgggccc tgcatcgcca gtag 804

<210> 107
<211> 2883
<212> DNA
<213> Ralstonia solanacearum

<400> 107
atgtcgcagc agattcagct ctctgatggc ttgccacagg tggagcgcag gctcttgcag 60

gacatcaagc ggttggcgac cggcgatctt gatgcactgc ttgctgactc gaccgtcccg 120

gaaccgccat cgttggatgg cgacctgacg atcgccgaag tcggagcgag cgcgctatac 180

ctgatgctgc atcgttgcgt tcaaagcatc gttgctgagg tgcttggacg cccaggttat 240

cagccgtcgt tacccgaact ggagcgtgtc gaagacatgt gcgcacagcc ggtcgtcttg 300

tcccatgacc gacttggcaa cagcggcttc cctgggcctc gccacttggc gtctttgttg 360

aaggccgtgg gtcgcgaact gccgatcgat agcttggctt gcctgccggc accgcctagt 420

gtcggcacac ccctttggat ttcgggcctg gcgctcattg cgaagactcg gccgtatctt 480

tgggcaaatc accggaatgc agtggagcag ggctacctga cccctggggt ttcagctgcc 540

attggctttc ccactggggc ggggaagtcg actctatctg agctcaagat tctttcgaac 600

ctgtgtcaag ggaaggacac catctttctg gcgcccactc tggcgctggt cgaccagacc 660

gcgcgcgcgc tccagaaggc ctttcctgag gcgcggctga gtcgagagcg ggcgagcgac 720

aacccgttcg attttgacct gggcactcga ctgccaccga tgtcggtgat gacgccggag 780

cgctgcttgg cgttgatggg gtttcagccg gagctcttca aggacgttgg cctgctcgtg 840

ttcgatgagt gccatctgct gcatgcgggc gtgaacgatc gtagccgtcg cgccgtcgat 900

gcgatgcttt gcattctgaa cctctccacg catgcacctg aggcagacta cttgctgctc 960

tcggccatga tgagcaacac tgaggaggtc gcggcttggt tggctgagct taccggtcgc 1020

aagtgcttgc cgctaaacat ggcgtggaag ccgacacggc aggtgcgcgg ctgtgtggtc 1080

tttccgagcg accaaattgc cgacctgcgc atccgactgg cggcgaagcg ccaagaattg 1140

ttggccgccg gcaagcgcaa gcctgggccg ccggtcgcgc tgaagcgtga actcaaggta 1200

cggccgctgg gcctgcttgg actgaagttc cggtgggact cgatgcgccg agatgattac 1260

gcgttactcc cgctttctga cagagagatg ccgctttcgg tgaatgcaac cacgtggaag 1320

ctgacgccga atgtgaacaa cgtggcagct gagcttgcgt cgctcgcatc ccgcaacaag 1380

ggaatcaaat cgctcatctt cgcgcagacc gtcccgtacg ctatgagcgc tcaggagacc 1440

gcaggtgagc atcttgggga ggcaaacatt gccttcacgg aagatgagca gcatctgtac 1500

aacctgacgc ttgatgagct gggcgacgag tcggcgctct acatcgatgc cgtcgaaggg 1560

aagttcgcgc ggtggccgag cctctgtcat catgggcttc ttctgccgac cgagcggcaa 1620

ctgcacgagt cgctcttcaa gcgtcccgat ggcgtgtccg tgctggttgc cacgtcgacg 1680

ctggcgcagg gaatgaacct gccgagtcac gtggtcatca tcgcgggcga cagtaagttc 1740

gatgaggaga acaagcagct cgcgcagatg aaggcgcatg atctgctcaa cgctgccggt 1800

cgcgctgggc gggccgggca gagttcgtat ggtctggtgc tggtgattcc cagcaacgtt 1860

gtggacatcg acgattcgaa taataggatt ggcaagcatt ggaaggatct tcaaggcatc 1920

tttgcgcaaa gcgaccaatg cttggcgatc gaagatccgc tggaagctat cctcgaccgt 1980

attcaagaac ttggcgagcg tgccggtggc gatgcggact accttttgcg ccggcttccc 2040

atctcgacgc aggccgagga cgtcgatggc ccggccagga cgctattgaa gaagtcgttc 2100

gcggcgttca agctgaggaa gcaggcgcgg gaagagcaac tgaacgattg cattaaccat 2160

gcactgcgcg cacgcaggaa gcttcaagaa ctggatgagg agcccgattg ggccgacaag 2220

atggcagcca actacggcgc gccgtcgaac gtgctgcgcg agctgaaggt tcgcctcaag 2280

acgtcgcctc ctgccgggcg cacgacgctg gactggttca cgtggatggc cacgtggctg 2340

accgagcggc ccatcctcat ctcgcagatg accaggcgtg agagcatgga cggattcatg 2400

ggcgagccgt tcaagtcgct tgaggacgac gcggagcgag gagagttcgc cgtcgacgtt 2460

gtgattgcag cgctgagggc gtgggttagc ggtcagacgt tagcggagat tcagaagctg 2520

aagcccacgt tgcaacagca gaaacgctgc gagcaagcgc gccgtttcgt cctgcgggtt 2580

ctcccggagc tcgcttacat cttctccctt ccggaacttg tgcgtcggca ttttgcggcg 2640

gaagaactgt ccttggacat ggacgagctt gcctcggtcg aactcgagaa gcttggaagc 2700

tgcgtccgtg agggctacga ttccgttgag aaactcgcac tagcgaaggt ccacggcaag 2760

accgcctcgc gcgttcgagt tcatcgtcag tgggacgaga tggactggtg gatgggcccc 2820

aaggtacccg gcgagacttg gcaggagtta ttcgagcgag tcaggcgtag taccgaggac 2880

taa 2883

<210> 108
<211> 1422
<212> DNA
<213> Ralstonia solanacearum

<400> 108
   ttgatcagcg gttccctaaa gtcatccgcc agcctctcga tctccgtcag caaccggcag 60
   ccggagaccg acaggccagg gcacgacgtc gaacggagcc cgtcgttttc gcctcggtct 120
   cggatgatgt ccccggatgc actgctgggc gaatattcgc agcgcagcga cagcagcctg 180
   agccagctgc gccgggcacc cgactccatg caggtgtcgt cggcccgatc cagtgcattg 240
   gattcgctcg ggggaaaccc gccaccgccc acggtccaca cgccccatcc cgggcaagac 300
   agccatggtg tcgacgttcc caaccggatg cagatcgccg agcagttgcg cgacatcttc 360
   agaccgcacg tcaacagtgc caacgatgcg caattcgaag ccctgatcca gcagcgagcc 420
   gagcggctcg acgagatggg agaaacgccg gcgagcctcg ccgaggtgct gtccaagggt 480
   gccaaccggg accggctggc gcaggccacg gtgggcttcg tgcgctcggt gccgttcggc 540
   gttgcctcgc gcctcttcga tgtgaagcag caactcacgg ccttcgccaa gacgccggcg 600
   caggtgggcg cggtggttgg cgccggatcg ggcgtggcgg acgccttcgg cggcagcctg 660
   gtgaccaagg ccaccagcaa cacgcaatgg ctggcggcct cggccgcgca cctcgaaccc 720
   gtcatggcgc aagcgcacca ggccgtgcag cccagcctgc gccggctggc ggtggaggtg 780
   agcggggcct tccaggccta ttcgctgcgc aacgtggtcc gcaccggcgt cgcgccgctg 840
   gcaacccatg tcctgggggc ggcaacggcg gccaacgtgg attcgtggat cgccgcggtg 900
   ggcgggccgg tggccggcgc ggcggcctac atggcgatgc agcacatgaa cgagacccag 960
   caccgcacgg gcgcggaata cctgctgggc cgaacggact gggaaacgca gttcacgctg 1020
   ctcaagcagt cgacgtggac ggatccgctg aagggggccg cccagcgcgc ggcaaaactg 1080
   cccgtcgatc tcctgaccga gacgctggcc gccacgcgca gcctgttcac cgccaccaac 1140
   atcatcaaga atgcgggcgc gctggccggc ggctttgccg gtgtgctgac cgcgcagacc 1200
   gccgccggcc ggatggcgac gaacgccggt gccagcgagg aagccgtcgt ggccatcaag 1260
   cgggccgtca gcacggtgct gtcggcaccg gtctatgcca cgtggacgac ggccgatgtc 1320
   gcggccggcc ccatcgtcga tgcggcggcc gggcgcatcc agcaagcgtt cgcacccagg 1380
   ccgcaagcgg tgccccccgc gcataccgcc atcgacatcg tg 1422

<210> 109
<211> 816
<212> DNA
<213> Ralstonia solanacearum

<400> 109

ttgtgggcgg aggggccggc gcgtcatgac atggttgcca aggttcgggc gttcgaagat 60

ggcagtgtcg agttcagcgg ttatcgtcgt accgtggtgc agcgcctgaa cgatctgcgt 120

gatcttcccc gccgtgcacg cggcgccaag cctgaaaatg aagacgataa agaggcacgg 180

gccgctagcg tcaaatctgc tgcgaagcga gctaaacaga acgttcgctt gcgctgcaag 240

actgctcgcg tcacgcacat gattacgctg actacgcgcg agtgcattac cgatctggac 300

tggttcctgg ggctctggga cgcttttcgg cgtgcgatgg ctcgctacag tcaattccac 360

tacatcgcgg ttcctgagct gcagaaacgc ggcgcatggc atatgcacgt cgccgtctca 420

ggtcgggtcg cgctcaacct cgcgcggcgg gtatggctga aggtcgtggg cggtcgtggc 480

aaggggtact gccacattcg caaccctcaa ggggcgcact tcggcaagca atggaagttg 540

gatgcgctgg ccagctatgt cgccaagtac atcggcaagg acattgctga gactcggttc 600

aacaagaaaa agtactacac cagtcgcggc atcaacgttc ctgaggccgt ggtctatgcg 660

atcgagaaca gtcgctcaga ctgtgcggac gcactgaagg acgtgctcac gacgctgtgc 720

gccgagttcg atatcgcgga cataagatgc ttcgtcgccc tcgatggctc gtcgtatttc 780

gctagtgcgt ctaaggctgt gctgctcgcg gcctag 816

<210> 110
<211> 2190
<212> DNA
<213> Ralstonia solanacearum

<400> 110
ttggggttgg gaatgaagcg atttgtcaaa cggcagcttt gggttgccgt attgggcctg 60

agtgcagcat ccggtgcatg ggcgggttcg tccgactctg gttttgacgg gttcatgtgg 120

atcaacagtg gcggcatcgg tcaaactctt actccacctg agcgcagccc ttcagagcag 180

tgcatggtat cggtcgctgc ccacgatgcc atatatggaa cccgctacaa atatgttggc 240

ctcgacacga cgaatagtaa gaacatccaa tgcatccttg atattggtag cggtacccca 300

gcggggtatg cgactgtgcg ccgcatcaat acatgcgtac ctgggtatgg gttgcaacct 360

gatggttcat gtaagccact gtctccgcgt acccccgtgc aagtctgtac ggccagcagc 420

tctgtcattc ccggtacagg caccaagacc ttcatcgagg gcgacgaggg gggcagtcca 480

gacctgtctg tcacacttgc ctaccagcag tcttacaacg actacggatc gaatgtcggc 540

atcggtcaat ggacaacaaa ctggcagcgc agcctggaca ctggttccgc cagcgagtcg 600

actccccaag tgtcggctca gcgtgaagat ggttcagtag tcaccttcag cggaaacagc 660

gcggcctgga gtgccatagg cagccaggac acgctgcggc caatcgcgga tgcaaatggc 720

aatacaaccg ggtggcagta caccgtggtt gatacggcgg tggttgagac ctacgatgtc 780

accggcaaac tgcaatcggt tcgggaacgc aacggccgca ccacgacact ggcctataac 840

gtggtagggc agttgaccgc tgtcacgacg ccaagcggcc gcagtgtgac gttcgcgtac 900

gacgcgtcag gccggacgac tagtactatg gctcccgacg gcgccgtcac tcgctacggc 960

tacaacagcg ctggcatgct gtcgacgatc acgcggccgg atggcgccgt acgccagtat 1020

gtctacgaag acagccgctt cccgaccgca ctgacgggga tcgtggatga aaacggcagc 1080

cgctacgcca cctatgcgta tgacgaccag ggccgggcca tcagcagcac gttggcgggc 1140

ggtgccaata cgtaccagtt ccagtatggc gacaactacc agaccactgt caccgacccc 1200

accggcaaga ccagcgtcta cagcttcctg aaacagaacg gcgtgctgct gccgacttcg 1260

atcagtgctc catgtggcct gtgtggcagc acccgccaga gcagcagcta cgacgccaac 1320

aacaacctga cccaggaaac cgactacaac ggcaccgtca cctcccacac ctacgacagc 1380

cagaaccgcg aaactcagcg cgtggacggc tcgggtacga ccagcgcccg caccacgaca 1440

acggtatggc actcgcaatt ctggaacctg ccgactcaaa tcgcctcgcc gaccaagctg 1500

gaaacgtaca gctacgatag caacggcaat ctgacccgct acagcgaaac gccgaccgcg 1560

gacagtgacg gcagccaggg cttcagcgca gcggcgaccg gcccggctcg gaccaccaac 1620

tggacctaca ccgccgacgg cctggtggcg accagcagcg gcccgcgaac cgatgtggcg 1680

accagcacca cctacgttta ccgcaccgcc gacgacaccg ccacgccgcc gcaataccgc 1740

aagggcgacc tgtaccagat cgttgacccg ctgggccacg ccaccacgat caaccagtac 1800

gacgccaacg gtcggccgct gcagatgacg gatgccaacg gcgcggtcac cgcgttcacc 1860

tattcgaacc gtggctggct gaccagccag acgatcaccc cggcgagcgg ggcggggcag 1920

accaccaact acagctatga cgccgtgggg caattgacca aggcgacgtt gccggacggc 1980

agcacggtca gcttcaccta tgacggtgca catcgtctga cgggcgcagc cgacagccag 2040

ggcaacagca ttgcttactc gctcgatgcg atgggcaacc gcacgcagga gcaggcgaag 2100

gatccgagcg gcaacttggc ccgccaagtc acgcgtgtct tcgattcgat gaaccggccg 2160

ctcaaggtca cggtcggcac tgcgtcttaa 2190

<210> 111
<211> 1128
<212> DNA
<213> Ralstonia solanacearum

<400> 111
ttgatcgaag ccttccgttc caccttcatg ccaggcttga aggttgatga gaatttcgct 60

gacttctggg tgatgcacag agacaaaggc aaccttgaac ttcacttcct tgttgccaat 120

accgaattga agaccggcca gcaactgaac atccatccgc ctggggagaa gaacatagag 180

ttcttcaatg ccttcagtgc cgtgatgaat gacaacttcg gttttgcaca ggtggtgccc 240

gatcccttga agatcagcct caagccattt gaagccaagt cccccaacgg taaacgcgag 300

aaggaagcca agaatgactt tgctgatgag cttcacactc gcatcgtcag tggctccata 360

gccaaccgta accagctcat tggctacatg aagaagcgag gcattggcat tgccttggta 420

ggtgacgagt tcatcaccgt tcgcttacct ggtgctggaa ggaacaccag attgaagggc 480

tcgctattca gcaaggacag tgactatgcg gccttggtga aggaacacca ccagtccaag 540

atacccaagt tcctcacccc ggccgagtcc gcggaccaac gttccaagct ggtggacttc 600

attgcagcca gaacagcctt caacaccaaa cgctacctca cccctagacc tggtgcaaaa 660

cgtgcatatg cggccaagaa agggccttct ggtggcaata ggaaggatgg caagacaccc 720

accaaagaga aggtgccaac tactcttgaa cgtgacacca ccgagatccg cagcaagctt 780

gaaaagaagc tcaaggagat caaggaacat gccaaggagg ttcagcttgg ccagcaaggc 840

atcaaccact ctgcccccca cagagtggcc cgcatgcagc acgagcgtga gaaccccacc 900

aacctgccca atgtgggttc tgctgttgga ggtttggagg ctcagatcgg tggtttgtcc 960

atgcagtatc acaacctgct gttgctgctg gctggtgcca ccggcacacg agccacgaag 1020

cttcaggctc agatcattgc cattgagcaa aaacttgtcg cattgaacct tgagcttgag 1080

aagaggaagc tcaaggaagc caaagacaat gaactcaaat ataaataa 1128

<210> 112
<211> 457
<212> DNA
<213> Ralstonia solanacearum

<400> 112
   atgaagcggc agatgagctt tgcagaagcg gaaagcgcag gtaagaagcg cgtgaccaaa 60
   cgccagcgct tcctggcaga gatggagaag gtcgtgccgt ggcagcgctt gctgtcggcg 120
   attgggcccc actatccgag aggcgagcga ggtcgcccgc cgattggtct ggagaggatg 180
   ctgcggatct acttcctgca gcagtggtac gggctgtcgg acgaaggtct ggaagacgcg 240
   ctgcacgaca gtatggcgat gcgagccttc gccggcatcg atctggcggt cgaggacgtg 300
   ccggatgcga ccacgctgtt gaagttccga cgcctgctca acgaacacga cttgacgcga 360
   aagctgttcg acgagatcgg catcatgctg tgcgagcggg ggctgatgat gaaggaaggc 420
   accatcgtgg atgcccccct cattgaagcg cccccgt 457

<210> 113
<211> 102
<212> DNA
<213> Ralstonia solanacearum

<400> 113
ttggccaaga acacggcgca actgttcagc ctgtttggtt tggccaatct ggtgctcgcc 60

aggaggcagt tgctggccag tcctgggagc attgcgtcct ga 102

<210> 114
<211> 867
<212> DNA
<213> Ralstonia solanacearum

<400> 114
gtggccttct acagcgacaa gtccagcgtg ttccgagtca atgccagggg cgccaccgcg 60

ggtcgcgact atacgcagtt cggccgagcg ctttatgaac tcaatatcga gagcatctgt 120

gccaattcca gccaggccaa gggtcgtgtc gaacgtatga atggcacgct gcaggatcgc 180

ctggtcaagg cgttgcgcct gcacaatatc agcacgctgg aggccgccaa tgcctttgcg 240

ccggtcttcc tggcggactt caacttgcgc tttgccaaga ttccccgcag cgatttcaac 300

gcgcatcgcc cggtacaacc tgaagaggat ctcgaacgca tttttacctg gcgggaatgg 360

cgcaaggtct cgaatcgact gaccctgcaa tacgatcgta cgctgtacct catcaccgat 420

cagcccgagc atcgtccgct ggtccaccgc tatgtcgagg tggcggaata tcccgacggc 480

cacatcgaac tgtgggccga cggcatctcc ctgccctatg tcctctatga ccggctttgc 540

gagatcgatc agggggcgat cgtcgagaac aagcggctcg gacacgtact gcagattgcc 600

caacgcgtgc aagcgctgcg tgataatcgg cgcaacctcg accggccgtc acgcaccctc 660

attggccagc caccacggca ccctgagttg cgtgcgccag gcaccaagtc accgcgacag 720

ttgagttcag aagacttgcg gcgcgccatc gaggagctgg caccaccgcc ccaccctgcg 780

gatgccgcac cgcctcaggc caactcgact gccctacgga aacatcccgg aaaggcacca 840

agaaagaaga gcaccacact gcactaa 867

<210> 115
<211> 660
<212> DNA
<213> Ralstonia solanacearum

<400> 115
atgccaaagc catcagtcat cgaagagcgc caggttcgcc acgtactgaa ggttgcagcc 60

gtcactgggc agaacagcaa gcgcgatgtc gccctgttat tggttgccta tggcactggc 120

cttacgccca acgaaatcgc caagctgcgc ctgtccgact atctgcaggc gaatggggag 180

acacgcatcg aatcgcacgt gagagcggag attgccttca acggcagagt gcgaccgctg 240

ctatgggcca gtgcaaaggt gcgcgctgcg atcgacgtct atctcgaaca ccggctggcg 300

tttcgacatg gcatcaccac aagtccagcc gcgtaccgtg gattggattg tcggagtccc 360

ctctttctga ctggcgaggg tgagcctttc ggctttacgc gtcgcgtgac tgcggccggt 420

gccactagct attcgtgcga gtccctggca gagatattca gacggctaca ccagcaggct 480

ggcatcgcca acggcaatgc cagttccgcg cgccggacat tcgcggtgag gcttcatcgg 540

caaggccgat cgaccaagct gatccaagag ctgattgggg tgtcgagcct ctccgccgtt 600

aagagactgg tagagagtga tcccgtgcgt ttggcctcca tcgtgagccg ggttatttga 660

<210> 116
<211> 522
<212> DNA
<213> Ralstonia solanacearum

<400> 116
atgcgaccgg cctgcgaact cgtttctgcg aagctcggtg atatcgaaac ggaccggcag 60

gacgatcact ggctgaacct ggtggggaag ggcagtcgcg ctggtaaggt tgcgctgccg 120

ccgctggctc gggcggcaat cgaccgctac ctggttgaac gcgggctacc gattacgcgc 180

gcgcgttggg atccgaagac gccgatgatt ggtgggttgg ggctcgatcc caatggcagc 240

attacgggaa cgcggttgtg gagtgtggtg cggcgttttt tctggtcggc cgccgaaatt 300

atcgacagcg atcacccttt gctggctgag aagctgcgca aggccagtcc acattggatg 360

cgccatacgc atgccaccca cgcgctcgca cgcggtgcgg aattgaccac cgtgcgcgac 420

aacctgcgac acgcatcagt ctcgaccacc tcgatctacc tgcacagcga tgaggccaag 480

cgcgcgcggc agctcgggga cgcctttggt accagaaaat ga 522

<210> 117
<211> 1167
<212> DNA
<213> Ralstonia solanacearum

<400> 117
atgcaccgga ttgatggggc cggaaacgtc aacggcacgt gggtggcgga agacgccggg 60

accaaccggc cgccgactga gattacggca ccgttcatga acgccatcca agaggaagtg 120

gcgtcgttca tcgagtggtg cggccttgtg ctgaacaagg ctgataacac ccagtttcgc 180

caagcgattg tggggctatt gcaggccgct accggctctg cgggctccac gtcgtaccgc 240

aacaagctca tcaacagcgg ggcgcaggtt gtcattcagg cgacatcgcc gtcgctttct 300

acgtcgcctc agtatgggcc ggttgagatg attgctggct gggcttctgg tggcgcgatt 360

acggcaggga cgctagtgca ggacacggcg gcccctgtgg gccgcagcgg caaggccgtc 420

cgctttcagg ggtgcacgct caccggggcg gggcaacttt catggcgtta ccgcatggag 480

gctactgacg cagcaaacct caagaatcag accgttacgt tccagattaa agtgcggcac 540

accgttggcg cggccatcaa ctacacggtg gtactgagaa cgcccacggc agccgacaat 600

ttcgcatcgg tcaacgcaat caacagcaag acggtggctg tggcgtccgg aacgtcgcag 660

ttactgacgt tcacagtggc gctcggtgac tgctcgaacg gcctggagat cggggtggac 720

gctacgtgtg gcgctgtcgt ggccaaagat ttctggttca ccgagtggag cctggaagaa 780

ggagctaccg caacggccgt ggagtttcgg cccattccaa ttgagctggt ggcgtgccag 840

cgctactacc aggcggatgc attccactgc ggcggcgcgc caatcgcaag tggcagtggg 900

agtgcagcaa tcgctgtcaa ctcgaccagt ttgcagggga caaagactct tccggttcaa 960

atgcgcgcgg ggccatcgat gacgtttaag gatatggctg gcaattctgg ggcgtacacc 1020

gtctacaacg gcgcgacaat ccacaaccag gggatgacga gcggcggcat cgggtgcagc 1080

gcatccacga tcgatatgga tgcaacttgg ggcgtgtacg gcggaacatg gtgccgggtt 1140

aactacatcc tgaatgcgag gctgtaa 1167

<210> 118
<211> 557
<212> DNA
<213> Ralstonia solanacearum

<400> 118
tagttacctc caactcccgt aatgtcttca aaccaaagaa acacgtgcgc ttgctggaat 60

gccgccaggg cttgccagag cgcagccgga tcgaccaccg agcggtagta gcgcacgcgc 120

agcacgtagc gggatcgggt gccccatagg cggttgccaa tgtggctgcc cacacgcgca 180

ggccccacca ggtggtccac ctgcaccaga tcaatgggga acatcagcgt tggcgcgtca 240

tggctccaca accggttgcc gaccgtgctg ccaatggcgg ctatccggcg cggcatcggc 300

tcgcccacgc cggcaatggc ctcggccgca acgcggcggt actcgctgat gtgccagctt 360

ccgaacttgg gccggtgcag ctcaatggcg gcgtccagaa ccgcttgcgt gtcggtgccc 420

aggcgctcca gctcggccgc agtgcccgcc agcatggcgt cgcccatgcc gccgtccggc 480

cattcccacg cggcgccagg cggcaacagc gccttgagcg cgtcggtgta gtcgcgtgtg 540

gtgtgcgccg tgagctt 557

<210> 119
<211> 951
<212> DNA
<213> Ralstonia solanacearum

<400> 119
atgtgggcac gtgccgcgca cggcatgcat ggccatctcg aatggcttca tgcccagtgc 60

tcgccgctca cgtgcgagct ggaacgcctc tacgactggg cggcgctcta cggtgttgac 120

cgcctcatgg ccaccgctag cacgggcaac gcgctggcca ccggtgtggc aggcatgccg 180

atccttgctg gcacgccgct gcgcggcccc aacgggctgg attacattgt ggcggcggca 240

gtcgatatcg gcgctggccc cactacccct gtttccattc gctgcaccac caccggcctg 300

gccagcaacc tggctgcggg tctggcactc acggtggtgg acccgattcc tggcgtcaat 360

ggcaccttga cggtaggcga cgcgggaatc acgggcggcg ctgaagacga gagcgtggac 420

gattggcgcg cacgggtggc cgacgagtgg accaccatca ccacccgtgg cgctcgttcc 480

ggcaaggacg acgactacag gttttgggcc agaagcgccc acccatccgt tagcacggcc 540

ctcgttcagc gccacgtgct gggcatggga acaatcatcg tccggccgat ctgcaacacg 600

ctggccgacc gcatgccgac tgctgccgta ctggccgcag tagctgcgca tctgcaagac 660

attgcccccg ccacggcgga ttggcgcgcg gtggcaccac tgcggcacga cgtgaacccg 720

tctattcatc tgctggccgg cagtgacacc gccgccaacc gcaccgccgt gtcttcggcg 780

ctgatggccg ccgtgctggc cgaggtcagc gaaacatcgg tgctggccat ggccgaggtg 840

gatgctgccg ttgccaccgt caccacccaa tacacccgcc tcgcgccgct ggccgatatc 900

gccgtcgcag cgggcgaagt gctggtgatg aatcctgtgg tgtgggcatg a 951

<210> 120
<211> 579
<212> DNA
<213> Ralstonia solanacearum

<400> 120
atgatcgggc aacaggtttg ggcacggctc cagttgctgt tcgcgcaggg cgttggcctt 60

gtagtcggcg cgagcaaggt gcaggtgcgc gtgctggacg aagagacgtt ggacaacatc 120

aaccgtgtgg agccctatgg cttttcctac cggccgaagg ccggctgcca aacgtacctg 180

gctttcccgt ctggtgaccg ctcctatggc gtggcgctca tcatcgggga caagcgctac 240

caaatggagc tggtggaggg cgaaatcgcc attcacgacg acgaacagaa ctgggtgcac 300

atcaagcgcg gcggcatcat cgaggtcaag gcgagcacga aggtgctggc cgatacgccc 360

atgttcgaga cgacgaaaga cgccaagatc ggcggtaacc ttacagtggc gggccaaacc 420

agctcggtgg gtggctactt cggcgacggc ggcggcgctg cgcagatgaa gggcggtgct 480

gatgtgaccg gagaactcag ggtgaatggt aaggacgtga gcgacaagca cacccacacg 540

agcacggccc ctggcacgcc cacttcggac gtgaattga 579

<210> 121
<211> 1056
<212> DNA
<213> Ralstonia solanacearum

<400> 121
   atggatgagg cattggcgga aatccgcttt gacggcacgc gctacggcta ctggcagaag 60
   gtggaaattc gggagtcggt ggacgatctg tgcgccgccg tacagctcgc catcgcgcgg 120
   ccgggcagcg gcgcgaacgg cggcagcggc atgggcatga cggcgaacac cgtcatcgac 180
   gtgctgatcg gcgacgagct ggtgacgaag gtgcgaccgg atgtcaaccg ccgccgcgtc 240
   gatcccacca gccacacaat caactttcac gcccggtcgc ttggtcgtga gcttgtggac 300
   tgccagtatt ccaagacgct atccggcctg aaactgagcg agatcgtgaa gcgcctttgc 360
   gccacgttca aagtgtctgt gaagatcgac gctgaaacgc ccgtggtgcc cagtttttcg 420
   atgcagtgcg aggtgcctgc caacgcgctc atcaacgctg tgcgggcggc aaacctgctg 480
   ctgtatccgt tgccggacgg tggcctggtg ctcacgtcgc ctaccagcgc tgcgcccgtg 540
   gccacgctgc aatacggcat gcacttcaag gtctacgagg tcatcgacga atacaagctg 600
   cggttttcgg actacgttgt gaagacgttc gactacagca gtgattccgc actcaagggt 660
   gccgtgaagg acaatggcat cacgtatttc cggcccatgc acgttgtggc ggaccgacac 720
   agcaacggcc tgggcggctg cgagcggcgc gcactcctgg agcggaaccg gcgcctggcg 780
   cgtgcccacc gcatcgaact ggaggtgcca ggctggcgct atcaggacac cgacggcaag 840
   tggcacccgt gggcactcaa cacccaggtg cgcgtcatca tcccggaaga agaaattgat 900
   ggtgtgttcc tgatcggtga acggacattc cgtatggatg acaagcacgg ccgcgtcacg 960
   ctgctacagg tgatgaaccg gggtgcgttc ctgggcgaag aaaagaagaa atccaagcgc 1020
   ggcgcaggcg tgcgcggcaa ggggcacaag aaatga 1056

<210> 122
<211> 1161
<212> DNA
<213> Ralstonia solanacearum

<400> 122
atgagctggg aagaccgttt ggtcggcgca agctttcgcg gcgtcgattt tctgaccgag 60

agccacgaag cacgcggcgg ccgtcgcctc gctgtgcacg agctgccggg cgcggaagag 120

cccgaggtgg aagacctggg cgcgaaggcg tgggactgga agctgaacgc ctatttcatc 180

gggccggact acgatctgga gcgcgatgcg ttcttggaac tgctcaacca gccgggcgcc 240

gattggctga cgcacccgtg gctgggtgac aagtgggtcc gtgcacatga ttggtctgtt 300

cacgagagca acgacaaggg cggctattgc tccatcacga ttgacttcgt gccgggcggc 360

ggcacggtag ccgctgggga aaccgatgcg gtggacgtgg cctatgaccg caacagcagc 420

ttggccgacg ccgccatgga cgacttcacg ctggagtcga tgagctttga cggcatgact 480

gcattcgttg cggccgtgag tcagcggctc gaagcgctgc gccaggtcat ttccctggcc 540

acgctgccgc tcacctggtc gagccaggtc atgaacctga ttgccggggc caagggcgat 600

ctggccacgc tgatggccat acccagcgcg tattccaacg cattgcgcag cctcaccaac 660

agcctgggcg gtggtgccga tgggacggac tacccggata cgtcgcgcgt gcgtgtggcg 720

gctcgcatcg catccgtggc caccacggct gcgccgctgg cgctgtctgg tgttgcctca 780

acggatggtg ccgtgcgtcg caatctgctg cgcgaagaag cgttgcgcgg ccggctgctg 840

gtgacggccg ccatcgaaat cgccatggcg gactaccgcg ccgaggcaga cagagatgca 900

gccctgggcg ccgtggtgac tgccctggat gcgctgctgc ccagcatgcc cgaccctgtg 960

tttgacgcgg ccgtggccgc gcgggcggcg gtcattgaag cgctgctggc ccaagacctg 1020

aagccgaccg cgttgcgcga cgtgaccaac ccgctgccgg ccatcgtgct tgcgcaccgg 1080

ctgggcgtgg atgaatcggt gttcctggcg cgcaatgcgg cgcgccatcc gctgtttgtg 1140

aaggggcgtg tgtatggatg a 1161

<210> 123
<211> 2145
<212> DNA
<213> Ralstonia solanacearum

<400> 123
atgtcgtcgc ctgctgttga tgtcgccgtc aagatcaaga ttgttgatga tggcagcgcg 60

aatgccgcga ttcagaagac tgccaaaacg gcagagcaga ctgctgccaa gacggccacg 120

gccaccgaga aggcggcgca gaaggctgcc gatgcagccg agaaaagcgc cgcgcgccaa 180

cgcagctcct atgaacggct tgcccaggcg cgcgaaacct tgggcgtgcg ctctgagcgc 240

gccattcagc gcgaaatcca gcaaaccgaa gcggcataca accggctggc gcgctccggc 300

accatgtcat ggcgcgagca agccgcagcg gtcggccaga tgcgccagaa ggtcactgag 360

ctgaccaacg aaatggggcg gctgactgct gcgcagaaga ccgctgcggg cctgaagttc 420

actggtgctg ccatcgccgg catcggtgct gccgcctaca cgctcagggg gcctgcggaa 480

cgctccatga gctatgaccg gcgtctggcc aacatggcca acaccgccta ttccgaacgc 540

gacgccgcgg gccgcaaggt cggcaccaag tcgctggagg atgctgtgaa caaggcgcgt 600

cgcgaaggcg gtggcacgcg cgagcaggca gcggaagcac ttgacaccat gatcgcgtcc 660

ggcaccgtgt ctgatacgga tgcaatgaag atgctgccgg gcatcatgaa ggccgccact 720

gcatccggca ccgacgccaa cgcactggcc accatcgcca tccgcgcgaa acagtcattc 780

aagatcagcg cggacgacat gccgcaaatc ctgagcgctg ccatggtggc cggccaagcg 840

ggcggcttcg agctgcgaga tatggcgaag tggctgccgc agcagatggc aatggccagc 900

aacctgggct tatcaggcaa ggaaggcttt gcgaagctgg ccgcctggaa tcaggcatcg 960

gtcattaccg caggcacccg cgacgaggca ggcaacaacc tgcgcgacct gctgaacgag 1020

ctgaacacac cgcacttccg caagtacatg gcggagcagt acctggccaa cggccagaag 1080

ctgaaacgcg gcgagaagga aaagcgccta aagggtgttg atgacgtgtt cctggactac 1140

cagagccgag gcattgacaa ggtgggcgcc accatcgaca tgatgcagtc gattttctcc 1200

aaggatgcca agtttcaagc gctgcaagcc aagctgcgtg ccaccgacaa gaacgacaag 1260

gaaggccagc ggcagattct ggaagccatg agcgcccagg tgcagggtac ggcggtcggc 1320

aaggtgttcc acaaccagca gtcgctgatg gcgttcctgg gcgtgatgaa caatcaggaa 1380

tacaccaaag acgtgttggg caaggtgcgg ggccagtatg gcttggcccc cgagcgctcg 1440

gaaatcgcca cctccttcaa gggcattgca gatacggccg acttcaagat ggagcaggcc 1500

aaggaagatg ccgccgtggc gcaaaagtcc gccatggaca accttctacg tacaattggc 1560

aaggcagctg aagcctttgg cgatctggcg aagaagcatc cgcttctggt cggcactacc 1620

acgctcgcca cggccgcact cggcgcactg gcgggtgtgg ctggcctggc atcgattgcg 1680

atgggcggga aaggtctgcc aggcggacag gccatcacca atgccgctgc ttgggcgaca 1740

ggtagcgcgg tcggccgggg cgcgatgaaa atgggcaagg tcggcggcat cgctggcgtg 1800

ggcgctctgg tgggtgacta cgcccttgag aaagcttttg gtgaagagtc ggctatttcc 1860

cgctacggct ccagtgcgct caatggcgct gctatgggcg ccatggtggg tagcgtggtg 1920

cccgtcctag gcaccggtgt tggtgccgct gtcggtggcg gtctgggctt agcctgggaa 1980

ggtctcaaag acctgctgaa gccagccgag cagaagccgg tggacgtgaa cgcccggatg 2040

acagtgggcc ttgcccccgg ccttgtgctg caaagccagt ctgtgcaggc cacgggcggc 2100

aacgtgcaga tgaacaccgg taatgtttgg aacggagcgc cctga 2145

<210> 124
<211> 354
<212> DNA
<213> Ralstonia solanacearum

<400> 124
atggacaaca tcgagaagga aattcagtcc atgggcctga ctgcgccgcg tgttacgccg 60

gccgacatcg aagcgaacat cgcaagcgag cattacttca ctgccgaaga cggcatgatg 120

gccgggcggg acggccgcgg ccactttcgg gatcgccacc cgtccctgca cctgctgacc 180

ttctgtgtgc tggtattgcg caatggtttc accgtgaccg gtgaatcggc ctgcgcaagc 240

cctgagaact tcaatgccga aatcggccgc aagattgcgc gccagaacgc tgcgcaaaag 300

gtgtggccgc tgatgggcta cgccttgcgc gacgcactgc acgccgcaac ttga 354

<210> 125
<211> 1473

<212>  DNA
<213>  Ralstonia solanacearum

<400>  125
gtgaccatta ccttcagtga agtgccttcg gcgctgcgct atccgggcgc ttacatcgag      60

attgatggga gccaggcggg cctgagcagt gatttgcctg tcgtactgct ggtaggccag     120

aaattggcca gcggcacggc accgaccggc gaactggtgc gtgtcgccag cgtagcggat     180

gccaagacgc tggcaggcga tggttcgatg ctggcgcaga tggccgcgcg ctatcgcaat     240

gtcgatcaga cgctcgacct gtacatgctg ccctacgcgg acaacgcggc cggcgtagca     300

gcaaccggtt ccatcaacgt caacagtgcg gccaccggtg acggcacgct cgcgctgtac     360

atcgcacaac gcgccatcac cgtgggcgtg gctgcgggca agacggctgc gcagattgcg     420

acggccattg cgcaagccat cacggacgca ggtgcggaca ttccggtgac tgccgttgcc     480

gctggtgctg cggtgacgct cagcgcgcgc cataaaggca cctgcggcaa cgccatcgac     540

ctgcggttga acctgtacgg cgaagacatg cccgcagggc tcgcgctcgc gctgaccgcc     600

atggcaggcg gtacgggcga cccgctgccg ggcgacctgg caacgctgat cggtcagaag     660

tggttccgct acgtcgtgct gggcatcaac gatgccgcca cgctggcggc atggcatgca     720

gagagccaac gccgctaccg ggtgcccgtg caggccggct tccgcgcatt cgctgcctat     780

cgtggggact acgcagcagc agccagcttt ggcgagacga agaactacga gcacatcacg     840

gacctgtcgc tgggcatcaa cccgcccact acctgggaag ccgccgcgac gctggccgcc     900

gccgctgcgc cgaagctgta caacaacccg gttatctcgc tggaaggtac atcgctgcct     960

gggcttgtgg ccacgagcta tcacgagtgg accagcggga acagcctgct cttcaagggc    1020

atgagcctca tggaggtagg cacggacggt tcctgctaca tcaagcggct gatttccatg    1080

tacctgcatc gctccgatgg cagcaccgac gacgcctatc tggatatcaa cgtggccgag    1140

gttacggagc gcatccgcta cgaacagcgc acgggcgcca tcaagaagtt ccggggcacc    1200

gttgccgcca agacggacga aggctaccgg ccgggcctgc ccatcaccac ggaagatggc    1260

gtgaaagcgt tcctgctgtc gctctacaag aacgtgctga tgtcgcgata cggctgggtg    1320

caggcgtatg actactacaa gagcactctg gtggtggagc aagacccgga caaccccagt    1380

cgcttcaact tcctagatga cccggtggtc aactcgccgt tctacatcct cgcgggccgc    1440

tcgcgcttcc ggaaggctgt accggtggtc tga                                 1473


<210>  126
<211>  480
<212>  DNA
<213>  Ralstonia solanacearum

<400>  126

gtgatttcgc tgacgccact gattgaacac gtgaagttga agcctgccga gttcgcaggc 60

atctggtttc ggcaggtagc tggtgctgcc gagttcgccc aggttcgccc tgaagcgttg 120

ccgctgccgg ccgcgtggat cgtccgggca gcagacaagg tgcagcacgc aggcgagcgg 180

gctgagaacg tgacgctggc cttcgatgtg gtgattgcca ttgaaaaccg ccgcacgcac 240

aagcaaggcg atacggacga tgagttgctg aagtaccgca ttgccgtgaa aacgctgctg 300

ctgggctggc agcttcagcc tggcgtgcgc cccatcaagt tcgtcggtgg ccaagtcctg 360

gagtacaccg acggcgattt gtactggcgc gatcgctacg agttcgacgc cttgattacc 420

aactacctac cggacccgcc tgcatttgac aggctcattt acacgggaga aaagctgtga 480

<210> 127
<211> 480
<212> DNA
<213> Ralstonia solanacearum

<400> 127
gtgatttcgc tgacgccact gattgaacac gtgaagttga agcctgccga gttcgcaggc 60

atctggtttc ggcaggtagc tggtgctgcc gagttcgccc aggttcgccc tgaagcgttg 120

ccgctgccgg ccgcgtggat cgtccgggca gcagacaagg tgcagcacgc aggcgagcgg 180

gctgagaacg tgacgctggc cttcgatgtg gtgattgcca ttgaaaaccg ccgcacgcac 240

aagcaaggcg atacggacga tgagttgctg aagtaccgca ttgccgtgaa aacgctgctg 300

ctgggctggc agcttcagcc tggcgtgcgc cccatcaagt tcgtcggtgg ccaagtcctg 360

gagtacaccg acggcgattt gtactggcgc gatcgctacg agttcgacgc cttgattacc 420

aactacctac cggacccgcc tgcatttgac aggctcattt acacgggaga aaagctgtga 480

<210> 128
<211> 903
<212> DNA
<213> Ralstonia solanacearum

<400> 128
atgggtgcac tcacgcaagc ccagttggac caactgaaga ccacgctgat tgcccgctgg 60

aatgctggct tggtgctgac gccggaagac tggaagaaga tcgccaagct agtcaccagt 120

accggcaaat ccaacaccta cgaatggctg agccagttcc cggccttccg ggaatgggtc 180

ggcgcacgcc tgcacaagaa gttcaaggaa accgcctatc aggtggtcaa ccgcaagttc 240

gagtgcacgg tggacgtgca gcgtacggac atcgaagacg acgaaatcgg ccagtacggc 300

accatcgccg agtcggccgg ccagtcggcc accgatctga agaacgatct ggtgtttcaa 360

gcgctgggcg ctggcttcgc ctccgtctgc tacgacggcc agtatttctt cgacaccgat 420

cattcggtgt acgagaacga agacggcacc ggtgccgtca ccaacgtgag caacatgcag 480

gatggtgcgg gcgcaccttg ggtgctgctg tgcacgaagc gcgcagcgtc tccgatctac 540

ctgcaacagc gcatgcccgc ggagttcaac agcatcacct ccacgcagaa caccaacgtc 600

ttcgacctgg acgtgtacag cttcggcggc cgttggcgcg gcgaggccgc ttacggcttc 660

tggcaatgcg cgttcggctc caaggctgcg ctcaacgctg ccaacttcaa cgcggcctac 720

gaggccatga tgaagttcag gggtgacggt cagcgcaagc tgggcatcgt gccggacacg 780

ctgctctgcg gcccggacaa catggccgat gccgaagcgc tgctgaaggc cgcgcagaac 840

gccaacggct ccagcaacac caactacaac aaggtggaac tggtcgttac gccctggctg 900

taa 903

<210> 129
<211> 984
<212> DNA
<213> Ralstonia solanacearum

<400> 129
gtgacgcaga atccccccat cgcacaaacc gcaatagccg cgctagcgtt cgagttggtg 60

cccggcagcg atggtgccgt tccgactgaa gcccatttgc tgccgctcgg cccgttccgg 120

gcaacggatg gtcgcccgtt cgactgcgcg gcttggcagc tcgacgccgc catcgccgcg 180

cgcgtgatcg cgctggccga gcagcagaag aacgacatcc tggtcgacta cgaccaccag 240

agcctgaaca aggaacggaa tggccagcgt gccgatgcgg ctggctggat tccccgcacg 300

ctcgaatggc gcgaaggcaa agggctctac gccaccaaca tcgcatgggt cggcgacgcc 360

gcccagctca tcgcgcagaa gaagtaccgc tacatcagca ctgtcttctt ctacagcccc 420

gtcacaggtg aagtgctcga aatcctttcg gtcgcactca ccaacacacc cgccctggat 480

gggctagaag ccgtggccgc tctggcccgc aagcaattca attttcgaga aggggagttc 540

gacatgtcgc agcaagaaat cgccgcgctc acgaccgagc gtgacggcct gaaatcgcag 600

ctcgcagcgc tgaccactga gcgcgatggc ctgaagacca acgttgctgc ccttacggca 660

gagcgcgaca cgctgaaggc caaggtggaa ggcatcgaga aggaagaagc cgaagccgcg 720

ctggccgcag agcgcaaaca acacggcgat ctgctgaaag ccgcgctgac cgatggccgc 780

ctgaccccgg cgcagaagcc gtgggctgaa aagcaatcgc tggccgcgct gacagagtac 840

ctggacgcca gcaagccgct ggccatcctg gacaagcagg ccgatggcaa gagcgaaggt 900

gcccatggcc tgacccacga agagcttgcg atgtgcaaga agatgggggt cacccccgag 960

cagtacgtcg ccgccaaacg ctga 984

<210> 130
<211> 948

<212>  DNA
<213>  Ralstonia solanacearum

<400>  130
atggacaagc aaacgacctg gcccgcccat tacgccgccg cagacgttgc gcttatcggc      60

cgcattggcc cctggatgga agagcgtggt tacacccaag ccgctctggc gcggctggcg     120

cgcatcagcg caagcagctt gaaccagatt ctcaaaggca gctatgccac cagtccgggc     180

aagctgctgg cgtcggtgga ctcggccatg cgccacgctg aagagacgaa ggccgatgtg     240

gtggccccgg tcgaaaccag cgtcttcaag ctcgcgcacg ccgcgtgcag catggcccgc     300

cgctatcgca acttcgcggt gttcaccggt tacgtgggca cgggcaagac ctttgccatc     360

aagcaatacg tggcatcgca tccgaacacg cacctgattg aggccacgcc caccatgacg     420

ccgcagagcc ttgtgcgtct gctggcccgc gtggtggccg gctatgacgg caagggcagc     480

attgatgaca agttccgctc cgtggtgacg gcgcttcgca acaccgacag tctgctcatc     540

gtggatgagg ccgaaacgct cacgccgcac cagctccaca cgctgcgccg cctccgtgat     600

ctagccaacg tcggcatcgt gctctgcggc accgaacacc tgtcgggcct catcaagccg     660

ctgcatggcc agtttgacca aatccgctcg cgcacgggct tctggcccga gacggtgcgc     720

gccatcaacc tggaagacgc cgccgccctg gtgcaagccg gcttcggcac ggaagacgta     780

cctgaagagg tggtgcaacg cctttaccaa tactgcaagg gcagcgcgcg gatgctggtg     840

gaggggttgg tcgctggcat caaggagttc cgccgtgggc gtccgctgga cgtgaagctg     900

gtggatgcgg tggccaaaca ggcgctgtgc ctgcaatcgc tggcctga                  948

<210>  131
<211>  531
<212>  DNA
<213>  Ralstonia solanacearum

<400>  131
gtggaccgct acgccaaccg cccgcaccca gaagaccaga acacgacacg cgccgcgctg      60

tggtcgcaac tggcaccgct tccgccgcac gccaacgtga ccgaactgaa gcgccaggcg     120

gtggtgctga cggtgaatcg cgcagccatc aagcacggca agcgtgccta cacgcacccc     180

gagctgcacg ccttcaacgg ccacagggtg gtcatggaat acgacctgat ggatgaccgt     240

gtcgccgtca tgcgcacgca ggaaggccgg tggatttgcg acgcacacct cgtcacggcc     300

attgacgcca tcgcgcccaa ccgcctggaa gaaaagcgcc aggcgcgcgc cgccgatgcc     360

atcaagcgcc tgcaacaaaa gatggacgag cagaaggccc gtgcgggcat ggtgctcgat     420

gtggacagcg tggccgacgg cgtgctgcct gcaatcgagg tggaagcccg cctggtggac     480

gacagcgacg acgcgccgct gctgctcgac ctgacgatga acgacgaata a              531

<210>  132
<211>  884
<212>  DNA
<213>  Ralstonia solanacearum

<400>  132
atggcacggg cacaactggt tcagcgcgtg gacgcaggcg gcgcaagtgg cgcggtagcg      60

gtcatgccca cagcaaaggt gctggcgctt cgtgctcgcg acccgtggcg cgaggcaacc     120

gacagggccc ggcaagtggc gacgtggcgt gagacggtgg tggcgtacgt gcgcgccatg     180

acggaagacg gtgtgacgca gaacaacgcg gtggcgctgc tgctggagcg cggcgaggcc     240

ggcagcctgc ccagccactt cgccatggcg ctggctggcg ccgccaaggc aggccggaag     300

acgccatcgc gctccgccat ctgcgaatgg tgcgctcagt atcgcgaagg cggcgcaact     360

gcgctgctgc ccgaccacaa gggccgcgtg gtggaggccg ccggctggtg ggggccggcg      420

ctggagtatt tcaacgcgcc cggcaaacca gatatggccg ctgtacatcg ccgcctggcg     480

gaagtggacg gcttcgcggt cagctacgac caagtacgca actacctgac gggggttcct     540

gccatgctcg gccgcaacag ccccgcacgc attggccgca acctgtaccg cctgaccgag     600

aaggcataca tccgccgctc gaccgaaaac gccttgcccg gcgatgtgta tgtggccgac     660

ggctaccgcg cggacgtgta cctggcgcac cccgtcacgg gcgatatctg gcgcccggag     720

ctgacggtgg caatcgacat gcgcagccgc ttccccgtgg gatggcgggc cgacgaacat     780

gaaggcacgt atgccgtgca gaacatgtgg gcagagtgct tcgcccgctg gaaccacgtg     840

ccgccgatgc tctacatcga caacgggtcc ggctacaaga acac                      884

<210>  133
<211>  1125
<212>  DNA
<213>  Ralstonia solanacearum

<400>  133
gtgaagccga ccatgacgaa gaaaaccaaa gccgtacaag caaccgaaga tatcgacctg      60

ggcatcacgg aaggcgctct agttcacgcc acgcagacgg acgacgcgca gccagtggat     120

attgcaaccg ctgcggcggt gctgcccatc gcgctcgcac cagtggcgaa tctcgccgca     180

gtcgcgtcgc atttcaacgt gaccaccgat gacctcggcg agctggcccg catcggcgct     240

gactctatcg gccgtgccat gttcgaaatt acgcgtgctg ggatggcatt tctgcgcgcc     300

caagagttga tttcgcttgg gcatggtggg gatcgcagat cgactcccga acgttcggga     360

gttgaaaagg acggctttct gacctggatc aacgcgcatg gacttgcaaa ccagcgcgtg     420

tacgaagcca tgcgcatcgc gaagtttgtg gctcaactgc cgcaagacga actggaaggc     480

gtgctggcgc tcggcaaggt caaggtgatg ctcttggcct cgctgccgca ggaagtcatc     540

gaccaagccg ccgagtccgg caacgacatg atcggcaaag ccgacctgat gacggtggcc 600

gaactcaagg aagagattcg cgcgctgaag cggcgtgaaa agaactacga agctgagctg 660

gagcgtgcgc actcgcaggt caaacgcctg tcgcaagtaa atcgcaccac cgaattcctc 720

ttgcgcacag aggaaatccg cgaagagtgc atggccctgc aacttggcgt tgaactcaac 780

acttccagcc tgaaaaagct gttcgatgag gtcaacggcg acgagtcgcc cgaatggcgc 840

ctccagatgg aacagatttg ggtgacggca cacgtcatgg cttcgcgcgg cctggatttg 900

atcgaccaca tggccagcct cgtgcgcgaa gacgacatgc ccgaacgcat ccaaggcacc 960

cacatcctca gccctgacga ggcccgccaa tggctgatcg actacccgat gatcgaaaac 1020

cgccacgccg ccgaagcagc aaaccggcaa gagaagcgtg atgcagccaa accgcgcggc 1080

gccggccgtc ccaagggttc caccaacaag gctaaggggg agtaa 1125

<210> 134
<211> 180
<212> DNA
<213> Ralstonia solanacearum

<400> 134
atgaccccgg agcaagtaaa ggcgcgcttt gagcgcgagg gcaagacctt tgcaaagtgg 60

gcaaaggaga agggctttga ataccgcacg gttatcgcgg tcatcaacgg cgtgaacaag 120

gggcgttacg gcgaggctca caaagtcgcc gtggcactgg gtctcaagaa agctgcgtga 180

<210> 135
<211> 369
<212> DNA
<213> Ralstonia solanacearum

<400> 135
   atgaccatcg gagagcgttt gaaggaagag cgcgagcgat tgcgcctcac ccagccggcc 60
   attgcctcgg ccgccgacac gacgaagcaa acacagcacg catacgaaac cgaccgcacg 120
   ccgccgaaag ccagctatct ggccgcaata gcgatcctgg gcgttgatgt ggcttatgtg 180
   ataacggggg agcgggcgct taatacggcg cgcacgccga tggaagttgc actgctggag 240
   aactaccggc acagcccagc agaggtgcag cgggggttta gcatgcttct cgcacaaacc 300
   tccggagccg cagattcagt cgccggaaag ggaaagaaaa atcgatcctc ggaggaagtg 360
   aagaagtga 369

<210> 136
<211> 318
<212> DNA
<213> Ralstonia solanacearum

<400>  136
gtggcaaaca acacgctgtt gagcatcaac ggcatgaaca agcgtttcga cggtgcttgg      60

ctgatgctgg acgcgagtgc attgccacat ccttccgact tgacgcgggc gggcgggttg     120

ttgctccgcg ttgcgaaggg ctaccactac ggcaaccatc tggcccttgc tcaggaggcg     180

attattggtg cgcaaagaag caaggggaag caatcctgca ttgacgatcc cgcatcaggt     240

agccgcctct gcgtctcgac cgataacggc acgatctaca acatgatctt ggagcaagcc     300

gagcccaaga agggctga                                                   318


<210>  137
<211>  336
<212>  DNA
<213>  Ralstonia solanacearum

<400>  137
atgaaactcc ctcgccttac cacgtggatc atcgccacca ttgcgctggc gatcatcatc      60

ggtctgctgt ctccgcagca actacccgtc agtctttaca agctctcgtt ggtcaccatg     120

gctgccgtgg tggcgtactg gctcgaccgc gcgctgttcc cctatgcccg gccggccggc     180

tacctgtcca gtgcggactg gcgcaaagac gggcctatgt gcgatgacgc cgaccacgcc     240

attgttaccg gctacgagct ggtgtttgcc gccgccatgc ttcgccgcgc cgtcatcgtg     300

gccggtgcga tgctcgccat cggtctgggg gcgtaa                               336


<210>  138
<211>  633
<212>  DNA
<213>  Ralstonia solanacearum

<400>  138
atgcgccgcc agtcgatcct cttgagcgtg ttcgccgcgc tccagctcgc ggcggcgctg      60

ctgctcgcat cggcagatgc attcggtgct gatgccgcca tcccgcacgt cgcgagacgc     120

taccgtgcag acctgacccg cattgcacgc gaggcatggg gacttgacgc acccgtggct     180

gtgttcgccg cacagctcca ccaggaaagc ggctggaacc ccgcagcggt ctcgcgtgtt     240

ggtgcgcaag gtatggcgca gttcatgccc gccacggcga aatggtggtg tgtgctgaat     300

ggccttgcca acgatgcgtg ccagcccacc aaccctgtgt gggctatgcg tgcattggtg     360

ggctatgacc gctggctgta tgagcgcgtg cgcggtgcat ccgagtttga ccggctgtgg     420

gctgcactac gtgcgtacaa cggcggactg ggccactggc aacaggaagc ggccacggtg     480

cggcctgcgc tgaaccgcgc aaccatcgat agcgcatgtg gcacagcgcg ccgccatcgc     540

agcttctgcc ctgagaacct gggctatccg catcgcatct tgaacgtctt gcagccgcgc     600

tatctggcgt gggggcgtgg ggtacggaca tga                                  633

<210> 139
<211> 495
<212> DNA
<213> Ralstonia solanacearum

<400> 139
atgacccgca cgctgattgc aacggcggta gccgtagcgt gcggtgccct tagcgcgtgg 60

agctacacac gcaatcacta cgtggccgag attgctggca tgacggccga tcaagccacg 120

gcccgagaga aagccgagaa gatcgcgcgt gagctgctcg aagccgagca aaagcgcggc 180

aacgcgctgt cggacacgtt ggccaagaaa gaaaccgcga tcaccgaaaa gacccaggag 240

cttgccaatg cactatctcg cctcactact ggccgcaagt gtctcgatgc tcgcgttgtg 300

cgcgtgctca acgactccag caccggcacc gctactgaca acgtgcgcgc caccaccggc 360

acatctgatg cagcggatgg acccgctgcc accgataccg acgtcgctag ctggatcaac 420

cacgccaaag gccaatacga aatctgccgg gctcgactcg gcgcactgat cgactttgaa 480

aaagggcgag ttcaa 495

<210> 140
<211> 153
<212> DNA
<213> Ralstonia solanacearum

<400> 140
atgaagcggc agatgagctt tgcagaagcg gaaagcgcag gtaagaagcg cgtgaccaaa 60

cgccagcgct tcctggcaga gatggagaag gtcgtgccgt ggcagcgctt gctgtcggcg 120

attgggcccc actatccgag aggcgagcga ggt 153

<210> 141
<211> 23
<212> DNA
<213> Artificial sequence

<220>
<223> Primer

<400> 141
ctgaattcga aaaggataga gca 23

<210> 142
<211> 23
<212> DNA
<213> Artificial sequence

<220>
<223> Primer

<400> 142
ctcgacaaac tcttgcaact gac 23

<210> 143
<211> 22
<212> DNA
<213> Artificial sequence

<220>
<223> Primer

<400> 143
ctatgcagaa gcgttgcttg tt 22


<210> 144
<211> 23
<212> DNA
<213> Artificial sequence

<220>
<223> Primer

<400> 144
ctttagcgag cacaagattg agt 23


<210> 145
<211> 23
<212> DNA
<213> Artificial sequence

<220>
<223> Primer

<400> 145
gagatcgttg gaaacatcaa gac 23


<210> 146
<211> 22
<212> DNA
<213> Artificial sequence

<220>
<223> Primer

<400> 146
gtgaaccact attgccggta tc 22


<210> 147
<211> 23
<212> DNA
<213> Artificial sequence

<220>
<223> Primer

<400> 147
agaactgcca agttcgacta cct 23

<210> 148
<211> 23
<212> DNA
<213> Artificial sequence

<220>
<223> Primer

<400> 148
cattccaacg ttcagatggt tat 23


<210> 149
<211> 22
<212> DNA
<213> Artificial sequence

<220>
<223> Primer

<400> 149
gcagaaaaga tatcccctgc ac 22


<210> 150
<211> 23
<212> DNA
<213> Artificial sequence

<220>
<223> Primer

<400> 150
ttcgagtaca aatgtaggct tcc 23


<210> 151
<211> 23
<212> DNA
<213> Artificial sequence

<220>
<223> Primer

<400> 151
gatcagatgg agcaaagaac act 23


<210> 152
<211> 23
<212> DNA
<213> Artificial sequence

<220>
<223> Primer

<400> 152
tattgaaact cttcacgggt cat 23

<210> 153
<211> 23
<212> DNA
<213> Artificial sequence

<220>
<223> Primer

<400> 153
tttcgaccaa gaaaagcata gag 23


<210> 154
<211> 23
<212> DNA
<213> Artificial sequence

<220>
<223> Primer

<400> 154
atttctgtgc ccactacgaa cta 23


<210> 155
<211> 23
<212> DNA
<213> Artificial sequence

<220>
<223> Primer

<400> 155
tggtgttcta actgtggaag gtt 23


<210> 156
<211> 23
<212> DNA
<213> Artificial sequence

<220>
<223> Primer

<400> 156
taccgccagt catatcagtt ctt 23


<210> 157
<211> 23
<212> DNA
<213> Artificial sequence

<220>
<223> Primer

<400> 157
attagactga tcaaggcatg gaa 23


<210> 158

<211>  23
<212>  DNA
<213>  Artificial sequence

<220>
<223>  Primer

<400>  158
ccttcattat tgagacggtc aag                                              23


<210>  159
<211>  23
<212>  DNA
<213>  Artificial sequence

<220>
<223>  Primer

<400>  159
atgtttgtgc tactggtcag tcc                                              23


<210>  160
<211>  23
<212>  DNA
<213>  Artificial sequence

<220>
<223>  Primer

<400>  160
ccttcacttg cagataatgg aac                                              23


<210>  161
<211>  23
<212>  DNA
<213>  Artificial sequence

<220>
<223>  Primer

<400>  161
aaagaagctc aaggagatca agg                                              23


<210>  162
<211>  23
<212>  DNA
<213>  Artificial sequence

<220>
<223>  Primer

<400>  162
aacagcaggt tgtgatactg cat                                              23


<210>  163
<211>  21

<212>  DNA
<213>  Artificial sequence

<220>
<223>  Primer

<400>  163
cgagctcatc gttatcgaca t                                                21


<210>  164
<211>  23
<212>  DNA
<213>  Artificial sequence

<220>
<223>  Primer

<400>  164
aagctcttgg actaggacga tct                                              23


<210>  165
<211>  23
<212>  DNA
<213>  Artificial sequence

<220>
<223>  Primer

<400>  165
tgaaatgctc aaagacaaac aga                                              23


<210>  166
<211>  22
<212>  DNA
<213>  Artificial sequence

<220>
<223>  Primer

<400>  166
atcgtacagg tcattgccaa at                                               22


<210>  167
<211>  23
<212>  DNA
<213>  Artificial sequence

<220>
<223>  Primer

<400>  167
tacaacctga agaggatctc gaa                                              23


<210>  168
<211>  23
<212>  DNA

<213>  Artificial sequence

<220>
<223>  Primer

<400>  168
aaagccggtc atagaggaca tag                                              23


<210>  169
<211>  23
<212>  DNA
<213>  Artificial sequence

<220>
<223>  Primer

<400>  169
caaccagacc atctacaaga tcc                                              23


<210>  170
<211>  23
<212>  DNA
<213>  Artificial sequence

<220>
<223>  Primer

<400>  170
gcttcatact caaatcgaac acc                                              23


<210>  171
<211>  23
<212>  DNA
<213>  Artificial sequence

<220>
<223>  Primer

<400>  171
aactccaact tgcttgactg ttc                                              23


<210>  172
<211>  22
<212>  DNA
<213>  Artificial sequence

<220>
<223>  Primer

<400>  172
ggatgaactt cgttcgattg ag                                               22


<210>  173
<211>  21
<212>  DNA
<213>  Artificial sequence

<220>
<223>  Primer

<400>  173
gaagctcggt gatatcgaaa c                                                21


<210>  174
<211>  21
<212>  DNA
<213>  Artificial sequence

<220>
<223>  Primer

<400>  174
ggtgatcgct gtcgataatt t                                                21


<210>  175
<211>  23
<212>  DNA
<213>  Artificial sequence

<220>
<223>  Primer

<400>  175
gtatcagaaa ggccagctac aca                                              23


<210>  176
<211>  23
<212>  DNA
<213>  Artificial sequence

<220>
<223>  Primer

<400>  176
ctgattgcca atattcgatt ctc                                              23


<210>  177
<211>  21
<212>  DNA
<213>  Artificial sequence

<220>
<223>  Primer

<400>  177
aagggcaatg gctttttctg t                                                21


<210>  178
<211>  23
<212>  DNA
<213>  Artificial sequence

<220>
<223>  Primer

<400>  178
gagactgata aatcagcgtt tcc                                              23


<210>  179
<211>  22
<212>  DNA
<213>  Artificial sequence

<220>
<223>  Primer

<400>  179
gctttctacg tcgcctcagt at                                               22


<210>  180
<211>  24
<212>  DNA
<213>  Artificial sequence

<220>
<223>  Primer

<400>  180
gtaacggtct gattcttgag gttt                                             24


<210>  181
<211>  21
<212>  DNA
<213>  Artificial sequence

<220>
<223>  Primer

<400>  181
ccactacccc tgtttccatt c                                                21


<210>  182
<211>  22
<212>  DNA
<213>  Artificial sequence

<220>
<223>  Primer

<400>  182
aaacctgtag tcgtcgtcct tg                                               22


<210>  183
<211>  21
<212>  DNA
<213>  Artificial sequence

<220>

<223>  Primer

<400>  183
agacgttgga caacatcaac c                                                21


<210>  184
<211>  22
<212>  DNA
<213>  Artificial sequence

<220>
<223>  Primer

<400>  184
agttctgttc gtcgtcgtga at                                               22


<210>  185
<211>  22
<212>  DNA
<213>  Artificial sequence

<220>
<223>  Primer

<400>  185
ccagtattcc aagacgctat cc                                               22


<210>  186
<211>  21
<212>  DNA
<213>  Artificial sequence

<220>
<223>  Primer

<400>  186
aacggataca gcagcaggtt t                                                21


<210>  187
<211>  20
<212>  DNA
<213>  Artificial sequence

<220>
<223>  Primer

<400>  187
atacccagcg cgtattccaa                                                  20


<210>  188
<211>  20
<212>  DNA
<213>  Artificial sequence

<220>
<223>  Primer

<400>  188
gttgaggcaa caccagacag                                                    20


<210>  189
<211>  23
<212>  DNA
<213>  Artificial sequence

<220>
<223>  Primer

<400>  189
cttgagaaag cttttggtga aga                                                23


<210>  190
<211>  22
<212>  DNA
<213>  Artificial sequence

<220>
<223>  Primer

<400>  190
ctttgagacc ttcccaggct aa                                                 22


<210>  191
<211>  23
<212>  DNA
<213>  Artificial sequence

<220>
<223>  Primer

<400>  191
taagagcagg ctatggacaa cat                                                23


<210>  192
<211>  23
<212>  DNA
<213>  Artificial sequence

<220>
<223>  Primer

<400>  192
aataccagca cacagaaggt cag                                                23


<210>  193
<211>  22
<212>  DNA
<213>  Artificial sequence

<220>
<223>  Primer

<400>  193
ctgatttcca tgtacctgca tc                                                22

<210>  194
<211>  23
<212>  DNA
<213>  Artificial sequence

<220>
<223>  Primer

<400>  194
acatcagcac gttcttgtag agc                                               23

<210>  195
<211>  22
<212>  DNA
<213>  Artificial sequence

<220>
<223>  Primer

<400>  195
gacgatgagt tgctgaagta cc                                                22

<210>  196
<211>  23
<212>  DNA
<213>  Artificial sequence

<220>
<223>  Primer

<400>  196
tgaaggtaat ggtcacagct ttt                                               23

<210>  197
<211>  22
<212>  DNA
<213>  Artificial sequence

<220>
<223>  Primer

<400>  197
atccggttcc ttctgatgat ct                                                22

<210>  198
<211>  23
<212>  DNA
<213>  Artificial sequence

<220>
<223>  Primer

<400>  198

cttcaacttc acgtgttcaa tca                                                23

<210>  199
<211>  23
<212>  DNA
<213>  Artificial sequence

<220>
<223>  Primer

<400>  199
gctagtccac acgacaaaat cat                                                23

<210>  200
<211>  22
<212>  DNA
<213>  Artificial sequence

<220>
<223>  Primer

<400>  200
gtgactagct tggcgatctt ct                                                 22

<210>  201
<211>  21
<212>  DNA
<213>  Artificial sequence

<220>
<223>  Primer

<400>  201
aggtgaagtg ctcgaaatcc t                                                  21

<210>  202
<211>  22
<212>  DNA
<213>  Artificial sequence

<220>
<223>  Primer

<400>  202
cttcttcctt ctcgatgcct tc                                                 22

<210>  203
<211>  23
<212>  DNA
<213>  Artificial sequence

<220>
<223>  Primer

<400>  203
ttatgtggct ttctctggca ata                                                23

<210> 204
<211> 21
<212> DNA
<213> Artificial sequence

<220>
<223> Primer

<400> 204
acaaacgtcc agtcgtcaat c 21


<210> 205
<211> 23
<212> DNA
<213> Artificial sequence

<220>
<223> Primer

<400> 205
catttatctc tggtcttggc ttg 23


<210> 206
<211> 21
<212> DNA
<213> Artificial sequence

<220>
<223> Primer

<400> 206
acagccaaac tgacaagatc g 21


<210> 207
<211> 23
<212> DNA
<213> Artificial sequence

<220>
<223> Primer

<400> 207
gcatgaaaat gtctacgttc ctc 23


<210> 208
<211> 21
<212> DNA
<213> Artificial sequence

<220>
<223> Primer

<400> 208
atggtgatag tgcggaatga c 21

<210>  209
<211>  21
<212>  DNA
<213>  Artificial sequence

<220>
<223>  Primer

<400>  209
cagacatgtt ctgcgaagga t                                                21


<210>  210
<211>  23
<212>  DNA
<213>  Artificial sequence

<220>
<223>  Primer

<400>  210
gaggaacgta gacattttca tgc                                              23


<210>  211
<211>  23
<212>  DNA
<213>  Artificial sequence

<220>
<223>  Primer

<400>  211
tgtaatgacc aagcaagaac tca                                              23


<210>  212
<211>  21
<212>  DNA
<213>  Artificial sequence

<220>
<223>  Primer

<400>  212
cacggtgtca agaatggttt c                                                21


<210>  213
<211>  23
<212>  DNA
<213>  Artificial sequence

<220>
<223>  Primer

<400>  213
atttcaaacg ccaagccttt aac                                              23

<210> 214
<211> 22
<212> DNA
<213> Artificial sequence

<220>
<223> Primer

<400> 214
gttcatcgaa agcgatgttc tc 22


<210> 215
<211> 22
<212> DNA
<213> Artificial sequence

<220>
<223> Primer

<400> 215
gcctatttgg accgaagaag ac 22


<210> 216
<211> 22
<212> DNA
<213> Artificial sequence

<220>
<223> Primer

<400> 216
acttcttgag gcatctcggt tt 22


<210> 217
<211> 21
<212> DNA
<213> Artificial sequence

<220>
<223> Primer

<400> 217
gcagcattga tgacaagttc c 21


<210> 218
<211> 22
<212> DNA
<213> Artificial sequence

<220>
<223> Primer

<400> 218
agtattggta aaggcgttgc ac 22


<210> 219

<211>  21
<212>  DNA
<213>  Artificial sequence

<220>
<223>  Primer

<400>  219
agcaaccgaa gatatcgacc t                                                21


<210>  220
<211>  21
<212>  DNA
<213>  Artificial sequence

<220>
<223>  Primer

<400>  220
gcccaagcga aatcaactct t                                                21


<210>  221
<211>  23
<212>  DNA
<213>  Artificial sequence

<220>
<223>  Primer

<400>  221
ctttcccagt caatacattc cag                                              23


<210>  222
<211>  23
<212>  DNA
<213>  Artificial sequence

<220>
<223>  Primer

<400>  222
cgttatcaca taagccacat caa                                              23


<210>  223
<211>  21
<212>  DNA
<213>  Artificial sequence

<220>
<223>  Primer

<400>  223
aaacaacacg ctgttgagca t                                                21


<210>  224
<211>  21

<212>  DNA
<213>  Artificial sequence

<220>
<223>  Primer

<400>  224
cttctttgcg caccaataat c                                                21


<210>  225
<211>  23
<212>  DNA
<213>  Artificial sequence

<220>
<223>  Primer

<400>  225
cgtgtatatc ggcagtcaag aag                                              23


<210>  226
<211>  23
<212>  DNA
<213>  Artificial sequence

<220>
<223>  Primer

<400>  226
ctaagaaatg aaaggtgggg ttc                                              23


<210>  227
<211>  23
<212>  DNA
<213>  Artificial sequence

<220>
<223>  Primer

<400>  227
ggatgacttg tgttagcgac tct                                              23


<210>  228
<211>  23
<212>  DNA
<213>  Artificial sequence

<220>
<223>  Primer

<400>  228
gaatacgatc ctccacaatc aaa                                              23


<210>  229
<211>  23
<212>  DNA

<213>  Artificial sequence

<220>
<223>  Primer

<400>  229
tgaagcactg tctatcaacc aga                                              23


<210>  230
<211>  23
<212>  DNA
<213>  Artificial sequence

<220>
<223>  Primer

<400>  230
tttgtcctag tcacagcact gaa                                              23


<210>  231
<211>  23
<212>  DNA
<213>  Artificial sequence

<220>
<223>  Primer

<400>  231
gacttcggct atctggagaa aat                                              23


<210>  232
<211>  23
<212>  DNA
<213>  Artificial sequence

<220>
<223>  Primer

<400>  232
tcttagcagg tttaggctga gtg                                              23


<210>  233
<211>  23
<212>  DNA
<213>  Artificial sequence

<220>
<223>  Primer

<400>  233
catcttcaag gatgactctc tgg                                              23


<210>  234
<211>  23
<212>  DNA
<213>  Artificial sequence

<220>
<223>  Primer

<400>  234
gaagaagtga ccaggctgaa ttt                                              23


<210>  235
<211>  23
<212>  DNA
<213>  Artificial sequence

<220>
<223>  Primer

<400>  235
actcagtgac gaagaggttg aag                                              23


<210>  236
<211>  23
<212>  DNA
<213>  Artificial sequence

<220>
<223>  Primer

<400>  236
acgagtagct tcaatggtgt ctt                                              23


<210>  237
<211>  22
<212>  DNA
<213>  Artificial sequence

<220>
<223>  Primer

<400>  237
acactatgcc tgctgacttg aa                                               22


<210>  238
<211>  23
<212>  DNA
<213>  Artificial sequence

<220>
<223>  Primer

<400>  238
aggtgacttc aacaatgtta ggc                                              23


<210>  239
<211>  23
<212>  DNA
<213>  Artificial sequence

<220>
<223>  Primer

<400>  239
caatagaaat tgccgaggtg ata                                              23


<210>  240
<211>  23
<212>  DNA
<213>  Artificial sequence

<220>
<223>  Primer

<400>  240
ccttgataag gatgttcaac gac                                              23


<210>  241
<211>  23
<212>  DNA
<213>  Artificial sequence

<220>
<223>  Primer

<400>  241
cttgacgtct gacaaccaag tag                                              23


<210>  242
<211>  23
<212>  DNA
<213>  Artificial sequence

<220>
<223>  Primer

<400>  242
ataagataaa caggtcggcc ttc                                              23


<210>  243
<211>  23
<212>  DNA
<213>  Artificial sequence

<220>
<223>  Primer

<400>  243
aattgggtat acgtgatctg tgg                                              23


<210>  244
<211>  22
<212>  DNA
<213>  Artificial sequence

<220>

<223>  Primer

<400>  244
tcgggtaaga cgaagctgac ta                                              22


<210>  245
<211>  21
<212>  DNA
<213>  Artificial sequence

<220>
<223>  Primer

<400>  245
agggaatcaa atcgctcatc t                                               21


<210>  246
<211>  23
<212>  DNA
<213>  Artificial sequence

<220>
<223>  Primer

<400>  246
agaagaagcc catgatgaca gag                                             23


<210>  247
<211>  70
<212>  DNA
<213>  Artificial sequence

<220>
<223>  Probe

<400>  247
ggctcgaaca acttggagag atttacggag gagacgtgtc attgctcatg ccgaattcac     60

gctacgccgc                                                            70


<210>  248
<211>  70
<212>  DNA
<213>  Artificial sequence

<220>
<223>  Probe

<400>  248
ccggaggaac cgctgcgatg gacgccatca ctggaaagtc tagtggccga accgcgaaag     60

cgcgcgcatg                                                            70


<210>  249
<211>  70
<212>  DNA

<213>  Artificial sequence

<220>
<223>  Probe

<400>  249
gtgacgatac cgctgttgcg tctctgtcgt ttgttgtcca agagggcttc aaaccagata      60

cgttctccgc                                                              70


<210>  250
<211>  70
<212>  DNA
<213>  Artificial sequence

<220>
<223>  Probe

<400>  250
gcgaacaatg tcaccgtcgc ctatgcagaa gcgttgcttg ttgccacgcc tgcctctcgt      60

cttgttgatg                                                              70


<210>  251
<211>  70
<212>  DNA
<213>  Artificial sequence

<220>
<223>  Probe

<400>  251
caaggtcttt gacgagatcg ttggaaacat caagaccatt gggctaaaga agccaatcac      60

cgtcacgccg                                                              70


<210>  252
<211>  70
<212>  DNA
<213>  Artificial sequence

<220>
<223>  Probe

<400>  252
gacgagaatt tcgtcaacct tctgcgagcg gaatctttgg cgacacttcc caaatatctg      60

gctgagcgtg                                                              70


<210>  253
<211>  70
<212>  DNA
<213>  Artificial sequence

<220>
<223>  Probe

<400>  253
cgcaaccgtg agggaggtgc tcaccaacga gaaatacatc ggcaacaaca tctataaccg      60

tgtgtcgtac                                                              70


<210>  254
<211>  70
<212>  DNA
<213>  Artificial sequence

<220>
<223>  Probe

<400>  254
gcgcctggtt gtcgcgatag tcgtcatcag agcgcaagtg ctgctgttgc caccgtcggc      60

gacgttggcg                                                              70


<210>  255
<211>  70
<212>  DNA
<213>  Artificial sequence

<220>
<223>  Probe

<400>  255
ggtgcgacgt tcttctcggc tccagataag gcggtcctgc tactgtcgtt cagatatctc      60

acagacgacc                                                              70


<210>  256
<211>  70
<212>  DNA
<213>  Artificial sequence

<220>
<223>  Probe

<400>  256
ggctgtgagt atgttctgag ggtagccgcg catcatcgtc ccgaaccacg ttcgtcaact      60

ggtaagttgg                                                              70


<210>  257
<211>  70
<212>  DNA
<213>  Artificial sequence

<220>
<223>  Probe

<400>  257
cctggttgtg cttgataacc atctgaacgt tggaatgcaa gtgctggaag actcgctatg      60

caactggcag                                                              70

<210> 258
<211> 70
<212> DNA
<213> Artificial sequence

<220>
<223> Probe

<400> 258
cggaactagg cacatacaag cacgccgtcg gaagcctaca tttgtactcg aagaacacgg 60

accaggcgac 70


<210> 259
<211> 70
<212> DNA
<213> Artificial sequence

<220>
<223> Probe

<400> 259
gcaggataag agccgtgagg ttcctgttca tctgcgtaat gcaccaacaa agctcatgaa 60

ggaactcggc 70


<210> 260
<211> 70
<212> DNA
<213> Artificial sequence

<220>
<223> Probe

<400> 260
gagcggttcc ttgaaagcta tgctggagcc atcatcactg atcctgctac ggcgattgtg 60

gagcttgtcg 70


<210> 261
<211> 70
<212> DNA
<213> Artificial sequence

<220>
<223> Probe

<400> 261
gtcttcatca accacattga agttcacgac aagccagcgg cagacaacat caggaacatt 60

gtgttcggcg 70


<210> 262
<211> 70
<212> DNA
<213> Artificial sequence

<220>
<223> Probe

<400> 262
ctgcctatca tcctggatag agggcgaagc cgaaccttgg agcgcacagt caaggaaggc 60

tttgaacatg 70


<210> 263
<211> 70
<212> DNA
<213> Artificial sequence

<220>
<223> Probe

<400> 263
cggctacagg cagtctcaaa gagacaggtc tgaagaagac tatgccatct attcggtatg 60

tggctggtgc 70


<210> 264
<211> 70
<212> DNA
<213> Artificial sequence

<220>
<223> Probe

<400> 264
cgttgaacat gtggatggta agcacggagg taaagtcagg cagttgatta gactgatcaa 60

ggcatggaag 70


<210> 265
<211> 70
<212> DNA
<213> Artificial sequence

<220>
<223> Probe

<400> 265
cgctggtgct gtggtgggct cccaagtgag taagagcagt gaagtgaaga atgtcactga 60

cttgcaagtg 70


<210> 266
<211> 70
<212> DNA
<213> Artificial sequence

<220>
<223> Probe

<400> 266

ccagcaatcc cactaatgtt tgtgctactg gtcagtccta cggcttcaac ttcgccaaca 60

atcaatacac 70

<210> 267
<211> 70
<212> DNA
<213> Artificial sequence

<220>
<223> Probe

<400> 267
ggaggtttgg aggctcagat cggtggtttg tccatgcagt atcacaacct gctgttgctg 60

ctggctggtg 70

<210> 268
<211> 70
<212> DNA
<213> Artificial sequence

<220>
<223> Probe

<400> 268
gctgtagcac cgactctgac cgccaatgac gaagttctat tgaacttcat tagtgaatac 60

ggtgatggtg 70

<210> 269
<211> 69
<212> DNA
<213> Artificial sequence

<220>
<223> Probe

<400> 269
cgtcttgtgc ctatagcaat gaaatgcaga aggcttggcg gacaatatca actatgccag 60

ctcgtggtg 69

<210> 270
<211> 70
<212> DNA
<213> Artificial sequence

<220>
<223> Probe

<400> 270
atcatggctg agatccagcg tattgccgca accgagctca tcgttatcga catgggcgac 60

aggcttggca 70

<210> 271
<211> 70
<212> DNA
<213> Artificial sequence

<220>
<223> Probe

<400> 271
gaggtcaagc tggatcaagc tggagtcgaa gtggaggaag cgaaagtcca caagttcagc 60

aagaacgatc 70


<210> 272
<211> 70
<212> DNA
<213> Artificial sequence

<220>
<223> Probe

<400> 272
ccacgatcaa ctactaccgt gtatcgacca aggatcaatc catcgaatcc cagcgtcaga 60

agctaggccc 70


<210> 273
<211> 70
<212> DNA
<213> Artificial sequence

<220>
<223> Probe

<400> 273
catcaacgac ccttactgtc cgagtaactg ccgatgggcc accaacaacg atcagcagaa 60

caacaagcgg 70


<210> 274
<211> 70
<212> DNA
<213> Artificial sequence

<220>
<223> Probe

<400> 274
gaccgactgg aacgagatgg ccaaggctcc gagcaaggtg gcaaagatcg cgaaagcagt 60

gggactcgac 70


<210> 275
<211> 70
<212> DNA
<213> Artificial sequence

<220>
<223> Probe

<400> 275
cttcatgacg gtgtagccgt gacccaggtg tctgaccttg atgcgcgact aagcgcacac 60

ttgatgctct 70


<210> 276
<211> 70
<212> DNA
<213> Artificial sequence

<220>
<223> Probe

<400> 276
gccggtgaag atacgggtca gcgtctcaag caggcgcgtg aaatgctcaa agacaaacag 60

atcttcgcgg 70


<210> 277
<211> 70
<212> DNA
<213> Artificial sequence

<220>
<223> Probe

<400> 277
gccacatcga actgtgggcc gacggcatct ccctgcccta tgtcctctat gaccggcttt 60

gcgagatcga 70


<210> 278
<211> 70
<212> DNA
<213> Artificial sequence

<220>
<223> Probe

<400> 278
ggagaacctc gacaaccaga ccatctacaa gatcctagtc aagtgcatca agaaggcgaa 60

ggagaggcgg 70


<210> 279
<211> 52
<212> DNA
<213> Artificial sequence

<220>
<223> Probe

<400> 279
ggcagatgga catgaatctt cctcttgaat ttcacgtccg cattgaggag ac 52

<210> 280
<211> 70
<212> DNA
<213> Artificial sequence

<220>
<223> Probe

<400> 280
gtgccactag ctattcgtgc gagtccctgg cagagatatt cagacggcta caccagcagg 60

ctggcatcgc 70


<210> 281
<211> 70
<212> DNA
<213> Artificial sequence

<220>
<223> Probe

<400> 281
gcgctgccgc cgctggctcg ggcggcaatc gaccgctacc tggttgaacg cgggctaccg 60

attacgcgcg 70


<210> 282
<211> 70
<212> DNA
<213> Artificial sequence

<220>
<223> Probe

<400> 282
gagaaacatc ccgtcgccat gcgccgcatg ttgctggttg ccgcaaccgt cacgctgcta 60

tcgctgactg 70


<210> 283
<211> 70
<212> DNA
<213> Artificial sequence

<220>
<223> Probe

<400> 283
cgtttcgtgc atacatcgac agccttgacc actacactga cgcccatgtg attgtccttc 60

cgtatgcgcc 70


<210> 284
<211> 70
<212> DNA

<213>  Artificial sequence

<220>
<223>  Probe

<400>  284
gatatctggc aggcatttgc caacccagac gaagatcgct tgcgctactt caagaaggcc      60

actgataagg                                                              70


<210>  285
<211>  70
<212>  DNA
<213>  Artificial sequence

<220>
<223>  Probe

<400>  285
gacgtcaagc tgcctatcgg gcaggtttgc ccagtgtcgt tgaacatcct tatcaagggt      60

gagtcgggcg                                                              70


<210>  286
<211>  70
<212>  DNA
<213>  Artificial sequence

<220>
<223>  Probe

<400>  286
cttgaccgat acgtcaaggg cagccgactg ccgacatatg cagcggcact catcctggcg      60

aaagaagcgg                                                              70


<210>  287
<211>  70
<212>  DNA
<213>  Artificial sequence

<220>
<223>  Probe

<400>  287
cgcaatgcat cctcaccgaa ggtgttgctg acgcaactgg cggcgtctcc gaacagatca      60

aggttggccg                                                              70


<210>  288
<211>  70
<212>  DNA
<213>  Artificial sequence

<220>
<223>  Probe

<400> 288
cgttcaggag gtcgtcatgg tcgatccgaa cgacaagtac acgatgcagt tgccgctgat 60

gcgttatcgc 70


<210> 289
<211> 70
<212> DNA
<213> Artificial sequence

<220>
<223> Probe

<400> 289
gggctcgtgc gtcaatgctt tggttatggc gctgttcgtt ctgctgctga cgctgttggt 60

cccggcgtgg 70


<210> 290
<211> 70
<212> DNA
<213> Artificial sequence

<220>
<223> Probe

<400> 290
gaggcgatgg acgacgctga ggctatcgag ttcttcggca gcctggatga gcgtgatgag 60

gcgattgagg 70


<210> 291
<211> 70
<212> DNA
<213> Artificial sequence

<220>
<223> Probe

<400> 291
ccacagagct gcagcggcta tctgctgttg cagccgagcg aatactcgaa cgtgatggct 60

ctgtccggcg 70


<210> 292
<211> 70
<212> DNA
<213> Artificial sequence

<220>
<223> Probe

<400> 292
gggtgctgcg atggcgcaaa ccacgaccag cggtatcgat ttctcgtcca tgacgggtgc 60

cgtgagcgcg 70

<210> 293
<211> 70
<212> DNA
<213> Artificial sequence

<220>
<223> Probe

<400> 293
cgacagtgtc ttcgtcgcta tcttcacggc gctttggcag atctcagagg atctgttcat 60

cgactcgctg 70


<210> 294
<211> 70
<212> DNA
<213> Artificial sequence

<220>
<223> Probe

<400> 294
cgagattcct gaagctgagg tgctgcgttg gaatgagctg gtcgagaacg acagcctggt 60

catcttggac 70


<210> 295
<211> 70
<212> DNA
<213> Artificial sequence

<220>
<223> Probe

<400> 295
cgccgcactg cattgaccaa gctcattcgt cagaagcgat ctaacaactt tcagattgcc 60

aaagagatgg 70


<210> 296
<211> 70
<212> DNA
<213> Artificial sequence

<220>
<223> Probe

<400> 296
ggaacaaatc aagatgttca acttcacggc tgatgaactt ggtttccatg ctgctcctga 60

tgcacgtgcc 70


<210> 297
<211> 70
<212> DNA
<213> Artificial sequence

<220>
<223> Probe

<400> 297
ctgtttacca accacatagc ctcacacacg gctgctgacg gatcagactt ggacgactac 60

ttgagtcgtg 70


<210> 298
<211> 70
<212> DNA
<213> Artificial sequence

<220>
<223> Probe

<400> 298
ggtggtcgag gtgagaagtg tctacgacaa acctgtgaaa gccgttgaga atcgaatatt 60

ggcaatcagg 70


<210> 299
<211> 70
<212> DNA
<213> Artificial sequence

<220>
<223> Probe

<400> 299
cgtcattcgt gagaaccaga tcaaagagct tctgattgct tacaacagct acttcatggt 60

ggctgctgcc 70


<210> 300
<211> 70
<212> DNA
<213> Artificial sequence

<220>
<223> Probe

<400> 300
gaagtatgct ggcctgatcg gtcagctcaa ctcagccgct agggaaacgc tgatttatca 60

gtctcagccg 70


<210> 301
<211> 70
<212> DNA
<213> Artificial sequence

<220>
<223> Probe

<400> 301

gcgttaccgc atggaggcta ctgacgcagc aaacctcaag aatcagaccg ttacgttcca 60

gattaaagtg 70

<210> 302
<211> 70
<212> DNA
<213> Artificial sequence

<220>
<223> Probe

<400> 302
ccggatagcc gccattggca gcacggtcgg caaccggttg tggagccatg acgcgccaac 60

gctgatgttc 70

<210> 303
<211> 70
<212> DNA
<213> Artificial sequence

<220>
<223> Probe

<400> 303
gtcaatggca ccttgacggt aggcgacgcg ggaatcacgg gcggcgctga agacgagagc 60

gtggacgatt 70

<210> 304
<211> 70
<212> DNA
<213> Artificial sequence

<220>
<223> Probe

<400> 304
gccttgtagt cggcgcgagc aaggtgcagg tgcgcgtgct ggacgaagag acgttggaca 60

acatcaaccg 70

<210> 305
<211> 70
<212> DNA
<213> Artificial sequence

<220>
<223> Probe

<400> 305
gcttggtcgt gagcttgtgg actgccagta ttccaagacg ctatccggcc tgaaactgag 60

cgagatcgtg 70

<210> 306
<211> 70
<212> DNA
<213> Artificial sequence

<220>
<223> Probe

<400> 306
gcgctgtctg gtgttgcctc aacggatggt gccgtgcgtc gcaatctgct gcgcgaagaa 60

gcgttgcgcg 70


<210> 307
<211> 70
<212> DNA
<213> Artificial sequence

<220>
<223> Probe

<400> 307
cggtgttggt gccgctgtcg gtggcggtct gggcttagcc tgggaaggtc tcaaagacct 60

gctgaagcca 70


<210> 308
<211> 70
<212> DNA
<213> Artificial sequence

<220>
<223> Probe

<400> 308
cgccggccga catcgaagcg aacatcgcaa gcgagcatta cttcactgcc gaagacggca 60

tgatggccgg 70


<210> 309
<211> 70
<212> DNA
<213> Artificial sequence

<220>
<223> Probe

<400> 309
ttcctgctac atcaagcggc tgatttccat gtacctgcat cgctccgatg gcagcaccga 60

cgacgcctat 70


<210> 310
<211> 70
<212> DNA
<213> Artificial sequence

<220>
<223>  Probe

<400>  310
cgtcggtggc caagtcctgg agtacaccga cggcgatttg tactggcgcg atcgctacga      60

gttcgacgcc                                                              70


<210>  311
<211>  70
<212>  DNA
<213>  Artificial sequence

<220>
<223>  Probe

<400>  311
ccgcatccga tgatccggtt ccttctgatg atctggccat catcgaaagc gcaccgcgcc      60

gctttggccg                                                              70


<210>  312
<211>  70
<212>  DNA
<213>  Artificial sequence

<220>
<223>  Probe

<400>  312
cggaagactg gaagaagatc gccaagctag tcaccagtac cggcaaatcc aacacctacg      60

aatggctgag                                                              70


<210>  313
<211>  70
<212>  DNA
<213>  Artificial sequence

<220>
<223>  Probe

<400>  313
ggtgaagtgc tcgaaatcct ttcggtcgca ctcaccaaca cacccgccct ggatgggcta      60

gaagccgtgg                                                              70


<210>  314
<211>  70
<212>  DNA
<213>  Artificial sequence

<220>
<223>  Probe

<400>  314
cgccgagtgg ttcgcgctct accgcgatga tggcgcgatt gacgactgga cgtttgtgag      60

tggcgtgcgg                                                          70

<210>  315
<211>  70
<212>  DNA
<213>  Artificial sequence

<220>
<223>  Probe

<400>  315
catgttcctt gtggtggcac tgctgctggc gatcttgtca gtttggctgt tcgaccgcta      60

cgtgtgtcgg                                                          70

<210>  316
<211>  70
<212>  DNA
<213>  Artificial sequence

<220>
<223>  Probe

<400>  316
cgtggtagac gcctacccgg ctgtcgatga agatttgctg agaaccttgc cacccgtgct      60

gcgtgcggtg                                                          70

<210>  317
<211>  70
<212>  DNA
<213>  Artificial sequence

<220>
<223>  Probe

<400>  317
gcgcgtcaag tgggccgtca cgtgccggac ttggacagct tggccgtagc tgaatgccaa      60

acgattgccg                                                          70

<210>  318
<211>  70
<212>  DNA
<213>  Artificial sequence

<220>
<223>  Probe

<400>  318
gcagacatgt tctgcgaagg atgggtgttt gccgtgcgcg agctggtggg tgccaccgtg      60

ccgcccgaag                                                          70

<210>  319

<211>  70
<212>  DNA
<213>  Artificial sequence

<220>
<223>  Probe

<400>  319
gaccgaaacc attcttgaca ccgtgcgcgc cggtaacgag ctgacgatca ccgacctggg      60

caagttcggc                                                            70


<210>  320
<211>  70
<212>  DNA
<213>  Artificial sequence

<220>
<223>  Probe

<400>  320
tcaccttgag cacgttcgac ggtcgccagc aggtagtcgt gtcccaggcc gagaacatcg      60

ctttcgatga                                                            70


<210>  321
<211>  70
<212>  DNA
<213>  Artificial sequence

<220>
<223>  Probe

<400>  321
ccgctgcctg aagtcgtctg cccgaactgc cgcgtgcgga tgagcctgga tgtggtgcta      60

gccgatgacg                                                            70


<210>  322
<211>  70
<212>  DNA
<213>  Artificial sequence

<220>
<223>  Probe

<400>  322
gaaacagcta gcaggcatca cccagggacc tgttgcagcc gaactgctgg cgggcttgcg      60

ccgaatctgc                                                            70


<210>  323
<211>  70
<212>  DNA
<213>  Artificial sequence

<220>

<223>  Probe

<400>  323
cgcttcttgg atgccatcgc caacggcgca ccttcagcag aggcagccgc gtttggcgcc      60

tggatcgaag                                                              70


<210>  324
<211>  70
<212>  DNA
<213>  Artificial sequence

<220>
<223>  Probe

<400>  324
cttcgcaaca ccgacagtct gctcatcgtg gatgaggccg aaacgctcac gccgcaccag      60

ctccacacgc                                                              70


<210>  325
<211>  70
<212>  DNA
<213>  Artificial sequence

<220>
<223>  Probe

<400>  325
cgctgtacat cgccgcctgg cggaagtgga cggcttcgcg gtcagctacg accaagtacg      60

caactacctg                                                              70


<210>  326
<211>  70
<212>  DNA
<213>  Artificial sequence

<220>
<223>  Probe

<400>  326
caagcaaccg aagatatcga cctgggcatc acggaaggcg ctctagttca cgccacgcag      60

acggacgacg                                                              70


<210>  327
<211>  70
<212>  DNA
<213>  Artificial sequence

<220>
<223>  Probe

<400>  327
gccgacacga cgaagcaaac acagcacgca tacgaaaccg accgcacgcc gccgaaagcc      60

agctatctgg                                                          70

<210>  328
<211>  70
<212>  DNA
<213>  Artificial sequence

<220>
<223>  Probe

<400>  328
ggcgatcttg gaatgtcaga ccgtggcaaa caacacgctg ttgagcatca acggcatgaa      60

caagcgtttc                                                          70

<210>  329
<211>  70
<212>  DNA
<213>  Artificial sequence

<220>
<223>  Probe

<400>  329
ggactgggcc actggcaaca ggaagcggcc acggtgcggc ctgcgctgaa ccgcgcaacc      60

atcgatagcg                                                          70

<210>  330
<211>  70
<212>  DNA
<213>  Artificial sequence

<220>
<223>  Probe

<400>  330
gtcgctagct ggatcaacca cgccaaaggc caatacgaaa tctgccgggc tcgactcggc      60

gcactgatcg                                                          70

<210>  331
<211>  70
<212>  DNA
<213>  Artificial sequence

<220>
<223>  Probe

<400>  331
ggaaggtcta tcacgatcat ctcagtgacc ctaacaagag gccaccagcg aacctggaac      60

ttgttgctgc                                                          70

<210>  332
<211>  70

<212>  DNA
<213>  Artificial sequence

<220>
<223>  Probe

<400>  332
cgcacttacc tcgacgccag cattgagtca ttggatcgcc acattaagct gctgactagc      60

ctaatcgaag                                                            70


<210>  333
<211>  70
<212>  DNA
<213>  Artificial sequence

<220>
<223>  Probe

<400>  333
caaattaaac actacaaagg gctggtagtt tatgcgcctc taatcgcgct atctgtggtc      60

tttgtggccg                                                            70


<210>  334
<211>  70
<212>  DNA
<213>  Artificial sequence

<220>
<223>  Probe

<400>  334
gcggtgcttg ccttgtctgg cgccaaacgc ttccagcact tcatcaaggt cgttgcagat      60

tggcaggagg                                                            70


<210>  335
<211>  70
<212>  DNA
<213>  Artificial sequence

<220>
<223>  Probe

<400>  335
acgcgaataa ggcatcgcta ccaggtgacg taagccgggt atatagaaat gctttagaca      60

tgatctggag                                                            70


<210>  336
<211>  70
<212>  DNA
<213>  Artificial sequence

<220>
<223>  Probe

<400> 336
ctatcggttc cgctgcgcac atgggcatga aacgtcccgc accggcgact acgccctccg 60

tagtctgatc 70


<210> 337
<211> 70
<212> DNA
<213> Artificial sequence

<220>
<223> Probe

<400> 337
cttcgctaaa taataatgct gaggctcatg atcttctata ctcgctgctt gcccatgaca 60

gtgaggcggc 70


<210> 338
<211> 70
<212> DNA
<213> Artificial sequence

<220>
<223> Probe

<400> 338
gacattgcag catccgtctt gcgtgacatt gaagtcggca aacacggctt aatgtcggct 60

tcggcgagcg 70


<210> 339
<211> 70
<212> DNA
<213> Artificial sequence

<220>
<223> Probe

<400> 339
gtgcgcaaac aaggcacgcg gcgaagcgtc tctgcgcaaa gacggattgg acgaactgcg 60

ccgcatcgcg 70


<210> 340
<211> 70
<212> DNA
<213> Artificial sequence

<220>
<223> Probe

<400> 340
ggccggagac atctttgcgt tccagttgga gcagtttccg gatcggtact tctttgggcg 60

agtcgtggac 70

<210>  341
<211>  70
<212>  DNA
<213>  Artificial sequence

<220>
<223>  Probe

<400>  341
cagcggacga gcaacgatag gttgcttcac gacgatctcg gcgcgtgtat atcggcagtc      60

aagaagttcg                                                              70


<210>  342
<211>  70
<212>  DNA
<213>  Artificial sequence

<220>
<223>  Probe

<400>  342
cgttgggaag acaaagagaa cgtctttctc tacgcatcag gatttcttgc cgcaatggat      60

ttcctcaggc                                                              70


<210>  343
<211>  70
<212>  DNA
<213>  Artificial sequence

<220>
<223>  Probe

<400>  343
cgcgggcgtc gcttgccctc ggtggcgtcg ggatcttcta ttcaagaact acgccctatt      60

cgtggcgggc                                                              70


<210>  344
<211>  70
<212>  DNA
<213>  Artificial sequence

<220>
<223>  Probe

<400>  344
caaggcaatt tgattgtgga ggatcgtatt caggttcaca tgagcttgcg acttctgcat      60

attccaacgg                                                              70


<210>  345
<211>  70
<212>  DNA

<213>  Artificial sequence

<220>
<223>  Probe

<400>  345
gtcgggttgt tgtacctcta cggcgagcaa accggtcatc catcagcggc tggtggcgct      60

ctactgctgt                                                              70


<210>  346
<211>  70
<212>  DNA
<213>  Artificial sequence

<220>
<223>  Probe

<400>  346
cgagtatcac cttgttgagg ctcgatcagt tactgaatgc cgcctatgcc aacctgttga      60

ccaagatgcg                                                              70


<210>  347
<211>  70
<212>  DNA
<213>  Artificial sequence

<220>
<223>  Probe

<400>  347
gatggcatgg cgaaccatca cctgattcct gaggagttga tgaaacatcc tcgatacgcg      60

gcgatgttcc                                                              70


<210>  348
<211>  70
<212>  DNA
<213>  Artificial sequence

<220>
<223>  Probe

<400>  348
ggcttgaaat ggtggagatt caactagaca agcatggccg cgctgccttt cgtctgaaca      60

ttggtgtcgc                                                              70


<210>  349
<211>  70
<212>  DNA
<213>  Artificial sequence

<220>
<223>  Probe

<400>  349
gccacacagc ttctcggcgg ctcaatgcgg tcgtgtgtct cttgagatcg ctgacagaac      60

gctcagggcg                                                              70


<210>  350
<211>  70
<212>  DNA
<213>  Artificial sequence

<220>
<223>  Probe

<400>  350
gtgcatacga gggactgtat atgctctcgt gcatggaaac actcacaaag gaacgcgccg      60

accacttggc                                                              70


<210>  351
<211>  70
<212>  DNA
<213>  Artificial sequence

<220>
<223>  Probe

<400>  351
caatagcctt gccaagcact gcaacatgaa ggcggtcacg gatttcaacg acctgcatca      60

gaagcaactg                                                              70


<210>  352
<211>  70
<212>  DNA
<213>  Artificial sequence

<220>
<223>  Probe

<400>  352
gcggaagtgt gggcagaagg aagctgttga gtatatcgct gatttgaatc ggtatacgac      60

gcttcgaccg                                                              70


<210>  353
<211>  70
<212>  DNA
<213>  Artificial sequence

<220>
<223>  Probe

<400>  353
cgatcacttc atcgagctat caaatattgg tgaagaagat cgttcagtgg aggcaatggc      60

ggttgggatg                                                              70

<210> 354
<211> 70
<212> DNA
<213> Artificial sequence

<220>
<223> Probe

<400> 354
gttggtgagc gaagcgcaga tgcgtgccgc tggtgaagcg atctatggtg aacgtaaagt 60

cgttacgtgc 70


<210> 355
<211> 70
<212> DNA
<213> Artificial sequence

<220>
<223> Probe

<400> 355
cgtcgtgaac acctcggttc gcaagtacga caagaagtac tacaagttct atcgcagcca 60

caccaagagc 70


<210> 356
<211> 70
<212> DNA
<213> Artificial sequence

<220>
<223> Probe

<400> 356
cggcgtgatc ggcgttgcga tcatcggtat ctgcctcggt ttcaaggcga tcgacctggg 60

caagcgcggc 70


<210> 357
<211> 70
<212> DNA
<213> Artificial sequence

<220>
<223> Probe

<400> 357
gcgctggtgg ctctcttcta cgcgttggtg gccatgatcg gtgctatcag cgcgctcgtc 60

ttcatttggg 70


<210> 358
<211> 70
<212> DNA
<213> Artificial sequence

<220>
<223>  Probe

<400>  358
gccgcaatgg aggtgtcggc cattggtatt gcggatatgg ctgctgttgt ggcgcgtgcc      60

tctacgccgg                                                              70


<210>  359
<211>  70
<212>  DNA
<213>  Artificial sequence

<220>
<223>  Probe

<400>  359
ggcaattacg gtgttcgcag catcgacgtg ccgtgctcgt tgttccagac catcggtctg      60

atcattctgg                                                              70


<210>  360
<211>  70
<212>  DNA
<213>  Artificial sequence

<220>
<223>  Probe

<400>  360
gctgctatct cggcgttgat cgccaaggtt gtcgggctgg ctacttggtt tgggcagctc      60

gcgattgccg                                                              70


<210>  361
<211>  70
<212>  DNA
<213>  Artificial sequence

<220>
<223>  Probe

<400>  361
gatctgattg agcggttgac ggaattggcg ttcggctacg ccgctgagat gcattaccac      60

caggagaagg                                                              70


<210>  362
<211>  70
<212>  DNA
<213>  Artificial sequence

<220>
<223>  Probe

<400>  362

ctgctgcact gctgagcagt ttgccgtgat cgggatcgat ggcccgacca tctttcagtg 60

gtggagctgg 70

<210> 363
<211> 70
<212> DNA
<213> Artificial sequence

<220>
<223> Probe

<400> 363
ctgcttgtga tggtcgtaat ggcgagtcgg gtggcgtact gggagcggcg tttctggcag 60

tcgctggtcg 70

<210> 364
<211> 70
<212> DNA
<213> Artificial sequence

<220>
<223> Probe

<400> 364
ggtggttatc agcgcaaggc gtaccagttc aagaacaagg acaccggcga ggtcgagacg 60

gtcacgccgg 70

<210> 365
<211> 70
<212> DNA
<213> Artificial sequence

<220>
<223> Probe

<400> 365
acagcgacaa gctccagtac gactttggca agcatccgcg agtggatgag gtgacggacg 60

cctatttgga 70

<210> 366
<211> 70
<212> DNA
<213> Artificial sequence

<220>
<223> Probe

<400> 366
gacgcaatac cgcaagggcg accgcaagat ggacaacgaa gcgtgcctgc gcctggcgca 60

actgctcgag 70

<210> 367
<211> 70
<212> DNA
<213> Artificial sequence

<220>
<223> Probe

<400> 367
cggcgttcta ccagccaagg aagtgctttc cactgcgaga tctgtaggca catggacatg 60

gagacacaag 70


<210> 368
<211> 70
<212> DNA
<213> Artificial sequence

<220>
<223> Probe

<400> 368
cggcagcact catgccagcg ggttcattgc ctctctacgt cccaagctca acttcattgt 60

cactgatacg 70


<210> 369
<211> 70
<212> DNA
<213> Artificial sequence

<220>
<223> Probe

<400> 369
cggcgttcgt gaggtacgtg gcttcactgg tgagcagcaa attcagcctg gtcacttctt 60

catcctccac 70


<210> 370
<211> 70
<212> DNA
<213> Artificial sequence

<220>
<223> Probe

<400> 370
gtggtggatt cggtcatcaa gaatggcgag atcaagcagg aaggtgacaa acgctttgtt 60

cgcatggccg 70


<210> 371
<211> 70
<212> DNA
<213> Artificial sequence

<220>
<223> Probe

<400> 371
ccacgaactg gatgtggtgg ttgagcagtg ctaccagatt aagccgttct acagcgatac 60

cgaacgcttg 70


<210> 372
<211> 70
<212> DNA
<213> Artificial sequence

<220>
<223> Probe

<400> 372
aggctatacg gacagcacag ccgcaaatta tgcctgccga ctgggaggtg aaagaggttg 60

gtggcacgtt 70


<210> 373
<211> 70
<212> DNA
<213> Artificial sequence

<220>
<223> Probe

<400> 373
acatgttgtt tatcggtgga tatggctggg atggctcgaa tctgtatatt gaaattaata 60

caggaaaggt 70


<210> 374
<211> 70
<212> DNA
<213> Artificial sequence

<220>
<223> Probe

<400> 374
caattgggta tacgtgatct gtgggtgcct gatgggattc gcgttcggct ggattgccat 60

gctctggctc 70


<210> 375
<211> 70
<212> DNA
<213> Artificial sequence

<220>
<223> Probe

<400> 375
gcggtagccg actggcacac gtatttggac ggcgttggcc gggaagattt ccctgaccaa 60

ccagacgatc                                                          70


<210>  376
<211>  70
<212>  DNA
<213>  Artificial sequence

<220>
<223>  Probe

<400>  376
cagccattct cttccaactt tatttctact ggatggatcg ctattacgtg tttgcgccga      60

tgtggagctc                                                          70


<210>  377
<211>  70
<212>  DNA
<213>  Artificial sequence

<220>
<223>  Probe

<400>  377
cttgaaccaa gcatagagcg cgtcgcatat cgccaggccg tgttcaagcg tgacctgatc      60

atcggtaagc                                                          70


<210>  378
<211>  70
<212>  DNA
<213>  Artificial sequence

<220>
<223>  Probe

<400>  378
gcctatcgtc aacatggttt cctatgttgc tcagaagtca gcgtggaata tgctcaattt      60

gaacgtgccg                                                          70


<210>  379
<211>  48
<212>  DNA
<213>  Artificial sequence

<220>
<223>  Probe

<400>  379
cgcgtgacca aacgccagcg cttcctggca gagatggaga aggtcttg                48


<210>  380
<211>  70
<212>  DNA

<213>  Artificial sequence

<220>
<223>  Probe

<400>  380
caaacattgc cttcacggaa gatgagcagc atctgtacaa cctgacgctt gatgagctgg      60

gcgacgagtc                                                              70


<210>  381
<211>  70
<212>  DNA
<213>  Artificial sequence

<220>
<223>  Probe

<400>  381
caatagcctt gccaagcact gcaacatgaa ggcggtcacg gatttcaacg acctgcatca      60

gaagcaactg                                                              70


<210>  382
<211>  70
<212>  DNA
<213>  Artificial sequence

<220>
<223>  Probe

<400>  382
gccggttgct gacggagatc gagaggctgg cggatgactt tagggaaccg ctgatcaatg      60

aagtcggcga                                                              70


<210>  383
<211>  70
<212>  DNA
<213>  Artificial sequence

<220>
<223>  Probe

<400>  383
cacaaccctg cgcatttccg cgccgaactc ttgatggaat acatggagaa tgcatccccc      60

tcaaaaaacg                                                              70


<210>  384
<211>  70
<212>  DNA
<213>  Artificial sequence

<220>
<223>  Probe

<400> 384
gatatcgcgg acataagatg cttcgtcgcc ctcgatggct cgtcgtattt cgctagtgcg 60

tctaaggctg 70


<210> 385
<211> 70
<212> DNA
<213> Artificial sequence

<220>
<223> Probe

<400> 385
gtccagacct gtctgtcaca cttgcctacc agcagtctta caacgactac ggatcgaatg 60

tcggcatcgg 70


<210> 386
<211> 70
<212> DNA
<213> Artificial sequence

<220>
<223> Probe

<400> 386
ggtcgccaca ttcacattga tggcccactc gacgtattga acaaagttcc aggcattgat 60

cattggtgga 70


<210> 387
<211> 23
<212> DNA
<213> Artificial sequence

<220>
<223> Primer

<400> 387
acttggagag atttacggag gag 23


<210> 388
<211> 23
<212> DNA
<213> Artificial sequence

<220>
<223> Primer

<400> 388
aagcaaacga gataagggag aac 23


<210> 389
<211> 23
<212> DNA

<213>  Artificial sequence

<220>
<223>  Primer

<400>  389
aaacacaatt caccttcctg atg                                              23


<210>  390
<211>  22
<212>  DNA
<213>  Artificial sequence

<220>
<223>  Primer

<400>  390
ggccactaga ctttccagtg at                                               22


<210>  391
<211>  23
<212>  DNA
<213>  Artificial sequence

<220>
<223>  Primer

<400>  391
agcttacctg ctgttcgaca tct                                              23


<210>  392
<211>  22
<212>  DNA
<213>  Artificial sequence

<220>
<223>  Primer

<400>  392
gttcttcgtg atagcggaga ct                                               22


<210>  393
<211>  23
<212>  DNA
<213>  Artificial sequence

<220>
<223>  Probe

<400>  393
tatgaatggg ttgatagcgt tct                                              23


<210>  394
<211>  23
<212>  DNA
<213>  Artificial sequence

<220>
<223>  Primer

<400>  394
ccatatccac cgataaacaa cat                                              23


<210>  395
<211>  23
<212>  DNA
<213>  Artificial sequence

<220>
<223>  Primer

<400>  395
cacgatatga ccacgatcaa cta                                              23


<210>  396
<211>  23
<212>  DNA
<213>  Artificial sequence

<220>
<223>  Primer

<400>  396
gtagacacga atcacgtctc cat                                              23


<210>  397
<211>  22
<212>  DNA
<213>  Artificial sequence

<220>
<223>  Primer

<400>  397
acatcaacga cccttactgt cc                                               22


<210>  398
<211>  23
<212>  DNA
<213>  Artificial sequence

<220>
<223>  Primer

<400>  398
gaccatagtc atcgctgctt aac                                              23


<210>  399
<211>  23
<212>  DNA
<213>  Artificial sequence

<220>
<223> Primer

<400> 399
ctatcccgca gaaggtattc aac 23


<210> 400
<211> 22
<212> DNA
<213> Artificial sequence

<220>
<223> Primer

<400> 400
agtcataggc gtctcggtac tt 22


<210> 401
<211> 22
<212> DNA
<213> Artificial sequence

<220>
<223> Primer

<400> 401
acactctgtt caccaagtac gg 22


<210> 402
<211> 23
<212> DNA
<213> Artificial sequence

<220>
<223> Primer

<400> 402
ctttgaaact ggaggaacag ctt 23


<210> 403
<211> 23
<212> DNA
<213> Artificial sequence

<220>
<223> Primer

<400> 403
aacactcaaa agctgaccat cat 23


<210> 404
<211> 22
<212> DNA
<213> Artificial sequence

<220>

<223>  Primer

<400>  404
caaccttgat ctgttcggag ac                                              22


<210>  405
<211>  21
<212>  DNA
<213>  Artificial sequence

<220>
<223>  Primer

<400>  405
gttgcaatgc tggtttccaa g                                               21


<210>  406
<211>  22
<212>  DNA
<213>  Artificial sequence

<220>
<223>  Primer

<400>  406
gtcatggacg agaaatcgat ac                                              22


<210>  407
<211>  23
<212>  DNA
<213>  Artificial sequence

<220>
<223>  Primer

<400>  407
ctcagaggat ctgttcatcg act                                             23


<210>  408
<211>  21
<212>  DNA
<213>  Artificial sequence

<220>
<223>  Primer

<400>  408
gttgaagacg ccgaagaaaa a                                               21


<210>  409
<211>  22
<212>  DNA
<213>  Artificial sequence

<220>
<223>  Primer

<400> 409
acgagattcc tgaagctgag gt 22

<210> 410
<211> 21
<212> DNA
<213> Artificial sequence

<220>
<223> Primer

<400> 410
cttgcagaac ctgacacatg a 21

<210> 411
<211> 23
<212> DNA
<213> Artificial sequence

<220>
<223> Primer

<400> 411
aaggctaagg gggagtaagt cat 23

<210> 412
<211> 23
<212> DNA
<213> Artificial sequence

<220>
<223> Primer

<400> 412
aggtagttgc gtacttggtc gta 23

<210> 413
<211> 23
<212> DNA
<213> Artificial sequence

<220>
<223> Primer

<400> 413
gcgcaaatta aacactacaa agg 23

<210> 414
<211> 23
<212> DNA
<213> Artificial sequence

<220>
<223> Primer

<400>  414
atccccaatc tcctttatca ctc                                              23


<210>  415
<211>  23
<212>  DNA
<213>  Artificial sequence

<220>
<223>  Primer

<400>  415
gaaggtaaat ccaaaggaaa tgg                                              23


<210>  416
<211>  23
<212>  DNA
<213>  Artificial sequence

<220>
<223>  Primer

<400>  416
aattgacttc ggctcgtatt ctt                                              23


<210>  417
<211>  24
<212>  DNA
<213>  Artificial sequence

<220>
<223>  Primer

<400>  417
aattccatga gtggagtgat tttt                                             24


<210>  418
<211>  24
<212>  DNA
<213>  Artificial sequence

<220>
<223>  Primer

<400>  418
agatcatgag cctcagcatt atta                                             24


<210>  419
<211>  22
<212>  DNA
<213>  Artificial sequence

<220>
<223>  Primer

<400>  419

gaacggagcc atagtgatga ag                                                22

<210>  420
<211>  23
<212>  DNA
<213>  Artificial sequence

<220>
<223>  Primer

<400>  420
agagtcgcta acacaagtca tcc                                               23

<210>  421
<211>  23
<212>  DNA
<213>  Artificial sequence

<220>
<223>  Primer

<400>  421
cacgatcata tatgggtcca gtt                                               23

<210>  422
<211>  23
<212>  DNA
<213>  Artificial sequence

<220>
<223>  Primer

<400>  422
ggttcttttt gatcgtagcc ttt                                               23

<210>  423
<211>  23
<212>  DNA
<213>  Artificial sequence

<220>
<223>  Primer

<400>  423
tctgttccga gtatcacctt gtt                                               23

<210>  424
<211>  23
<212>  DNA
<213>  Artificial sequence

<220>
<223>  Primer

<400>  424
catagtattc gcagtccaga tcc                                               23

<210>  425
<211>  23
<212>  DNA
<213>  Artificial sequence

<220>
<223>  Primer

<400>  425
ggatgtgggg aggtttatta gtc                                                23


<210>  426
<211>  23
<212>  DNA
<213>  Artificial sequence

<220>
<223>  Primer

<400>  426
ttcatcaact cctcaggaat cag                                                23


<210>  427
<211>  23
<212>  DNA
<213>  Artificial sequence

<220>
<223>  Primer

<400>  427
aagatatgtg ccagctacca ctg                                                23


<210>  428
<211>  23
<212>  DNA
<213>  Artificial sequence

<220>
<223>  Primer

<400>  428
agcatataca gtccctcgta tgc                                                23


<210>  429
<211>  21
<212>  DNA
<213>  Artificial sequence

<220>
<223>  Primer

<400>  429
atttcaacga cctgcatcag a                                                  21

<210> 430
<211> 22
<212> DNA
<213> Artificial sequence

<220>
<223> Primer

<400> 430
ccacaccagg ttcttcttgt tc 22


<210> 431
<211> 23
<212> DNA
<213> Artificial sequence

<220>
<223> Primer

<400> 431
gtaaggtctg caaggacatc atc 23


<210> 432
<211> 23
<212> DNA
<213> Artificial sequence

<220>
<223> Primer

<400> 432
gtggctgcga tagaacttgt agt 23


<210> 433
<211> 20
<212> DNA
<213> Artificial sequence

<220>
<223> Primer

<400> 433
gaccatcttt cagtggtgga 20


<210> 434
<211> 23
<212> DNA
<213> Artificial sequence

<220>
<223> Primer

<400> 434
taatgcccta aactttcctg atg 23

<210> 435
<211> 23
<212> DNA
<213> Artificial sequence

<220>
<223> Primer

<400> 435
tgtttgatct gagcaagttt gtg 23


<210> 436
<211> 20
<212> DNA
<213> Artificial sequence

<220>
<223> Primer

<400> 436
agaaactcat ccgcaaggtc 20


<210> 437
<211> 23
<212> DNA
<213> Artificial sequence

<220>
<223> Primer

<400> 437
gtaaggagaa gtgatgtcgg aac 23


<210> 438
<211> 23
<212> DNA
<213> Artificial sequence

<220>
<223> Primer

<400> 438
gtccttgttc ttgaactggt acg 23


<210> 439
<211> 21
<212> DNA
<213> Artificial sequence

<220>
<223> Primer

<400> 439
catgccaagg aacacatcaa g 21


<210> 440

<211>  23
<212>  DNA
<213>  Artificial sequence

<220>
<223>  Primer

<400>  440
aagtatttgt tgccgtggta ctc                                              23


<210>  441
<211>  23
<212>  DNA
<213>  Artificial sequence

<220>
<223>  Primer

<400>  441
aggtactcgg ctatcacaat cac                                              23


<210>  442
<211>  23
<212>  DNA
<213>  Artificial sequence

<220>
<223>  Primer

<400>  442
atgcattctc catgtattcc atc                                              23


<210>  443
<211>  23
<212>  DNA
<213>  Artificial sequence

<220>
<223>  Primer

<400>  443
gagactcggt tcaacaagaa aaa                                              23


<210>  444
<211>  23
<212>  DNA
<213>  Artificial sequence

<220>
<223>  Primer

<400>  444
cttagacgca ctagcgaaat acg                                              23